# EUROPEAN PATENT APPLICATION

(11) **EP 4 293 019 A1**
(43) Date of publication of application: **20.12.2023**
(21) Application number: 22178710.4
(22) Date of filing: 13.06.2022
(51) Int. Cl.: C07D 403/14, C07D 409/14, C07D 401/14, A61P 35/00

(54) **4-SUBSTITUTED (1H-BENZO[D]IMIDAZOL-2-YL)-1H-PYRAZOLES AS BUB1 INHIBITORS USEFUL FOR TREATING CANCERS**

(71) Applicant: Netherlands Translational Research Center Holding B.V., 5349 AB Oss (NL); Pivot Park Screening Centre, 5349 AB Oss (NL); Universiteit Leiden, 2311 EZ Leiden (NL)
(72) Inventor: Bosman, Robertus, Eduardes, Johannes,, 2718KP Zoetermeer (NL); Van Boeckel, Constant, Adriaan, Anton,, 5345LX Oss (NL); Buijsman, Rogier, Christian,, 5351MK Berghem (NL); De Man, Adrianus, Petrus, Antonius,, 5327AH Hurwenen (NL); van der Stelt, Marcelis, 2333AB, Leiden (NL)
(74) Representative: Arth, Hans-Lothar

(57) **Abstract**

The present invention relates to 4-substituted (1*H*-benzo[d]imidazol-2-yl)-1*H-*pyrazole compounds of the formula (I) and pharmaceutically acceptable salts thereof. These compounds are useful in the treatment or prevention of cancers associated with and/or caused by budding uninhibited by benzimidazole 1 (BUB1) kinase.

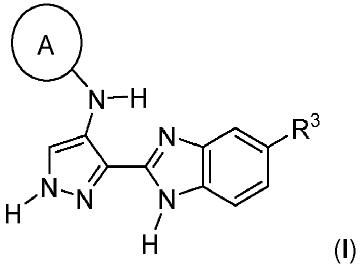

## Description

### Field of the Invention

The present invention relates to 4-substituted (1*H*-benzo[d]imidazol-2-yl)-1*H-*pyrazole compounds of the formula (I) and pharmaceutically acceptable salts thereof. These compounds are useful in the treatment or prevention of a cancer or a carcinoma associated with and/or caused by budding uninhibited by benzimidazole 1 kinase (BUB1 kinase).

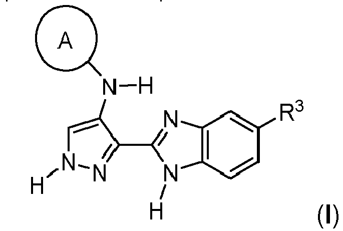

### Background of the Invention

The eukaryotic cell division cycle (or cell cycle) ensures the duplication of the genome and its distribution to the daughter cells by passing through a coordinated and regulated sequence of events. The cell cycle is divided into four successive phases:
1. The G1 phase represents the time before the DNA replication, in which the cell grows and is sensitive to external stimuli.
2. In the S phase the cell replicates its DNA, and
3. in the G2 phase preparations are made for entry into mitosis.
4. In mitosis (M phase), the duplicated chromosomes get separated supported by a spindle device built from microtubules, and cell division into two daughter cells is completed.

To ensure the extraordinary high fidelity required for an accurate distribution of the chromosomes to the daughter cells, the passage through the cell cycle is strictly regulated and controlled. The enzymes that are necessary for the progression through the cycle must be activated at the correct time and are also turned off again as soon as the corresponding phase is passed. Corresponding control points ("checkpoints") stop or delay the progression through the cell cycle if DNA damage is detected, or the DNA replication or the creation of the spindle device is not yet completed. The mitotic checkpoint (also known as spindle checkpoint or spindle assembly checkpoint) controls the accurate attachment of microtubules of the spindle device to the kinetochores (the attachment site for microtubules) of the duplicated chromosomes. The mitotic checkpoint is active as long as unattached kinetochores are present and generates a wait-signal to give the dividing cell the time to ensure that each kinetochore is attached to a spindle pole, and to correct attachment errors. Thus the mitotic checkpoint prevents a mitotic cell from completing cell division with unattached or erroneously attached chromosomes. Once all kinetochores are attached with the mitotic spindle poles in a correct bipolar (amphitelic) fashion, the checkpoint is satisfied and the cell enters anaphase and proceeds through mitosis. [for a recent review see Musacchio A, Curr. Biol. 25, R1002-R1018, 2015].

The mitotic checkpoint is established by a complex network of a number of essential proteins, including members of the MAD (mitotic arrest deficient, MAD 1 - 3) and BUB (Budding uninhibited by benzimidazole, BUB 1 - 3) families, Mps1 kinase, cdc20, as well as other components [reviewed in Bolanos-Garcia VM and Blundell TL, Trends Biochem. Sci. 36, 141 , 2010], many of these being over-expressed in proliferating cells (e.g. cancer cells) and tissues [Yuan B et al., Clin. Cancer Res. 12, 405, 2006]. The major function of an unsatisfied mitotic checkpoint is to keep the anaphase-promoting complex/cyclosome (APC/C) in an inactive state. As soon as the checkpoint gets satisfied the APC/C ubiquitin-ligase targets cyclin B and securin for proteolytic degradation leading to separation of the paired chromosomes and exit from mitosis.

Cell cycle deregulation represents one of the classical hallmarks of cancer. The concept of cell cycle checkpoint regulation offers a novel approach to cancer treatment: inactivation of cell cycle checkpoints is considered to drive tumor cells into cell division despite DNA damage or unattached/misattached chromosomes resulting in a lethal degree of DNA damage or aneuploidy.

The SAC signal is initiated by multipolar-spindle 1 (Mps1)-mediated phosphorylation of Met-Glu-Leu-Thr (MELT) motifs on the kinetochore scaffold 1 (KNL-1) protein to generate docking sites for Bub1/Bub3 dimers. The N-terminal non-catalytic part of BUB1 represents a scaffold for the assembly of mitotic arrest deficient 1 (MAD1)/MAD2, BUBR1 and centromere protein E (CENP-E) proteins. The inactive open from of MAD2 (o-MAD2) gets converted to the active closed conformation (c-MAD2) which binds cell-division cycle 20 homolog (CDC20) and BUBR1 to form the diffusible mitotic checkpoint complex (MCC) which inhibits the ubiquitin ligase anaphase-promoting complex/cyclosome (APC/C) and subsequently leads to mitotic arrest. Note that MCC generation requires BUB1 protein, however, its kinase function is not involved.

Budding uninhibited by benzimidazole 1 kinase (BUB1 kinase) phosphorylates histone H2A at Thr120 within the centromeric region of the duplicated chromosomes thereby creating binding sites for shugoshin proteins (SGO) 1 and 2, which protect centromeric cohesin from premature degradation, and for the chromosomal passenger complex consisting of INCENP, survivin, borealin and Aurora B (AurB) [Kawashima et al. Science 327, 172, 2010; Watanabe Y, Cold Spring Harb. Symp. Quant. Biol. 75, 419, 2010]. Specific inhibition of BUB1 kinase activity results in reduced levels of SGO1 and SGO2 at mitotic centromeres. Furthermore, AurB fails to concentrate at the centromeres and was instead found to be spread over the chromosome arms [Baron et al. eLife 5, e12187, 2016]. AurB activity localized at the centromeric region is essential for the resolution of microtubule - kinetochore attachment errors such as syntelic and merotelic attachments. Dyslocatization of AurB due to BUB1 kinase inactivation strongly compromizes the cells ability to resolve attachment errors and results in an increased rate of chromosome alignment defects [Ricke et al. J. Cell Biol. 199, 931 , 2012], in particular in presence of attachment error inducing agents such as microtubule stabilizers paclitaxel or docetaxel.

Complete inhibition of the mitotic checkpoint has been recognised to result in severe chromosome missegregation and induction of apoptosis in tumour cells [Kops GJ et al., Nature Rev. Cancer 5, 773, 2005; Schmidt M and Medema RH, Cell Cycle 5, 159, 2006; Schmidt M and Bastians H, Drug Res. Updates 10, 162, 2007]. Thus, mitotic checkpoint abrogation through pharmacological inhibition of components of the mitotic checkpoint, such as BUB1 kinase, represents a new approach for the treatment of proliferative disorders, including solid tumours such as carcinomas, sarcomas, leukaemias and lymphoid malignancies or other disorders, associated with uncontrolled cellular proliferation.

It is object of the present invention to provide compounds as BUB1 kinase inhibitors, and/or pharmaceutically acceptable salts thereof which can be used as pharmaceutically active agents, and use thereof especially for the treatment of cell proliferative disease, especially a cancer or a carcinoma associated with and/or caused by BUB1 kinase, a combination comprising the inventive compound and at least one anticancer drug; as well as compositions comprising at least one of those compounds and/or pharmaceutically acceptable salts thereof as pharmaceutically active ingredients.

The present invention provides novel compound(s) that inhibit BUB1 kinase. The objective of the present invention is solved by the teaching of the independent claims. Further advantageous features, aspects and details of the invention are evident from the dependent claims, the description, the figures, and the examples of the present application.

### Summary of this invention

The present invention provides compounds that inhibit budding uninhibited by benzimidazole 1 kinase (BUB1 kinase) and pharmaceutically acceptable salts thereof as pharmaceutical active ingredient can be used for treatment of a cell proliferative disease, in particular, a cancer or a carcinoma associated with and/or caused by BUB1 kinase.

Thus, the present invention is directed to a compound of the general formula (I) 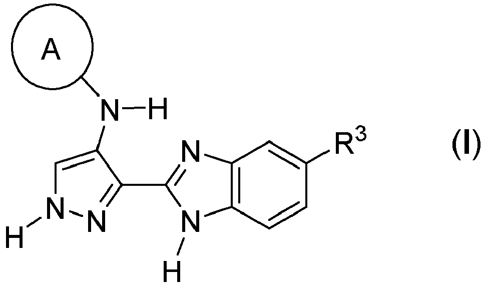 wherein
A represents 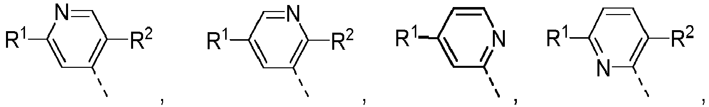 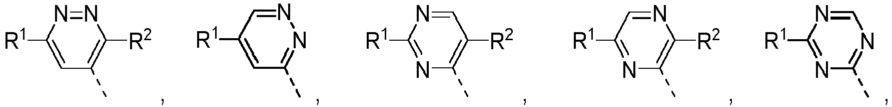 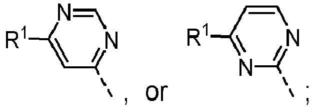
**R¹** represents -**R⁴** or -O-**L¹**-**R⁴**;
**L¹** represents a covalent bond, C₁-C₃ alkylene which is optionally substituted with one or more of -F, -CI, -Br, -CN, C₁-C₃ alkyl, -CHF₂, -CF₃, and -cyclo-C₃H₅;
**R²** represents -H, -**R⁵**, or -O-**R⁵**;
**R³** represents -H, -**R⁶**, -(CH₂)ₘ-**R⁶**, or -O-(CH₂)ₘ-**R⁶**;
m represents an integer selected from 1, 2, and 3;
**R⁴** represents -C=C-**L²**-**R***, C₆-C₁₀ aryl, or C₁-C₁₀ heteroaryl, wherein C₆-C₁₀ aryl and C₁-C₁₀ heteroaryl are optionally substituted with one or more of **R^{N1}**, **Z¹**, **Z²**, **Z³**, **Z⁴** and **Z⁵**;
**L²** represents a covalent bond, -CH₂-, -CF₂-, or -CH₂CH₂-;
**R*** represents -H, C₁-C₆ alkyl, C₃-C₆ cycloalkyl, C₆-C₁₀ aryl, or C₁-C₁₀ heteroaryl, wherein C₃-C₆ cycloalkyl, C₆-C₁₀ aryl, and C₁-C₁₀ heteroaryl are optionally substituted with one or more of **R^{N1}**, **Z¹**, **Z²**, **Z³**, **Z⁴** and **Z⁵**;
**R⁵** represents -H, -CH₃, -CD₃, -C₂H₅, -C₃H₇, -CH(CH₃)₂, -CHF₂, -CF₃, -CN, -CH₂CN, -C₄H₉, -cyclo-C₃H₅, or -cyclo-C₄H₇;
**R⁶** represents -OH, -OCH₃, -OC₂H₅, -N(CH₃)₂, -N(C₂H₅)₂, -C₂H₄OH -OC₂H₄OH -NR⁷R⁸, -CH₂NR⁷R⁸, -CH₂CH₂NR⁷R⁸, -CH₂CH₂CH₂NR⁷R⁸, -NHCOR⁷, -NHSO₂R⁷, -OCH₂NR⁷R⁸, -OCH₂CH₂NR⁷R⁸, -OCH₂CH₂CH₂NR⁷R⁸, -NR⁹CH₂NR⁷R⁸, -NR⁹CH₂CH₂NR⁷R⁸, -NR⁹CH₂CH₂CH₂NR⁷R⁸, -CONR⁷R⁸, -CONHCH₂NR⁷R⁸, -CONHCH₂CH₂NR⁷R⁸, -CONHCH₂CH₂CH₂NR⁷R⁸, -(OC₂H₄)ₙOH, -(OC₂H₄)ₙOCH₃, -CN, 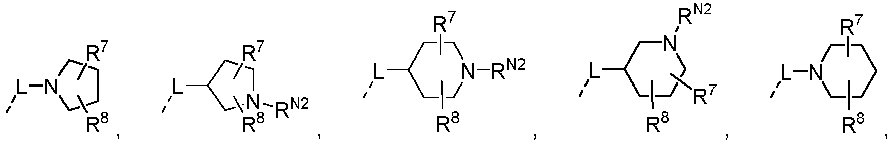 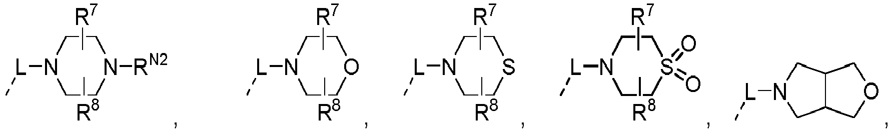 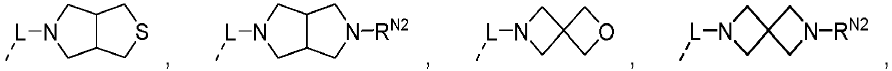 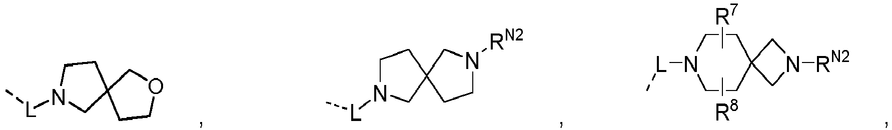 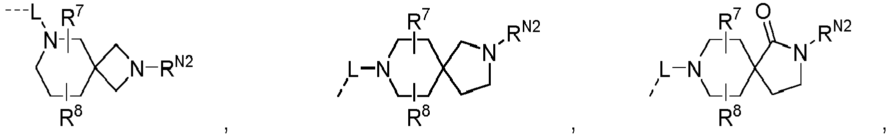 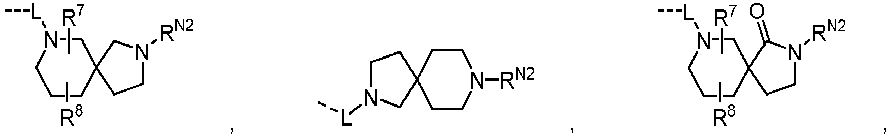 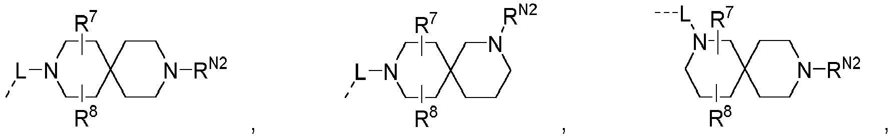 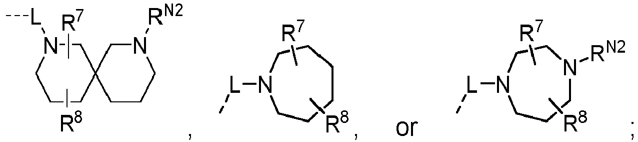
**n** represents an integer selected from 1, 2, and 3;
L represents a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO- -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OC-O-, -O-CO-, -SO₂-, -CH₂O-CO-, -CH₂-CO-O-, -CH₂CH₂O-CO-, or -CH₂CH₂-CO-O-;
**R⁷** and **R⁸** represent independently of each other -H, -F, -Br, -CI, -CH₃, -C₂H₅, -C₃H₇, -CH(CH₃)₂, -CH₂F, -CHF₂, -CF₃, -CH₂CN, -CH₂Cl, -CH₂Br, -C(CH₃)₂-CN, -CH₂-C(CH₃)₂-CN, -C₄H₉, -CH₂-CH(CH₃)₂, -CH(CH₃)-C₂H₅, -C(CH₃)₃, -OCH₃, -OC₂H₅, -OC₃H₇, -OCH(CH₃)₂, -OCH₂F, -OCHF₂, -OCF₃, cyclo-C₃H₅, cyclo-C₄H₇, cyclo-C₅H₉, cyclo-C₆H₁₁, -O-cyclo-C₃H₅, -O-cyclo-C₄H₇, -O-cyclo-C₅H₉, -O-cyclo-C₆H₁₁, -CH=CH₂, -CH₂-CH=CH₂, -C(CH₃)=CH₂, -CH=CH-CH₃, -C(CH₃)=CH-CH₃, -CH=C(CH₃)₂, -C(CH₃)=C(CH₃)₂, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -C=CH, -CH₂-C=CH, -C₂H₄-C≡CH, -CH₂-C≡C-CH₃, -C≡C-CH₃, -C≡C-C₂H₅, -CH₂-OCF₃, -C₂H₄-OCF₃, -C₃H₆-OCF₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -CH₂-SCH₃, -C₂H₄-SCH₃, -C₃H₆-SCH₃, -CH₂-SC₂H₅, -C₂H₄-SC₂H₅, -C₃Hₑ-SC₂H₅, -CH₂-SH, -C₂H₄-SH, or -C₃H₆-SH; or **R⁷** and **R⁸** form together -CH₂-, -CH₂CH₂-, or -CH₂CH₂CH₂-;
**R⁹** and **R^{N1}** represent independently of each other -H, -CH₃, or -C₂H₅;
**R^{N2}** represents -H, -CH₃, -C₂H₅, -C₃H₇, -CH(CH₃)₂, -CF₃, 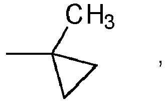 -cyclo-C₃H₅, -cyclo-C₄H₇, -cyclo-C₅H₉, -cyclo-C₆H₁₁, -cyclo-C₇H₁₃, -CH₂-cyclo-C₃H₅, -C₄H₉, -CH₂-CH(CH₃)₂, -CH(CH₃)-C₂H₅, -C(CH₃)₃, -C_{S}H₁₁, -CH(CH₃)-C₃H₇, -Ph, -CH₂-Ph, -CH₂-CH₂-Ph, -CH=CH₂, -CH₂-CH=CH₂, -C(CH₃)=CH₂, -CH=CH-CH₃;
**Z¹** - **Z⁵** represent independently of each other 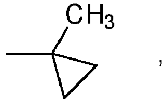 -cyclo-C₃H₅, -cyclo-C₄H₇, -cyclo-C₅H₉, -cyclo-C₆H₁₁, cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -CI, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, O0-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C_{S}H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C=C-CH₃, -CH₂-CH=CH-CH=CH-CH₃, -CH=CH-CH=CH-C₂H₅, -CH(CH₃)-CH₂-C=CH, -CH(CH₃)-C=C-CH₃, -C₂H₄-CH(CH₃)-C=CH, -CH=CH-CH=C(CH₃)₂, -CH₂-CH(CH₃)-CH₂-C=CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅;
or an enantiomer, a diastereomer, a tautomer, a mixture of enantiomers, a mixture of diastereomers, a hydrate, a solvate, or a racemate of the above mentioned compounds or a pharmaceutically acceptable salt thereof.

As used herein, the term **"C₁-C₁₀-heteroaryl"** refers to aromatic residues with one or more heteroatoms such as O, S, N and especially N and refers preferably to 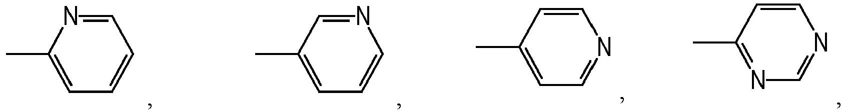 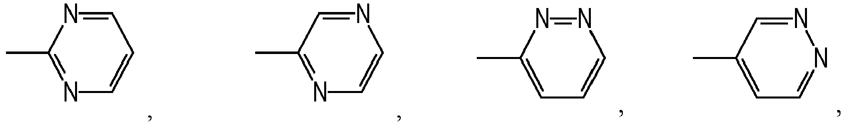 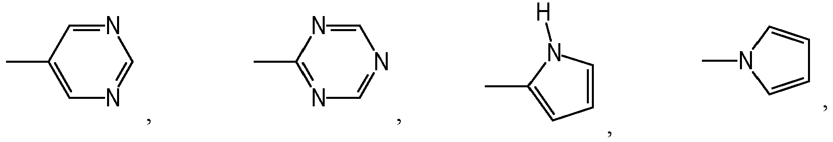 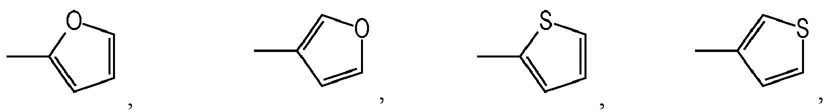 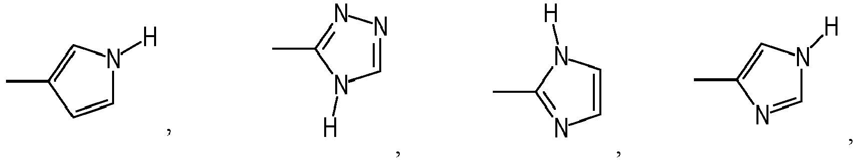 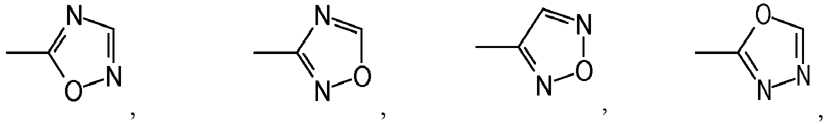 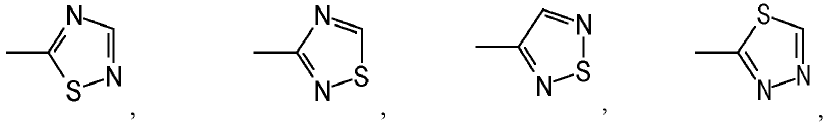 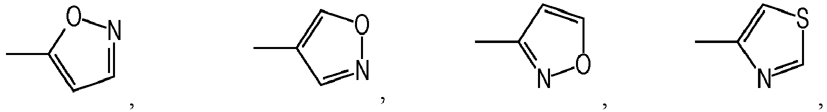 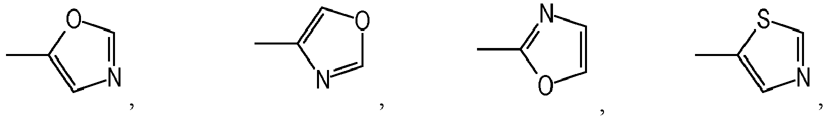 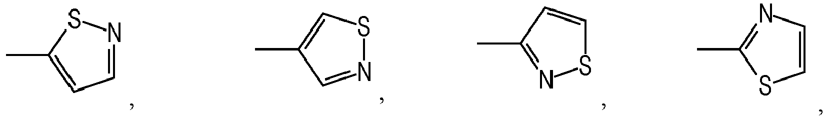 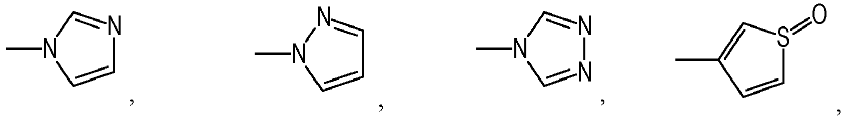 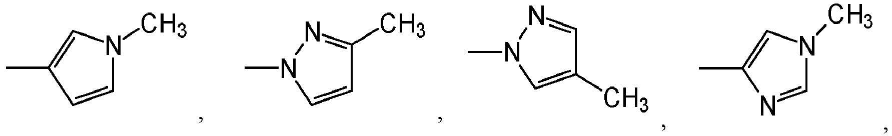 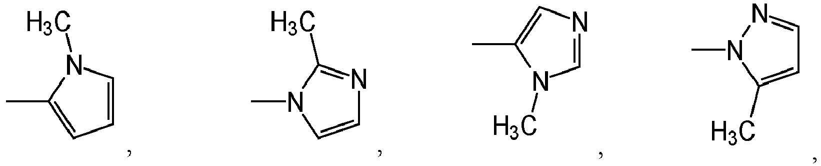 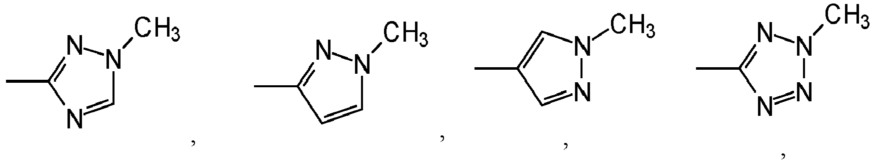 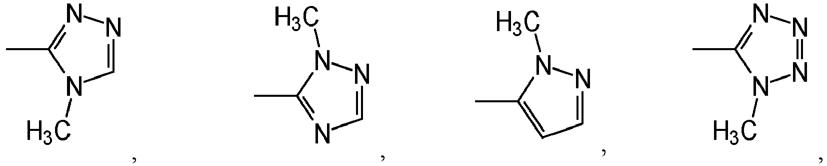 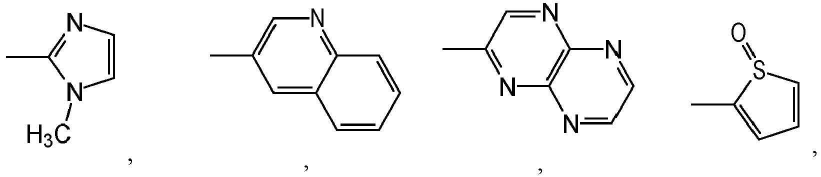 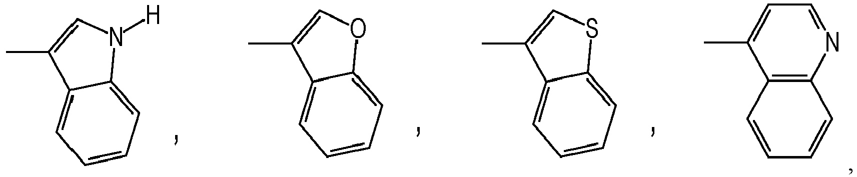 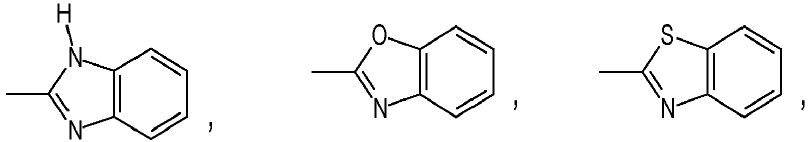 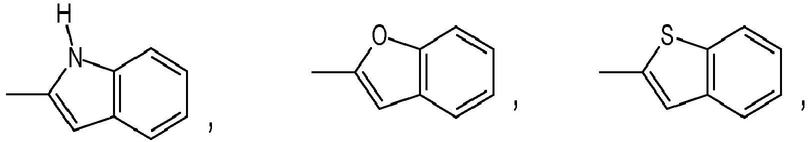 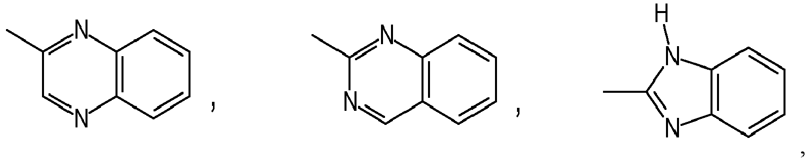 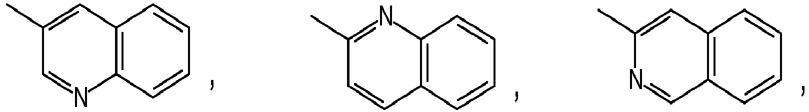 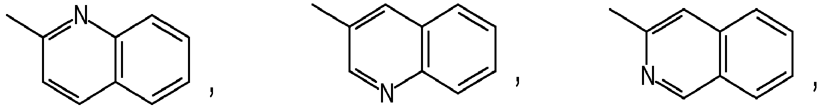 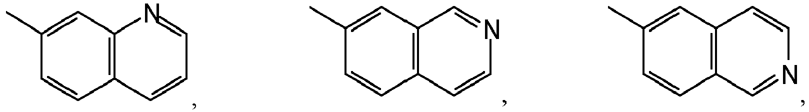 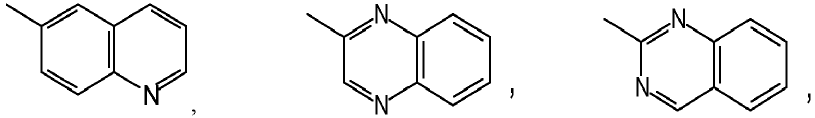 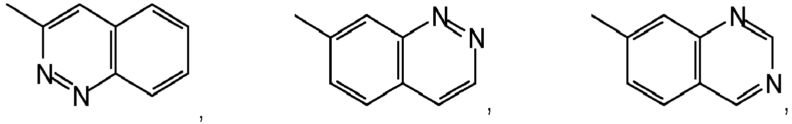 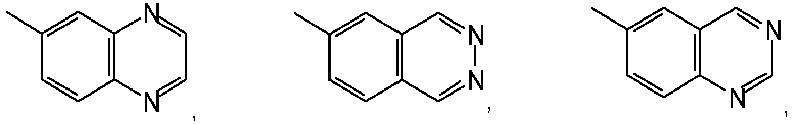 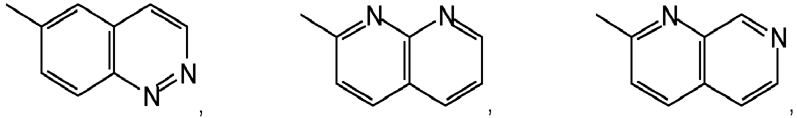 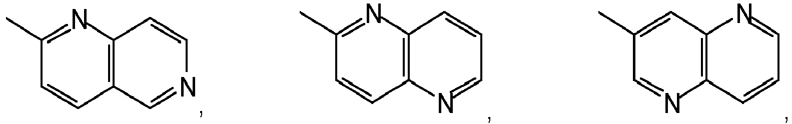 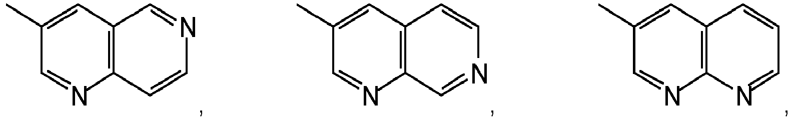 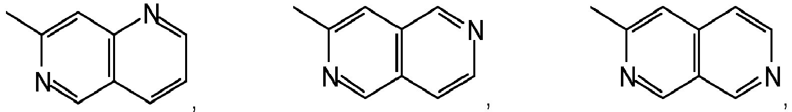 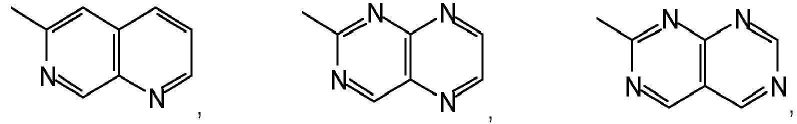 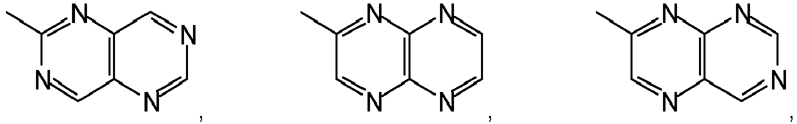 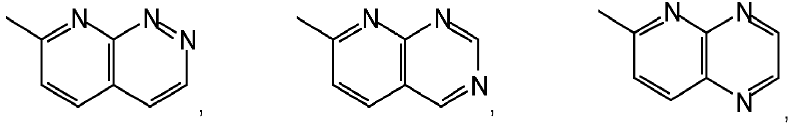 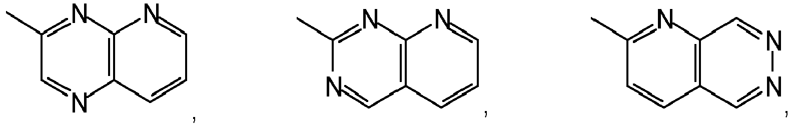 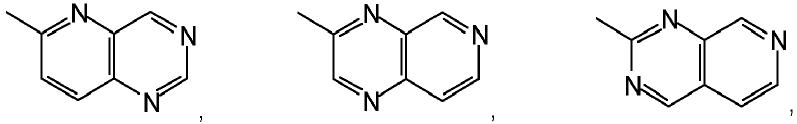 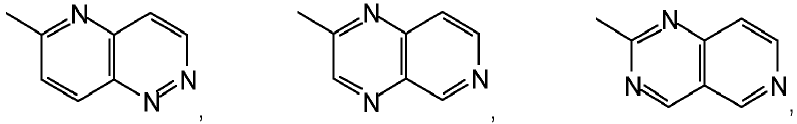 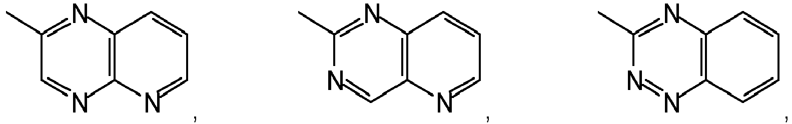 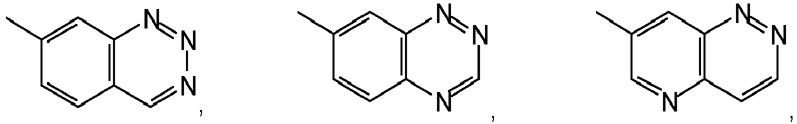 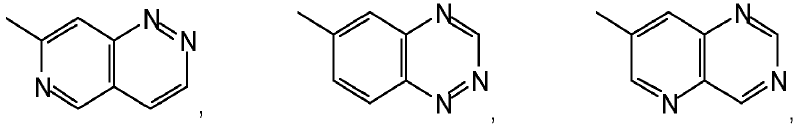 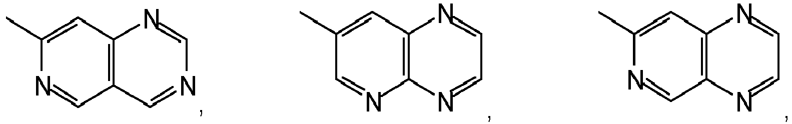 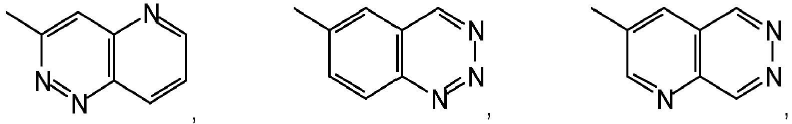 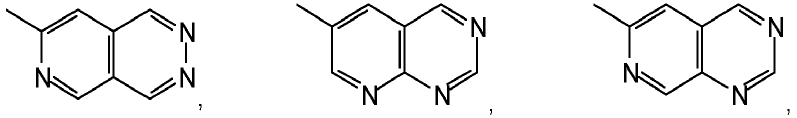 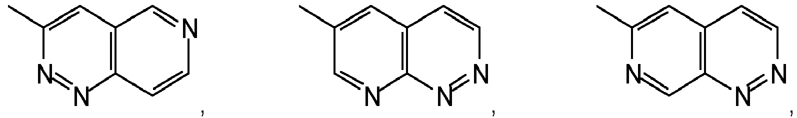 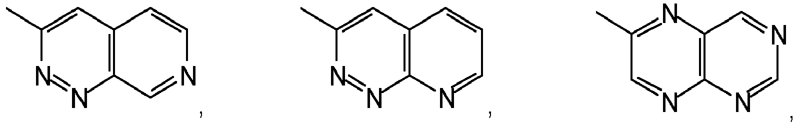 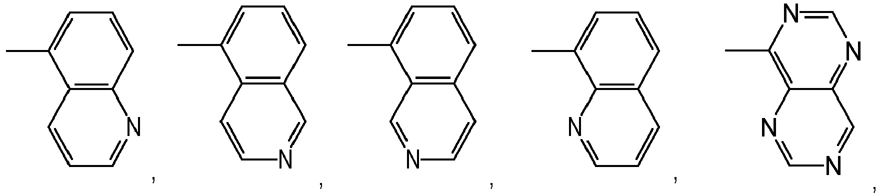 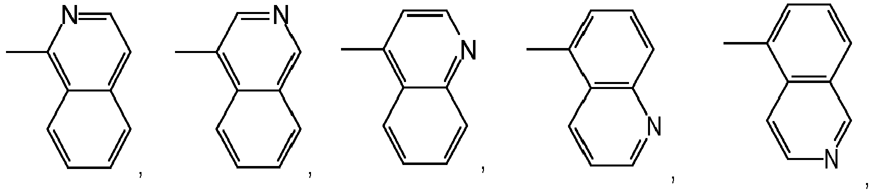 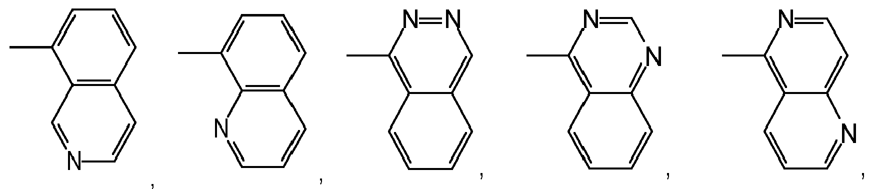 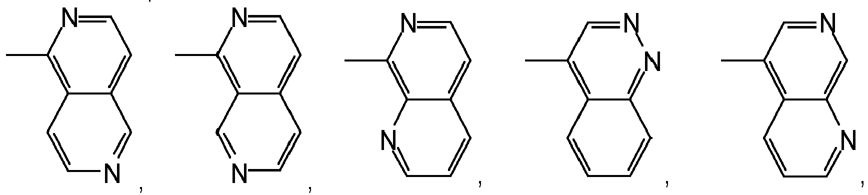 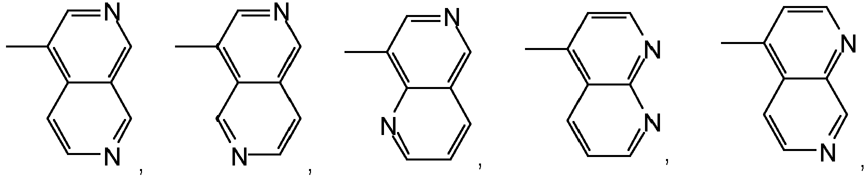 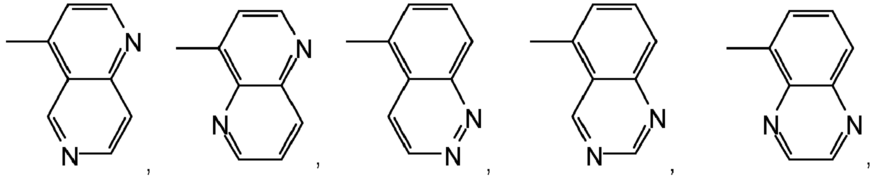 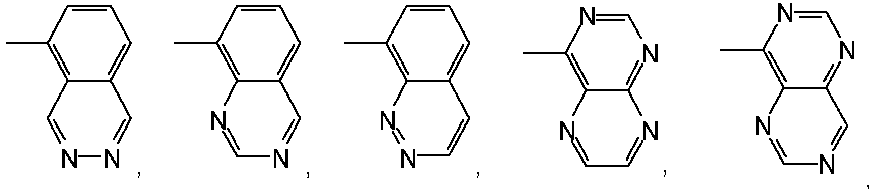 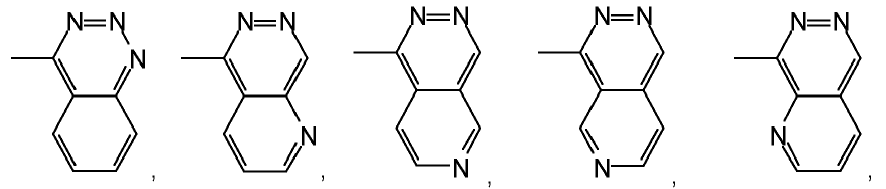 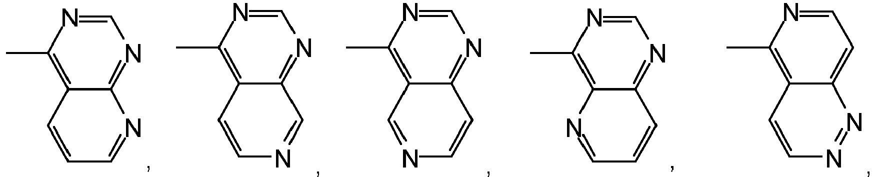 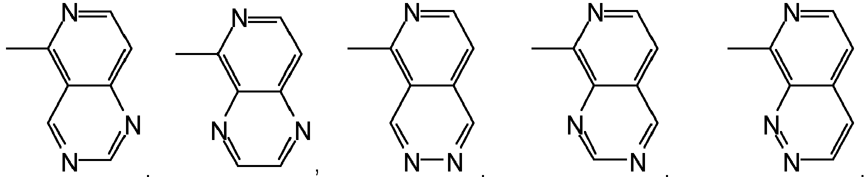 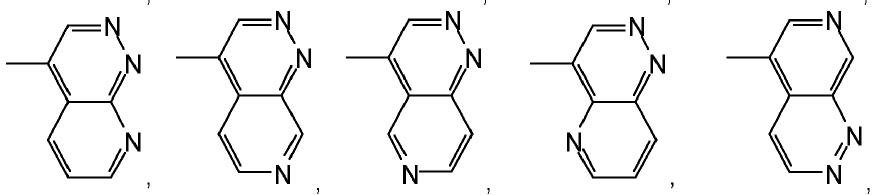 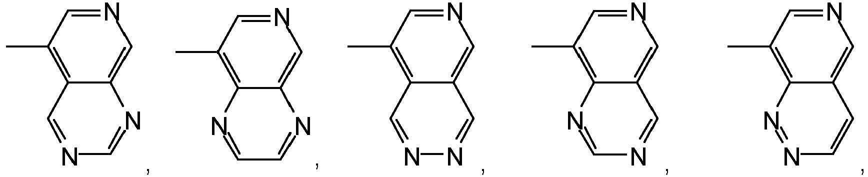 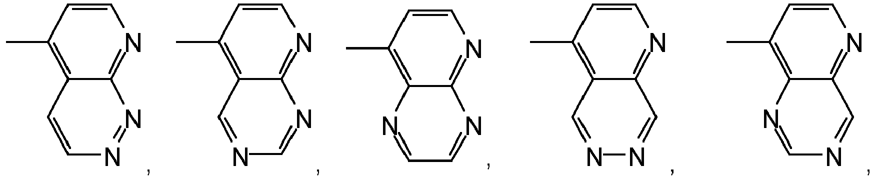 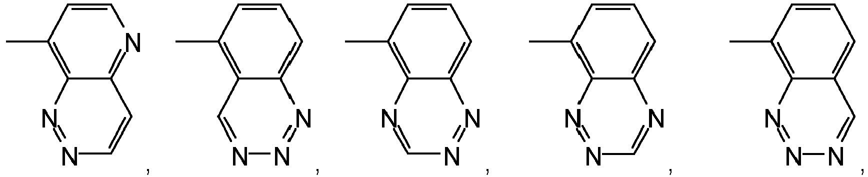 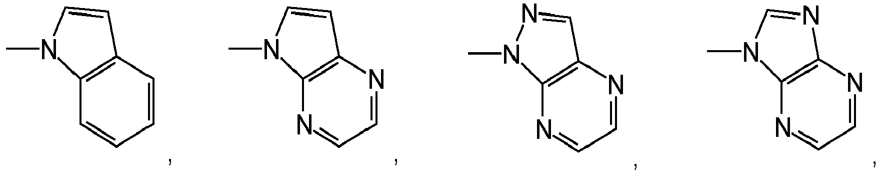 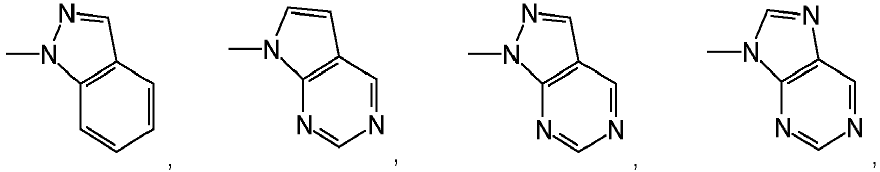 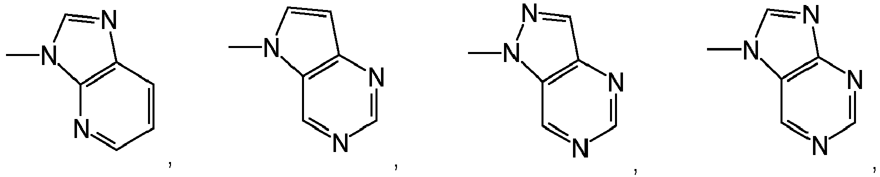 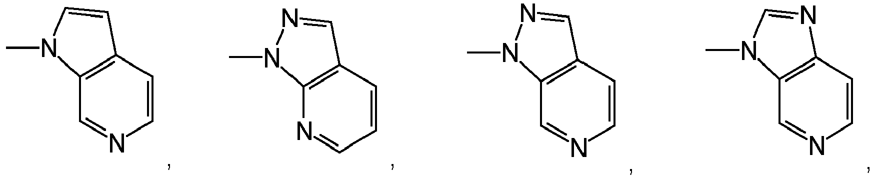 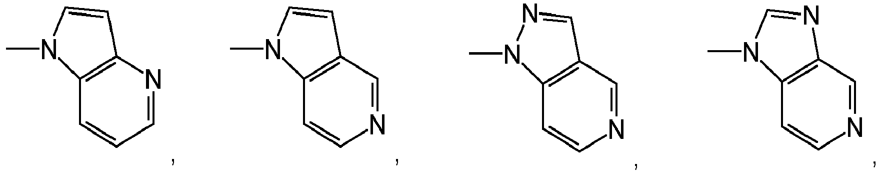 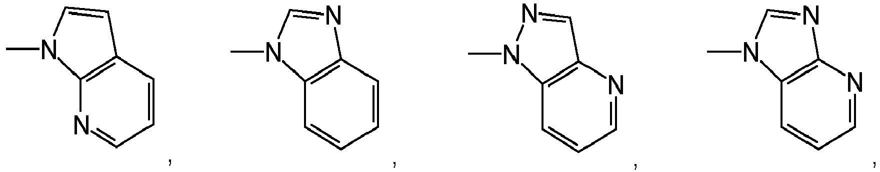 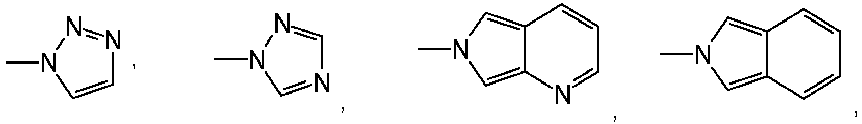 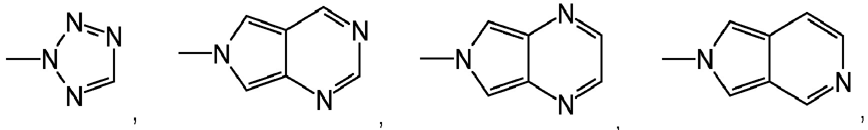 wherein these C₁-C₁₀ heteroaryl residues can be substituted with 1 to 5 substituents selected from **R^{N1}**, **Z¹**, **Z²**, **Z³**, **Z⁴** and **Z⁵**. However it is clear to a skilled person that the term "can be substituted" refers to the replacement of a hydrogen atom by one of the substituents **R^{N1}**, **Z¹**, **Z²**, **Z³**, **Z⁴** and **Z⁵**. Moreover it is clear to a skilled person that only these hydrogen atoms which are present in the residue can be replaced by the substituents **R^{N1}**, **Z¹**, **Z²**, **Z³**, **Z⁴** and **Z⁵**. In case of secondary amine group in these heteroaryl residues, a hydrogen atom of secondary amine group is replaced by the substituent **R^{N1}**. Thus, since the oxadiazole group has only one hydrogen atom, only one hydrogen atom can be replaced by one substituent selected from **R^{N1}**, **Z¹**, **Z²**, **Z³**, **Z⁴** and **Z⁵**. The carbon atom number of C₁-C₁₀ refers only to the carbon atoms of the heteroaromatic ring system (heteroaryl) and does not include the carbon atoms of the substituents **R^{N1}**, **Z¹** to **Z⁵**. Moreover, the number of heteroatoms does preferably not exceed 4 and the heteroatoms are preferably selected from nitrogen, oxygen and sulphur.

Examples of preferred substituted C₁-C₁₀-heteroaryl residues are

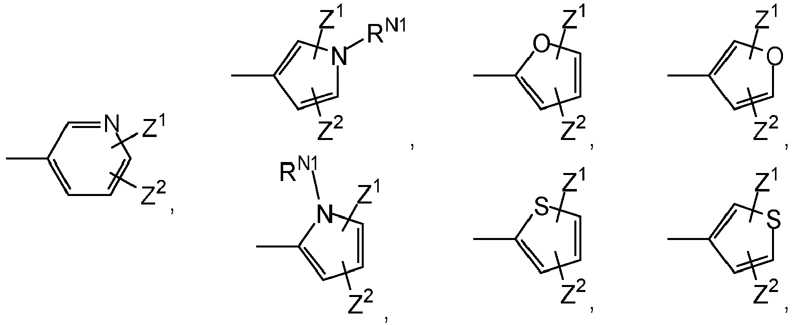

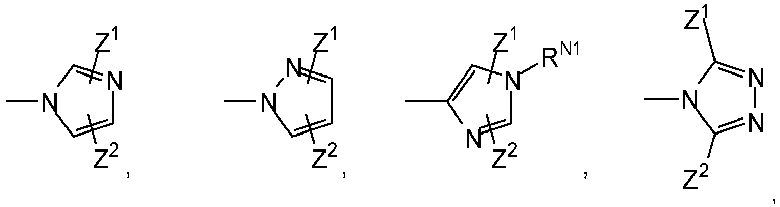

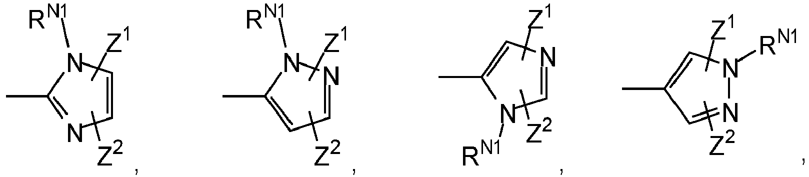

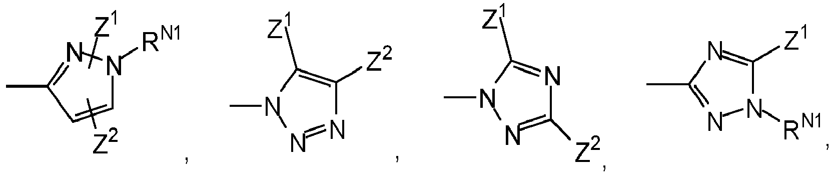

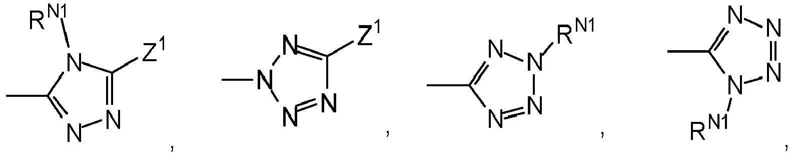

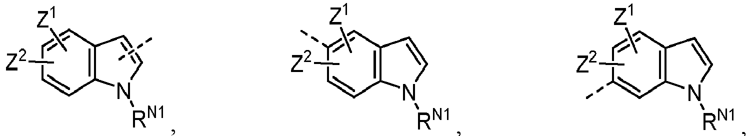

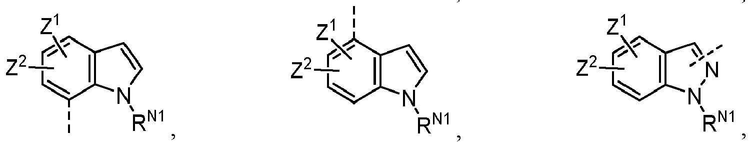

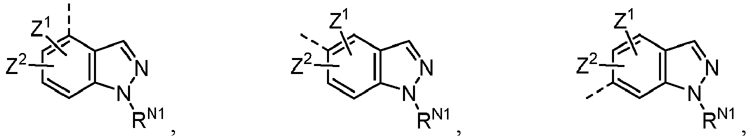

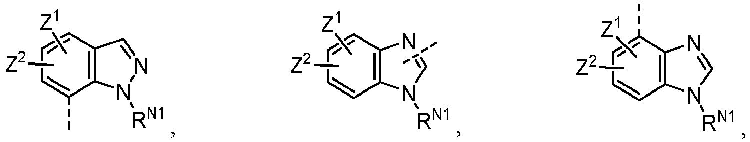

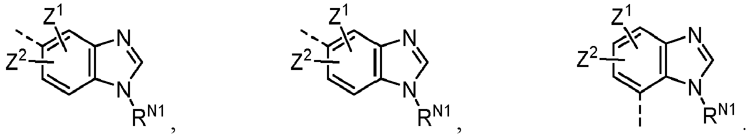

As used herein, the term **"C₆-C₁₀-aryl"** refers to aromatic residues or more specific to aromatic carbocyclic residues with one, or two aromatic rings and refers preferably to phenyl and naphthyl, wherein these phenyl and naphthyl residues can be substituted with 1 to 5 substituents selected from **R^{N1}**, **Z¹**, **Z²**, **Z³**, **Z⁴** and **Z⁵**. However it is clear to a skilled person that the term "can be substituted" refers to the replacement of a hydrogen atom by one of the substituents **Z¹**, **Z²**, **Z³**, **Z⁴** and **Z⁵**. The carbon atom number of **C₆-C₁₀** refers only to the carbon atoms of the aromatic ring system (aryl) and does not include the carbon atoms of the substituents **Z¹** to **Z⁵**.

Examples of preferred C₆-C₁₀-aryl groups and substituted C₆-C₁₀-aryl residues are

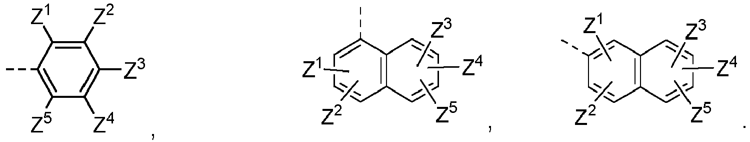

As used herein, the term **"C₃-C₆-cycloalkyl"** refers to 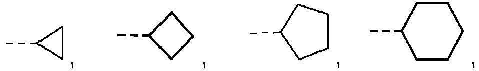 wherein these ring systems can be substituted with 1 to 5 substituents selected from **Z¹**, **Z²**, **Z³**, **Z⁴** and **Z⁵**. However it is clear to a skilled person that the term "can be substituted" refers to the replacement of a hydrogen atom by one of the substituents **Z¹**, **Z²**, **Z³**, **Z⁴** and **Z⁵**. The carbon atom number of **C₃-C₆** refers only to the carbon atoms of the cycloalkyl ring system and does not include the carbon atoms of the substituents **Z¹** to **Z⁵**.

As used herein, the term **"C₁-C₆-alkyl"** refers to -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, and -CH(CH₃)-C(CH₃)₃.

Preferably **R⁴** represents -C=CH, -C≡C-CH₃, 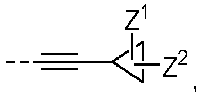 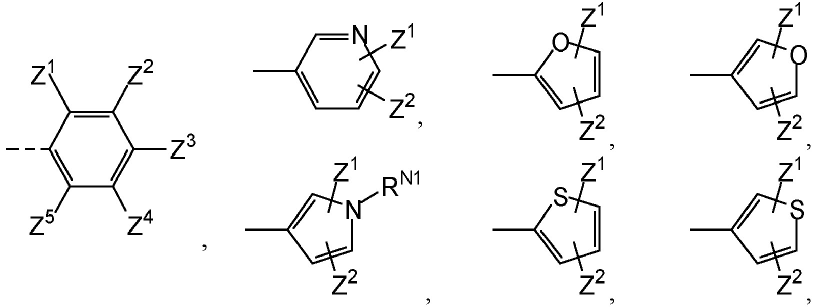 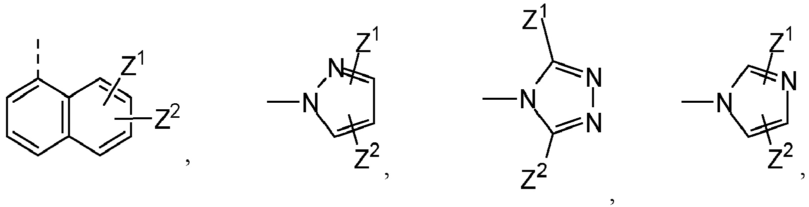 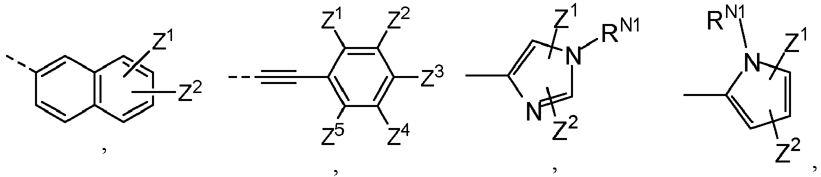 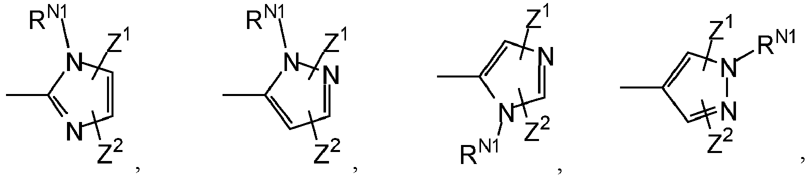 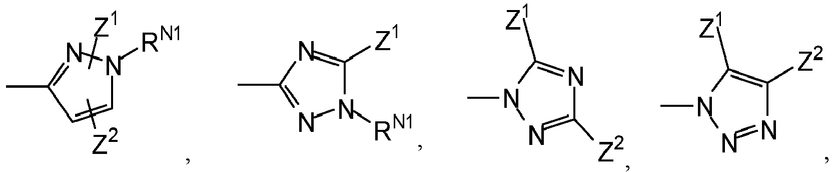 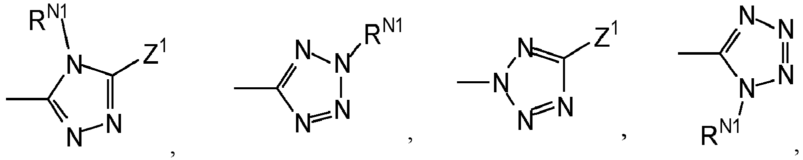 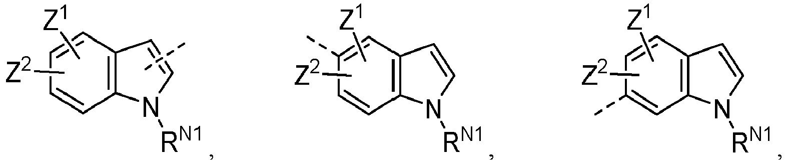 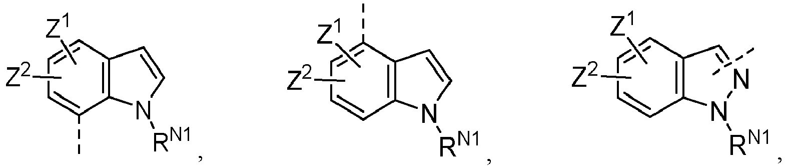 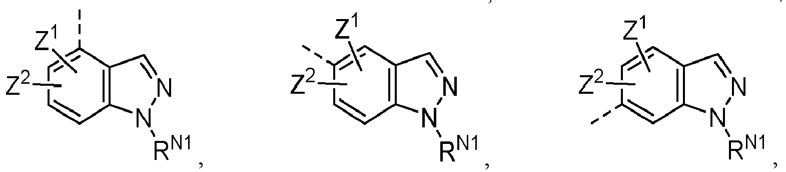 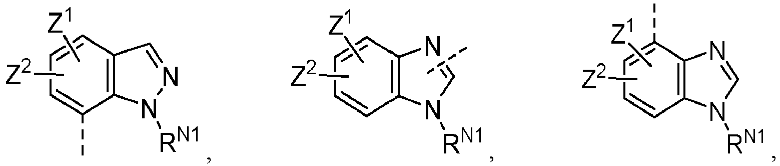 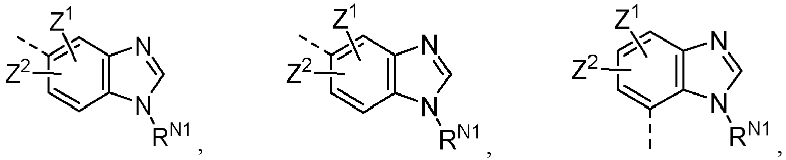 and **Z¹**, **Z²**, **Z³**, **Z⁴**, **Z⁵**, and **R^{N1}** have the meanings as defined herein.

Also preferred is the compound of the formula (I), wherein
**R¹** represents -**R⁴**, -O-**R⁴**, -O-CH₂-**R⁴**, -O-C₂H₄-**R⁴**, or -O-C₃H₆-**R⁴**;
**R⁴** represents -C=CH, -C≡C-CH₃, 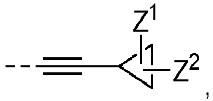 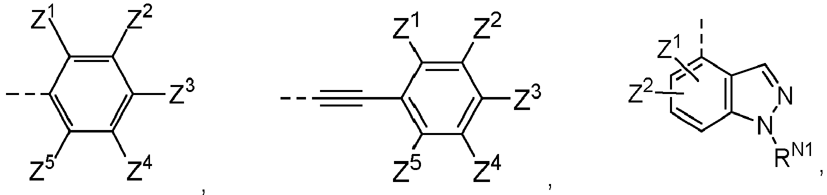 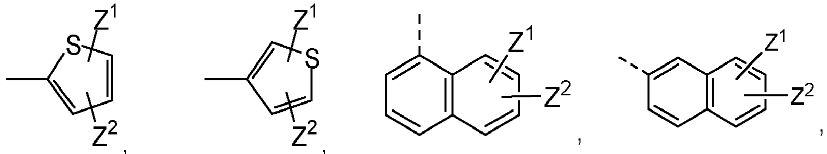 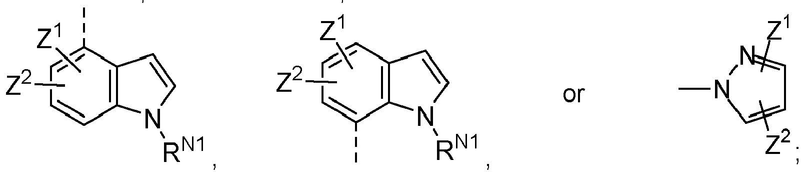
and **Z¹**, **Z²**, **Z³**, **Z⁴**, **Z⁵**, and **R^{N1}** have the meanings as defined herein.

Also preferred are the compounds of the formula (I), wherein
**R⁶** represents -OH, -OCH₃, -OC₂H₅, -N(CH₃)₂, -N(C₂H₅)₂, -C₂H₄OH, -OC₂H₄OH -(OC₂H₄)ₙOH, -(OC₂H₄)ₙOCH₃, -NR⁷R⁸, -CH₂NR⁷R⁸, -CH₂CH₂NR⁷R⁸ -CH₂CH₂CH₂NR⁷R⁸ -NHCOR⁷, -NHSO₂R⁷, -OCH₂NR⁷R⁸, -OCH₂CH₂NR⁷R⁸, -OCH₂CH₂CH₂NR⁷R⁸, -NR⁹CH₂NR⁷R⁸, -NR⁹CH₂CH₂NR⁷R⁸ -NR⁹CH₂CH₂CH₂NR⁷R⁸, -CONR⁷R⁸, -CONHCH₂NR⁷R⁸ -CONHCH₂CH₂NR⁷R⁸ -CONHCH₂CH₂CH₂NR⁷R⁸ -CN, 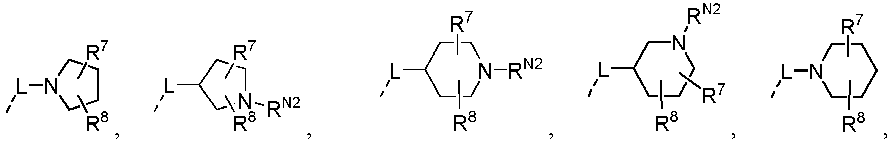 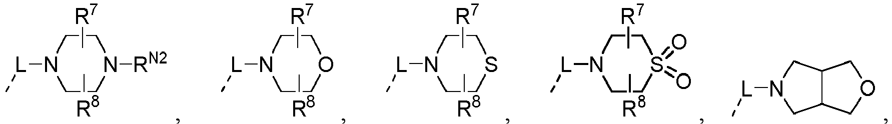 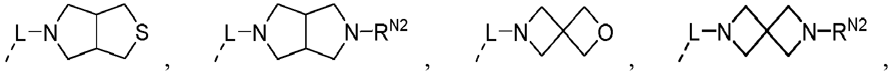 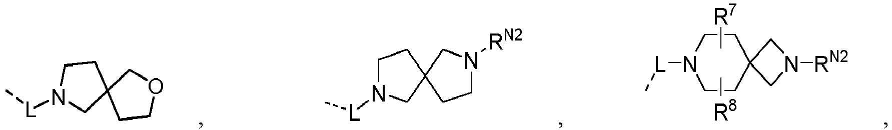 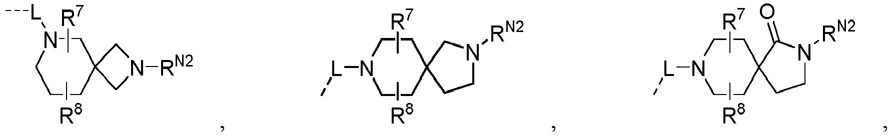 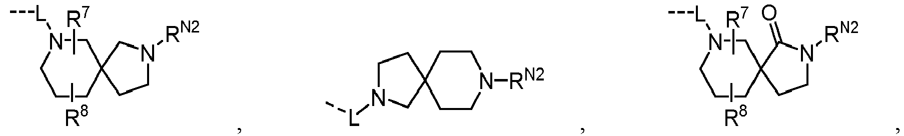 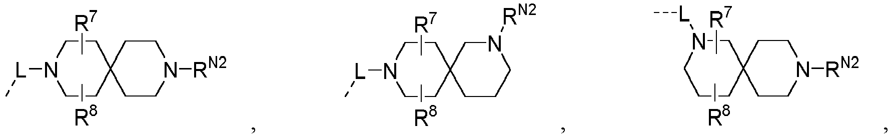 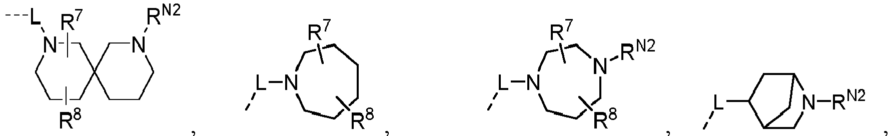 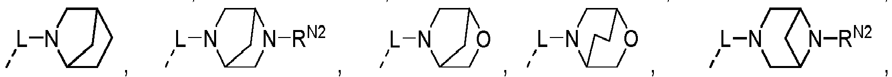 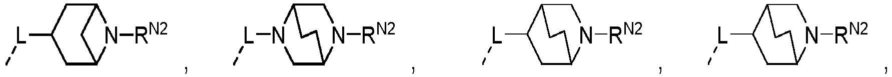 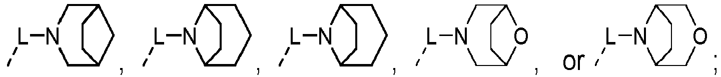 and
**L,** n, **R⁷**, **R⁸**, **R⁹** and **R^{N2}** have the same meanings as defined herein or as defined in formula (I).

More preferably, **R⁶** represents -OH, -OCH₃, -OC₂H₅, -N(CH₃)₂, -N(C₂H₅)₂, -NR⁷R⁸, -CH₂NR⁷R⁸, -NR⁹CH₂CH₂NR⁷R⁸, -(OC₂H₄)ₙOH, -(OC₂H₄)ₙOCH₃, 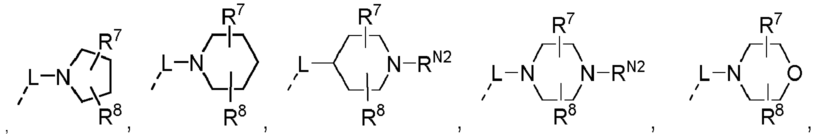 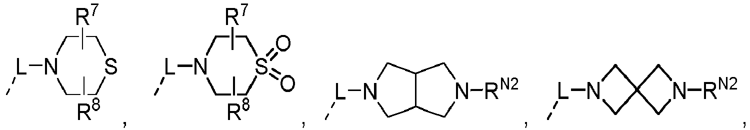 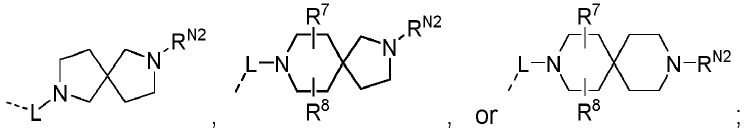 L represents a bond, -CH₂-, -CH₂CH₂-, -CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO- or -CH₂CH₂OCO-; and
n, **R⁷**, **R⁸**, **R⁹** and **R^{N2}** have the same meanings as defined herein or as defined in formula (I), and preferably **R⁷**, **R⁸**, **R⁹** and **R^{N2}** represent independently of each other -H, -CH₃, -C₂H₅, or -C₃H₇.

More preferred is the compound of the formula (I), wherein
**R⁶** represents -OH, -OCH₃, -OC₂H₅, -N(CH₃)₂, -N(C₂H₅)₂,

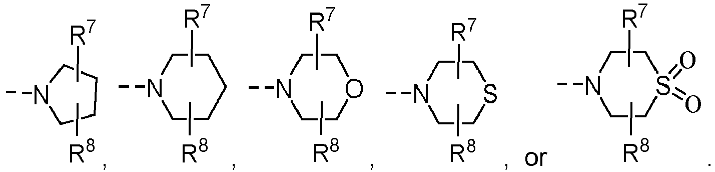

More preferred is the compound of the formula (I), wherein
**R³** represents -H, -**R⁶**, -(CH₂)ₘ-**R⁶**, or -O-(CH₂)ₘ-**R⁶**;
**R⁶** represents -OH, -OCH₃, -OC₂H₅, -N(CH₃)₂, -N(C₂H₅)₂, 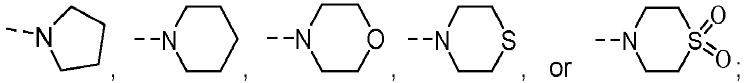 and
m represents the integer 1, 2 or 3.

Still more preferred is the compound of the formula (I), wherein
**R¹** represents -**R⁴**, -O-**R⁴**, -O-CH₂-**R⁴**, -O-C₂H₄-**R⁴**, or -O-C₃H₆-**R⁴**;
**R³** represents -H, -**R⁶**, -(CH₂)ₘ-**R⁶**, or -O-(CH₂)ₘ-**R⁶**;
**R⁴** represents -C=CH, -C≡C-CH₃, 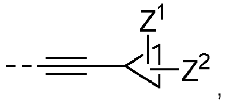 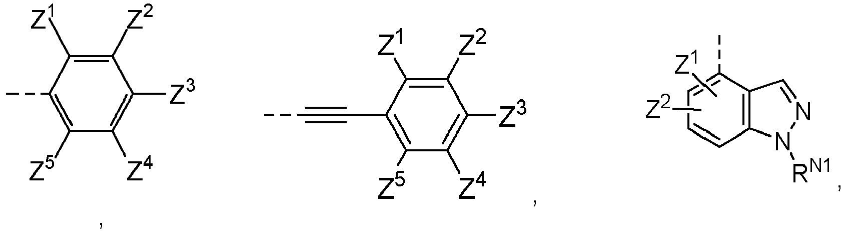 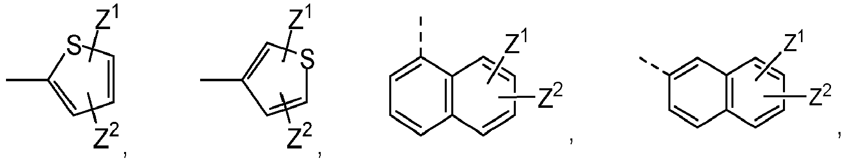 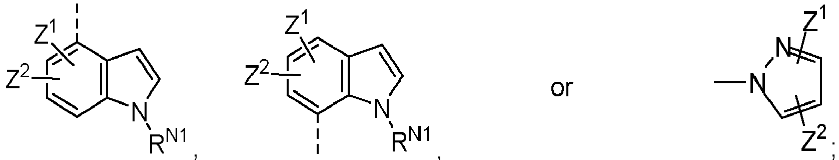
**R⁶** represents -OH, -OCH₃, -OC₂H₅, -N(CH₃)₂, -N(C₂H₅)₂, 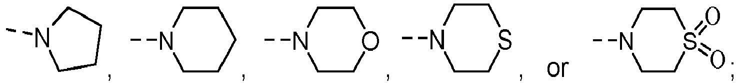
m represents the integer 1, 2 or 3,
and **Z¹**, **Z²**, **Z³**, **Z⁴**, **Z⁵**, and **R^{N1}** have the meanings as defined herein or as defined in formula (I).

Also preferred is the compound of the formula (**Ia**), 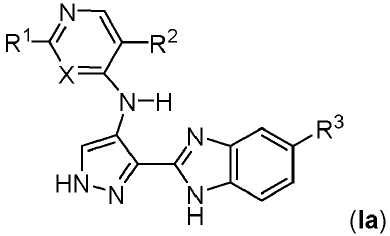 wherein
X represents N or CH;
**R¹**, **R²** and **R²** have the same meanings as defined in the formula (I),

Preferably,
**R¹** represents -C=CH, -C≡C-CH₃, -C≡C-cyclo-C₃H₅, -C=C-Ph, 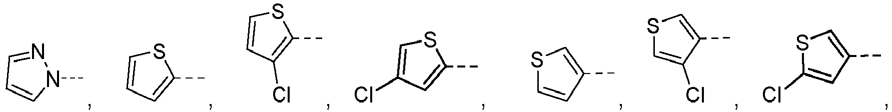 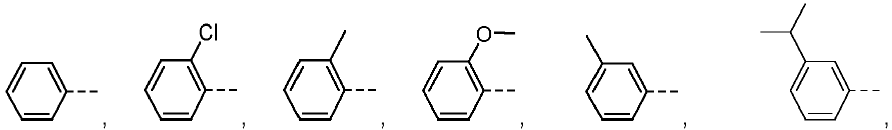 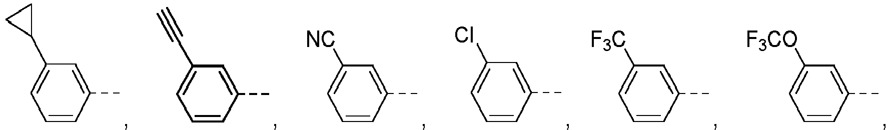 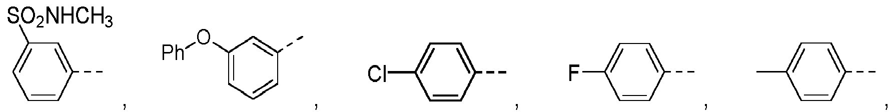 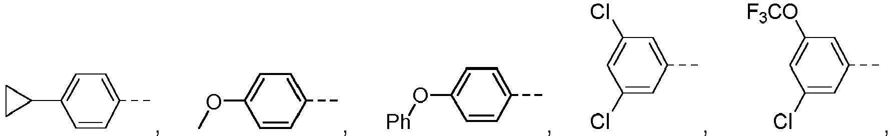 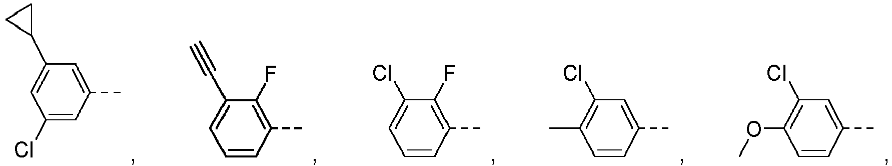 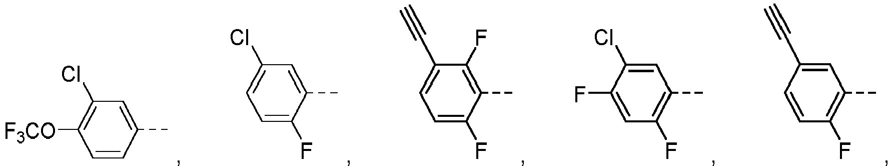 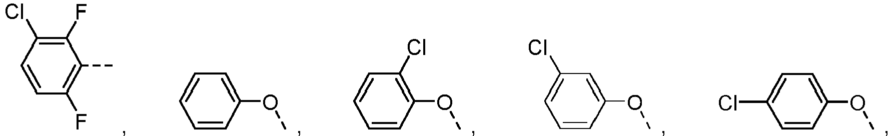 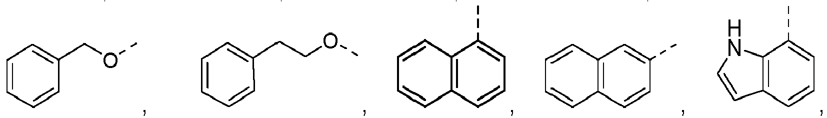 or 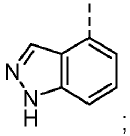
**R²** represents -H, -CH₃, -OCH₃, or -OCF₃;
**R³** represents -H, -CH₂-OH, -CH₂CH₂-OH, -CH₂-OCH₃, -CH₂CH₂-OCH₃, -CH₂N(CH₃)₂, -CH₂CH₂N(CH₃)₂, -CH₂N(C₂H₅)₂, -CH₂CH₂N(C₂H₅)₂, -OCH₂CH₂-OH, -OCH₂CH₂-OCH₃, -OCH₂CH₂N(CH₃)₂, -OCH₂CH₂N(C₂H₅)₂, 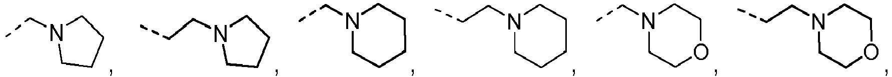 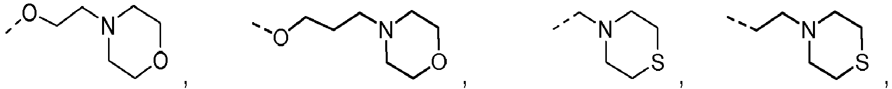 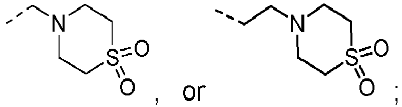
preferably **R³** represents -H, -CH₂-OH, -CH₂CH₂-OCH₃, -CH₂N(CH₃)₂, -OCH₂CH₂-OCH₃,

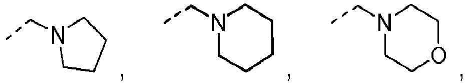

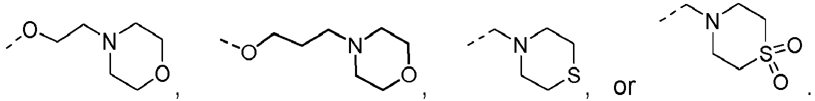

Preferably, the compound of the present invention has any one of the formulae (**I-1**) to (**I-8**): 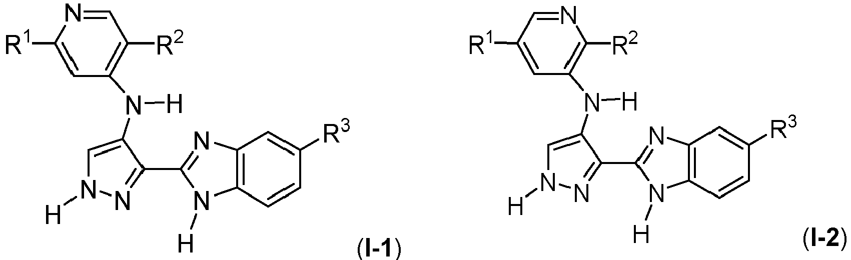 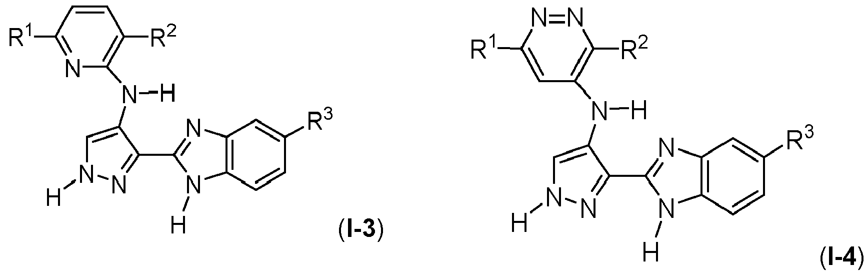 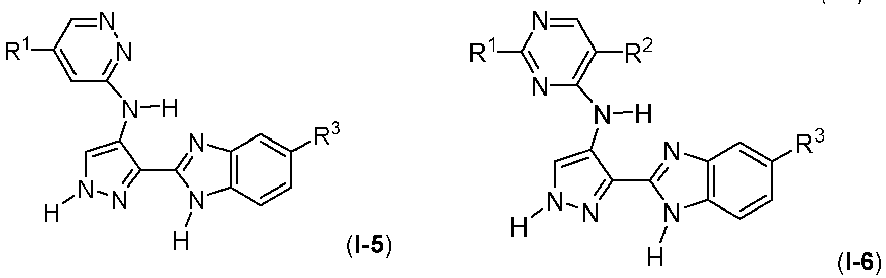 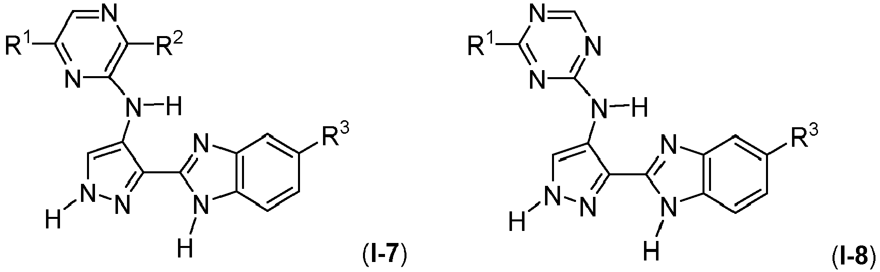 and **R¹**, **R²**, and **R³** have the meanings as defined herein.

Preferred is the compound of any one of the formulae (I), (**I-1**) to (**I-8**), wherein
**R¹** represents -**R⁴** -O-**R⁴**, -O-CH₂-**R⁴**, or -O-C₂H₄-**R⁴**;
**R⁴** represents -C≡CH, -C≡C-CH₃, -C≡C-cyclo-C₃H₅, -C=C-Ph, 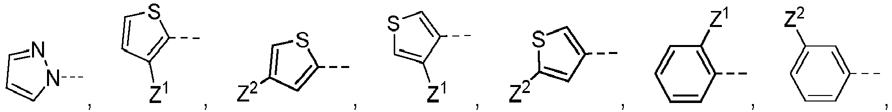 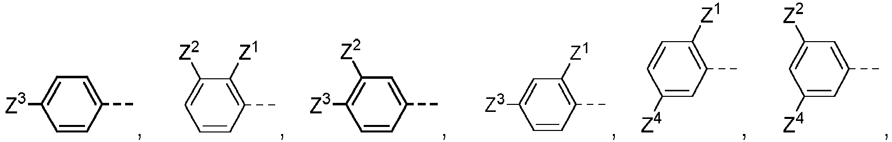 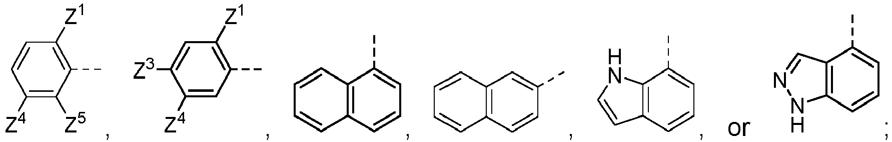 and
**Z¹**, **Z²**, **Z³**, **Z⁴**, and **Z⁵** represent independently of each other -H, -F, -CI, -CN, -CH₃, -CH(CH₃)₂, -cyclo-C₃H₅, -CF₃, -OCH₃, -OCF₃, -OPh, -SO₂NHCH₃, or -C=CH.

More preferred is the compound of any one of the formulae (I), (**I-1**) to (**I-8**), wherein
**R¹** represents -C≡CH, -C≡C-CH₃, -C≡C-cyclo-C₃H₅, -C=C-Ph, 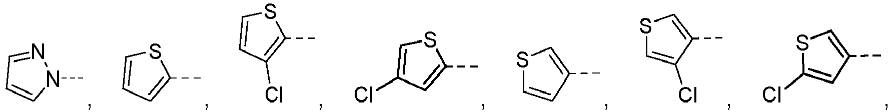 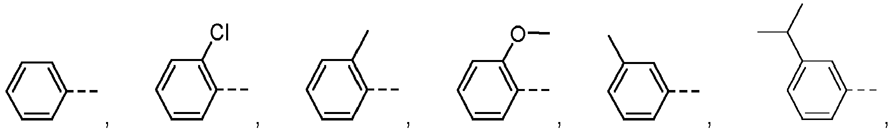 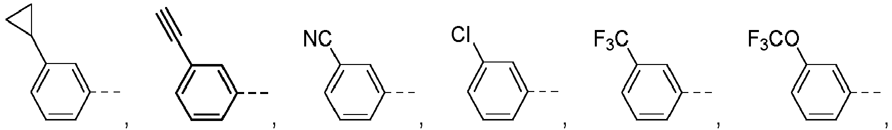 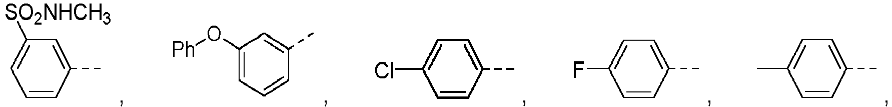 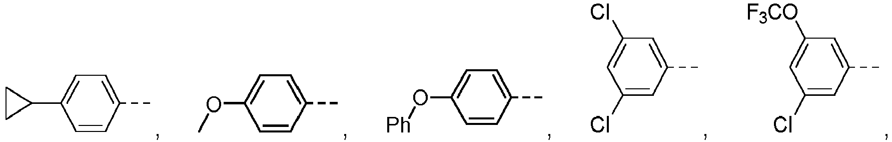 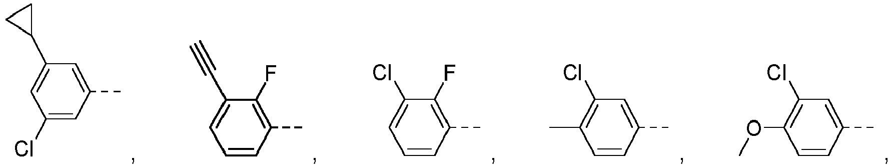 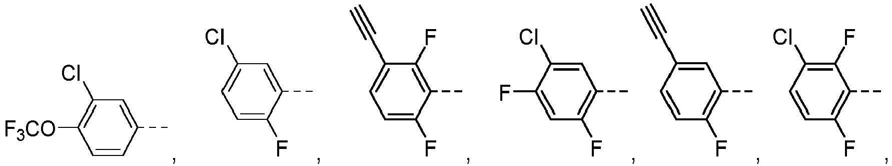 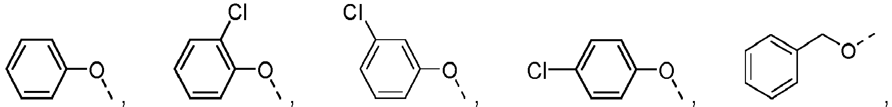 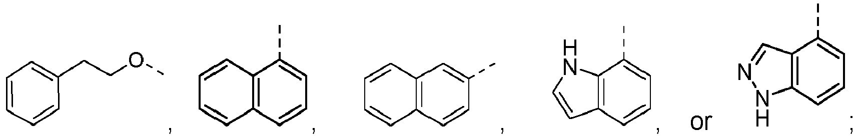
**R²** represents -H, -CH₃, -OCH₃, or -OCF₃;
**R³** represents -H, -CH₂-OH, -CH₂CH₂-OH, -CH₂-OCH₃, -CH₂CH₂-OCH₃, -CH₂N(CH₃)₂, -CH₂CH₂N(CH₃)₂, -CH₂N(C₂H₅)₂, -CH₂CH₂N(C₂H₅)₂, -OCH₂CH₂-OH, -OCH₂CH₂-OCH₃, -OCH₂CH₂N(CH₃)₂, -OCH₂CH₂N(C₂H₅)₂, 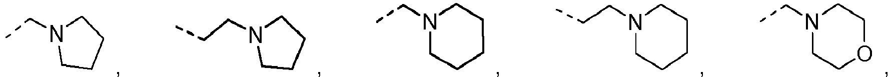 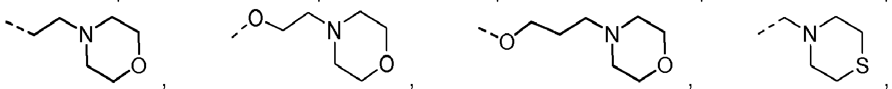 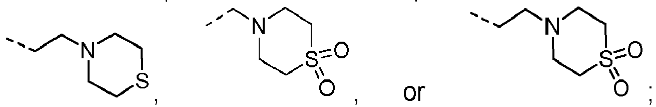
preferably **R³** represents -H, -CH₂-OH, -CH₂CH₂-OCH₃, -CH₂N(CH₃)₂, -OCH₂CH₂-OCH₃,

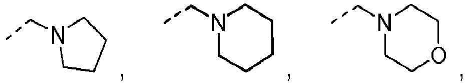

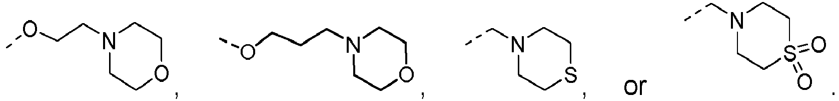

Also preferred is the compound of any one of the formulae (**II-1**), (**II-2**), (**II-3**), (**II-4**), (**II-5**), and (**II-6**): 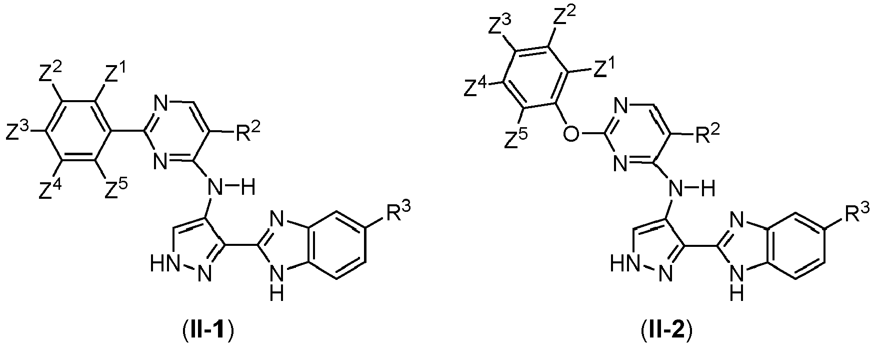 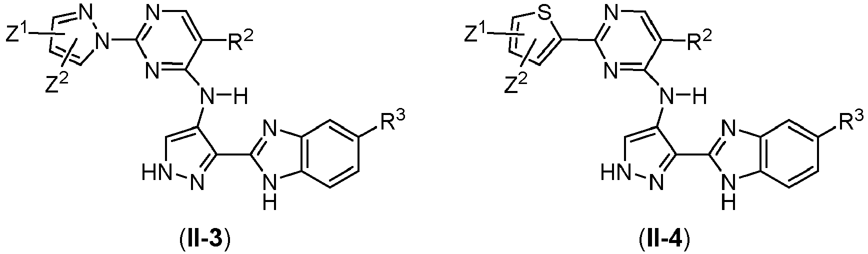 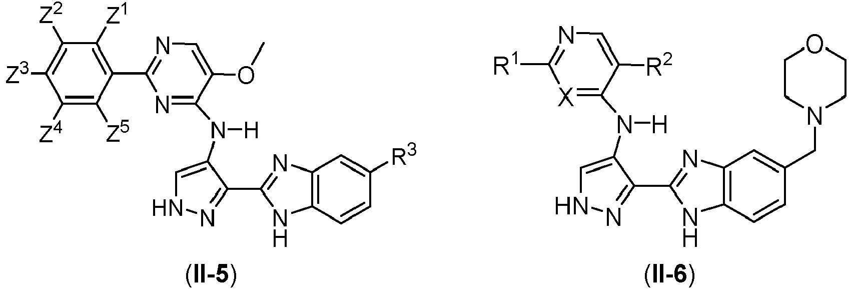 wherein X represents CH or N ; and **R¹**, **R²**, **R³**, **Z¹**, **Z²**, **Z³**, **Z⁴**, and **Z⁵** have the meanings as defined herein.

More preferred is the compound of any one of the formulae (**III-1**) -(**III-10**): 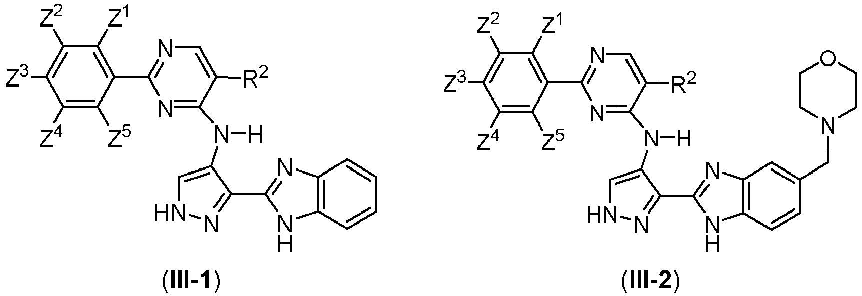 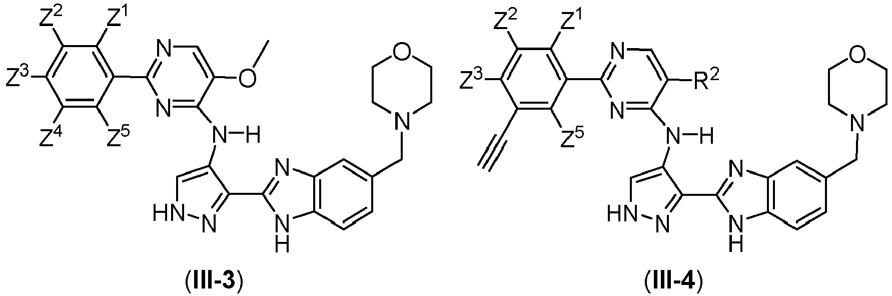 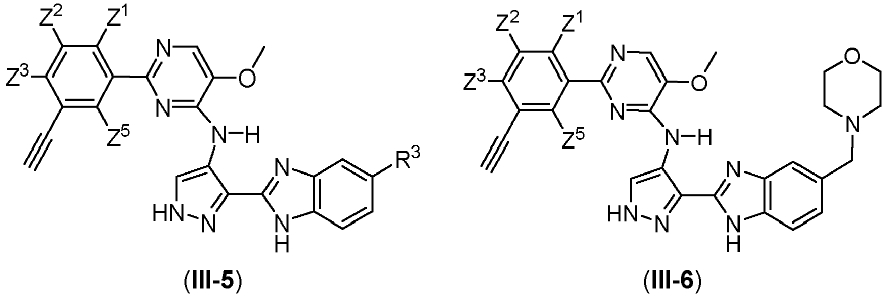 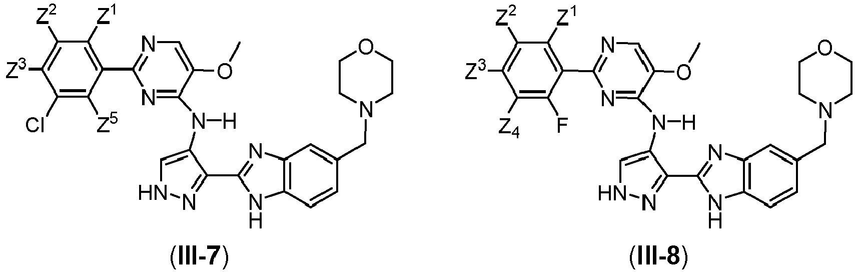 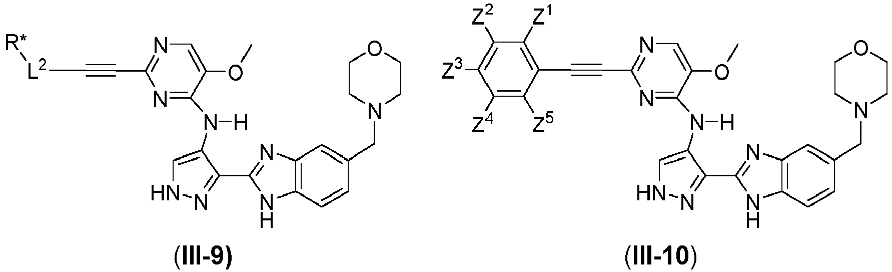 wherein **L²**, **R²**, **R³**, **R^{*}**, **Z¹**, **Z²**, **Z³**, **Z⁴**, and **Z⁵** have the meanings as defined herein.

Especially preferred compounds according to the present invention include compounds presented by **Table 1.**

**Table 1**

| **Compound** | **Structure** | **Nomenclature^{a)}** |
|---|---|---|
| **15** | 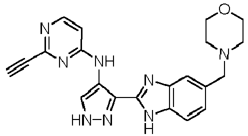 | 2-ethynyl-N-(3-(5-(morpholinomethyl)-1H-benzo[d]imidazol-2-yl)-1H-pyrazol-4-yl)pyrimidin-4-amine |
| **16** | 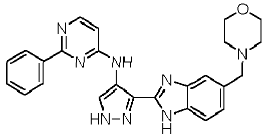 | N-(3-(5-(morpholinomethyl)-1H-benzo[d]imidazol-2-yl)-1H-pyrazol-4-yl)-2-phenylpyrimidin-4-amine |
| **17** | 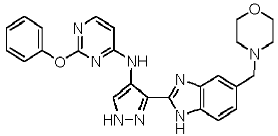 | N-(3-(5-(morpholinomethyl)-1H-benzo[d]imidazol-2-yl)-1H-pyrazol-4-yl)-2-phenoxypyrimidin-4-amine |
| **18** | 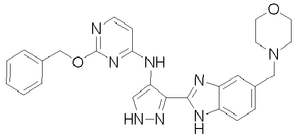 | 2-(benzyloxy)-N-(3-(5-(morpholinomethyl)-1H-benzo[d]imidazol-2-yl)-1H-pyrazol-4-yl)pyrimidin-4-amine |
| **19** | 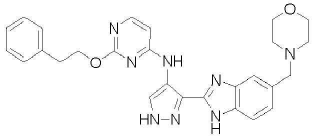 | N-(3-(5-(morpholinomethyl)-1H-benzo[d]imidazol-2-yl)-1H-pyrazol-4-yl)-2-phenethoxypyrimidin-4-amine |
| **20** | 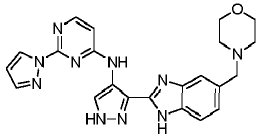 | N-(3-(5-(morpholinomethyl)-1H-benzo[d]imidazol-2-yl)-1H-pyrazol-4-yl)-2-(1 H-pyrazol-1-yl)pyrimidin-4-amine |
| **21** | 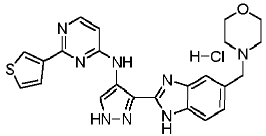 | N-(3-(5-(morpholinomethyl)-1H-benzo[d]imidazol-2-yl)-1H-pyrazol-4-yl)-2-(thiophen-3-yl)pyrimidin-4-amine hydrochloride |
| **22** | 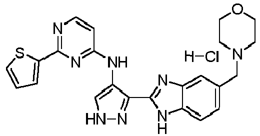 | N-(3-(5-(morpholinomethyl)-1H-benzo[d]imidazol-2-yl)-1H-pyrazol-4-yl)-2-(thiophen-2-yl)pyrimidin-4-amine hydrochloride |
| **23** | 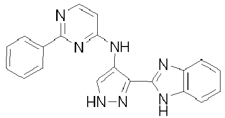 | N-(3-(1H-benzo[d]imidazol-2-yl)-1H-pyrazol-4-yl)-2-phenylpyrimidin-4-amine |
| **24** | 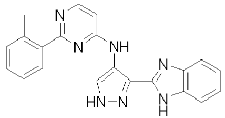 | N-(3-(1H-benzo[d]imidazol-2-yl)-1H-pyrazol-4-yl)-2-(o-tolyl)pyrimidin-4-amine |
| **25** | 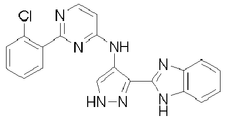 | N-(3-(1H-benzo[d]imidazol-2-yl)-1H-pyrazol-4-yl)-2-(2-chlorophenyl)pyrimidin-4-amine |
| **26** | 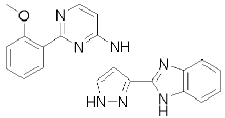 | N-(3-(1H-benzo[d]imidazol-2-yl)-1H-pyrazol-4-yl)-2-(2-methoxyphenyl)pyrimidin-4-amine |
| **27** | 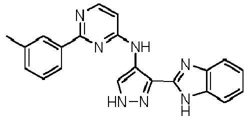 | N-(3-(1H-benzo[d]imidazol-2-yl)-1H-pyrazol-4-yl)-2-(m-tolyl)pyrimidin-4-amine |
| **28** | 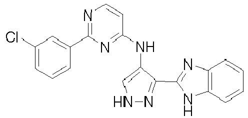 | N-(3-(1H-benzo[d]imidazol-2-yl)-1H-pyrazol-4-yl)-2-(3-chlorophenyl)pyrimidin-4-amine |
| **29** | 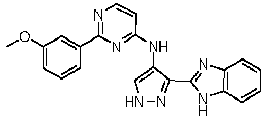 | N-(3-(1H-benzo[d]imidazol-2-yl)-1H-pyrazol-4-yl)-2-(3-methoxyphenyl)pyrimidin-4-amine |
| **30** | 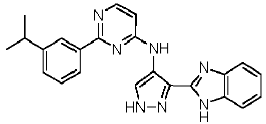 | N-(3-(1H-benzo[d]imidazol-2-yl)-1H-pyrazol-4-yl)-2-(3-isopropylphenyl)pyrimidin-4-amine |
| **32** | 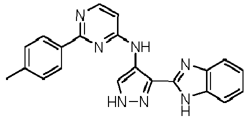 | N-(3-(1H-benzo[d]imidazol-2-yl)-1H-pyrazol-4-yl)-2-(p-tolyl)pyrimidin-4-amine |
| **33** | 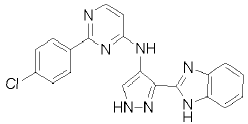 | N-(3-(1H-benzo[d]imidazol-2-yl)-1H-pyrazol-4-yl)-2-(4-chlorophenyl)pyrimidin-4-amine |
| **34** | 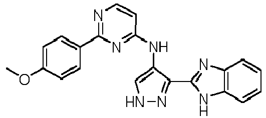 | N-(3-(1H-benzo[d]imidazol-2-yl)-1H-pyrazol-4-yl)-2-(4-methoxyphenyl)pyrimidin-4-amine |
| **36** | 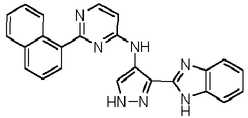 | N-(3-(1H-benzo[d]imidazol-2-yl)-1H-pyrazol-4-yl)-2-(naphthalen-1-yl)pyrimidin-4-amine |
| **37** | 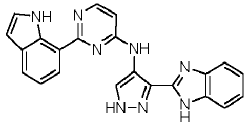 | N-(3-(1H-benzo[d]imidazol-2-yl)-1H-pyrazol-4-yl)-2-(1H-indol-7-yl)pyrimidin-4-amine |
| **38** | 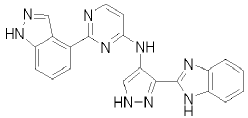 | N-(3-(1H-benzo[d]imidazol-2-yl)-1H-pyrazol-4-yl)-2-(1H-indazol-4-yl)pyrimidin-4-amine |
| **39** | 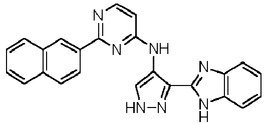 | N-(3-(1H-benzo[d]imidazol-2-yl)-1H-pyrazol-4-yl)-2-(naphthalen-2-yl)pyrimidin-4-amine |
| **41** | 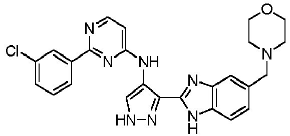 | 2-(3-chlorophenyl)-N-(3-(5-(morpholinomethyl)-1H-benzo[d]imidazol-2-yl)-1H-pyrazol-4-yl)pyrimidin-4-amine |
| **42** | 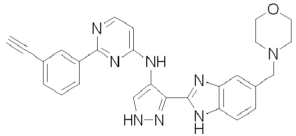 | 2-(3-ethynylphenyl)-N-(3-(5-(morpholinomethyl)-1H-benzo[d]imidazol-2-yl)-1H-pyrazol-4-yl)pyrimidin-4-amine |
| **43** | 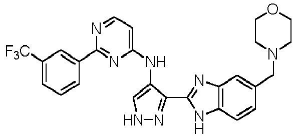 | N-(3-(5-(morpholinomethyl)-1H-benzo[d]imidazol-2-yl)-1H-pyrazol-4-yl)-2-(3-(trifluoromethyl)phenyl)pyrimidin-4-amine |
| **44** | 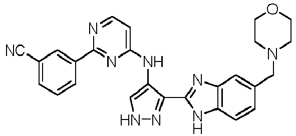 | 3-(4-((3-(5-(morpholinomethyl)-1H-benzo[d]imidazol-2-yl)-1H-pyrazol-4-yl)amino)pyrimidin-2-yl)benzonitrile |
| **45** | 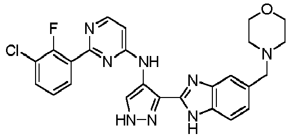 | 2-(3-chloro-2-fluorophenyl)-N-(3-(5-(morpholinomethyl)-1H-benzo[d]imidazol-2-yl)-1H-pyrazol-4-yl)pyrimidin-4-amine |
| **46** | 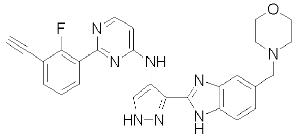 | 2-(3-ethynyl-2-fluorophenyl)-N-(3-(5-(morpholinomethyl)-1H-benzo[d]imidazol-2-yl)-1H-pyrazol-4-yl)pyrimidin-4-amine |
| **47** | 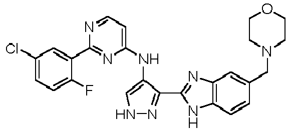 | 2-(5-chloro-2-fluorophenyl)-N-(3-(5-(morpholinomethyl)-1H-benzo[d]imidazol-2-yl)-1H-pyrazol-4-yl)pyrimidin-4-amine |
| **48** | 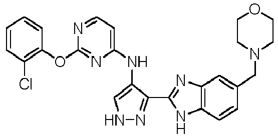 | 2-(2-chlorophenoxy)-N-(3-(5-(morpholinomethyl)-1H-benzo[d]imidazol-2-yl)-1H-pyrazol-4-yl)pyrimidin-4-amine |
| **49** | 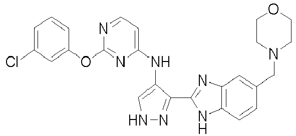 | 2-(3-chlorophenoxy)-N-(3-(5-(morpholinomethyl)-1H-benzo[d]imidazol-2-yl)-1H-pyrazol-4-yl)pyrimidin-4-amine |
| **50** | 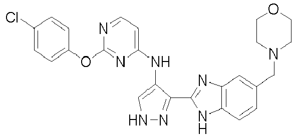 | 2-(4-chlorophenoxy)-N-(3-(5-(morpholinomethyl)-1H-benzo[d]imidazol-2-yl)-1H-pyrazol-4-yl)pyrimidin-4-amine |
| **51** | 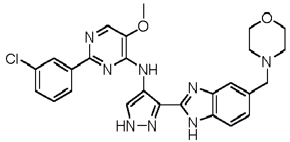 | 2-(3-chlorophenyl)-5-methoxy-N-(3-(5-(morpholinomethyl)-1H-benzo[d]imidazol-2-yl)-1H-pyrazol-4-yl)pyrimidin-4-amine |
| **52** | 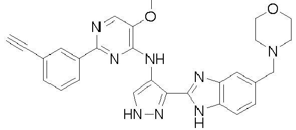 | 2-(3-ethynylphenyl)-5-methoxy-N-(3-(5-(morpholinomethyl)-1H-benzo[d]imidazol-2-yl)-1H-pyrazol-4-yl)pyrimidin-4-amine |
| **53** | 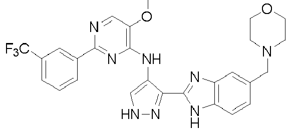 | 5-methoxy-N-(3-(5-(morpholinomethyl)-1H-benzo[d]imidazol-2-yl)-1H-pyrazol-4-yl)-2-(3-(trifluoromethyl)phenyl)pyrimidin-4-amine |
| **54** | 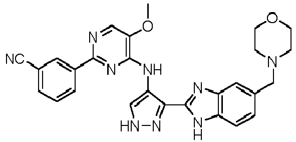 | 3-(5-methoxy-4-((3-(5-(morpholinomethyl)-1H-benzo[d]imidazol-2-yl)-1H-pyrazol-4-yl)amino)pyrimidin-2-yl)benzonitrile |
| **55** | 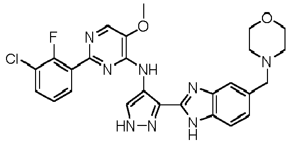 | 2-(3-chloro-2-fluorophenyl)-5-methoxy-N-(3-(5-(morpholinomethyl)-1H-benzo[d]imidazol-2-yl)-1H-pyrazol-4-yl)pyrimidin-4-amine |
| **56** | 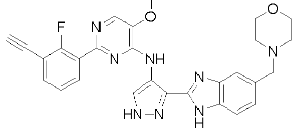 | 2-(3-ethynyl-2-fluorophenyl)-5-methoxy-N-(3-(5-(morpholinomethyl)-1H-benzo[d]imidazol-2-yl)-1H-pyrazol-4-yl)pyrimidin-4-amine |
| **57** | 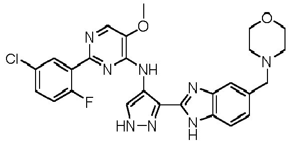 | 2-(5-chloro-2-fluorophenyl)-5-methoxy-N-(3-(5-(morpholinomethyl)-1H-benzo[d]imidazol-2-yl)-1H-pyrazol-4-yl)pyrimidin-4-amine |
| **58** | 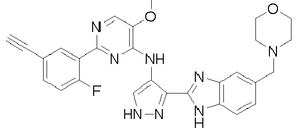 | 2-(5-ethynyl-2-fluorophenyl)-5-methoxy-N-(3-(5-(morpholinomethyl)-1H-benzo[d]imidazol-2-yl)-1H-pyrazol-4-yl)pyrimidin-4-amine |
| **59** | 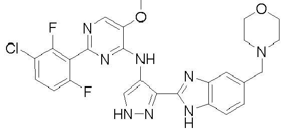 | 2-(3-chloro-2,6-difluorophenyl)-5-methoxy-N-(3-(5-(morpholinomethyl)-1H-benzo[d]imidazol-2-yl)-1H-pyrazol-4-yl)pyrimidin-4-amine |
| **60** | 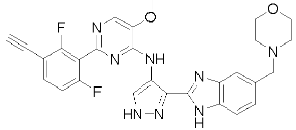 | 2-(3-ethynyl-2,6-difluorophenyl)-5-methoxy-N-(3-(5-(morpholinomethyl)-1H-benzo[d]imidazol-2-yl)-1H-pyrazol-4-yl)pyrimidin-4-amine |
| **61** | 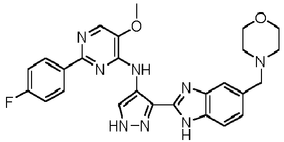 | 2-(4-fluorophenyl)-5-methoxy-N-(3-(5-(morpholinomethyl)-1H-benzo[d]imidazol-2-yl)-1H-pyrazol-4-yl)pyrimidin-4-amine |
| **144** | 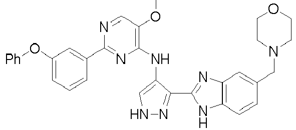 | 5-methoxy-N-(3-(5-(morpholinomethyl)-1H-benzo[d]imidazol-2-yl)-1H-pyrazol-4-yl)-2-(3-phenoxyphenyl)pyrimidin-4-amine |
| **145** | 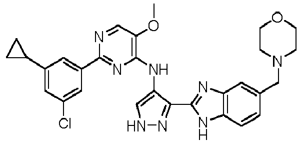 | 2-(3-chloro-5-cyclopropylphenyl)-5-methoxy-N-(3-(5-(morpholinomethyl)-1H-benzo[d]imidazol-2-yl)-1H-pyrazol-4-yl)pyrimidin-4-amine |
| **146** | 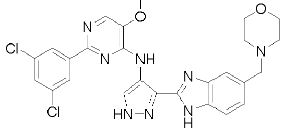 | 2-(3,5-dichlorophenyl)-5-methoxy-N-(3-(5-(morpholinomethyl)-1H-benzo[d]imidazol-2-yl)-1H-pyrazol-4-yl)pyrimidin-4-amine |
| **147** | 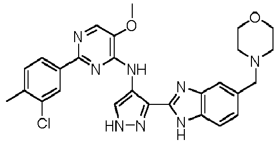 | 2-(3-chloro-4-methylphenyl)-5-methoxy-N-(3-(5-(morpholinomethyl)-1H-benzo[d]imidazol-2-yl)-1H-pyrazol-4-yl)pyrimidin-4-amine |
| **148** | 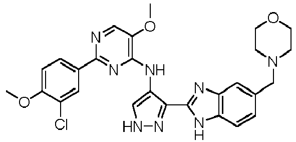 | 2-(3-chloro-4-methoxyphenyl)-5-methoxy-N-(3-(5-(morpholinomethyl)-1H-benzo[d]imidazol-2-yl)-1H-pyrazol-4-yl)pyrimidin-4-amine |
| **149** | 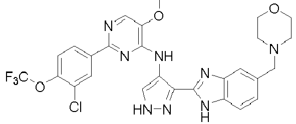 | 2-(3-chloro-4-(trifluoromethoxy)phenyl)-5-methoxy-N-(3-(5-(morpholinomethyl)-1H-benzo[d]imidazol-2-yl)-1H-pyrazol-4-yl)pyrimidin-4-amine |
| **150** | 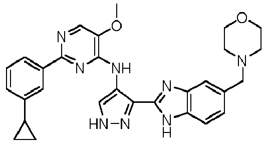 | 2-(3-cyclopropylphenyl)-5-methoxy-N-(3-(5-(morpholinomethyl)-1H-benzo[d]imidazol-2-yl)-1H-pyrazol-4-yl)pyrimidin-4-amine |
| **151** | 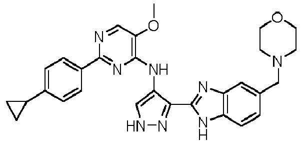 | 2-(4-cyclopropylphenyl)-5-methoxy-N-(3-(5-(morpholinomethyl)-1H-benzo[d]imidazol-2-yl)-1H-pyrazol-4-yl)pyrimidin-4-amine |
| **152** | 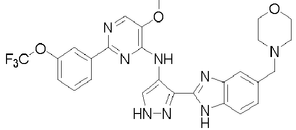 | 5-methoxy-N-(3-(5-(morpholinomethyl)-1H-benzo[d]imidazol-2-yl)-1H-pyrazol-4-yl)-2-(3-(trifluoromethoxy)phenyl)pyrimidin-4-amine |
| **153** | 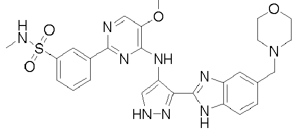 | 3-(5-methoxy-4-((3-(5-(morpholinomethyl)-1H-benzo[d]imidazol-2-yl)-1H-pyrazol-4-yl)amino)pyrimidin-2-yl)-N-methylbenzenesulfonamide |
| **154** | 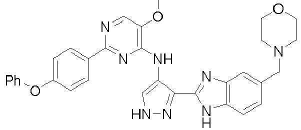 | 5-methoxy-N-(3-(5-(morpholinomethyl)-1H-benzo[d]imidazol-2-yl)-1H-pyrazol-4-yl)-2-(4-phenoxyphenyl)pyrimidin-4-amine |
| **155** | 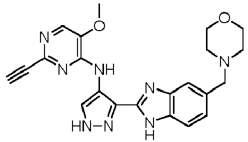 | 2-ethynyl-5-methoxy-N-(3-(5-(morpholinomethyl)-1H-benzo[d]imidazol-2-yl)-1H-pyrazol-4-yl)pyrimidin-4-amine |
| **156** | 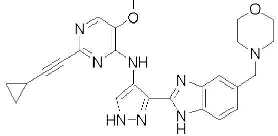 | 2-(cyclopropylethynyl)-5-methoxy-N-(3-(5-(morpholinomethyl)-1H-benzo[d]imidazol-2-yl)-1H-pyrazol-4-yl)pyrimidin-4-amine |
| **157** | 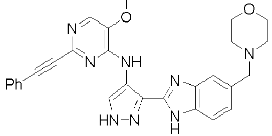 | 5-methoxy-N-(3-(5-(morpholinomethyl)-1H-benzo[d]imidazol-2-yl)-1H-pyrazol-4-yl)-2-(phenylethynyl)pyrimidin-4-amine |
| **158** | 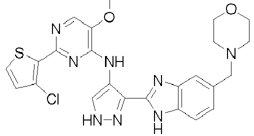 | 2-(3-chlorothiophen-2-yl)-5-methoxy-N-(3-(5-(morpholinomethyl)-1H-benzo[d]imidazol-2-yl)-1H-pyrazol-4-yl)pyrimidin-4-amine |
| **159** | 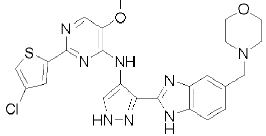 | 2-(4-chlorothiophen-2-yl)-5-methoxy-N-(3-(5-(morpholinomethyl)-1H-benzo[d]imidazol-2-yl)-1H-pyrazol-4-yl)pyrimidin-4-amine |
| **160** | 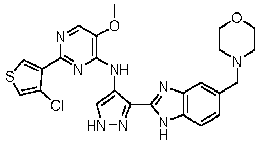 | 2-(4-chlorothiophen-3-yl)-5-methoxy-N-(3-(5-(morpholinomethyl)-1H-benzo[d]imidazol-2-yl)-1H-pyrazol-4-yl)pyrimidin-4-amine |
| **161** | 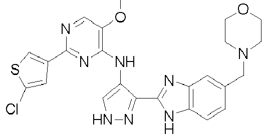 | 2-(5-chlorothiophen-3-yl)-5-methoxy-N-(3-(5-(morpholinomethyl)-1H-benzo[d]imidazol-2-yl)-1H-pyrazol-4-yl)pyrimidin-4-amine |
| **162** | 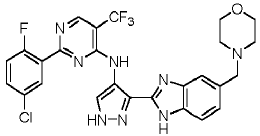 | 2-(5-chloro-2-fluorophenyl)-N-(3-(5-(morpholinomethyl)-1H-benzo[d]imidazol-2-yl)-1H-pyrazol-4-yl)-5-(trifluoromethyl)pyrimidin-4-amine |
| **163** | 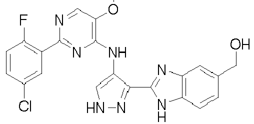 | (2-(4-((2-(5-chloro-2-fluorophenyl)-5-methoxypyrimidin-4-yl)amino)-1H-pyrazol-3-yl)-1H-benzo[d]imidazol-5-yl)methanol |
| **164** | 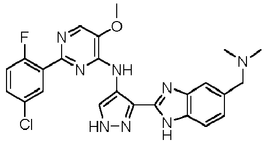 | 2-(5-chloro-2-fluorophenyl)-N-(3-(5-((dimethylamino)methyl)-1H-benzo[d]imidazol-2-yl)-1H-pyrazol-4-yl)-5-methoxypyrimidin-4-amine |
| **165** | 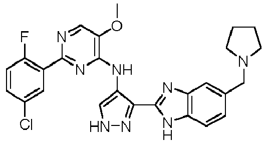 | 2-(5-chloro-2-fluorophenyl)-5-methoxy-N-(3-(5-(pyrrolidin-1-ylmethyl)-1H-benzo[d]imidazol-2-yl)-1H-pyrazol-4-yl)pyrimidin-4-amine |
| **166** | 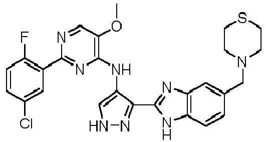 | 2-(5-chloro-2-fluorophenyl)-5-methoxy-N-(3-(5-(thiomorpholinomethyl)-1H-benzo[d]imidazol-2-yl)-1H-pyrazol-4-yl)pyrimidin-4-amine |
| **167** | 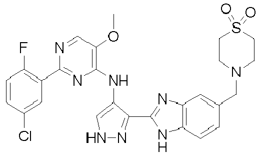 | 4-((2-(4-((2-(5-chloro-2-fluorophenyl)-5-methoxypyrimidin-4-yl)amino)-1H-pyrazol-3-yl)-1H-benzo[d]imidazol-5-yl)methyl)thiomorpholine 1,1-dioxide |
| **168** | 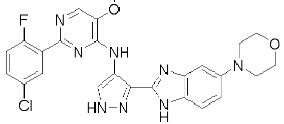 | 2-(5-chloro-2-fluorophenyl)-5-methoxy-N-(3-(5-morpholino-1H-benzo[d]imidazol-2-yl)-1H-pyrazol-4-yl)pyrimidin-4-amine |
| **169** | 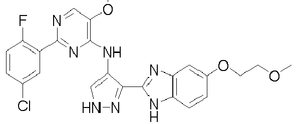 | 2-(5-chloro-2-fluorophenyl)-5-methoxy-N-(3-(5-(2-methoxyethoxy)-1H-benzo[d]imidazol-2-yl)-1H-pyrazol-4-yl)pyrimidin-4-amine |
| **170** | 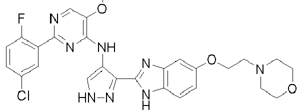 | 2-(5-chloro-2-fluorophenyl)-5-methoxy-N-(3-(5-(2-morpholinoethoxy)-1H-benzo[d]imidazol-2-yl)-1H-pyrazol-4-yl)pyrimidin-4-amine |
| **171** | 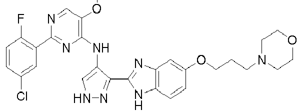 | 2-(5-chloro-2-fluorophenyl)-5-methoxy-N-(3-(5-(3-morpholinopropoxy)-1H-benzo[d]imidazol-2-yl)-1H-pyrazol-4-yl)pyrimidin-4-amine |
| **172** | 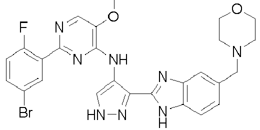 | 2-(5-bromo-2-fluorophenyl)-5-methoxy-N-(3-(5-(morpholinomethyl)-1H-benzo[d]imidazol-2-yl)-1H-pyrazol-4-yl)pyrimidin-4-amine |
| **173** | 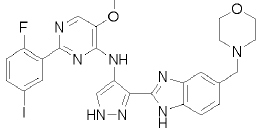 | 2-(2-fluoro-5-iodophenyl)-5-methoxy-N-(3-(5-(morpholinomethyl)-1H-benzo[d]imidazol-2-yl)-1H-pyrazol-4-yl)pyrimidin-4-amine |
| **174** | 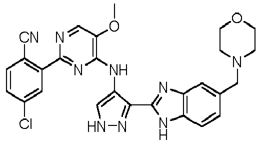 | 4-chloro-2-(5-methoxy-4-((3-(5-(morpholinomethyl)-1H-benzo[d]imidazol-2-yl)-1H-pyrazol-4-yl)amino)pyrimidin-2-yl)benzonitrile |
| **175** | 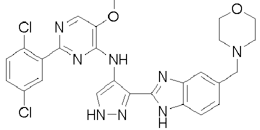 | 2-(2,5-dichlorophenyl)-5-methoxy-N-(3-(5-(morpholinomethyl)-1H-benzo[d]imidazol-2-yl)-1H-pyrazol-4-yl)pyrimidin-4-amine |
| **176** | 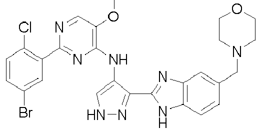 | 2-(5-bromo-2-chlorophenyl)-5-methoxy-N-(3-(5-(morpholinomethyl)-1H-benzo[d]imidazol-2-yl)-1H-pyrazol-4-yl)pyrimidin-4-amine |
| **177** | 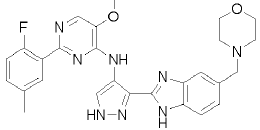 | 2-(2-fluoro-5-methylphenyl)-5-methoxy-N-(3-(5-(morpholinomethyl)-1H-benzo[d]imidazol-2-yl)-1H-pyrazol-4-yl)pyrimidin-4-amine |
| **178** | 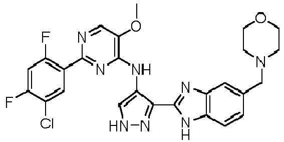 | 2-(5-chloro-2,4-difluorophenyl)-5-methoxy-N-(3-(5-(morpholinomethyl)-1H-benzo[d]imidazol-2-yl)-1H-pyrazol-4-yl)pyrimidin-4-amine |
| **179** | 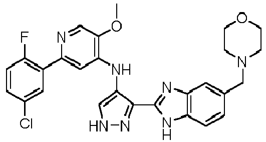 | 2-(5-chloro-2-fluorophenyl)-5-methoxy-N-(3-(5-(morpholinomethyl)-1H-benzo[d]imidazol-2-yl)-1H-pyrazol-4-yl)pyridin-4-amine |

| | | |
|---|---|---|
| **^{a)}** Nomenclature was generated by ChemDraw v. 19.1.21 | | |

or a tautomer, or a pharmaceutically acceptable salt thereof.

The present invention also includes within its scope N-oxides of the compounds of formula (I) above. In general, such N-oxides may be formed by conventional means, such as reacting the compound of formula (I) with oxone in the presence of wet alumina.

The expression tautomer is defined as an organic compound that is interconvertible by a chemical reaction called tautomerization. Tautomerization can be catalyzed preferably by bases or acids or other suitable compounds.

The pyrazole ring of the formula (I) may exhibit tautomerism and can exist in the two tautomeric forms. Further benzimidazole group of the formula (I) may also exist in the two tautomeric forms. Thus, the compounds of the formula (**Ia**) may have any of the following tautomeric forms (A) - (C).

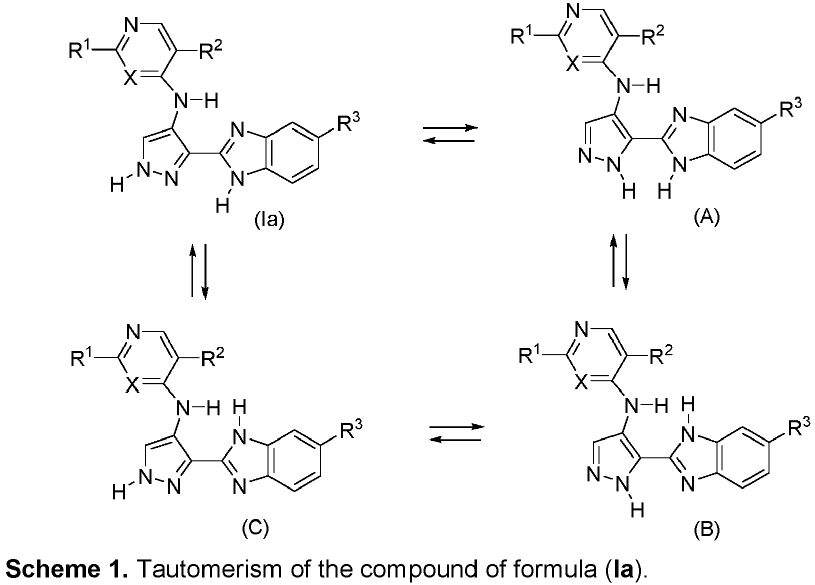

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

The inventive compounds may exist in a number of different polymorphic forms.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

### Syntheses of compounds

The compound of formula (**Ia**) is prepared by reference to the methods illustrated in the following schemes 2-4. The compound of formula (**Ia**) is produced by Schemes 2-4 by the suitable selection of reagents with appropriate substitution. Solvents, temperatures, pressures, and other reaction conditions may readily be selected by one of ordinary skill in the art. Starting materials are commercially available or readily prepared by one of ordinary skill in the art.

As shown in scheme 2, the present invention is directed to a method for producing the compound of the formula (**Ia**) comprising the steps:
**Step 1:** providing a compound **1*** wherein **X'** is Br, Cl, or I; 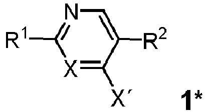
**Step 2:** performing a coupling reaction of compound **1*** with a compound **2*** 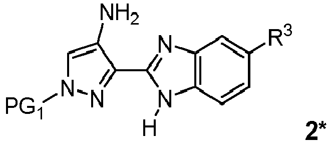 to obtain a compound **3*** 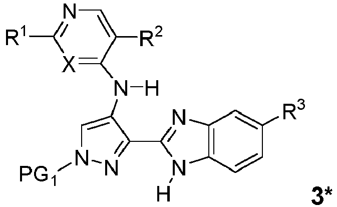
**Step** 3: removing a protecting group PG₁ from the compound **3*** to obtain the compound of the formula (Ia) 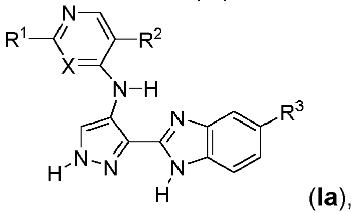 wherein **R¹**, **R², R³,** and **X** have the same meanings as defined in the formula (I) and
**PG₁** is an amine protecting group.

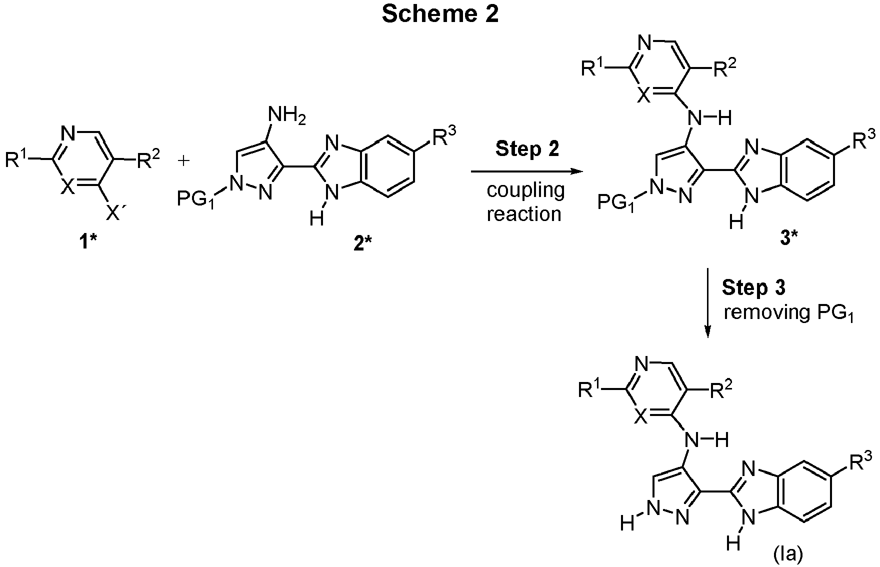

As shown in scheme 3, the present invention is also directed to a method for producing the compound of the formula (**Ia**) comprising the steps:
**Step 1A**: providing a compound **1b*** 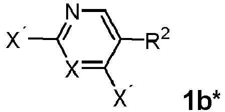 wherein **X'** is Br, Cl, or I;
**Step 2A:** performing a coupling reaction of compound **1b*** with a compound **2a*** 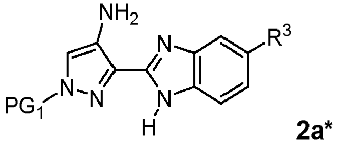 to obtain a compound **3a*** 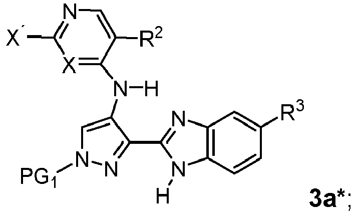
**Step 2B:** performing a coupling reaction of the compound **3a*** with a boronic acid compound **R¹**-B(O**R'**)(O**R**)^{^}) **4*** to obtain a compound **3***; 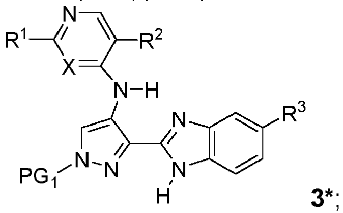
**Step 3:** removing a protecting group PG₁ from the compound **3*** to obtain the compound of the formula (**Ia**) 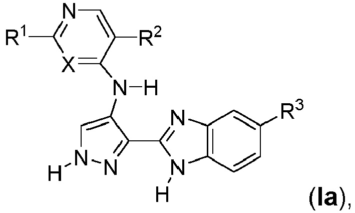 wherein
   **R¹**, **R², R³** and **X** have the same meanings as defined in the formula (I); **PG₁** is an amine protecting group; and
   **R'** and **R"** represent independently of each other H or an alkyl chain with 1-10 carbon atoms or a cycloalkyl chain with 3 to 12 carbon atoms or both residues **R'** and **R"** form together a residue derived from pinacol.

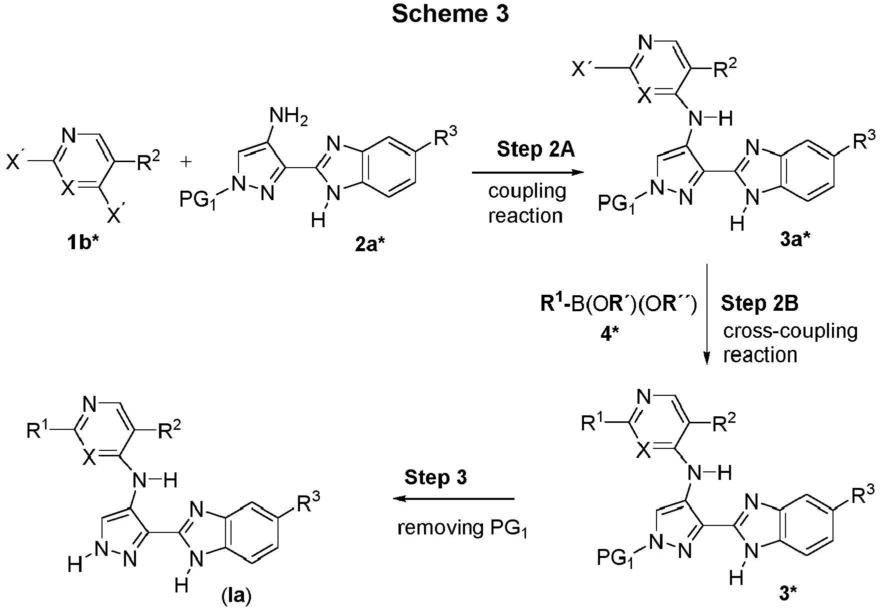

As shown in scheme 4, the present invention is also directed to a method for producing the compound of the formula (**Ia**) comprising the steps:
**Step 1A**: providing a compound **1b*** 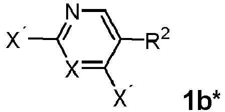 wherein **X'** is Br, Cl, or I;
**Step 2A**: performing a coupling reaction of compound **1b*** with a compound **2b*** 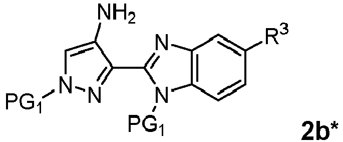 to obtain a compound **3b*** 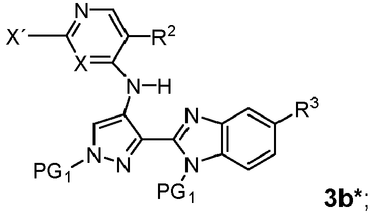
**Step 2B:** performing a coupling reaction of the compound **3b*** with a boronic acid compound **R¹**-B(O**R'**)(O**R**^{^}) **4*** to obtain a compound **5b***; 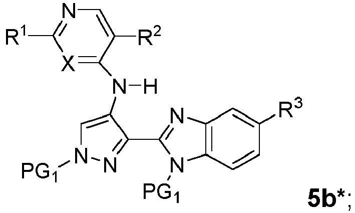
**Step 3B:** removing two protecting groups PG₁ from the compound **5b*** to obtain the compound of the formula (**Ia**) 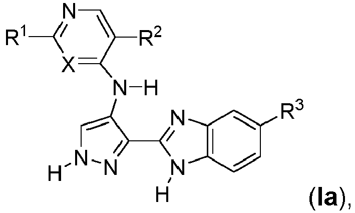 wherein
   **R¹**, **R², R³** and **X** have the same meanings as defined in the formula (I); **PG₁** is an amine protecting group; and
   **R'** and **R"** represent independently of each other H or an alkyl chain with 1-10 carbon atoms or a cycloalkyl chain with 3 to 12 carbon atoms or both residues **R'** and **R"** form together a residue derived from pinacol.

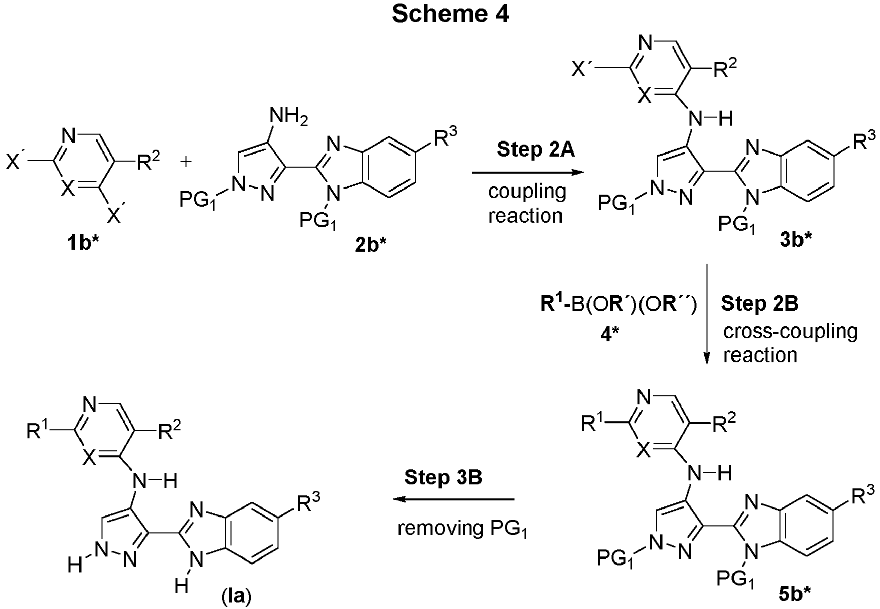

An alternative method for producing the compound of formula (**Ia**) is described in **Scheme 5.**

Thus, the present invention relates to a method for producing a compound of the formula (**Ia**) comprising the steps:
**Step 1C:** providing a compound **6*** 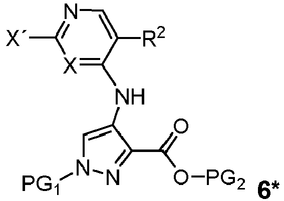 wherein **X'** is Br, Cl, or I;
**Step 2C:**
   i) performing a coupling reaction of compound **6*** with a compound **R¹**-B(O**R'**)(O**R**^{^}) **4*** to obtain a compound **7a*** 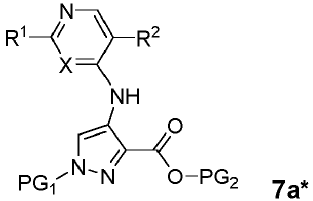
   ii) removing a protecting group **PG₂** to obtain a compound **7b*** 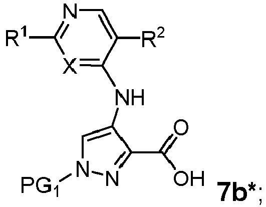
**Step 2D:** performing a condensation reaction of compound **7b*** with a compound **8*** 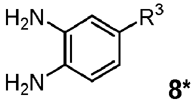 to obtain a compound **3*** 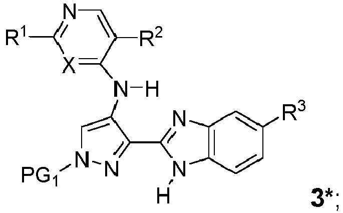
**Step 3:** removing a protecting group PG₁ from the compound **3*** to obtain the compound of the formula (**Ia**) 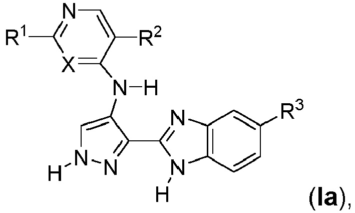 wherein
   **R¹**, **R², R³** and **X** have the same meanings as defined in the formula (I); **PG₁** is an amine protecting group;
   **PG₂** is a carboxyl protecting group;and
   **R'** and **R"** represent independently of each other H or an alkyl chain with 1-10 carbon atoms or a cycloalkyl chain with 3 to 12 carbon atoms or both residues **R'** and **R"** form together a residue derived from pinacol.

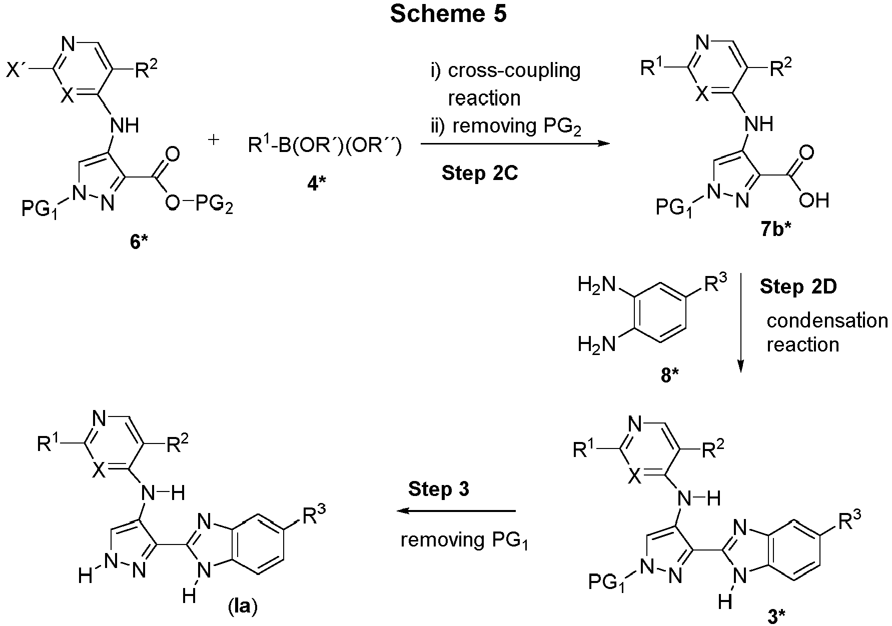

For the coupling reaction performed in Steps **2,** and **2A,** the coupling reaction as used herein refers to commonly used in aromatic substitution reaction in the presence of a base selected from trialkylamine such as Et₃N, or DIPEA, a metal hydroxide such as NaOH, KOH and a metal carbonate such as Li₂CO₃, Na₂CO₃, K₂CO₃, in a polar protic solvent including alcohol such as ethanol at a reaction temperature in a range of 20 to 150°C, preferred, 30 to 100°C, more preferred 30 to 70°C, and most preferred 40 to 60 °C. This coupling reaction may be carried out in an oil bath or by means of a microwave reactor.

In each of the steps **2B,** and **2C,** coupling reaction is performed in the present of the palladium catalyst, and the base. The palladium catalyst for C-C coupling reaction (Suzuki reaction) is Pd(0) or Pd(II) catalyst, preferred PdCl₂, Pd(PPh₃)₄, Pd(acac)₂, PdCl₂(CH₃CN)₂, PdCl₂(PCy₃)₂, PdCl₂(PPh₃)₂, Pd(dppf)Cl₂, [(π-allyl)PdCl]₂, (SIPr)PdCl₂(TEA). Optionally, further phosphine ligand may be used together with the first palladium catalyst.

A ratio of the palladium catalyst to the starting material is in the range of 0.01 to 20 mol-%, preferred 0.01-10 mol-%, more preferred 0.01-5 mol-%, most preferred 0.01-1 mol-%.

Preferred, the boronic acid derivative **4*** may be a boronic acid (R' = -H) or an ester of the boronic acid, e.g. its isopropyl ester (R' = -CH(CH₃)₂), an ester derived from pinacol (R'-R' = -C(CH₃)₂-C(CH₃)₂-).

Preferred, this reaction is performed in a polar aprotic solvent such as dioxane, DMF or DME, THF, a mixture thereof, or a mixture of said solvent and water in the presence of the base under N₂ atmosphere at a temperature in a range of 50 to 200°C, preferred, 50 to 150°C, more preferred -50 to 100, and most preferred 70 to 100°C.

The specific catalysis for C-N coupling reaction is selected from Pd, Cu, and Ni catalyses. Preferred, the palladium catalyst for C-N coupling reaction (Buchwald Reaction) is Pd(0) or Pd(II) catalyst, The Pd(0) catalyst may be tetrakis(triphenylphosphine)palladium(0) [Pd(PPh₃)₄], tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or more preferred [1,1'-bis(diphenylphosphino)ferrocene]palladium dichloride.

Preferred Pd catalyses with various phosphine ligands, especially any of Buchwald type Pd catalyses, for example, BrettPhosPd G1, BrettPhosPd G2,

BrettPhosPd G3, BrettPhosPd G4, RuPhosPd G1, RuPhosPd G2, RuPhosPd G3, RuPhosPd G4, RuPhosPd(allyl)CI, RuphosPd(crotyl)cl, RuphosPd(cinamyl)CI, SPhosPd G1, SPhosPd G2, SPhosPd G3, SPhosPd G4, XantPhos Pd G1, XantPhos Pd G2, XantPhos Pd G3, XantPhos Pd G4, XPhos Pd G1, XPhos Pd G2, XPhos Pd G3, XPhos Pd G4, XPhosPd(crotyl)CI, tBuXPhos Pd G1, tBuXPhos Pd G2, tBuXPhos Pd G3, tBuXPhos Pd G4, is used.

A ratio of the specific catalysis to the starting material is in the range of 0.01 to 20 mol-%, preferred 0.1-10 mol-%, more preferred 0.1-5 mol-%, most preferred 0.5-1 mol-%.

The cross-coupling reaction is preferably carried out in a polar solvent like DCM, DMF, DME, dioxane, THF, or isopropanol and a mixture thereof and in the presence of the base.

The base may be an organic base or inorganic bases. The organic base may be tertiary amine such as Et₃N, and DIPEA, DABCO, DBU, pyrrolidine or piperidine. The inorganic base may be K₂CO₃, Cs₂CO₃ or K₃PO₄. A ratio of the first base to the starting material is in the range of 1.0 to 5.0 equivalents, preferred 1.0 to 3.0 equivalents, more preferred 1.0 to 3.0 equivalents, most preferred 1.0 to 1.5 equivalents.

Preferred, this reaction is performed at a reactions temperature in a range of 50 to 200°C, preferred, 50 to 150°C, more preferred -50 to 100, and most preferred 70 to 100°C.

The term "protecting group" as used herein refers to commonly used protection groups in organic synthesis, preferably for amino and carboxyl groups.

A protecting group (PG₁) is an amino protecting group. The amine protecting group may be [2-(trimethylsilyl)ethoxy]methyl (SEM), t-butyloxycarbonyl (Boc) or benzyloxycarbonyl (Cbz) and removed under acidic condition, for example, treating with HCl, TBAF, or TFA. Preferably the protecting group PG₁ is [2-(trimethylsilyl)ethoxy]methyl (SEM).

In the **step 3** and the **step 3B,** any one of the general procedures A - D as described in the example is applied for removing the protecting group PG₁.

PG₂ is suitable protecting group for a carboxyl group. Preferably, -O-PG₂ may be selected from the group consisting of or comprising: methoxy, ethoxy, isobutoxy, tert-butoxy, benzyloxy; preferably, methoxy or ethoxy group. In the **step 2C**, PG₂ group is removed under basic condition such as LiOH.

In another aspects, the present invention is directed to an intermediate compound selected from the group consisting of the compounds **3***, **3a***, **3b***, **5b***, **6***, **7a***, and **7b***, 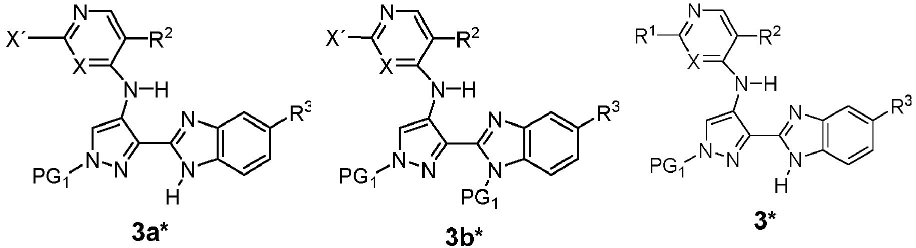 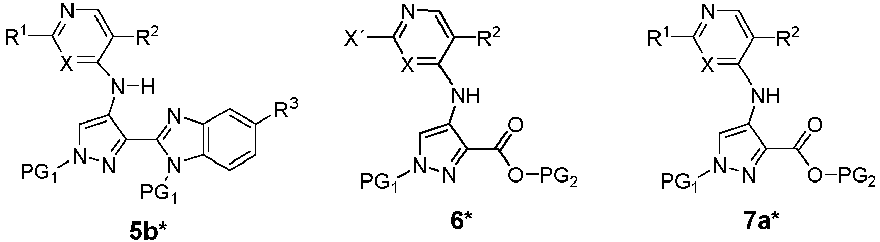 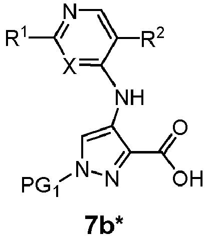 wherein **R¹**, **R², R³,** and **X** have the same meanings as defined in the formula (I) and **PG₁** is an amino protecting group as defined above, preferably, [2-(trimethylsilyl)ethoxy]methyl (SEM); **PG₂** is a protecting group for a carboxyl group as defined above, preferably methyl or ethyl.

It is found that the compound of the present invention is a selective inhibitor budding uninhibited by benzimidazole 1 kinase (BUB1 kinase) against and effectively inhibit the activity of BUB1 kinase as shown in Table 2.

Furthermore, it is also demonstrated that the compound of the present invention inhibit the proliferation of certain cancer or carcinoma cells effectively.

Thus, the compound of the present invention is useful as a medicament.

Thus, it is useful for the the treatment of cell proliferative disease, in particular, a cancer or a carcinoma associated with and/or caused by BUB1 kinase.

Further aspect of the present invention relates to the use of the compound of general formula (I) for the preparation of a pharmaceutical composition useful for treatment of a cancer or a carcinoma associated with and/or caused by BUB1 kinase.

Another aspect of the present invention relates to a method of treatment. This method comprises administering a therapeutically effective amount of at least one compound of general formula (I) to a patient suffering from a cancer or a carcinoma associated with and/or caused by BUB1 kinase

The proliferation disorders, especially the cancer or the carcinoma associated with and/or caused by BUB1 kinase is selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, estrogen dependent and independent breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma in situ, lobular carcinoma in situ, small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma, canine mammary carcinoma, and feline mammary carcinoma.

Preferred, is the compound of the present invention, or the pharmaceutical composition thereof is useful in the treatment of the following cancer or the carcinoma associated with and/or caused by BUB1 kinase: breast cancer, esophageal cancer, glioblastoma, hepatocellular carcinoma, gastric carcinomas, ovarian cancer, pancreatic cancer, prostate cancer, osteosarcoma, bladder cancer, cervical cancer, colon cancer, colorectal cancer, liver cancer, melanoma, and non-small cell lung cancer (NSCLC).

### Pharmaceutical Composition

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or the pharmaceutically acceptable salt as an active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anti-cancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to 1 weight %.

The compounds of the present invention are suitable for use in medicine, particulary in human medicine, but also in veterinary medicine. The dosage of the compounds may be determined by a skilled practitioner according to the type and severity of the disorder to be treated.

The compounds of the present invention may be administered as a monotherapy or together with further active agents, particulary chemotherapeutic agents or antitumor antibodies. Furthermore they may be used in combination with surgery and/or irradiation.

### Examples

### Synthetic procedures

All reagents were purchased from chemical suppliers (Fluorochem, Sigma-Aldrich, Merck, Fisher Scientific) and used without further purification. Solvents (Honeywell, VWR, Biosolve) indicated with "dry" were stored on activated 3Å (EtOH) or 4Å (other solvents) molecular sieves (8 to 12 mesh, Acros Organics). Solvents indicated by "degassed" were sonicated while bubbling N₂ through the solvent for 20 min. All reactions were performed at room temperature (RT) under a nitrogen atmosphere, unless stated otherwise. Reactions were monitored by thin layer chromatography (TLC, silica gel 60, UV₂₅₄, Macherey-Nagel, ref: 818333) and compounds were visualized by UV absorption (254 nm and/or 366 nm) or spray reagent (permanganate (5 g/L KMnO₄, 25 g/L K₂CO₃)) followed by heating. Alternatively, reactions were monitored by liquid chromatography mass spectrometry (LCMS), either on a Thermo Finnigan (Thermo Finnigan LCQ Advantage MAX ion-trap mass spectrometer (ESI+) coupled to a Surveyor HPLC system (Thermo Finnigan) equipped with a Nucleodur C18 Gravity column (50x4.6 mm, 3 µm particle size, Macherey-Nagel)) or a Thermo Fleet (Thermo LCQ Fleet ion-trap mass spectrometer (ESI+) coupled to a Vanquish UHPLC system). LCMS eluent consisted of MeCN in 0.1% TFA (aq.) and LCMS methods were as follows: 0.5 min cleaning with starting gradient, 8 min using specified gradient (linear), 2 min cleaning with 90% MeCN in 0.1% TFA (aq.). LCMS data is reported as follows: instrument (Finnigan or Fleet), gradient (% MeCN in 0.1% TFA (aq.)), retention time (tᵣ) and mass (as m/z: [M+H]⁺). Purity of final compounds was determined to be ≥ 95% by integrating UV intensity of spectra generated by either of the LCMS instruments. ¹H and ¹³C NMR spectra were recorded on a Bruker AV300 (300 and 75 MHz, respectively), Bruker AV400 (400 and 101 MHz, respectively), Bruker AV500 (500 and 126 MHz, respectively) or Bruker AV600 (600 and 150 MHz, respectively) NMR spectrometer. NMR samples were prepared in deuterated chloroform, methanol or DMSO. Chemical shifts are given in ppm (δ) relative to residual protonated solvent signals (CDCl₃ → δ 7.260 (¹H), δ 77.160 (¹³C), MeOD → δ 3.310 (¹H), δ 49.000 (¹³C), DMSO → δ 2.500 (¹H), δ 39.520 (¹³C)). Data was processed by using MestReNova (v. 14) and is reported as follows: chemical shift (δ), multiplicity, coupling constant (J in Hz) and integration. Multiplicities are abbreviated as follows: s = singlet, br s = broad singlet, d = doublet, dd = doublet of doublets, ddd = doublet of doublet of doublets, td = triplet of doublets, t = triplet, dt = doublet of triplets, q = quartet, hept = heptet, m = multiplet, br m = broad multiplet. For some molecules about 1:1 rotamer and/or tautomer peaks were observed, resulting in extra splitting of peaks. For these compounds, chemical shifts were reported as ranges and multiplicity was denoted by "2x", followed by the multiplicities specified above (i.e. 2x d = twice a doublet). The reported coupling constant belongs to either of the multiplet peaks (of note, coupling constants were the same for both multiplet peaks). Purification was done either by manual silica gel column chromatography (using 40-63 µm, 60 Å silica gel, Macherey-Nagel) or automated flash column chromatography on a Biotage Isolera machine (using pre-packed cartridges with 40-63 µm, 60 Å silica gel (4, 12, 25 or 40 g), Screening Devices). High-performance liquid chromatography (HPLC) purifications were performed on either an Agilent 1200 preparative HPLC system (equipped with a Gemini C18 column (250x10 mm, 5 µm particle size, Phenomenex) coupled to a 6130 quadrupole mass spectrometer) or a Waters Acquity UPLC system (equipped with a Gemini C18 column (150x21 mm, 5 µm particle size, Phenomenex) coupled to a SQ mass spectrometer). Specified gradients for HPLC purifications (MeCN in 0.2% TFA (aq.)) were linear (5 mL/min for 12 min (Agilent) or 25 mL/min for 10 min (Waters)). High resolution mass spectrometry (HRMS) spectra were recorded through direct injection of a 1 µM sample either on a Thermo Scientific Q Exactive Orbitrap equipped with an electrospray ion source in positive mode coupled to an Ultimate 3000 system (source voltage = 3.5 kV, capillary temperature = 275 °C, resolution R = 240,000 at m/z 400, external lock, mass range m/z = 150-2000) or on a Synapt G2-Si high definition mass spectrometer (Waters) equipped with an electrospray ion source in positive mode (ESI-TOF) coupled to a NanoEquity system with Leu-enkephalin (m/z = 556.2771) as internal lock mass. The eluent for HRMS measurements consisted of a 1:1 (v/v) mixture of MeCN in 0.1% formic acid (aq.) using a flow of 25 mL/min. Compound names were generated by ChemDraw v. 19.1.21.

**Abbreviations used in the description of the chemistry and in the Examples that follow are:**
MeCN (acetonitrile); br (broad); CDCl₃ (deuterated chloroform); DCM (dichloromethane); DIPEA (di-iso-propylethylamine); DMF (dimethylformamide); DMSO (dimethyl sulfoxide); EDC (1-ethyl-3-(3-dimethylaminopropyl)carbodiimide), eq. (equivalent); ES (electrospray); EtOAc (ethyl acetate); EtOH (ethanol); HCl (hydrochloric acid); HOAc (acetic acid); H₂O (water); HOBt (1-Hydroxybenzotriazole), K₂CO₃ (potassium carbonate); KOH (potassium hydroxide); MeOH (methanol); MS (mass spectrometry); NaHCO₃ (sodium hydrogencarbonate); NMR (nuclear magnetic resonance); Pd(dppf)Cl₂ ([1,1'-bis(diphenylphosphino)ferrocene]dichloro palladium(II) complex with dichloromethane); RT (room temperature); sat. aq. (saturated aqueous), SEM ([2-(trimethylsilyl)ethoxy]methyl); TBAF (tetra-n-butylammonium fluoride); TFA (trifluoroacetic acid); THF (tetrahydrofurane), TMS (trimethylsilyl).

### General procedure A - SEM deprotection

SEM-protected amine starting material was dissolved in DCM (2 mL) after which TFA (2 mL) was added dropwise. The mixture was stirred for the indicated time and subsequently concentrated under a flow of N₂. The mixture was suspended in EtOAc (25 mL) and poured into 1 M NaHCO₃ (aq.) (20 mL). The organic layer was separated and the water layer extracted with EtOAc (1 or 2x25 mL). The combined organic layers were concentrated as such, suspended/dissolved in 1:1 MeOH/DCM (2 mL) and transferred to a microwave vial. Ethylenediamine (50 µL, 746 µmol) was added after which the vial was sealed and the mixture was stirred at 50°C for 30 - 60 min. The mixture was poured into H₂O (20 mL) and when required, brine (0.5 - 1 mL), and the product extracted with EtOAc (2x20 mL). The combined organic layers were concentrated as such. Purification was performed as indicated.

### General procedure B - SEM deprotection

SEM-protected amine starting material was dissolved in DCM (0.5 mL) after which TFA (0.5 mL) was added dropwise. The mixture was stirred for the indicated time and subsequently quenched by addition of sat. NaHCO₃ (aq.) (20 mL). The product was extracted with EtOAc (2x20 mL) and the combined organic layers were concentrated as such. The mixture was suspended/dissolved in 1:1 MeOH/DCM (3 mL) and ethylenediamine (50 µL, 746 µmol) was added after which the mixture was stirred for 1 h. The mixture was poured into H₂O (20 mL) and when required, brine (1 mL), and the product extracted with EtOAc (2x20 mL). The combined organic layers were concentrated as such. Purification was performed as indicated.

### General procedure C - SEM deprotection

A microwave vial was charged with SEM-protected amine starting material dissolved in DCM (± 0.2 M) after which HCl (4 M in dioxane, 24 eq.) was added and the vial was sealed. The mixture was stirred at 50°C for the indicated time and subsequently concentrated. The mixture was dissolved in CHCl₃ (20 mL) and poured into 1 M NaHCO₃ (aq.) (20 mL). The organic layer was separated and the water layer extracted with CHCl₃ (20 mL). The combined organic layers were concentrated as such and purified as indicated.

### General procedure D - TMS and SEM deprotection

TMS- and SEM-protected starting material was dissolved in TBAF (1 M in THF, 0.5 mL) and the mixture was stirred for 1.5 h. The mixture was poured into H₂O (20 mL) and brine (1 mL), and the intermediate extracted with EtOAc (2x15 mL). The combined organic layers were concentrated as such. The intermediate was dissolved in DCM (0.5 mL) after which TFA (0.5 mL) was added dropwise. The mixture was stirred for the indicated time and subsequently quenched by addition of sat. NaHCO₃ (aq.) (15 mL). The product was extracted with EtOAc (2x15 mL) and the combined organic layers were concentrated as such. The mixture was suspended/dissolved in 1:1 MeOH/DCM (3 mL) and ethylenediamine (50 µL, 746 µmol) was added after which the mixture was stirred for 1 h. The mixture was poured into H₂O (20 mL) and when required, brine (1 mL), and the product extracted with EtOAc (2x15 mL). The combined organic layers were concentrated as such. Purification was performed as indicated.

### General procedure F - Suzuki coupling

A microwave vial was charged with **77** (300 mg, 524 µmol), K₂CO₃ (290 mg, 2.10 mmol), corresponding boronic acid (786 µmol) and Pd(dppf)Cl₂·DCM (30 mg, 37 µmol) after which the vial was sealed. The tube was evacuated and backfilled with argon (3x) via a Schlenk setup after which degassed 4:1 dioxane/H₂O (2.6 mL) was added. The mixture was heated to 90°C, stirred for 16 h and subsequently poured into H₂O (30 mL). The product was extracted with EtOAc (2x30 mL) and the combined organic layers were concentrated as such. Purification was performed as indicated.

### General procedure G - Suzuki coupling

A microwave vial was charged with 2-chloropyrimidine analogue (1 eq.), K₂CO₃ (4 eq.), corresponding boronic acid (pinacol ester) (1.02 - 1.5 eq.) and 4:1 dioxane/H₂O (0.2 M). N₂ was bubbled through the mixture for 1 min after which Pd(dppf)Cl₂·DCM (0.07 eq.) was added. N₂ was bubbled through the mixture for 30 sec after which the vial was sealed. The mixture was heated to 90°C, stirred for indicated time and subsequently poured into H₂O (20 mL) and when required, brine (1 mL). The product was extracted with DCM (2x20 mL) and the combined organic layers were concentrated as such. Purification was performed as indicated.

### General procedure H - Stille coupling

A microwave vial was charged with chlorophenyl analogue (1 eq.), XPhos (0.6 eq.) and Pd₂(dba)₃ (0.15 eq.). THF (0.15 M) was added and N₂ was bubbled through the mixture for 30 sec after which the vial was sealed and trimethyl((tributylstannyl)ethynyl)silane (1.5 eq.) was added via syringe. The vial was put into a heating block and the top of the vial was covered with cotton and aluminum foil. The mixture was heated to 135°C and stirred for 1 h. The mixture was cooled down to RT and filtered over a pre-wetted mixture of K₂CO₃/silica gel (~750 mg/10 mL). Elution was done by EtOAc (10 mL) and subsequently 5% MeOH/EtOAc (4×10 mL). Product containing fractions were concentrated and purified as indicated.

### General procedure I - Nucleophilic aromatic substitution

A microwave vial was charged with **116** (90.0 mg, 166 µmol), K₂CO₃ (46.0 mg, 333 µmol) and dry dioxane (0.2 mL) after which corresponding phenol analogue (1.05 eq.) was added. The vial was sealed, stirred at 120°C for 16 h and subsequently poured into H₂O (20 mL). The product was extracted with DCM (2x20 mL) and the combined organic layers were concentrated as such. Purification was done by automated column chromatography (0 - 25% MeOH/DCM) to afford the product.

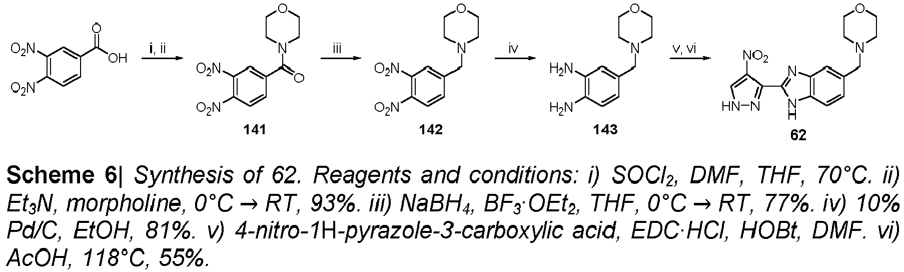

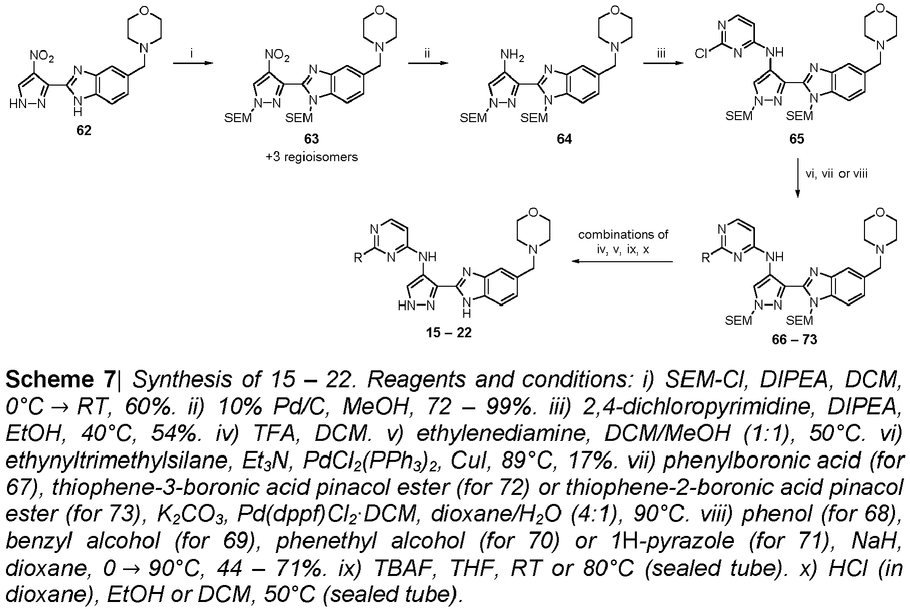

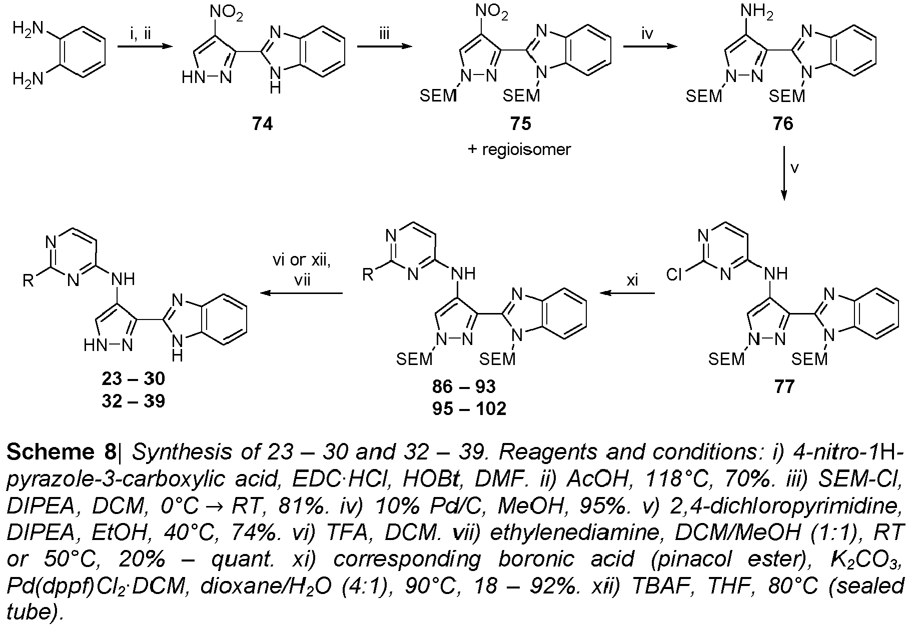

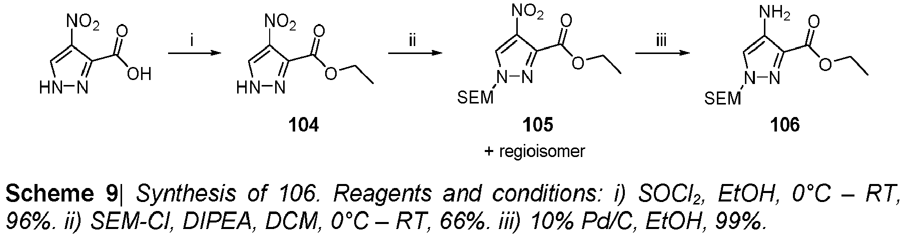

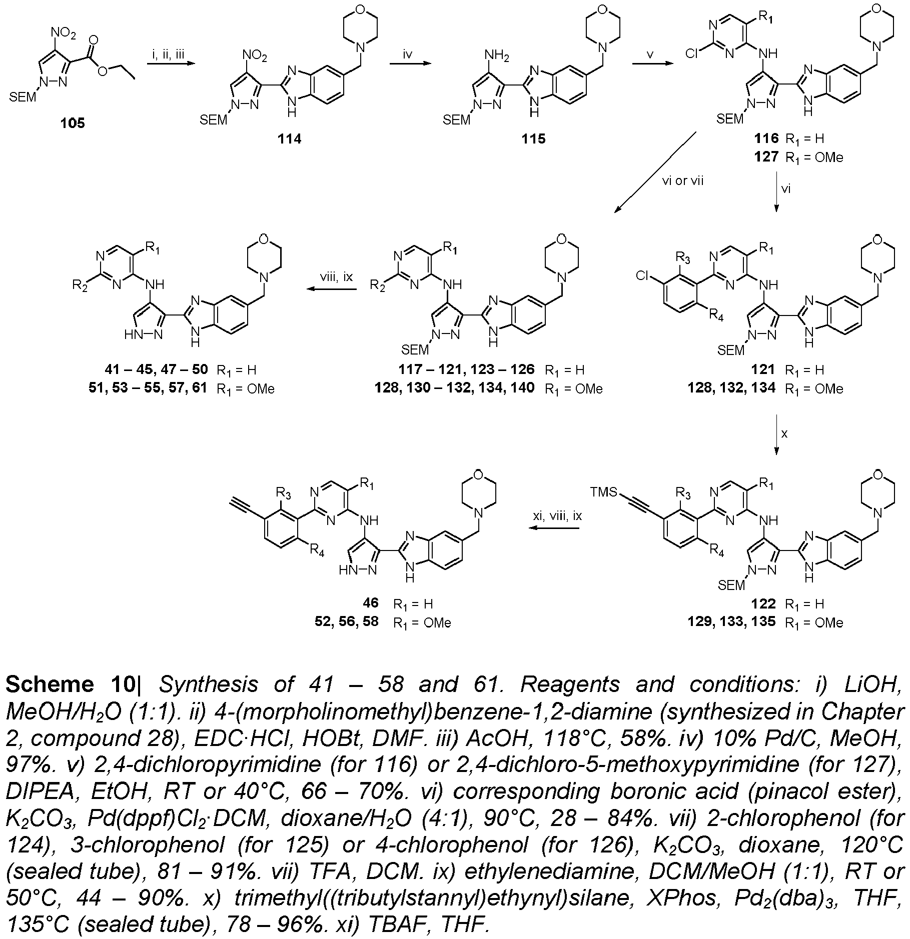

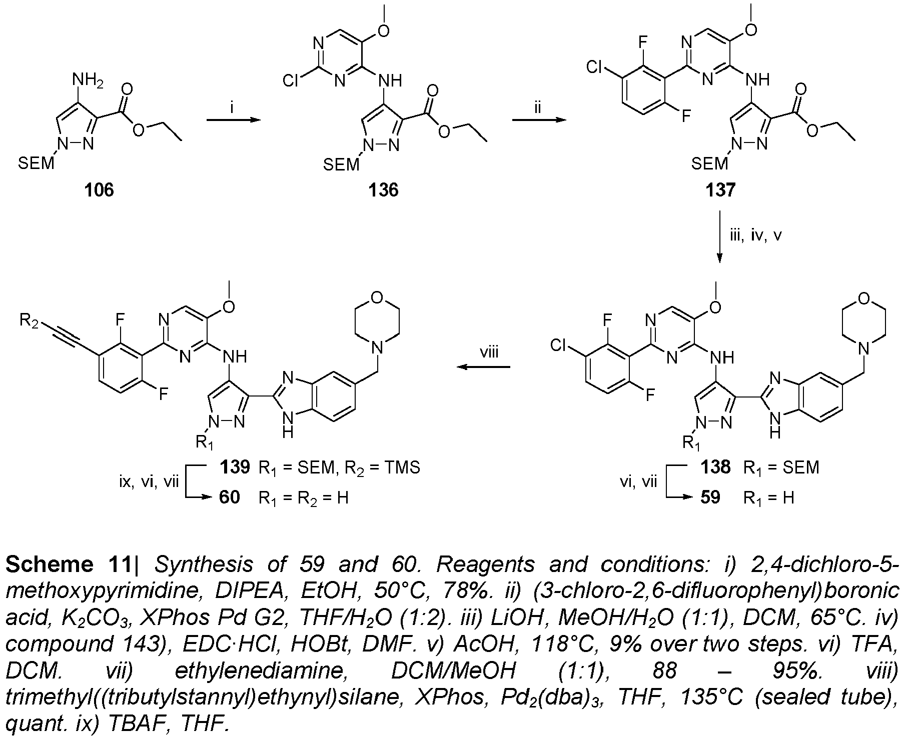

### 2-Ethynyl-N-(3-(5-(morpholinomethyl)-1H-benzo[d]imidazol-2-yl)-1H-pyrazol-4-yl)pyrimidin-4-amine (15)

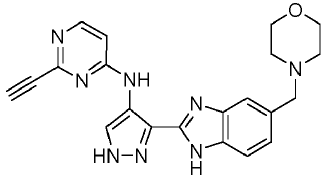

**66** (19.1 mg, 26.1 µmol) was dissolved in TBAF (1 M in THF, 0.5 mL) and stirred for 2.5 h. The mixture was poured into H₂O (10 mL) and the intermediate extracted with 10% MeOH/EtOAc (3x4 mL). The combined organic layers were concentrated as such and subsequently dissolved in DCM (1.2 mL) after which TFA (0.3 mL) was added dropwise. The mixture was stirred for 6 h, subsequently poured into 1 M NaHCO₃ (aq.) (10 mL) and the product extracted with 10% MeOH/EtOAc (3x3 mL). The combined organic layers were concentrated as such and purified by automated column chromatography (2 - 14% MeOH/DCM) to afford the product (5.5 mg, 14 µmol, 53%). ¹H NMR (500 MHz, MeOD) δ 8.48 (br s, 1H), 8.18 (d, *J* = 6.1 Hz, 1H), 7.76 - 7.42 (br m, 2H), 7.25 (d, *J* = 8.2 Hz, 1H), 6.95 (d, *J* = 6.1 Hz, 1H), 3.72 - 3.68 (m, 4H), 3.66 (s, 1H), 3.64 (s, 2H), 2.54 - 2.47 (m, 4H). ¹³C NMR (126 MHz, MeOD) δ 160.22, 155.23, 152.51, 149.02, 132.70 (br), 125.67 (br), 122.62, 121.89, 120.58 (br), 119.05 (br), 113.49 (br), 112.13 (br), 108.11, 83.10, 82.70, 76.34, 67.73, 64.73, 54.63 (not all quaternary carbons were observed). LCMS (Fleet, 0 → 50%): tᵣ = 4.25 min, m/z: 401.3. HRMS [C₂₁H₂₀N₈O + H]⁺: 401.18328 calculated, 401.18305 found.

### N-(3-(5-(Morpholinomethyl)-1H-benzo[d]imidazol-2-yl)-1H-pyrazol-4-yl)-2-phenylpyrimidin-4-amine (16)

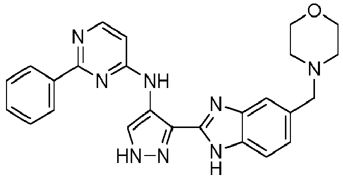

The title compound was synthesized from **67** (29.8 mg, 41.8 µmol) according to general procedure C (reaction time: 4.5 h). The crude was purified by automated column chromatography (3 - 15% MeOH/DCM) to afford the product (8.6 mg, 19 µmol, 46%). ¹H NMR (500 MHz, MeOD) δ 8.64 (s, 1H), 8.30 (d, *J =* 5.9 Hz, 1H), 8.29 - 8.25 (m, 2H), 7.70 (br s, 1H), 7.51 - 7.43 (m, 4H), 7.27 - 7.20 (br m, 1H), 6.81 (d, *J* = 5.9 Hz, 1H), 3.71 (t, *J* = 4.4 Hz, 4H), 3.64 (s, 2H), 2.58 - 2.43 (m, 4H). ¹³C NMR (126 MHz, MeOD) δ 165.28, 159.50, 154.94, 148.06, 143.62, 143.08, 138.77, 133.85, 133.18, 132.33, 131.57, 131.14, 130.86, 128.86, 128.48, 125.47, 124.61, 122.71, 120.80, 120.08, 118.65, 112.57, 111.40, 106.03, 67.10, 64.16, 53.80. LCMS (Finnigan, 0 → 50%): tᵣ = 5.53 min, m/z: 453.1. HRMS [C₂₅H₂₄N₈O + H]⁺: 453.21458 calculated, 453.2146 found.

### N-(3-(5-(Morpholinomethyl)-1H-benzo[d]imidazol-2-yl)-1H-pyrazol-4-yl)-2-phenoxypyrimidin-4-amine(17)

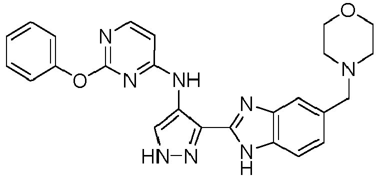

The title compound was synthesized from **68** (28.0 mg, 38.4 µmol) according to general procedure C (reaction time: 6 h). The crude was dissolved in 1:1 MeOH/DCM (1 mL) and transferred to a microwave vial. Ethylenediamine (50 µL, 746 µmol) was added after which the vial was sealed and the mixture was stirred at 50°C for 40 min. The mixture was poured into H₂O (20 mL) and the product extracted with CHCl₃ (2x20 mL). The combined organic layers were concentrated as such and purified by automated column chromatography (2 - 15% MeOH/DCM) to afford the product (7.5 mg, 16 µmol, 42%). ¹H NMR (500 MHz, CDCl₃) δ 11.21 - 10.75 (br m, 2H), 10.11 (s, 1H), 8.11 (d, *J* = 5.5 Hz, 1H), 7.70 (s, 1H), 7.54 (d, *J* = 15.5 Hz, 1H), 7.41 - 7.29 (m, 3H), 7.24 - 7.13 (m, 4H), 6.51 - 6.43 (2x d, *J* = 5.4 Hz, 1H), 3.79 - 3.52 (m, 6H), 2.56 - 2.39 (m, 4H). ¹³C NMR (126 MHz, CDCl₃) δ 165.83, 160.33, 156.95, 153.50, 147.26 (br), 143.40, 142.78, 133.18, 133.05, 132.21, 132.01, 131.21 (br), 129.63, 125.20, 124.37, 122.63, 122.52, 120.72 (br), 119.94, 118.83, 111.69, 110.72, 102.41 (br), 67.09, 66.95, 63.89, 63.85, 53.70. LCMS (Finnigan, 0 → 50%): tᵣ = 5.88 min, m/z: 469.1. HRMS [C₂₅H₂₄N₈O₂ + H]⁺: 469.20950 calculated, 469.2097 found.

### 2-(Benzyloxy)-N-(3-(5-(morpholinomethyl)-1H-benzo[d]imidazol-2-yl)-1H-pyrazol-4-yl)pyrimidin-4-amine(18)

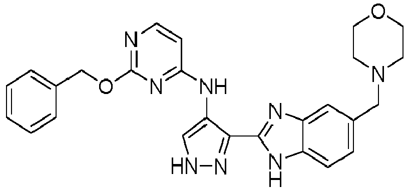

A microwave vial was charged with **69** (55 mg, 74 µmol) and TBAF (1 M in THF, 0.5 mL) was added. The vial was sealed and the mixture was stirred at 80°C for 7 days. The mixture was poured into H₂O (20 mL) and the product extracted with 10% MeOH/CHCl₃ (2x20 mL). The combined organic layers were concentrated as such, dissolved in 1:1 MeOH/DCM (1 mL) and transferred to a microwave vial. Ethylenediamine (50 µL, 746 µmol) was added after which the vial was sealed and the mixture was stirred at 50°C for 1 h. The mixture was poured into H₂O (20 mL) and the product extracted with CHCl₃ (2x20 mL). The combined organic layers were concentrated as such and purified by automated column chromatography (twice, 0 - 10% MeOH/EtOAc) to afford the product (6.9 mg, 14 µmol, 19%). ¹H NMR (600 MHz, MeOD) δ 8.30 (s, 1H), 8.03 (d, *J =* 5.9 Hz, 1H), 7.66 (br s, 1H), 7.50 (br s, *J* = 1.2 Hz, 1H), 7.47 - 7.44 (m, 2H), 7.38 - 7.34 (m, 2H), 7.31 - 7.28 (m, 1H), 7.24 (d, *J* = 8.1 Hz, 1H), 6.58 (d, *J =* 5.9 Hz, 1H), 5.45 (s, 2H), 3.72 - 3.68 (m, 4H), 3.65 (s, 2H), 2.55 - 2.48 (m, 4H). ¹³C NMR (151 MHz, MeOD) δ 166.08, 161.99, 156.83, 148.77, 137.90, 129.32, 128.74, 128.32, 125.85, 125.12, 122.55, 121.55, 120.43, 119.15, 113.16, 111.85, 102.12, 69.69, 67.52, 64.56, 54.34. LCMS (Fleet, 0 → 50%): tᵣ = 5.48 min, m/z: 483.4. HRMS [C₂₆H₂₆N₈O₂ + H]⁺: 483.22515 calculated, 483.22509 found.

### N-(3-(5-(Morpholinomethyl)-1H-benzo[d]imidazol-2-yl)-1H-pyrazol-4-yl)-2-phenethoxypyrimidin-4-amine(19)

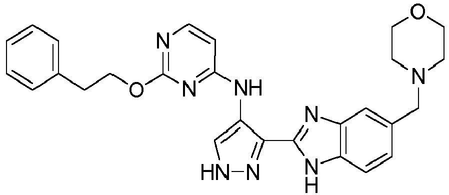

**70** (30 mg, 40 µmol) was dissolved in DCM (1 mL) after which TFA (1 mL) was added dropwise and the mixture was stirred for 2.5 h. The mixture was concentrated under a flow of N₂, dissolved in 5% MeOH/DCM (20 mL), poured into 1 M NaHCO₃ (aq.) (20 mL) and the layers were separated. The water layer was extracted with 5% MeOH/DCM (20 mL) and the combined organic layers were concentrated as such. The mixture was dissolved in 1:1 MeOH/DCM (1 mL) and transferred to a microwave vial. Ethylenediamine (50 µL, 746 µmol) was added after which the vial was sealed and the mixture was stirred at 50°C for 80 min. The mixture was poured into H₂O (20 mL) and the product extracted with 5% MeOH/DCM (2x20 mL). The combined organic layers were concentrated as such and purified by HPLC (Agilent, 19 - 25% MeCN in 0.2% TFA (aq.)). The fractions were concentrated, coevaporated with 1:1 MeCN/H₂O (20 mL), subsequently dissolved in CHCl₃ (20 mL) and poured into 1 M NaHCO₃ (aq.) (20 mL). The organic layer was separated and the water layer extracted with CHCl₃ (20 mL). The combined organic layers were washed with brine (30 mL), dried over Na₂SO₄, filtered and concentrated to afford the product (8.6 mg, 17 µmol, 44%). ¹H NMR (500 MHz, CDCl₃) δ 11.46 - 11.12 (br m, 1H), 10.00 (s, 1H), 8.39 (s, 1H), 7.99 (d, *J* = 5.2 Hz, 1H), 7.70 (d, *J* = 5.8 Hz, 1H), 7.40 - 7.22 (m, 6H), 7.23 - 7.14 (m, 2H), 6.36 - 6.30 (m, 1H), 4.50 (t, *J =* 7.3 Hz, 2H), 3.74 - 3.64 (m, 4H), 3.63 - 3.55 (m, 2H), 3.08 (t, *J* = 7.3 Hz, 2H), 2.52 - 2.41 (m, 4H). ¹³C NMR (126 MHz, CDCl₃) δ 165.26, 160.63, 156.33, 147.25 (br), 143.46, 142.85, 138.20, 133.29, 132.37, 131.91, 131.26 (br), 129.13, 128.63, 126.63, 125.19, 124.31, 122.82, 120.71 (br), 119.91, 118.82, 111.73, 110.75, 101.68, 67.87, 67.05, 66.95, 63.88, 53.71, 35.51. LCMS (Fleet, 0 → 50%): tᵣ = 5.76 min, m/z: 497.4. HRMS [C₂₇H₂₈N₈O₂ + H]⁺: 497.24080 calculated, 497.24093 found.

### N-(3-(5-(Morpholinomethyl)-1H-benzo[d]imidazol-2-yl)-1H-pyrazol-4-yl)-2-(1H-pyrazol-1-yl)pyrimidin-4-amine (20)

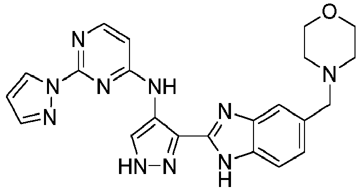

**71** (60 mg, 85 µmol) was dissolved in DCM (1 mL) after which TFA (1 mL) was added dropwise and the mixture was stirred for 3 h. The mixture was concentrated under a flow of N₂, dissolved in CHCl₃ (20 mL) and poured into 1 M NaHCO₃ (aq.) (20 mL). The organic layer was separated and the water layer extracted with CHCl₃ (20 mL). The combined organic layers were concentrated as such, dissolved in 1:1 MeOH/DCM (1 mL) and transferred to a microwave vial. Ethylenediamine (50 µL, 746 µmol) was added after which the vial was sealed and the mixture was stirred at 50°C for 1 h. The mixture was poured into H₂O (20 mL) and the product extracted with CHCl₃ (2x20 mL). The combined organic layers were concentrated as such and purified by automated column chromatography (2 - 15% MeOH/DCM) to afford the product (16.5 mg, 37.3 µmol, 44%). ¹H NMR (500 MHz, MeOD) δ 8.60 (br s, 1H), 8.54 (d, *J* = 2.5 Hz, 1H), 8.22 (d, *J* = 5.9 Hz, 1H), 7.78 - 7.77 (m, 1H), 7.67 (br s, 1H), 7.45 (br s, 1H), 7.22 (d, *J* = 8.0 Hz, 1H), 6.77 (d, *J* = 5.8 Hz, 1H), 6.50 (dd, *J* = 2.6, 1.7 Hz, 1H), 3.72 - 3.68 (m, 4H), 3.63 (s, 2H), 2.55 - 2.46 (m, 4H). ¹³C NMR (126 MHz, MeOD) δ 160.31 (br), 156.08, 155.93 (br), 147.96 (br), 143.61 (br), 143.35, 143.05 (br), 133.92 (br), 133.27 (br), 132.37 (br), 131.74 (br), 131.18 (br), 129.55, 125.53 (br), 124.67 (br), 122.11 (br), 121.41 (br), 120.08 (br), 118.66 (br), 112.65 (br), 111.47 (br), 108.70, 105.74 (br), 67.11, 64.17, 53.82. LCMS (Fleet, 0 → 50%): tr = 4.78 min, m/z: 443.3. HRMS [C₂₂H₂₂N₁₀O + H]⁺: 443.20508 calculated, 443.20488 found.

### N-(3-(5-(Morpholinomethyl)-1H-benzo[d]imidazol-2-yl)-1H-pyrazol-4-yl)-2-(thiophen-3-yl)pyrimidin-4-amine hydrochloride (21)

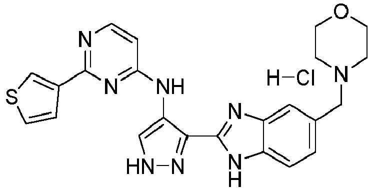

**72** (43.2 mg, 60.0 µmol) was dissolved in EtOH (0.5 mL) and HCl (4 M in dioxane, 0.5 mL) was added. The mixture was stirred at 50°C for 16 h. The reaction was concentrated under a flow of N₂, subsequently 2 M K₂CO₃ (2 mL) was added and the mixture was stirred for 30 min. The mixture was diluted with DCM (10 mL) and H₂O (10 mL), the organic layer was separated and subsequently concentrated as such. The crude was purified by HPLC (Agilent, 13 - 19% MeCN in 0.2% TFA (aq.)) after which the fractions were concentrated, coevaporated with 1:1 MeCN/H₂O (3x20 mL) and subsequently by 1:1 MeCN/H₂O (2×40 mL) to which 0.4 mL HCl (2 M aq.) was added to afford the product as HCl salt (14.8 mg, 32.3 µmol, 54%). ¹H NMR (500 MHz, MeOD) δ 8.46 (s, 1H), 8.37 (s, 1H), 8.25 (d, *J* = 4.5 Hz, 1H), 8.17 (s, 1H), 7.86 (d, *J* = 6.1 Hz, 1H), 7.80 (d, *J* = 6.3 Hz, 1H), 7.60 (d, *J* = 4.1 Hz, 1H), 7.58 - 7.53 (m, 1H), 7.32 (d, *J* = 4.0 Hz, 1H), 4.55 (s, 2H), 4.05 - 3.89 (m, 4H), 3.31 - 3.21 (m, 4H). ¹³C NMR (126 MHz, MeOD) δ 162.80, 155.49, 145.12, 143.67, 133.98, 133.36, 132.64, 130.50, 130.43, 129.39, 127.65, 127.51, 126.81, 119.69, 118.55, 115.54, 106.94, 64.41, 60.91, 52.42. LCMS (Finnigan, 0 → 50%): tᵣ = 5.44 min, m/z: 459.1. HRMS [C₂₃H₂₃ClN₈OS + H]⁺: 459.17100 calculated, 459.1712 found.

### N-(3-(5-(Morpholinomethyl)-1H-benzo[d]imidazol-2-yl)-1H-pyrazol-4-yl)-2-(thiophen-2-yl)pyrimidin-4-amine hydrochloride (22)

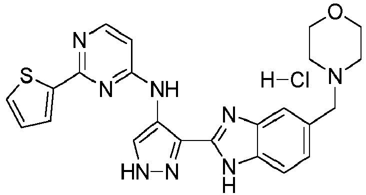

**73** (59.6 mg, 82.8 µmol) was dissolved in EtOH (0.5 mL) and HCl (4 M in dioxane, 0.5 mL) was added. The mixture was stirred at 50°C for 16 h. The reaction was concentrated under a flow of N₂, subsequently 2 M K₂CO₃ (2 mL) was added and the mixture was stirred for 30 min. The mixture was diluted with DCM (10 mL) and H₂O (10 mL), the organic layer was separated and subsequently concentrated as such. The crude was purified by HPLC (Agilent, 13 - 19% MeCN in 0.2% TFA (aq.)) after which the fractions were concentrated, coevaporated with 1:1 MeCN/H₂O (3x20 mL) and subsequently by 1:1 MeCN/H₂O (2×40 mL) to which 0.4 mL HCl (2 M aq.) was added to afford the product as HCl salt (21.7 mg, 47.3 µmol, 57%). ¹H NMR (500 MHz, MeOD) δ 8.37 (s, 1H), 8.24 (d, *J* = 5.1 Hz, 1H), 8.20 - 8.12 (m, 2H), 7.87 (s, 2H), 7.81 - 7.76 (m, 1H), 7.26 (d, *J* = 5.0 Hz, 1H), 7.24 - 7.20 (m, 1H), 4.56 (s, 2H), 4.05 - 3.97 (m, 2H), 3.96 - 3.87 (m, 2H), 3.33 - 3.21 (m, 4H). ¹³C NMR (126 MHz, MeOD) δ 162.45, 155.24, 145.39, 143.75, 136.75, 135.01, 133.72, 133.41, 133.04, 130.70, 130.44, 127.53, 119.83, 118.69, 115.63, 106.79, 64.50, 61.06, 52.53. LCMS (Finnigan, 0 → 50%): tᵣ = 5.54 min, m/z: 459.1. HRMS [C₂₃H₂₃ClN₈OS + H]⁺: 459.17100 calculated, 459.1710 found.

### N-(3-(1H-Benzo[d]imidazol-2-yl)-1H-pyrazol-4-yl)-2-phenylpyrimidin-4-amine (23)

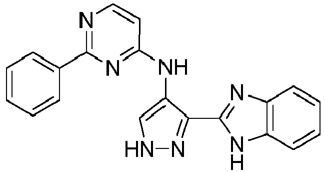

The title compound was synthesized from **86** (114 mg, 186 µmol) according to general procedure A (reaction time: 4.5 h). The crude was loaded onto Celite and purified by silica gel column chromatography (2 - 4% MeOH/DCM) to afford the product (50.6 mg, 143 µmol, 77%). ¹H NMR (400 MHz, DMSO) δ 13.20 (br s, 2H), 10.20 (br s, 1H), 8.63 (br s, 1H), 8.46 (d, *J* = 5.8 Hz, 1H), 8.44 - 8.39 (m, 2H), 7.75 (br s, 1H), 7.65 - 7.45 (m, 4H), 7.29 - 7.20 (m, 2H), 7.01 (d, *J* = 5.8 Hz, 1H). ¹³C NMR (101 MHz, DMSO) δ 163.42, 158.66, 155.68, 147.55, 142.92 (br), 138.11, 133.65 (br), 130.86 (br), 130.51, 128.59, 127.77, 122.53 (br), 121.84, 119.80, 118.57 (br), 111.56 (br), 105.74 (br). LCMS (Fleet, 10 → 90%): tᵣ = 3.08 min, m/z: 354.3. HRMS [C₂₀H₁₅N₇ + H]⁺: 354.14617 calculated, 354.14637 found.

### N-(3-(1H-Benzo[d]imidazol-2-yl)-1H-pyrazol-4-yl)-2-(o-tolyl)pyrimidin-4-amine (24)

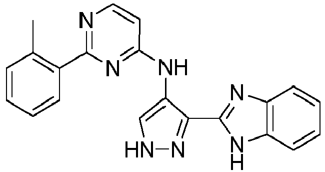

The title compound was synthesized from **87** (177 mg, 281 µmol) according to general procedure A (reaction time: 2 h). The crude was loaded onto Celite and purified by automated column chromatography (40 - 70% EtOAc/DCM) to afford the product (75.2 mg, 205 µmol, 73%). ¹H NMR (500 MHz, DMSO) δ 13.16 (br s, 2H), 10.19 (br s, 1H), 8.47 - 8.43 (m, 2H), 7.80 (dd, *J* = 7.2, 1.4 Hz, 1H), 7.77 - 7.49 (br m, 2H), 7.38 - 7.29 (m, 3H), 7.28 - 7.22 (m, 2H), 7.00 (d, *J* = 5.9 Hz, 1H), 2.52 (s, 3H). ¹³C NMR (126 MHz, DMSO) δ 166.47, 158.41 (br), 155.27 (br), 147.59 (br), 142.94 (br), 139.19, 136.38, 133.74 (br), 130.98, 129.92, 128.86, 125.71, 122.16 (br), 121.95, 119.79 (br), 118.50 (br), 111.63 (br), 104.79 (br), 20.80 (not all quaternary carbons were observed). LCMS (Fleet, 10 → 90%): tᵣ = 3.21 min, m/z: 368.3. HRMS [C₂₁H₁₇N₇ + H]⁺: 368.16182 calculated, 368.16163 found.

### N-(3-(1H-Benzo[d]imidazol-2-yl)-1H-pyrazol-4-yl)-2-(2-chlorophenyl)pyrimidin-4-amine (25)

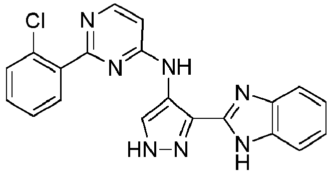

The title compound was synthesized from **88** (120 mg, 185 µmol) according to general procedure A (reaction time: 4.5 h). The crude was loaded onto Celite and purified by silica gel column chromatography (2 - 4% MeOH/DCM) to afford the product (48.1 mg, 124 µmol, 67%). ¹H NMR (400 MHz, DMSO) δ 13.14 (br s, 2H), 10.22 (br s, 1H), 8.50 (s, 1H), 8.47 (d, *J* = 5.9 Hz, 1H), 7.80 - 7.75 (m, 1H), 7.65 (br s, 2H), 7.62 - 7.58 (m, 1H), 7.52 - 7.44 (m, 2H), 7.27 - 7.21 (m, 2H), 7.08 (d, *J* = 6.0 Hz, 1H). ¹³C NMR (101 MHz, DMSO) δ 164.33, 158.21 (br), 155.43 (br), 147.49 (br), 138.55, 131.68, 131.37, 130.72 (br), 130.46, 130.26, 127.17, 122.26 (br), 121.72, 120.23 (br), 105.83 (not all quaternary carbons were observed, neither were two -CH's of the benzimidazole). LCMS (Fleet, 10 → 90%): tᵣ = 3.01 min, m/z: 388.3. HRMS [C₂₀H₁₄ClN₇ + H]⁺: 388.10720 calculated, 388.10741 found.

### N-(3-(1H-Benzo[d]imidazol-2-yl)-1H-pyrazol-4-yl)-2-(2-methoxyphenyl)pyrimidin-4-amine (26)

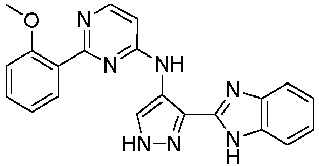

The title compound was synthesized from **89** (110 mg, 171 µmol) according to general procedure A (reaction time: 3 h). The crude was purified by silica gel column chromatography (2 - 3% MeOH/DCM) to afford the product (60.0 mg, 156 µmol, 91%). ¹H NMR (400 MHz, DMSO) δ 13.43 - 12.94 (br m, 2H), 10.07 (s, 1H), 8.67 (s, 1H), 8.44 (d, *J* = 5.8 Hz, 1H), 7.76 (d, *J* = 7.4 Hz, 2H), 7.53 (br s, 1H), 7.46 (ddd, *J* = 8.3, 7.3, 1.8 Hz, 1H), 7.27 - 7.22 (m, 2H), 7.20 (dd, *J* = 8.4, 1.0 Hz, 1H), 7.07 (td, *J* = 7.5, 1.0 Hz, 1H), 7.00 (d, *J* = 5.9 Hz, 1H), 3.87 (s, 3H). ¹³C NMR (101 MHz, DMSO) δ 164.51, 157.98 (br), 157.48, 155.44 (br), 147.69, 142.96 (br), 133.68 (br), 131.33, 130.70, 130.51 (br), 128.73, 122.65 (br), 122.13, 121.78 (br), 120.23, 120.14, 118.53 (br), 112.34, 111.50 (br), 104.95 (br), 55.57. LCMS (Fleet, 10 → 90%): tᵣ = 3.22 min, m/z: 384.3. HRMS [C₂₁H₁₇N₇O + H]⁺: 384.15673 calculated, 384.15685 found.

### N-(3-(1H-Benzo[d]imidazol-2-yl)-1H-pyrazol-4-yl)-2-(m-tolyl)pyrimidin-4-amine (27)

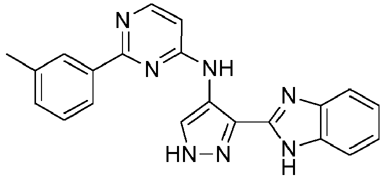

The title compound was synthesized from **90** (120 mg, 191 µmol) according to general procedure A (reaction time: 4 h). The crude was loaded onto Celite and purified by silica gel chromatography (3 - 5% MeOH/DCM) to afford the product (59.5 mg, 162 µmol, 85%). ¹H NMR (400 MHz, DMSO) δ 13.59 - 12.82 (br m, 2H), 10.18 (br s, 1H), 8.63 (br s, 1H), 8.45 (d, *J* = 5.9 Hz, 1H), 8.26 - 8.18 (m, 2H), 7.77 (br s, 1H), 7.57 (br s, 1H), 7.43 (t, *J* = 7.6 Hz, 1H), 7.33 (d, *J* = 7.5 Hz, 1H), 7.30 - 7.21 (m, 2H), 6.99 (d, *J* = 5.8 Hz, 1H), 2.43 (s, 3H). ¹³C NMR (101 MHz, DMSO) δ 163.55, 158.65 (br), 155.65 (br), 147.58 (br), 142.94 (br), 138.11, 137.64, 133.69 (br), 131.15, 130.86 (br), 128.48, 128.41, 124.99, 122.55 (br), 121.90, 119.78 (br), 118.59 (br), 111.58 (br), 105.63 (br), 21.24. LCMS (Fleet, 10 → 90%): tr = 3.33 min, m/z: 368.3. HRMS [C₂₁H₁₇N₇ + H]⁺: 368.16182 calculated, 368.16206 found.

### N-(3-(1H-Benzo[d]imidazol-2-yl)-1H-pyrazol-4-yl)-2-(3-chlorophenyl)pyrimidin-4-amine (28)

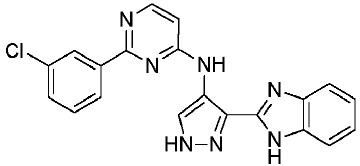

The title compound was synthesized from **91** (111 mg, 171 µmol) according to general procedure A (reaction time: 4 h), using 10% MeOH/EtOAc as organic layers in the work-up. The crude was purified by HPLC (Waters, 15 - 25% MeCN in 0.2% TFA (aq.)). The fractions were concentrated, coevaporated with 1:1 MeCN/H₂O (20 mL), subsequently dissolved in EtOAc (20 mL) and poured into 1 M NaHCO₃ (aq.) (20 mL). The organic layer was separated and the water layer extracted with EtOAc (20 mL). The combined organic layers were washed with brine (30 mL), dried over Na₂SO₄, filtered and concentrated to afford the product (53.0 mg, 137 µmol, 80%). ¹H NMR (500 MHz, DMSO) δ 13.53 - 12.90 (br m, 2H), 10.21 (br s, 1H), 8.56 (br s, 1H), 8.46 (d, *J* = 5.8 Hz, 1H), 8.37 - 8.32 (m, 2H), 7.76 (br s, 1H), 7.62 - 7.57 (m, 2H), 7.53 (br s, 1H), 7.27 - 7.21 (m, 2H), 7.04 (d, *J* = 5.8 Hz, 1H). ¹³C NMR (126 MHz, DMSO) δ 161.99, 158.72 (br), 155.62 (br), 147.45, 142.93 (br), 140.18, 133.63 (br), 133.44, 131.03 (br), 130.56, 130.25, 127.32, 126.29, 122.68 (br), 121.79 (br), 121.57, 119.87, 118.53 (br), 111.51 (br), 106.32 (br). LCMS (Fleet, 10 → 90%): tᵣ = 3.66 min, m/z: 388.3. HRMS [C₂₀H₁₄ClN₇ + H]⁺: 388.10720 calculated, 388.10706 found.

### N-(3-(1H-Benzo[d]imidazol-2-yl)-1H-pyrazol-4-yl)-2-(3-methoxyphenyl)pyrimidin-4-amine (29)

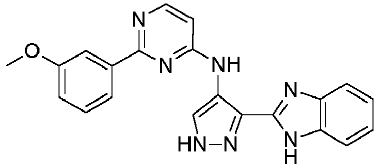

The title compound was synthesized from **92** (184 mg, 286 µmol) according to general procedure A (reaction time: 2 h). The crude was loaded onto Celite and purified by automated column chromatography (30 - 60% EtOAc/DCM) to afford the product (76.8 mg, 200 µmol, 70%). ¹H NMR (500 MHz, DMSO) δ 13.23 (br s, 2H), 10.21 (br s, 1H), 8.64 (br s, 1H), 8.46 (d, *J* = 5.8 Hz, 1H), 8.03 (d, *J* = 7.7 Hz, 1H), 7.99 - 7.97 (m, 1H), 7.75 (br s, 1H), 7.61 (br s, 1H), 7.46 (t, *J* = 7.9 Hz, 1H), 7.29 - 7.23 (m, 2H), 7.09 (dd, *J* = 8.1, 2.5 Hz, 1H), 7.00 (d, *J =* 5.8 Hz, 1H), 3.88 (s, 3H). ¹³C NMR (126 MHz, DMSO) δ 163.21, 159.47, 158.62 (br), 155.59 (br), 147.56 (br), 142.92 (br), 139.63, 133.79 (br), 130.91 (br), 129.61, 122.21 (br), 121.89, 120.15, 119.78 (br), 118.56 (br), 116.31, 112.76, 111.49 (br), 105.80 (br), 55.09. LCMS (Fleet, 10 → 90%): tᵣ = 3.44 min, m/z: 384.3. HRMS [C₂₁H₁₇N₇O + H]⁺: 384.15673 calculated, 384.15663 found.

### N-(3-(1H-Benzo[d]imidazol-2-yl)-1H-pyrazol-4-yl)-2-(3-isopropylphenyl)pyrimidin-4-amine (30)

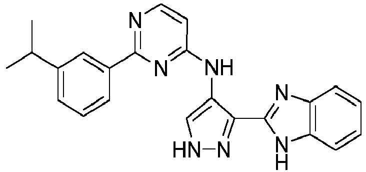

The title compound was synthesized from **93** (72.4 mg, 110 µmol) according to general procedure A (reaction time: 2 h). The crude was loaded onto Celite and purified by automated column chromatography (20 - 55% EtOAc/DCM) to afford the product (35.5 mg, 89.8 µmol, 81%). ¹H NMR (500 MHz, DMSO) δ 13.24 (br s, 2H), 10.19 (br s, 1H), 8.63 (br s, 1H), 8.46 (d, *J* = 5.8 Hz, 1H), 8.30 (t, *J* = 1.8 Hz, 1H), 8.22 (dt, *J* = 7.6, 1.5 Hz, 1H), 7.66 (br s, 2H), 7.45 (t, *J* = 7.6 Hz, 1H), 7.39 (dt, *J* = 7.7, 1.5 Hz, 1H), 7.28 - 7.22 (m, 2H), 7.01 (d, *J* = 5.9 Hz, 1H), 3.01 (hept, *J* = 6.9 Hz, 1H), 1.29 (d, *J* = 6.9 Hz, 6H). ¹³C NMR (126 MHz, DMSO) δ 163.61, 158.60, 155.66 (br), 148.56, 147.55, 142.89 (br), 138.15, 133.73 (br), 130.83 (br), 128.75, 128.56, 125.53, 125.39, 122.22 (br), 121.91, 119.68 (br), 118.50 (br), 111.57 (br), 105.68 (br), 33.51, 23.98. LCMS (Fleet, 10 → 90%): tᵣ = 4.11 min, m/z: 396.3. HRMS [C₂₃H₂₁N₇ + H]⁺: 396.19312 calculated, 396.19308 found.

### N-(3-(1H-Benzo[d]imidazol-2-yl)-1H-pyrazol-4-yl)-2-(p-tolyl)pyrimidin-4-amine (32)

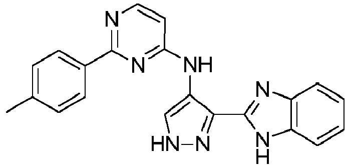

The title compound was synthesized from **95** (100 mg, 190 µmol) according to general procedure A (reaction time: 3 h). The crude was loaded onto Celite and purified by silica gel column chromatography (2% MeOH/DCM) to afford the product (40.0 mg, 106 µmol, 56%). ¹H NMR (400 MHz, DMSO) δ 13.19 (br s, 2H), 10.15 (br s, 1H), 8.60 (s, 1H), 8.43 (d, *J* = 5.8 Hz, 1H), 8.32 - 8.28 (m, 2H), 7.65 (br s, 2H), 7.37 - 7.33 (m, 2H), 7.28 - 7.22 (m, 2H), 6.98 (d, *J* = 5.8 Hz, 1H), 2.39 (s, 3H). ¹³C NMR (101 MHz, DMSO) δ 163.47, 158.61, 155.65 (br), 147.52 (br), 142.68 (br), 140.23, 135.43, 134.07 (br), 130.77 (br), 129.20, 127.75, 122.21 (br), 121.89, 119.71 (br), 118.27 (br), 111.60 (br), 105.45 (br), 21.05. LCMS (Fleet, 10 → 90%): tᵣ = 3.35 min, m/z: 368.3. HRMS [C₂₁H₁₇N₇ + H]⁺: 368.16182 calculated, 368.16204 found.

### N-(3-(1H-Benzo[d]imidazol-2-yl)-1H-pyrazol-4-yl)-2-(4-chlorophenyl)pyrimidin-4-amine (33)

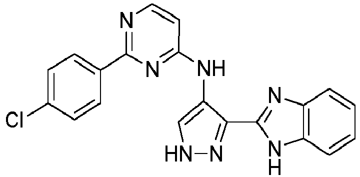

The title compound was synthesized from **96** (141 mg, 218 µmol) according to general procedure A (reaction time: 5 h). The crude was purified by HPLC (Waters, 15 - 25% MeCN in 0.2% TFA (aq.)). The fractions were concentrated, coevaporated with 1:1 MeCN/H₂O (20 mL), subsequently dissolved in EtOAc (20 mL) and poured into 1 M NaHCO₃ (aq.) (20 mL). The organic layer was separated and the water layer extracted with EtOAc (20 mL). The combined organic layers were washed with brine (30 mL), dried over Na₂SO₄, filtered and concentrated to afford the product (24 mg, 62 µmol, 28%). ¹H NMR (500 MHz, DMSO) δ 12.74 (br s, 2H), 10.25 (br s, 1H), 8.56 (br s, 1H), 8.44 (d, *J* = 5.8 Hz, 1H), 8.42 - 8.39 (m, 2H), 7.66 - 7.62 (m, 2H), 7.61 - 7.57 (m, 2H), 7.25 - 7.21 (m, 2H), 7.01 (d, *J* = 5.8 Hz, 1H). ¹³C NMR (126 MHz, DMSO) δ 162.40, 158.68, 155.60 (br), 147.52, 138.38 (br), 136.91, 135.32, 130.65 (br), 129.50, 128.63, 122.10, 121.64, 120.42 (br), 114.94 (br), 105.96 (br). LCMS (Fleet, 10 → 90%): tᵣ = 3.72 min, m/z: 388.3. HRMS [C₂₀H₁₄ClN₇ + H]⁺: 388.10720 calculated, 388.10714 found.

### N-(3-(1H-Benzo[d]imidazol-2-yl)-1H-pyrazol-4-yl)-2-(4-methoxyphenyl)pyrimidin-4-amine (34)

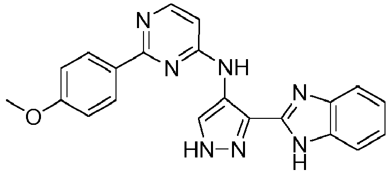

The title compound was synthesized from **97** (110 mg, 171 µmol) according to general procedure A (reaction time: 3 h), using 10% MeOH/EtOAc as organic layers in the work-up. The crude was purified by silica gel column chromatography (2% MeOH/DCM) to afford the product (66 mg, 171 µmol, quant.). ¹H NMR (400 MHz, DMSO) δ 13.35 (br s, 1H), 13.06 (br s, 1H), 10.16 (br s, 1H), 8.61 (br s, 1H), 8.41 (d, *J* = 5.8 Hz, 1H), 8.39 - 8.35 (m, 2H), 7.83 - 7.73 (br m, 1H), 7.58 - 7.50 (br m, 1H), 7.28 - 7.21 (m, 2H), 7.12 - 7.06 (m, 2H), 6.93 (d, *J* = 5.9 Hz, 1H), 3.84 (s, 3H). ¹³C NMR (101 MHz, DMSO) δ 163.27, 161.30, 158.55, 155.64 (br), 147.60, 142.94, 133.66, 130.82 (br), 130.60, 129.41, 122.72, 121.97, 121.79, 119.65, 118.56, 113.90, 111.53, 104.92 (br), 55.29. LCMS (Fleet, 10 → 90%): tᵣ = 3.26 min, m/z: 384.3. HRMS [C₂₁H₁₇N₇O + H]⁺: 384.15673 calculated, 384.15699 found.

### N-(3-(1H-Benzo[d]imidazol-2-yl)-1H-pyrazol-4-yl)-2-(naphthalen-1-yl)pyrimidin-4-amine (36)

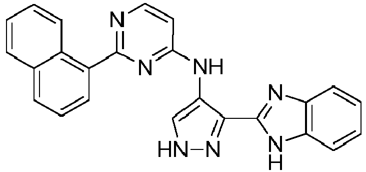

The title compound was synthesized from **99** (134 mg, 201 µmol) according to general procedure A (reaction time: 2 h). The crude was purified by automated column chromatography (20 - 55% EtOAc/DCM) to afford the product (49.3 mg, 122 µmol, 61%). ¹H NMR (500 MHz, DMSO) δ 13.15 (br s, 2H), 10.27 (br s, 1H), 8.77 - 8.72 (m, 1H), 8.56 (d, *J* = 5.9 Hz, 1H), 8.42 (s, 1H), 8.09 - 8.05 (m, 2H), 8.04 - 7.99 (m, 1H), 7.74 (br s, 2H), 7.66 (dd, *J* = 8.2, 7.1 Hz, 1H), 7.59 - 7.55 (m, 2H), 7.29 - 7.24 (m, 2H), 7.11 (d, *J* = 5.9 Hz, 1H). ¹³C NMR (126 MHz, DMSO) δ 165.78, 158.37 (br), 155.55 (br), 147.57 (br), 142.97 (br), 136.79, 133.80 (br), 133.64, 130.85 (br), 130.49, 129.78, 128.48, 128.41, 126.46, 126.10, 125.90, 125.34, 122.47 (br), 121.87, 119.89 (br), 118.55 (br), 111.55 (br), 105.33 (br). LCMS (Fleet, 10 → 90%): tᵣ = 3.52 min, m/z: 404.3. HRMS [C₂₄H₁₇N₇ + H]⁺: 404.16182 calculated, 404.16171 found.

### N-(3-(1H-Benzo[d]imidazol-2-yl)-1H-pyrazol-4-yl)-2-(1H-indol-7-yl)pyrimidin-4-amine (37)

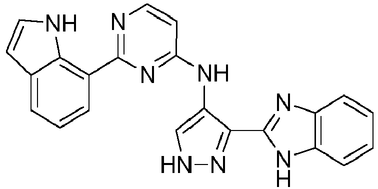

A microwave vial was charged with **100** (92.6 mg, 142 µmol) after which TBAF (1 M in THF, 2.5 mL) and ethylenediamine (28.6 µL, 425 µmol) were added. The mixture heated to 80°C, stirred for 2 days and subsequently poured into H₂O (20 mL). The product was extracted with EtOAc (2x20 mL) and the combined organic layers were concentrated as such. The mixture was dissolved in 1:1 MeOH/DCM (2 mL) and transferred to a microwave vial. Ethylenediamine (50 µL, 746 µmol) was added after which the vial was sealed and the mixture was stirred at 50°C for 1 h. The mixture was poured into H₂O (20 mL) and the product extracted with EtOAc (2x20 mL). The combined organic layers were concentrated as such, the crude was loaded onto Celite and purified by automated column chromatography (25 - 100% EtOAc/DCM) to afford the product (16.5 mg, 42.0 µmol, 30%). ¹H NMR (400 MHz, DMSO) δ 13.40 (br s, 1H), 13.05 (br s, 1H), 11.57 (br s, 1H), 10.14 (br s, 1H), 8.65 (br s, 1H), 8.53 (d, *J* = 5.9 Hz, 1H), 8.30 (dd, *J* = 7.5, 1.1 Hz, 1H), 7.78 (d, *J* = 7.6 Hz, 1H), 7.75 (br s, 1H), 7.53 (br s, 1H), 7.47 (t, *J* = 2.8 Hz, 1H), 7.29 - 7.20 (m, 3H), 6.98 (br s, 1H), 6.57 (dd, *J* = 3.1, 2.1 Hz, 1H). ¹³C NMR (101 MHz, DMSO) δ 164.17, 158.70 (br), 155.22 (br), 147.43 (br), 143.04 (br), 134.39, 133.72 (br), 131.46 (br), 129.23, 126.06, 123.35, 122.74 (br), 121.78 (br), 121.57 (br), 121.43, 120.68, 120.58 (br), 118.88, 118.62 (br), 111.55 (br), 105.11 (br), 101.47. LCMS (Fleet, 10 → 90%): tᵣ = 3.77 min, m/z: 393.3. HRMS [C₂₂H₁₆N₈ + H]⁺: 393.15707 calculated, 393.15677 found.

### N-(3-(1H-Benzo[d]imidazol-2-yl)-1H-pyrazol-4-yl)-2-(1H-indazol-4-yl)pyrimidin-4-amine (38)

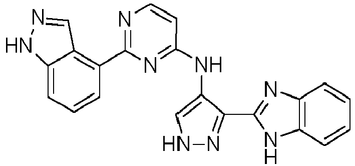

The title compound was synthesized from **101** (101 mg, 155 µmol) according to general procedure A (reaction time: 2.5 h). The crude was loaded onto Celite and purified by automated column chromatography (60 - 100% EtOAc/DCM) to afford the product (36.5 mg, 92.8 µmol, 60%). ¹H NMR (500 MHz, DMSO) δ 13.66 - 12.82 (br m, 3H), 10.22 (br s, 1H), 8.87 (br s, 1H), 8.59 (br s, 1H), 8.57 (d, *J* = 5.8 Hz, 1H), 8.23 (d, *J* = 7.2 Hz, 1H), 7.77 (br s, 1H), 7.75 (d, *J* = 8.3 Hz, 1H), 7.58 (br s, 1H), 7.55 (dd, *J* = 8.3, 7.2 Hz, 1H), 7.29 - 7.22 (m, 2H), 7.04 (d, *J* = 5.9 Hz, 1H). ¹³C NMR (126 MHz, DMSO) δ 164.02, 158.77, 155.86 (br), 147.54, 143.00 (br), 140.85, 135.20, 133.71 (br), 131.23, 125.79, 122.65 (br), 121.80, 121.16, 121.11, 120.10, 118.56 (br), 112.58, 111.58 (br), 105.48 (br). LCMS (Fleet, 10 → 90%): tᵣ = 2.83 min, m/z: 394.3. HRMS [C₂₁H₁₅N₉ + H]⁺: 394.15232 calculated, 394.15214 found.

### N-(3-(1H-Benzo[d]imidazol-2-yl)-1H-pyrazol-4-yl)-2-(naphthalen-2-yl)pyrimidin-4-amine (39)

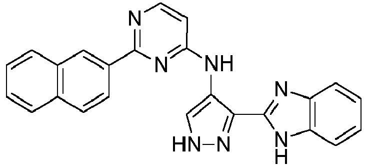

The title compound was synthesized from **102** (68.5 mg, 103 µmol) according to general procedure A (reaction time: 2 h). The crude was loaded onto Celite and purified by automated column chromatography (25 - 100% EtOAc/DCM) to afford the product (30.8 mg, 76.3 µmol, 74%). ¹H NMR (500 MHz, DMSO) δ 13.23 (br s, 2H), 10.23 (br s, 1H), 9.00 (s, 1H), 8.72 (br s, 1H), 8.53 (dd, *J* = 8.7, 1.8 Hz, 1H), 8.51 (d, *J* = 5.8 Hz, 1H), 8.17 - 8.12 (m, 1H), 8.07 (d, *J* = 8.6 Hz, 1H), 8.02 - 7.96 (m, 1H), 7.67 (br s, 2H), 7.62 - 7.55 (m, 2H), 7.28 - 7.23 (m, 2H), 7.05 (d, *J* = 5.8 Hz, 1H). ¹³C NMR (126 MHz, DMSO) δ 163.44, 158.76 (br), 155.74 (br), 147.56 (br), 142.82 (br), 135.56, 134.10, 133.87 (br), 132.84, 130.92 (br), 129.08, 128.06, 127.81, 127.65, 127.22, 126.49, 124.98, 122.27 (br), 121.83, 119.98 (br), 118.48 (br), 111.59 (br), 105.76 (br). LCMS (Finnigan, 10 → 90%): tᵣ = 4.53 min, m/z: 404.4. HRMS [C₂₄H₁₇N₇ + H]⁺: 404.16182 calculated, 404.16163 found.

### 2-(3-Chlorophenyl)-N-(3-(5-(morpholinomethyl)-1H-benzo[d]imidazol-2-yl)-1H-pyrazol-4-yl)pyrimidin-4-amine (41)

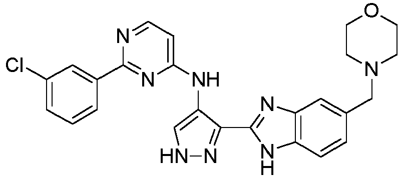

The title compound was synthesized from **117** (87.3 mg, 141 µmol) according to general procedure A (reaction time: 1.5 h). The crude was purified by automated column chromatography (0 - 10% MeOH/EtOAc) to afford the product (53.6 mg, 110 µmol, 78%). ¹H NMR (500 MHz, DMSO) δ 13.73 - 12.63 (br m, 2H), 10.22 (br s, 1H), 8.55 (s, 1H), 8.44 (d, *J* = 5.8 Hz, 1H), 8.35 - 8.33 (m, 2H), 7.67 - 7.44 (m, 4H), 7.17 (dd, *J* = 8.3, 0.9 Hz, 1H), 7.02 (d, *J* = 5.9 Hz, 1H), 3.58 - 3.55 (m, 4H), 3.54 (s, 2H), 2.40 - 2.32 (m, 4H). ¹³C NMR (126 MHz, DMSO) δ 162.02, 158.70, 155.56 (br), 147.54 (br), 142.27 (br), 140.20, 133.70 (br), 133.45, 132.07 (br), 130.92 (br), 130.48, 130.21, 127.35, 126.28, 123.61 (br), 121.62, 119.80 (br), 118.04 (br), 111.54 (br), 106.31 (br), 66.27, 62.98, 53.22. LCMS (Fleet, 10 → 90%): tᵣ = 3.19 min, m/z: 487.3. HRMS [C₂₅H₂₃ClN₈O + H]⁺: 487.17561 calculated, 487.17545 found.

### 2-(3-Ethynylphenyl)-N-(3-(5-(morpholinomethyl)-1H-benzo[d]imidazol-2-yl)-1H-pyrazol-4-yl)pyrimidin-4-amine (42)

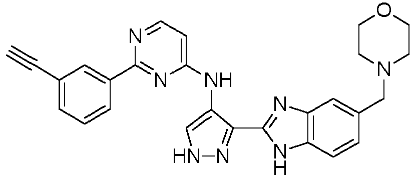

The title compound was synthesized from **118** (44.0 mg, 64.8 µmol) according to general procedure D (reaction time: 2 h). The crude was purified by automated column chromatography (0 - 10% MeOH/EtOAc) to afford the product (13.7 mg, 28.7 µmol, 44%). ¹H NMR (500 MHz, DMSO) δ 13.40 (br s, 1H), 13.02 (br s, 1H), 10.20 (br s, 1H), 8.55 (br s, 1H), 8.46 (d, *J* = 5.8 Hz, 1H), 8.44 - 8.41 (m, 2H), 7.67 (br s, 1H), 7.65 (dt, *J* = 7.6, 1.5 Hz, 1H), 7.58 (t, *J* = 7.7 Hz, 1H), 7.45 (br s, 1H), 7.18 (d, *J* = 8.2 Hz, 1H), 7.04 (d, *J* = 5.9 Hz, 1H), 4.30 (s, 1H), 3.61 - 3.54 (m, 6H), 2.44 - 2.32 (m, 4H). ¹³C NMR (126 MHz, DMSO) δ 162.40, 158.74 (br), 155.65 (br), 147.48 (br), 142.91 (br), 142.19 (br), 138.49, 133.73 (br), 133.57, 132.54 (br), 131.32 (br), 130.88 (br), 130.77, 129.18, 128.26, 124.11 (br), 123.19 (br), 122.04, 121.64 (br), 119.98 (br), 118.96 (br), 118.01 (br), 111.67 (br), 111.04 (br), 106.37 (br), 83.35, 81.09, 66.27, 62.97, 53.24 (not all quaternary carbons were observed). LCMS (Fleet, 10 → 90%): tᵣ = 3.13 min, m/z: 477.3. HRMS [C₂₇H₂₄N₈O + H]⁺: 477.21458 calculated, 477.21467 found.

### N-(3-(5-(Morpholinomethyl)-1H-benzo[d]imidazol-2-yl)-1H-pyrazol-4-yl)-2-(3-(trifluoromethyl)phenyl)pyrimidin-4-amine (43)

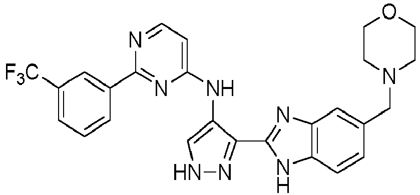

The title compound was synthesized from **119** (112 mg, 173 µmol) according to general procedure A (reaction time: 2.5 h). The crude was purified by automated column chromatography (0 - 10% MeOH/EtOAc) to afford the product (62.6 mg, 120 µmol, 70%). ¹H NMR (400 MHz, DMSO) δ 13.59 - 12.90 (br m, 2H), 10.26 (br s, 1H), 8.68 - 8.63 (m, 2H), 8.54 (br s, 1H), 8.45 (d, *J* = 5.8 Hz, 1H), 7.86 (d, *J* = 7.7 Hz, 1H), 7.76 (t, *J* = 8.0 Hz, 1H), 7.63 (br s, 1H), 7.50 (br s, 1H), 7.16 (d, *J* = 8.2 Hz, 1H), 7.03 (d, *J* = 5.9 Hz, 1H), 3.58 - 3.54 (m, 4H), 3.53 (s, 2H), 2.40 - 2.30 (m, 4H). ¹³C NMR (101 MHz, DMSO) δ 161.86, 158.75, 155.59 (br), 147.56 (br), 142.95 (br), 142.25 (br), 139.01, 133.76 (br), 132.49 (br), 131.48, 130.98 (br), 129.85, 129.58, 129.27, 128.95, 128.35, 126.96 (br), 126.92 (br), 126.89 (br), 126.85 (br), 125.65, 124.20 (br), 124.04 (br), 124.00 (br), 123.97 (br), 123.93 (br), 123.39 (br), 122.94, 121.62, 119.83 (br), 118.91 (br), 118.05 (br), 111.65 (br), 111.08 (br), 106.48 (br), 66.28, 63.00, 53.25. LCMS (Fleet, 10 → 90%): tᵣ = 3.60 min, m/z: 521.25. HRMS [C₂₆H₂₃F₃N₈O + H]⁺: 521.20197 calculated, 521.20212 found.

### 3-(4-((3-(5-(Morpholinomethyl)-1H-benzo[d]imidazol-2-yl)-1H-pyrazol-4-yl)amino)pyrimidin-2-yl)benzonitrile (44)

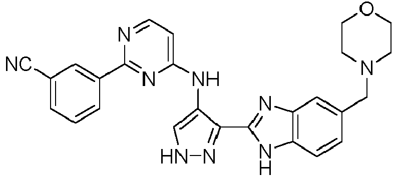

**120** (100 mg, 165 mmol) was dissolved in DCM (1 mL) after which TFA (0.33 mL) was added dropwise. The mixture was stirred for 7.5 h and subsequently sat. NaHCO₃ (aq.) (10 mL) was added. The mixture was poured into H₂O (10 mL) and the product extracted with EtOAc (2x20 mL). The combined organic layers were concentrated as such and suspended in 1:1 MeOH/DCM (5 mL). Ethylenediamine (50 µL, 746 µmol) was added after which the mixture was stirred for 30 min. The mixture was poured into H₂O (20 mL), the product extracted with DCM (20 mL) and subsequently with 5% MeOH/DCM (20 mL). The combined organic layers were concentrated as such. The crude was purified by automated column chromatography (1 - 20% MeOH/DCM) to afford the product (34.5 mg, 72.2 µmol, 44%). ¹H NMR (400 MHz, DMSO) δ 13.39 (s, 1H), 13.03 - 12.96 (2x s, 1H), 10.35 - 10.16 (2x s, 1H), 8.69 - 8.62 (m, 2H), 8.56 (br s, 1H), 8.47 - 8.42 (2x d, *J* = 5.9 Hz, 1H), 7.96 (dt, *J* = 7.7, 1.5 Hz, 1H), 7.73 (t, *J* = 8.1 Hz, 1H), 7.69 - 7.64 (m, 1H), 7.47 - 7.42 (m, 1H), 7.21 - 7.13 (2x dd, *J* = 8.3, 1.5 Hz, 1H), 7.07 - 6.99 (2x d, *J* = 5.9 Hz, 1H), 3.61 - 3.51 (m, 6H), 2.43 - 2.31 (m, 4H). ¹³C NMR (101 MHz, DMSO) δ 161.50, 158.72, 155.52 (br), 147.68 (br), 147.55 (br), 142.95, 142.23, 139.14, 133.87, 133.71, 132.82, 132.49 (br), 132.24, 131.24 (br), 131.06, 129.96, 124.14, 123.18, 121.52, 121.42, 120.02 (br), 118.93, 118.78, 118.03, 111.82, 111.68, 111.05, 106.62 (br), 66.26, 63.01, 62.93, 53.25, 53.20. LCMS (Fleet, 10 → 90%): tᵣ = 3.04 min, m/z: 478.2. HRMS [C₂₆H₂₃N₉O + H]⁺: 478.20983 calculated, 478.21008 found.

### 2-(3-Chloro-2-fluorophenyl)-N-(3-(5-(morpholinomethyl)-1H-benzo[d]imidazol-2-yl)-1H-pyrazol-4-yl)pyrimidin-4-amine (45)

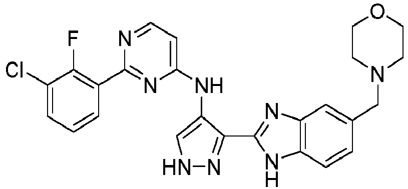

The title compound was synthesized from **121** (86.8 mg, 137 µmol) according to general procedure B (reaction time: 3 h). The crude was purified by automated column chromatography (0 - 10% MeOH/EtOAc) to afford the product (56.1 mg, 111 µmol, 81%). ¹H NMR (500 MHz, DMSO) δ 13.31 (br s, 1H), 12.99 (br s, 1H), 10.40 - 10.16 (br m, 1H), 8.55 (s, 1H), 8.46 (d, *J* = 5.8 Hz, 1H), 8.08 - 8.03 (m, 1H), 7.71 (ddd, *J* = 8.3, 6.7, 1.7 Hz, 1H), 7.68 (s, 1H), 7.45 (br s, 1H), 7.35 (td, *J* = 7.9, 1.0 Hz, 1H), 7.21 - 7.13 (br m, *J* = 8.4 Hz, 1H), 7.10 - 7.03 (br m, 1H), 3.61 - 3.51 (m, 6H), 2.42 - 2.31 (m, 4H). ¹³C NMR (126 MHz, DMSO) δ 161.16, 161.12, 158.28, 156.74, 155.42 (br), 154.71, 147.62 (br), 142.95 (br), 142.22 (br), 133.72 (br), 132.81 (br), 132.53 (br), 131.90, 131.32 (br), 130.75 (br), 130.53, 128.47, 128.39, 125.15, 125.11, 124.10 (br), 123.17 (br), 121.70 (br), 121.15, 121.01, 119.82 (br), 118.89 (br), 117.99 (br), 111.66 (br), 111.03 (br), 106.25 (br), 66.27, 62.98, 53.23. LCMS (Fleet, 10 → 90%): tr = 3.07 min, m/z: 505.3. HRMS [C₂₅H₂₂ClFN₈O + H]⁺: 505.16619 calculated, 505.16584 found.

### 2-(3-Ethynyl-2-fluorophenyl)-N-(3-(5-(morpholinomethyl)-1H-benzo[d]imidazol-2-yl)-1H-pyrazol-4-yl)pyrimidin-4-amine (46)

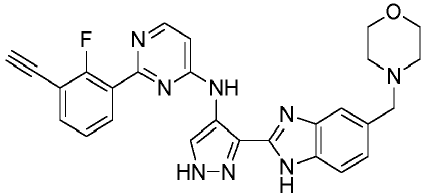

The title compound was synthesized from **122** (74.7 mg, 107 µmol) according to general procedure D (reaction time with TFA: 4 h). The crude was purified by automated column chromatography (0 - 10% MeOH/EtOAc) to afford the product (33.7 mg, 68.1 µmol, 64%). ¹H NMR (400 MHz, DMSO) δ 13.31 (br s, 1H), 13.02 (br s, 1H), 10.25 (br s, 1H), 8.56 (s, 1H), 8.47 (d, *J* = 5.9 Hz, 1H), 8.13 (td, *J* = 7.6, 1.8 Hz, 1H), 7.71 (ddd, *J* = 8.0, 6.4, 1.8 Hz, 1H), 7.66 (br s, 1H), 7.46 (br s, 1H), 7.36 (t, *J* = 7.7 Hz, 1H), 7.18 (d, *J* = 8.3 Hz, 1H), 7.08 (d, *J* = 5.9 Hz, 1H), 4.59 (s, 1H), 3.59 - 3.53 (m, 6H), 2.42 - 2.32 (m, 4H). ¹³C NMR (101 MHz, DMSO) δ 162.15, 161.31, 161.27, 159.58, 158.27, 155.48 (br), 147.58 (br), 142.89 (br), 142.20 (br), 135.27, 133.73 (br), 132.83 (br), 132.56, 132.54, 131.35 (br), 130.51 (br), 127.24, 127.15, 124.56, 124.51, 124.11 (br), 123.29 (br), 121.74, 120.02 (br), 118.90 (br), 118.07 (br), 111.69 (br), 111.55, 111.38, 111.04 (br), 106.16 (br), 86.59, 86.55, 77.03, 77.02, 66.28, 62.99, 53.25. LCMS (Fleet, 10 → 90%): tr = 3.01 min, m/z: 495.2. HRMS [C₂₇H₂₃FN₈O + H]⁺: 495.20516 calculated, 495.20496 found.

### 2-(5-Chloro-2-fluorophenyl)-N-(3-(5-(morpholinomethyl)-1H-benzo[d]imidazol-2-yl)-1H-pyrazol-4-yl)pyrimidin-4-amine (47)

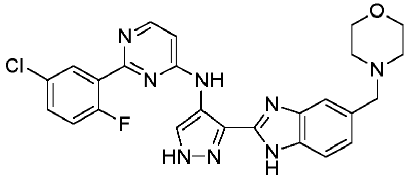

The title compound was synthesized from **123** (65.5 mg, 103 µmol) according to general procedure B (reaction time: 4 h). The crude was purified by automated column chromatography (0 - 10% MeOH/EtOAc) to afford the product (41.3 mg, 81.7 µmol, 79%). ¹H NMR (400 MHz, DMSO) δ 13.09 (br s, 2H), 10.28 (br s, 1H), 8.55 (s, 1H), 8.46 (d, *J* = 5.9 Hz, 1H), 8.09 (dd, *J* = 6.6, 2.8 Hz, 1H), 7.61 (ddd, *J* = 8.8, 4.0, 2.8 Hz, 1H), 7.55 (br s, 2H), 7.44 (dd, *J* = 10.8, 8.8 Hz, 1H), 7.17 (dd, *J* = 8.3, 1.5 Hz, 1H), 7.07 (d, *J* = 5.9 Hz, 1H), 3.59 - 3.52 (m, 6H), 2.41 - 2.32 (m, 4H). ¹³C NMR (101 MHz, DMSO) δ 160.65, 160.60, 160.49, 158.28, 157.97, 155.42 (br), 147.59, 131.94 (br), 131.53, 131.44, 130.92, 130.90, 130.66 (br), 128.33, 128.30, 128.19, 123.62 (br), 121.69, 119.99 (br), 119.16, 118.91, 106.35 (br), 66.28, 62.99, 53.25 (not all quaternary carbons were observed, neither were two -CH's of the benzimidazole). LCMS (Fleet, 10 → 90%): tr = 3.04 min, m/z: 505.2. HRMS [C₂₅H₂₂ClFN₈O + H]⁺: 505.16619 calculated, 505.16603 found.

### 2-(2-Chlorophenoxy)-N-(3-(5-(morpholinomethyl)-1H-benzo[d]imidazol-2-yl)-1H-pyrazol-4-yl)pyrimidin-4-amine (48)

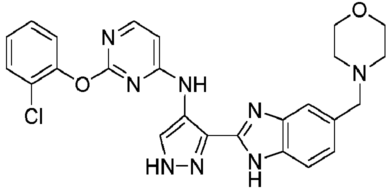

The title compound was synthesized from **124** (95.5 mg, 151 µmol) according to general procedure B (reaction time: 4 h). The crude was purified by automated column chromatography (0 - 50% MeOH/DCM) to afford the product (38.1 mg, 75.8 µmol, 50%). ¹H NMR (400 MHz, DMSO) δ 13.15 (br s, 1H), 13.03 - 12.94 (2x s, 1H), 10.35 - 10.14 (2x s, 1H), 8.19 - 8.15 (2x d, *J* = 5.8 Hz, 1H), 7.69 - 7.62 (m, 2H), 7.53 - 7.28 (m, 5H), 7.20 - 7.11 (2x dd, *J* = 8.3, 1.5 Hz, 1H), 6.88 - 6.79 (2x d, *J* = 5.8 Hz, 1H), 3.59 - 3.51 (m, 6H), 2.40 - 2.30 (m, 4H). ¹³C NMR (101 MHz, DMSO) δ 164.42, 159.96 (br), 157.22 (br), 149.01, 147.58 (br), 147.45 (br), 142.85, 142.12, 133.65, 132.75, 132.57, 131.34, 130.58 (br), 130.29, 128.66, 127.04, 126.77, 124.76, 124.16, 123.21, 121.34, 121.25, 119.46 (br), 118.90, 118.00, 111.66, 111.03, 102.80 (br), 66.27, 62.99, 62.92, 53.25, 53.21. LCMS (Fleet, 10 → 90%): tr = 3.43 min, m/z: 503.2. HRMS [C₂₅H₂₃ClN₈O₂ + H]⁺: 503.17053 calculated, 503.17069 found.

### 2-(3-Chlorophenoxy)-N-(3-(5-(morpholinomethyl)-1H-benzo[d]imidazol-2-yl)-1H-pyrazol-4-yl)pyrimidin-4-amine (49)

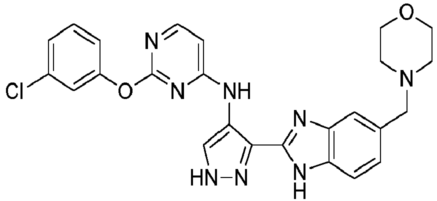

The title compound was synthesized from **125** (85.8 mg, 135 µmol) according to general procedure B (reaction time: 3 h). The crude was purified by automated column chromatography (2 - 40% MeOH/DCM) to afford the product (46 mg, 91 µmol, 67%). ¹H NMR (400 MHz, DMSO) δ 13.22 (br s, 1H), 13.00 (br s, 1H), 10.40 - 10.18 (2x s, 1H), 8.17 (d, *J* = 5.8 Hz, 1H), 7.67 - 7.62 (m, 1H), 7.57 (br s, 1H), 7.52 (t, *J* = 8.1 Hz, 1H), 7.44 (t, *J* = 2.2 Hz, 1H), 7.42 (br s, 1H), 7.39 (dd, *J* = 7.9, 1.8 Hz, 1H), 7.26 (dd, *J* = 7.9, 2.1 Hz, 1H), 7.20 - 7.12 (m, 1H), 6.87 - 6.78 (2x d, *J* = 5.8 Hz, 1H), 3.58 - 3.51 (m, 6H), 2.39 - 2.30 (m, 4H). ¹³C NMR (101 MHz, DMSO) δ 164.86, 160.01 (br), 157.09 (br), 153.86, 147.58 (br), 147.48 (br), 142.87, 142.14, 133.68, 133.58, 132.77, 132.58, 131.35, 131.02, 130.65 (br), 125.34, 124.16, 123.22, 122.72, 121.35 (br), 121.25, 119.65 (br), 118.90, 118.00, 111.68, 111.04, 102.89 (br), 66.28, 62.97, 53.24. LCMS (Fleet, 10 → 90%): tr = 3.50 min, m/z: 503.2. HRMS [C₂₅H₂₃ClN₈O₂ + H]⁺: 503.17053 calculated, 503.17040 found.

### 2-(4-Chlorophenoxy)-N-(3-(5-(morpholinomethyl)-1H-benzo[d]imidazol-2-yl)-1H-pyrazol-4-yl)pyrimidin-4-amine (50)

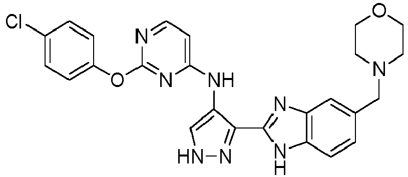

The title compound was synthesized from **126** (95.5 mg, 151 µmol) according to general procedure B (reaction time: 3 h). The crude was purified by automated column chromatography (2 - 14% MeOH/DCM) to afford the product (59.6 mg, 118 µmol, 79%). ¹H NMR (400 MHz, DMSO) δ 13.24 (br s, 1H), 13.01 (br s, 1H), 10.39 - 10.18 (2x s, 1H), 8.15 (d, *J* = 5.8 Hz, 1H), 7.71 (br s, 1H), 7.67 - 7.63 (m, 1H), 7.55 - 7.50 (m, 2H), 7.46 - 7.40 (m, 1H), 7.33 - 7.27 (m, 2H), 7.16 (t, *J* = 8.8 Hz, 1H), 6.85 - 6.77 (2x d, *J* = 5.8 Hz, 1H), 3.59 - 3.51 (m, 6H), 2.39 - 2.31 (m, 4H). ¹³C NMR (101 MHz, DMSO) δ 164.88, 160.12 (br), 157.02 (br), 151.89, 147.61 (br), 147.50 (br), 142.88, 142.15, 133.68, 132.78, 132.59, 131.36, 130.68 (br), 129.52, 129.27, 124.17, 124.07, 123.23, 121.40, 121.31, 119.69 (br), 118.90, 118.01, 111.69, 111.05, 102.84 (br), 66.28, 63.01, 62.96, 53.25, 53.23. LCMS (Fleet, 10 → 90%): tr = 3.44 min, m/z: 503.2. HRMS [C₂₅H₂₃ClN₈O₂ + H]⁺: 503.17053 calculated, 503.17050 found.

### 2-(3-Chlorophenyl)-5-methoxy-N-(3-(5-(morpholinomethyl)-1H-benzo[d]imidazol-2-yl)-1H-pyrazol-4-yl)pyrimidin-4-amine (51)

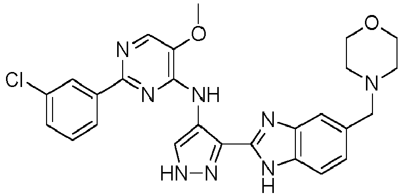

The title compound was synthesized from **128** (50 mg, 77 µmol) according to general procedure B (reaction time: 5.5 h). The crude was purified by automated column chromatography (1 - 20% MeOH/EtOAc) to afford the product (34.4 mg, 66.5 µmol, 86%). ¹H NMR (500 MHz, DMSO) δ 13.67 - 12.76 (br m, 2H), 10.72 (br s, 1H), 8.61 (s, 1H), 8.31 - 8.25 (m, 2H), 8.19 (s, 1H), 7.64 (br s, 1H), 7.57 - 7.50 (m, 2H), 7.45 (br s, 1H), 7.21 - 7.17 (br m, 1H), 4.14 (s, 3H), 3.61 - 3.53 (m, 6H), 2.42 - 2.33 (m, 4H). ¹³C NMR (126 MHz, DMSO) δ 154.03, 149.70, 147.62 (br), 142.87 (br), 142.11 (br), 140.27, 139.76, 133.69 (br), 133.51, 133.38, 132.78 (br), 132.57 (br), 131.39 (br), 130.84 (br), 130.48, 129.22, 126.77, 125.76, 124.14 (br), 123.20 (br), 121.70, 119.00 (br), 118.90 (br), 117.99 (br), 111.71 (br), 111.11 (br), 66.28, 62.96, 56.62, 53.25. LCMS (Fleet, 10 → 90%): tr = 3.79 min, m/z: 517.3. HRMS [C₂₆H₂₅ClN₈O₂ + H]⁺: 517.18618 calculated, 517.18628 found.

### 2-(3-Ethynylphenyl)-5-methoxy-N-(3-(5-(morpholinomethyl)-1H-benzo[d]imidazol-2-yl)-1H-pyrazol-4-yl)pyrimidin-4-amine (52)

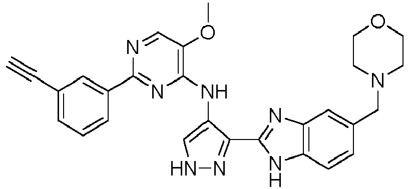

The title compound was synthesized from **129** (52.9 mg, 74.6 µmol) according to general procedure D (reaction time with TFA: 5 h). The crude was purified by automated column chromatography (1 - 15% MeOH/EtOAc) to afford the product (30.1 mg, 59.4 µmol, 80%). ¹H NMR (400 MHz, DMSO) δ 13.39 (br s, 1H), 13.03 (br s, 1H), 10.81 - 10.61 (2x s, 1H), 8.65 - 8.62 (2x s, 1H), 8.39 - 8.36 (m, 2H), 8.23 - 8.20 (2x s, 1H), 7.68 - 7.62 (m, 1H), 7.59 (dt, *J* = 7.6, 1.6 Hz, 1H), 7.57 - 7.53 (m, 1H), 7.49 - 7.43 (m, 1H), 7.25 - 7.15 (2x d, *J* = 8.2 Hz, 1H), 4.29 (s, 1H), 4.17 - 4.12 (2x s, 3H), 3.61 - 3.53 (m, 6H), 2.42 - 2.33 (m, 4H). ¹³C NMR (101 MHz, DMSO) δ 154.46, 149.72, 147.70 (br), 147.61 (br), 142.87, 142.12, 139.69, 138.56, 133.68 (br), 133.61, 132.78 (br), 132.60, 131.39 (br), 130.84 (br), 130.22, 129.10, 127.76, 124.17 (br), 123.20 (br), 121.96, 121.75, 118.91 (br), 118.00 (br), 111.69 (br), 111.09 (br), 83.54, 80.93, 66.28, 62.96, 56.68, 56.60, 53.25. LCMS (Fleet, 10 → 90%): tr = 3.68 min, m/z: 507.3. HRMS [C₂₈H₂₆N₈O₂ + H]⁺: 507.22515 calculated, 507.22522 found.

### 5-Methoxy-N-(3-(5-(morpholinomethyl)-1H-benzo[d]imidazol-2-yl)-1H-pyrazol-4-yl)-2-(3-(trifluoromethyl)phenyl)pyrimidin-4-amine (53)

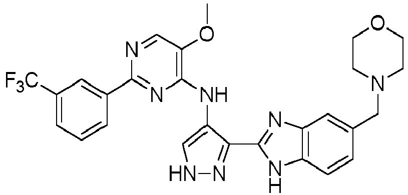

The title compound was synthesized from **130** (74.0 mg, 109 µmol) according to general procedure B (reaction time: 7 h). The crude was purified by automated column chromatography (1 - 20% MeOH/EtOAc) to afford the product (52.4 mg, 95.2 µmol, 88%). ¹H NMR (400 MHz, DMSO) δ 13.66 - 12.74 (br m, 2H), 10.74 (s, 1H), 8.63 - 8.56 (m, 3H), 8.19 (s, 1H), 7.82 - 7.78 (m, 1H), 7.73 (t, *J* = 7.7 Hz, 1H), 7.67 - 7.60 (br m, 1H), 7.48 - 7.42 (br m, 1H), 7.23 - 7.14 (br m, 1H), 4.14 (s, 3H), 3.62 - 3.52 (m, 6H), 2.43 - 2.30 (m, 4H). ¹³C NMR (101 MHz, DMSO) δ 153.88, 149.75, 147.63 (br), 142.90 (br), 142.15 (br), 139.87, 139.07, 133.71 (br), 133.44, 132.81 (br), 132.58 (br), 131.38 (br), 130.93, 129.83 (br), 129.79, 129.51, 129.20, 128.88, 128.43, 125.93 (br), 125.90 (br), 125.86 (br), 125.83 (br), 125.73, 124.16 (br), 123.42 (br), 123.39 (br), 123.34 (br), 123.30 (br), 123.21 (br), 123.02, 121.71, 118.94 (br), 118.00 (br), 111.70 (br), 111.10 (br), 66.29, 62.98, 56.61, 53.26. LCMS (Fleet, 10 → 90%): tr = 4.22 min, m/z: 551.3. HRMS [C₂₇H₂₅F₃N₈O₂ + H]⁺: 551.21253 calculated, 551.21229 found.

### 3-(5-Methoxy-4-((3-(5-(morpholinomethyl)-1H-benzo[d]imidazol-2-yl)-1H-pyrazol-4-yl)amino)pyrimidin-2-yl)benzonitrile (54)

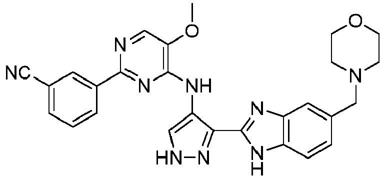

The title compound was synthesized from 131 (62.4 mg, 97.8 µmol) according to general procedure B (reaction time: 7 h). The crude was purified by automated column chromatography (1 - 20% MeOH/EtOAc) to afford the product (44.6 mg, 87.9 µmol, 90%). ¹H NMR (400 MHz, DMSO) δ 13.50 - 12.66 (br m, 2H), 10.71 (s, 1H), 8.60 (s, 1H), 8.60 - 8.55 (m, 2H), 8.14 (s, 1H), 7.87 (dt, *J* = 7.7, 1.4 Hz, 1H), 7.67 (t, *J* = 7.8 Hz, 1H), 7.62 (br s, 1H), 7.45 (br s, 1H), 7.22 - 7.14 (m, 1H), 4.13 (s, 3H), 3.61 - 3.53 (m, 6H), 2.43 - 2.32 (m, 4H). ¹³C NMR (101 MHz, DMSO) δ 153.43, 149.71, 147.61 (br), 142.85 (br), 142.17 (br), 139.89, 139.18, 133.69 (br), 133.31, 132.82, 132.53 (br), 131.67, 131.44 (br), 130.80 (br), 130.42, 129.83, 124.14 (br), 123.21 (br), 121.60, 119.25 (br), 118.94, 118.01 (br), 111.71, 111.08 (br), 66.30, 62.99, 56.60, 53.27. LCMS (Fleet, 10 → 90%): tr = 3.66 min, m/z: 508.2. HRMS [C₂₇H₂₅N₉O₂ + H]⁺: 508.22040 calculated, 508.22051 found.

### 2-(3-Chloro-2-fluorophenyl)-5-methoxy-N-(3-(5-(morpholinomethyl)-1H-benzo[d]imidazol-2-yl)-1H-pyrazol-4-yl)pyrimidin-4-amine (55)

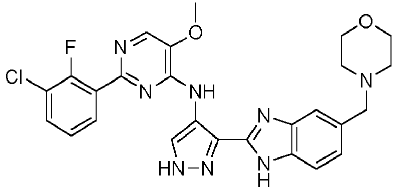

The title compound was synthesized from **132** (60.3 mg, 90.6 µmol) according to general procedure B (reaction time: 4 h). The crude was purified by automated column chromatography (2 - 20% MeOH/EtOAc) to afford the product (42.1 mg, 78.7 µmol, 87%). ¹H NMR (400 MHz, DMSO) δ 13.72 - 12.72 (br m, 2H), 10.74 - 10.68 (2x s, 1H), 8.60 (s, 1H), 8.23 (s, 1H), 8.05 (td, *J* = 7.8, 1.6 Hz, 1H), 7.70 - 7.60 (m, 2H), 7.48 - 7.42 (m, 1H), 7.33 (td, *J* = 7.9, 1.0 Hz, 1H), 7.24 - 7.14 (m, 1H), 4.18 - 4.12 (2x s, 3H), 3.60 - 3.53 (m, 6H), 2.41 - 2.33 (m, 4H). ¹³C NMR (101 MHz, DMSO) δ 156.79, 154.26, 152.83, 152.78, 149.38, 147.68, 147.59, 142.89, 142.15, 139.41, 133.71, 133.58, 132.81, 132.56, 131.37, 131.18, 130.57, 130.29, 130.28, 128.37, 128.28, 125.09, 125.04, 124.16, 123.20, 121.77, 121.17, 120.98, 119.37 (br), 118.93, 117.99, 111.71, 111.09, 66.29, 62.97, 56.64, 56.57, 53.26. LCMS (Fleet, 10 → 90%): tr = 3.70 min, m/z: 535.3. HRMS [C₂₆H₂₄ClFN₈O₂ + H]⁺: 535.17675 calculated, 535.17673 found.

### 2-(3-Ethynyl-2-fluorophenyl)-5-methoxy-N-(3-(5-(morpholinomethyl)-1H-benzo[d]imidazol-2-yl)-1H-pyrazol-4-yl)pyrimidin-4-amine (56)

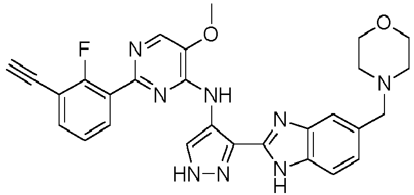

The title compound was synthesized from **133** (85.7 mg, 118 µmol) according to general procedure D (reaction time with TFA: 3 h). The crude was purified by automated column chromatography (1 - 20% MeOH/EtOAc) to afford the product (50.2 mg, 95.7 µmol, 81%). ¹H NMR (400 MHz, DMSO) δ 13.32 (s, 1H), 13.02 (s, 1H), 10.73 - 10.66 (2x s, 1H), 8.64 - 8.61 (2x s, 1H), 8.27 - 8.22 (2x s, 1H), 8.13 (td, *J* = 7.7, 1.8 Hz, 1H), 7.68 - 7.63 (m, 2H), 7.47 - 7.43 (m, 1H), 7.34 (t, *J* = 7.8 Hz, 1H), 7.23 - 7.16 (2x dd, *J* = 8.2, 1.5 Hz, 1H), 4.58 (s, 1H), 4.17 - 4.13 (2x s, 3H), 3.60 - 3.55 (m, 6H), 2.42 - 2.33 (m, 4H). ¹³C NMR (101 MHz, DMSO) δ 161.95, 159.38, 152.98, 152.94, 149.34, 147.72, 147.62, 142.87, 142.13, 139.34, 134.53, 133.69, 133.65, 132.78, 132.58, 132.31, 132.28, 131.38, 130.74, 127.09, 127.00, 124.45, 124.41, 124.16, 123.19, 121.77, 119.13, 118.92, 117.99, 111.69, 111.51, 111.34, 111.08, 86.41, 86.38, 77.19, 77.17, 66.38, 66.28, 62.96, 56.65, 56.57, 53.26, 53.24. LCMS (Fleet, 10 → 90%): tr = 3.61 min, m/z: 525.3. HRMS [C₂₈H₂₅FN₈O₂ + H]⁺: 525.21573 calculated, 525.21579 found.

### 2-(5-Chloro-2-fluorophenyl)-5-methoxy-N-(3-(5-(morpholinomethyl)-1H-benzo[d]imidazol-2-yl)-1H-pyrazol-4-yl)pyrimidin-4-amine (57)

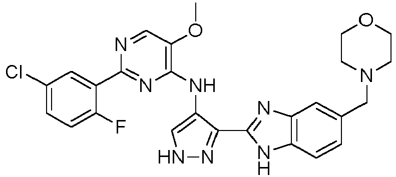

The title compound was synthesized from **134** (56.6 mg, 85.1 µmol) according to general procedure B (reaction time: 7 h). The crude was purified by automated column chromatography (1 - 20% MeOH/EtOAc) to afford the product (40.9 mg, 76.5 µmol, 90%). ¹H NMR (400 MHz, DMSO) δ 13.61 - 12.81 (br m, 2H), 10.70 (s, 1H), 8.61 - 8.60 (2x s, 1H), 8.22 (s, 1H), 8.07 (dd, *J* = 6.7, 2.8 Hz, 1H), 7.68 - 7.59 (br m, 1H), 7.55 (ddd, *J* = 8.8, 4.0, 2.8 Hz, 1H), 7.45 (br s, 1H), 7.41 (dd, *J* = 10.9, 8.8 Hz, 1H), 7.24 - 7.14 (br m, 1H), 4.15 (s, 3H), 3.61 - 3.53 (m, 6H), 2.42 - 2.32 (m, 4H). ¹³C NMR (101 MHz, DMSO) δ 160.27, 157.76, 152.33, 152.28, 149.35, 147.64 (br), 142.89 (br), 142.14 (br), 139.43, 133.70 (br), 133.54, 132.80 (br), 132.58 (br), 131.40, 130.72, 130.63, 130.60, 130.58, 128.24, 128.21, 128.20, 128.09, 124.17 (br), 123.22 (br), 121.76, 119.12, 118.87, 118.00 (br), 111.71 (br), 111.09 (br), 66.29, 62.98, 56.61, 53.26. LCMS (Fleet, 10 → 90%): tr = 3.68 min, m/z: 535.2. HRMS [C₂₆H₂₄ClFN₈O₂ + H]⁺: 535.17675 calculated, 535.17674 found.

### 2-(5-Ethynyl-2-fluorophenyl)-5-methoxy-N-(3-(5-(morpholinomethyl)-1H-benzo[d]imidazol-2-yl)-1H-pyrazol-4-yl)pyrimidin-4-amine (58)

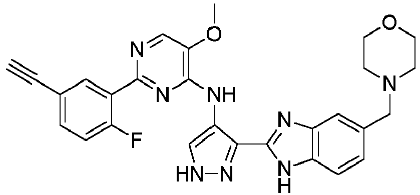

The title compound was synthesized from **135** (86.1 mg, 118 µmol) according to general procedure D (reaction time with TFA: 5 h). The crude was purified by automated column chromatography (1 - 20% MeOH/EtOAc) to afford the product (46.9 mg, 89.4 µmol, 75%). ¹H NMR (500 MHz, DMSO) δ 13.31 (br s, 1H), 13.02 (br s, 1H), 10.74 - 10.65 (2x s, 1H), 8.63 - 8.60 (2x s, 1H), 8.25 - 8.22 (2x s, 1H), 8.18 (dd, *J* = 7.5, 2.3 Hz, 1H), 7.68 - 7.60 (m, 2H), 7.48 - 7.43 (m, 1H), 7.40 (dd, *J* = 11.1, 8.5 Hz, 1H), 7.21 (2x dd, *J* = 8.2, 1.5 Hz, 1H), 4.24 (s, 1H), 4.18 - 4.13 (2x s, 3H), 3.62 - 3.53 (m, 6H), 2.43 - 2.32 (m, 4H). ¹³C NMR (126 MHz, DMSO) δ 161.29, 159.26, 152.66, 152.63, 149.37, 147.70, 147.60, 142.88, 142.13, 139.38, 134.73, 134.34, 134.27, 133.69, 133.63, 132.79, 132.58, 131.38, 130.76, 127.08, 126.99, 124.16, 123.20, 121.80, 119.10, 118.92, 118.07, 118.04, 117.99, 117.84, 117.65, 111.69, 111.08, 82.37, 80.75, 66.28, 62.96, 56.65, 56.57, 53.26. LCMS (Fleet, 10 → 90%): tr = 3.59 min, m/z: 525.2. HRMS [C₂₈H₂₅FN₈O₂ + H]⁺: 525.21573 calculated, 525.21586 found.

### 2-(3-Chloro-2,6-difluorophenyl)-5-methoxy-N-(3-(5-(morpholinomethyl)-1H-benzo[d]imidazol-2-yl)-1H-pyrazol-4-yl)pyrimidin-4-amine (59)

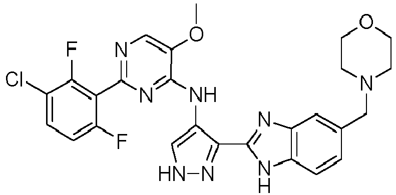

The title compound was synthesized from **138** (10 mg, 15 µmol) according to general procedure B (reaction time: 5 h). The crude was purified by automated column chromatography (1 - 15% MeOH/EtOAc) to afford the product (7.1 mg, 13 µmol, 88%). ¹H NMR (600 MHz, DMSO) δ 13.41 - 12.84 (br m, 2H), 10.72 (br s, 1H), 8.36 (s, 1H), 8.26 (s, 1H), 7.77 (td, *J* = 8.7, 5.5 Hz, 1H), 7.71 - 7.57 (br m, 1H), 7.52 - 7.40 (br m, 1H), 7.34 (td, *J* = 9.0, 1.7 Hz, 1H), 7.23 - 7.18 (br m, 1H), 4.17 (s, 3H), 3.61 - 3.56 (m, 6H), 2.43 - 2.36 (m, 4H). ¹³C NMR (151 MHz, DMSO) δ 159.49, 159.46, 157.84, 157.80, 156.10, 156.05, 154.43, 154.38, 149.50, 148.65, 147.52 (br), 142.04 (br), 139.64, 134.67 (br), 133.56, 132.77 (br), 131.43 (br), 130.84, 130.77, 124.11 (br), 123.15 (br), 121.50, 119.14 (br), 119.07, 118.95, 118.94, 118.82, 117.97 (br), 116.06, 116.03, 115.94, 115.91, 113.36, 113.34, 113.20, 113.18, 111.67 (br), 111.05 (br), 66.25, 62.91, 56.66, 53.23. LCMS (Fleet, 10 → 90%): tr = 3.80 min, m/z: 553.3. HRMS [C₂₆H₂₃ClF₂N₈O₂ + H]⁺: 553.16733 calculated, 553.16754 found.

### 2-(3-Ethynyl-2,6-difluorophenyl)-5-methoxy-N-(3-(5-(morpholinomethyl)-1H-benzo[d]imidazol-2-yl)-1H-pyrazol-4-yl)pyrimidin-4-amine (60)

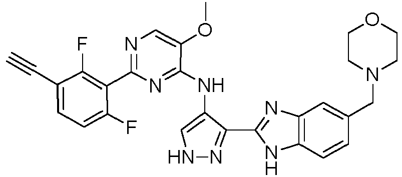

The title compound was synthesized from **139** (24 mg, 32 µmol) according to general procedure D (reaction time with TFA: 5 h). The crude was purified by automated column chromatography (1 - 15% MeOH/EtOAc) to afford the product (16.6 mg, 30.6 µmol, 95%). ¹H NMR (400 MHz, DMSO) δ 13.38 - 12.90 (br m, 2H), 10.72 (s, 1H), 8.36 (s, 1H), 8.26 (s, 1H), 7.73 (td, *J* = 8.2, 6.1 Hz, 1H), 7.69 - 7.60 (br m, 1H), 7.49 - 7.41 (br m, 1H), 7.31 (td, *J* = 8.9, 1.3 Hz, 1H), 7.25 - 7.16 (br m, 1H), 4.57 (s, 1H), 4.17 (s, 3H), 3.64 - 3.53 (m, 6H), 2.44 - 2.33 (m, 4H). ¹³C NMR (101 MHz, DMSO) δ 161.99, 161.92, 161.33, 161.27, 159.46, 159.39, 158.81, 158.75, 149.50, 148.72, 147.55 (br), 142.88 - 142.61 (m), 142.16 - 142.02 (m), 139.61, 134.45, 134.35, 133.67 (br), 133.58, 132.76 (br), 132.62 (br), 131.43 (br), 130.73 (br), 124.15 (br), 123.19 (br), 121.56, 119.18 (br), 118.93 (br), 118.27, 118.09, 118.01 (br), 117.90, 112.86, 112.82, 112.63, 112.60, 111.70 (br), 111.11 (br), 107.21, 107.17, 107.04, 107.00, 86.36, 76.02, 66.27, 62.94, 56.67, 53.25. LCMS (Fleet, 10 → 90%): tr = 3.69 min, m/z: 543.3. HRMS [C₂₈H₂₄F₂N₈O₂ + H]⁺: 543.20630 calculated, 543.20646 found.

### 2-(4-Fluorophenyl)-5-methoxy-N-(3-(5-(morpholinomethyl)-1H-benzo[d]imidazol-2-yl)-1H-pyrazol-4-yl)pyrimidin-4-amine (61)

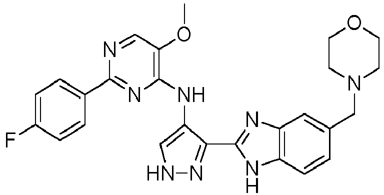

The title compound was synthesized from **140** (69.2 mg, 110 µmol) according to general procedure B (reaction time: 5 h). The crude was loaded onto Celite and purified by automated column chromatography (1 - 20% MeOH/EtOAc) to afford the product (49.4 mg, 98.7 µmol, 90%). ¹H NMR (400 MHz, DMSO) δ 13.50 - 12.88 (br m, 2H), 10.71 (br s, 1H), 8.65 (s, 1H), 8.43 - 8.33 (m, 2H), 8.18 (s, 1H), 7.70 - 7.60 (br m, 1H), 7.51 - 7.41 (br m, 1H), 7.37 - 7.29 (m, 2H), 7.23 - 7.15 (br m, 1H), 4.13 (s, 3H), 3.61 - 3.54 (m, 6H), 2.43 - 2.32 (m, 4H). ¹³C NMR (101 MHz, DMSO) δ 164.42, 161.97, 154.68, 149.71, 147.64 (br), 142.90 (br), 142.15 (br), 139.38, 134.71, 134.69, 133.71 (br), 133.60, 132.81 (br), 132.57 (br), 131.38 (br), 130.79 (br), 129.46, 129.37, 124.14 (br), 123.19 (br), 121.80, 119.11 (br), 118.90 (br), 117.99 (br), 115.45, 115.23, 111.69 (br), 111.09 (br), 66.29, 62.97, 56.57, 53.25. LCMS (Fleet, 10 → 90%): tr = 3.53 min, m/z: 501.3.

### 4-((2-(4-Nitro-1H-pyrazol-3-yl)-1H-benzo[d]imidazol-5-yl)methyl)morpholine (62)

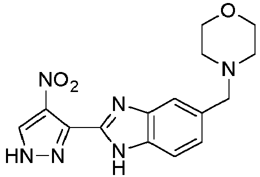

**143** (3.55 g, 17.1 mmol), 4-nitro-1*H*-pyrazole-3-carboxylic acid (2.53 g, 16.1 mmol), EDC·HCl (3.38 g, 17.7 mmol) and HOBt (2.32 g, 17.1 mmol) were mixed in dry DMF (39 mL) and stirred for 20 h. The mixture was concentrated at 60°C after which AcOH (49 mL) was added. The mixture was heated to 118°C and stirred for 2.5 h. The mixture was concentrated at 80°C and subsequently coevaporated with toluene (4×20 mL). The crude was brought onto Celite and purified by silica gel chromatography (5 - 9% MeOH/DCM) to afford the product (3.07 g, 9.35 mmol, 55%). ¹H NMR (400 MHz, MeOD) δ 8.60 (s, 1H), 7.68 (s, 1H), 7.60 (d, *J* = 8.3 Hz, 1H), 7.28 (d, *J* = 8.4 Hz, 1H), 3.98 (s, 2H), 3.78 - 3.66 (m, 4H), 2.89 - 2.78 (m, 4H). ¹³C NMR (101 MHz, MeOD) δ 144.63, 139.63, 139.44, 136.69, 134.62, 133.79, 128.93, 126.95, 118.88, 116.50, 66.10, 63.04, 53.34. LCMS (Finnigan, 0 → 50%): tr = 4.60 min, m/z: 329.1.

### 4-((2-(4-Nitro-1-((2-(trimethylsilyl)ethoxy)methyl)-1H-pyrazol-3-yl)-1-((2-(trimethylsilyl)ethoxy)methyl)-1H-benzo[d]imidazol-5-yl)methyl)morpholine (63) & 3 regioisomers

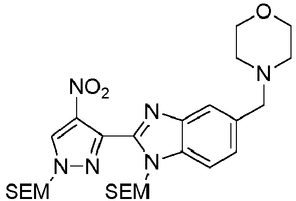

**62** (3.07 g, 9.35 mmol) was suspended in dry DCM (45 mL) and cooled down to 0°C. DIPEA (5.1 mL, 29 mmol) was added after which SEM-Cl (3.56 mL, 20.1 mmol) was added dropwise. The mixture was left to stir at 0°C and allowed to warm to RT overnight. The mixture was poured into 0.05 M NaHCO₃ (aq.) (150 mL) and the product extracted with DCM (2x100 mL). The combined organic layers were washed with brine (200 mL) and the layers were separated. The brine layer was extracted with DCM (30 mL) after which the combined organic layers were dried over Na₂SO₄, filtered and concentrated. The crude was purified several times either by manual or automated column chromatography to separate all four regioisomers (total yield: 3.26 g, 5.54 mmol, 60%). NMR data (sorted on descending isomer lipophilicity as determined by TLC analysis): Regioisomer 1 - yield (1.34 g, 2.28 mmol, 25%). ¹H NMR (500 MHz, CDCl₃) δ 8.29 (s, 1H), 7.79 (d, *J* = 8.3 Hz, 1H), 7.61 (s, 1H), 7.39 (dd, *J* = 8.4, 1.3 Hz, 1H), 5.83 - 5.40 (br m, 2H), 5.32 (br s, 2H), 3.75 - 3.70 (m, 4H), 3.67 (s, 2H), 3.62 (br s, 2H), 3.43 - 3.36 (m, 2H), 2.56 - 2.45 (m, 4H), 0.89 - 0.72 (m, 4H), - 0.05 (s, 9H), -0.08 (s, 9H). ¹³C NMR (126 MHz, CDCl₃) δ 142.58, 139.22, 136.37, 136.13, 135.38, 135.30, 131.10, 125.13, 120.56, 111.35, 79.55, 74.39, 68.22, 67.14, 67.05, 63.81, 53.82, 17.94, 17.83, -1.33, -1.42. LCMS (Finnigan, 10 → 90%): tᵣ = 7.89 min, m/z: 589.1. Regioisomer 2 - yield (765 mg, 1.30 mmol, 14%). ¹H NMR (500 MHz, CDCl₃) δ 8.28 (s, 1H), 7.78 (s, 1H), 7.57 (d, *J* = 8.4 Hz, 1H), 7.45 (dd, *J* = 8.4, 1.2 Hz, 1H), 5.77 - 5.38 (br m, 2H), 5.31 (br s, 2H), 3.74 - 3.70 (m, 4H), 3.65 (s, 2H), 3.60 (br s, 2H), 3.42 - 3.35 (m, 2H), 2.52 - 2.46 (m, 4H), 0.87 - 0.72 (m, 4H), -0.06 (s, 9H), -0.09 (s, 9H). ¹³C NMR (126 MHz, CDCl₃) δ 143.24, 139.27, 136.31, 136.11, 134.47, 133.60, 131.05, 126.36, 121.20, 110.85, 79.53, 74.45, 68.17, 67.12, 67.01, 63.57, 53.71, 17.89, 17.77, -1.38, -1.46. LCMS (Finnigan, 10 → 90%): tr = 8.00 min, m/z: 589.1. Regioisomer 3 - yield (502 mg, 0.853 mmol, 9%). ¹H NMR (500 MHz, CDCl₃) δ 8.48 (s, 1H), 7.75 (d, *J* = 8.3 Hz, 1H), 7.55 (s, 1H), 7.31 (dd, *J* = 8.3, 1.3 Hz, 1H), 5.51 (s, 2H), 5.50 (s, 2H), 3.72 - 3.68 (m, 4H), 3.68 - 3.64 (m, 2H), 3.63 (s, 2H), 3.36 - 3.31 (m, 2H), 2.50 - 2.42 (m, 4H), 0.97 - 0.92 (m, 2H), 0.76 - 0.71 (m, 2H), -0.02 (s, 9H), -0.15 (s, 9H). ¹³C NMR (126 MHz, CDCl₃) δ 143.02, 142.38, 137.77, 135.63, 135.12, 134.29, 130.01, 124.66, 120.26, 111.10, 82.14, 73.76, 68.47, 67.04, 66.36, 63.79, 53.69, 17.90, 17.65, -1.36, -1.48. LCMS (Finnigan, 10→ 90%): tᵣ = 7.53 min, m/z: 589.1. Regioisomer 4 - yield (649 mg, 1.10 mmol, 12%). ¹H NMR (500 MHz, CDCl₃) δ 8.49 (s, 1H), 7.76 (s, 1H), 7.53 (d, *J* = 8.4 Hz, 1H), 7.38 (dd, *J* = 8.4, 1.2 Hz, 1H), 5.51 (s, 2H), 5.50 (s, 2H), 3.71 - 3.68 (m, 4H), 3.68 - 3.64 (m, 2H), 3.63 (s, 2H), 3.36 - 3.31 (m, 2H), 2.50 - 2.44 (m, 4H), 0.98 - 0.93 (m, 2H), 0.77 - 0.72 (m, 2H), -0.01 (s, 9H), -0.15 (s, 9H). ¹³C NMR (126 MHz, CDCl₃) δ 143.15, 143.09, 137.79, 135.69, 134.31, 132.95, 129.99, 125.68, 121.13, 110.56, 82.19, 73.88, 68.53, 67.09, 66.40, 63.64, 53.64, 17.94, 17.65, -1.33, -1.46. LCMS (Finnigan, 10 → 90%): tr = 7.70 min, m/z: 589.1.

### 3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-amine (64)

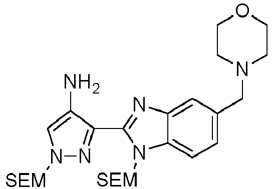

**63** (502 mg, 853 µmol) was dissolved in degassed MeOH (10 mL). 10% Pd/C (60 mg) was added and the atmosphere was exchanged for H₂. The reaction was vigorously stirred for 3.5 h while bubbling H₂ through the mixture. The atmosphere was exchanged for N₂, the mixture was filtered over Celite and concentrated to afford the product, which was used as such in subsequent reaction (471 mg, 853 µmol, 99%). LCMS (Finnigan, 10 → 90%): tᵣ = 6.59 min, m/z: 559.1.

### 2-Chloro-N-(3-(5-(morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)pyrimidin-4-amine (65)

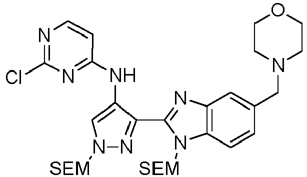

**64** (471 mg, 843 µmol) was dissolved in EtOH (2 mL) after which DIPEA (450 µL, 2.69 mmol) and 2,4-dichloropyrimidine (119 mg, 801 µmol) were added. The mixture was stirred at 40°C for 3 days and subsequently poured into H₂O (50 mL). The product was extracted with DCM (2x50 mL) and the combined organic layers were washed with brine (100 mL), dried over Na₂SO₄, filtered and concentrated. The crude was purified by silica gel column chromatography (1 - 4% MeOH/DCM) to afford the product (305 mg, 455 µmol, 54%). ¹H NMR (500 MHz, CDCl₃) δ 11.00 (s, 1H), 8.57 (s, 1H), 8.11 (d, *J* = 5.8 Hz, 1H), 7.70 (d, *J* = 8.2 Hz, 1H), 7.56 (s, 1H), 7.32 (dd, *J* = 8.3, 1.4 Hz, 1H), 6.68 (d, *J* = 5.8 Hz, 1H), 6.26 (s, 2H), 5.50 (s, 2H), 3.75 - 3.69 (m, 4H), 3.69 - 3.60 (m, 6H), 2.54 - 2.44 (m, 4H), 0.99 - 0.94 (m, 2H), 0.90 - 0.85 (m, 2H), 0.00 (s, 9H), - 0.12 (s, 9H). ¹³C NMR (126 MHz, CDCl₃) δ 160.95, 159.52, 156.33, 146.84, 141.60, 135.03, 133.84, 131.70, 124.71, 124.39, 121.18, 118.78, 111.47, 106.27, 81.29, 73.92, 67.17, 67.10, 66.15, 63.87, 53.76, 17.96, 17.83, -1.29, - 1.34. LCMS (Finnigan, 10 → 90%): tr = 8.73 min, m/z: 671.1.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-((trimethylsilyl)ethynyl)pyrimidin-4-amine (66)

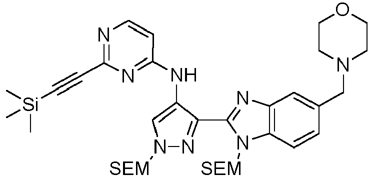

A microwave vial was charged with **65** (104 mg, 155 µmol) and Et₃N (1 mL). N₂ was bubbled through the mixture for 1 min after which PdCl₂(PPh₃)₂ and Cu(I)I were added. N₂ was bubbled through the mixture for 30 sec after which ethynyltrimethylsilane (100 µL, 722 µmol) was added and the vial was sealed. The mixture was heated to 89°C and stirred for 5 h. Ethynyltrimethylsilane (50 µL, 361 µmol) was added via syringe and the mixture was stirred at 90°C for 16 h. The mixture was diluted in MeOH (15 mL), filtered over Celite and subsequently concentrated. The crude was purified by automated column chromatography (0 - 10% MeOH/EtOAc) to afford the product (19.1 mg, 26.1 µmol, 17%). ¹H NMR (500 MHz, CDCl₃) δ 8.16 (d, *J* = 6.0 Hz, 1H), 8.05 (br s, 1H), 7.76 (br s, 1H), 7.72 (s, 1H), 7.44 (d, *J* = 8.3 Hz, 1H), 7.39 (dd, *J* = 8.4, 1.3 Hz, 1H), 6.49 (d, *J* = 6.0 Hz, 1H), 5.54 (s, 2H), 5.38 (s, 2H), 3.73 - 3.69 (m, 4H), 3.63 (s, 2H), 3.58 - 3.49 (m, 4H), 2.52 - 2.43 (m, 4H), 0.98 - 0.92 (m, 2H), 0.79 - 0.73 (m, 2H), 0.24 (s, 9H), -0.06 (s, 9H), -0.09 (s, 9H). ¹³C NMR (126 MHz, CDCl₃) δ 156.37, 152.04, 143.31, 142.29, 134.75, 134.56, 133.52, 125.97, 120.95, 110.24, 102.89, 92.42, 79.05, 73.79, 67.68, 67.30, 67.13, 63.58, 53.70, 17.99, 17.95, -0.30, -1.34, -1.42 (not all quaternary carbons were observed). LCMS (Finnigan, 10 → 90%): tr = 6.64 min, m/z: 661.2.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-phenylpyrimidin-4-amine (67)

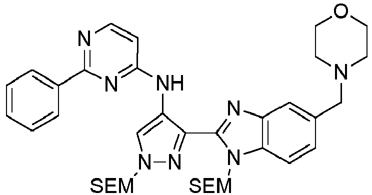

A microwave vial was charged with **65** (65.8 mg, 98.0 µmol), phenylboronic acid (20.3 mg, 167 µmol), K₂CO₃ (4.7 M (aq.), 83 µL, 392 µmol) and dioxane (0.33 mL). N₂ was bubbled through the mixture for 1 min after which Pd(dppf)Cl₂·DCM (4.0 mg, 4.9 µmol) was added. N₂ was bubbled through the mixture for 30 sec after which the vial was sealed. The mixture was heated to 90°C, stirred for 3.5 h and subsequently poured into H₂O (20 mL). The product was extracted with DCM (2x20 mL) and the combined organic layers were washed with brine (40 mL), dried over Na₂SO₄, filtered and concentrated. The crude was purified by automated column chromatography (50 - 100% EtOAc/pentane) to afford the product (34.2 mg, 47.9 µmol, 49%). ¹H NMR (400 MHz, CDCl₃) δ 10.72 (s, 1H), 8.79 (s, 1H), 8.44 - 8.38 (m, 3H), 7.75 (d, *J* = 8.2 Hz, 1H), 7.58 (s, 1H), 7.56 - 7.48 (m, 3H), 7.35 (dd, *J* = 8.3, 1.4 Hz, 1H), 6.74 (d, *J* = 5.8 Hz, 1H), 6.30 (s, 2H), 5.56 (s, 2H), 3.77 - 3.73 (m, 4H), 3.72 - 3.62 (m, 6H), 2.54 - 2.48 (m, 4H), 1.02 - 0.96 (m, 2H), 0.92 - 0.87 (m, 2H), -0.01 (s, 9H), -0.10 (s, 9H). ¹³C NMR (101 MHz, CDCl₃) δ 164.85, 158.81, 155.36, 147.17, 141.86, 138.87, 135.14, 133.62, 131.67, 130.41, 128.60, 128.22, 125.52, 124.69, 120.44, 118.90, 111.51, 106.05, 81.46, 73.99, 67.20, 67.15, 66.14, 63.96, 53.80, 18.01, 17.92, - 1.24, -1.29. LCMS (Finnigan, 10 → 90%): tr = 7.03 min, m/z: 713.3.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-phenoxypyrimidin-4-amine (68)

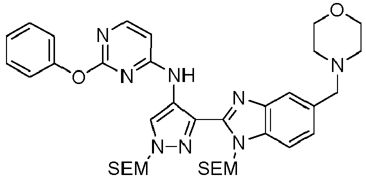

NaH (60% in mineral oil, 46 mg, 1.15 mmol) was suspended in dioxane (4 mL) and cooled down to 0°C. Phenol (116 mg, 1.23 mmol) was carefully added (H₂ evolution) and the mixture was allowed to warm to RT and stirred for 30 min. Of this mixture, 400 µL was added to a microwave vial charged with **65** (27.6 mg, 41.1 µmol) after which the vial was sealed and the mixture was stirred at 100°C for 16 h. Extra sodium phenolate was prepared freshly as described above, of which 100 µL was added to the mixture which was continued to stir at 100°C for 24 h. The mixture was poured into 1 M NaHCO₃ (aq.) (20 mL) and the product extracted with DCM (2x20 mL). The combined organic layers were concentrated as such and purified by automated column chromatography (0 - 15% MeOH/DCM) to afford the product (18 mg, 24.7 µmol, 60%). ¹H NMR (500 MHz, CDCl₃) δ 10.76 (s, 1H), 8.19 (d, *J* = 5.8 Hz, 1H), 7.73 (s, 1H), 7.56 - 7.45 (m, 4H), 7.36 - 7.31 (m, 2H), 7.30 - 7.27 (m, 2H), 6.52 (d, *J* = 5.8 Hz, 1H), 6.23 (s, 2H), 5.19 (s, 2H), 3.76 - 3.71 (m, 4H), 3.66 (s, 2H), 3.59 - 3.55 (m, 2H), 3.54 - 3.50 (m, 2H), 2.55 - 2.45 (m, 4H), 0.94 - 0.89 (m, 2H), 0.88 - 0.83 (m, 2H), - 0.00 (s, 9H), -0.13 (s, 9H). ¹³C NMR (126 MHz, CDCl₃) δ 166.10, 160.02, 157.35, 153.79, 147.16, 142.48, 134.32, 131.71, 129.76, 125.20, 124.60, 123.03, 121.44, 119.76, 110.78, 102.42, 80.96, 74.03, 67.16, 66.95, 66.06, 63.85, 53.75, 17.95, 17.87, -1.21, -1.33. LCMS (Finnigan, 10 → 90%): tr = 7.37 min, m/z: 729.2.

### 2-(Benzyloxy)-N-(3-(5-(morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)pyrimidin-4-amine (69)

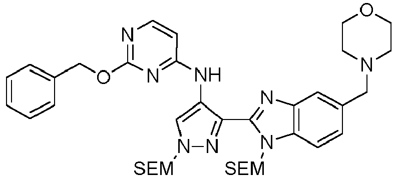

NaH (60% in mineral oil, 90.0 mg, 2.25 mmol) was suspended in dioxane (3 mL) and cooled down to 0°C. Benzyl alcohol (250 µL, 2.41 mmol) was carefully added (H₂ evolution) and the mixture was allowed to warm to RT and stirred for 2 h. Of this mixture, 300 µL was added to a microwave vial charged with **65** (80.9 mg, 120 µmol) after which the vial was sealed and the mixture was stirred at 90°C for 16 h. The mixture was poured into H₂O (20 mL) and brine (0.5 mL), and the product extracted with DCM (2x20 mL). The combined organic layers were concentrated as such and purified by automated column chromatography (1 - 10% MeOH/DCM) to afford the product (63.3 mg, 85.2 µmol, 71%). ¹H NMR (400 MHz, CDCl₃) δ 8.12 (br s, 1H), 8.05 (br s, 1H), 8.01 (d, *J* = 5.8 Hz, 1H), 7.65 (d, *J* = 8.3 Hz, 1H), 7.46 - 7.40 (m, 3H), 7.35 - 7.30 (m, 2H), 7.30 - 7.26 (m, 2H), 6.21 (d, *J* = 5.8 Hz, 1H), 5.52 (s, 2H), 5.35 (s, 2H), 5.34 (s, 2H), 3.74 - 3.67 (m, 4H), 3.61 (s, 2H), 3.54 - 3.46 (m, 4H), 2.53 - 2.41 (m, 4H), 0.93 - 0.86 (m, 2H), 0.78 - 0.70 (m, 2H), -0.09 (s, 9H), -0.10 (s, 9H). ¹³C NMR (101 MHz, CDCl₃) δ 165.11, 162.37 (br), 157.83 (br), 142.48, 142.37, 136.83, 135.17, 134.75 (br), 134.53, 128.42, 127.91, 127.86, 124.89, 124.49 (br), 119.84, 110.94, 99.29 (br), 78.83, 73.75, 68.68, 67.38, 67.12, 67.06, 63.74, 53.72, 17.99, 17.87, -1.40, -1.46. LCMS (Finnigan, 10 → 90%): tr = 6.82 min, m/z: 743.3.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-phenethoxypyrimidin-4-amine (70)

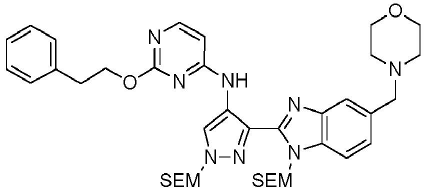

NaH (60% in mineral oil, 120 mg, 3.02 mmol) was suspended in dioxane (6 mL), phenethyl alcohol (400 µL, 3.34 mmol) was carefully added and the mixture was stirred at 45°C for 3 h. The mixture was cooled down to RT and 300 µL was added to a microwave vial charged with **65** (61.3 mg, 91.3 µmol) after which the vial was sealed and the mixture was stirred at 90°C for 16 h. The mixture was poured into H₂O (20 mL) and the product extracted with DCM (2x20 mL). The combined organic layers were concentrated as such and purified by automated column chromatography (50 - 100% EtOAc/pentane) to afford the product (30.6 mg, 40.4 µmol, 44%). ¹H NMR (400 MHz, CDCl₃) δ 10.72 (s, 1H), 8.57 (s, 1H), 8.11 (d, *J* = 5.7 Hz, 1H), 7.75 (s, 1H), 7.54 (d, *J* = 8.3 Hz, 1H), 7.38 - 7.30 (m, 5H), 7.26 - 7.22 (m, 1H), 6.46 (d, *J* = 5.8 Hz, 1H), 6.27 (s, 2H), 5.45 (s, 2H), 4.61 (t, *J* = 7.5 Hz, 2H), 3.77 - 3.71 (m, 4H), 3.67 (s, 2H), 3.66 - 3.57 (m, 4H), 3.19 (t, *J* = 7.5 Hz, 2H), 2.55 - 2.47 (m, 4H), 0.98 - 0.90 (m, 2H), 0.93 - 0.84 (m, 2H), -0.01 (s, 9H), -0.11 (s, 9H). ¹³C NMR (101 MHz, CDCl₃) δ 165.42, 160.49, 156.54, 147.18, 142.49, 138.32, 134.32, 132.80, 131.60, 129.13, 128.62, 126.57, 125.22, 125.20, 120.69, 119.79, 110.73, 101.95, 81.26, 74.02, 67.77, 67.14, 67.09, 66.08, 63.84, 53.73, 35.52, 17.94, 17.85, -1.25, -1.33. LCMS (Finnigan, 10 → 90%): tr = 7.75 min, m/z: 757.3.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-(1H-pyrazol-1-yl)pyrimidin-4-amine (71)

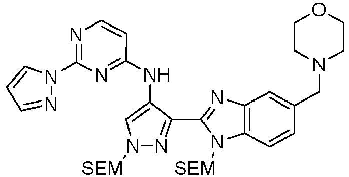

NaH (60% in mineral oil, 74 mg, 1.85 mmol) was suspended in dioxane (2 mL) and cooled down to 0°C. 1*H*-Pyrazole (155 mg, 2.28 mmol) was carefully added (H₂ evolution) and the mixture was allowed to warm to RT and stirred for 1.5 h. Of this mixture, 300 µL was added to a microwave vial charged with **65** (136 mg, 203 µmol) after which the vial was sealed and the mixture was stirred at 90°C for 45 min. The mixture was poured into H₂O (20 mL) and the product extracted with DCM (2x20 mL). The combined organic layers were concentrated as such and purified by automated column chromatography (70 - 100% EtOAc/pentane, then 0 - 10% MeOH/EtOAc) to afford the product (78.2 mg, 111 µmol, 55%). ¹H NMR (500 MHz, CDCl₃) δ 10.98 (s, 1H), 8.86 (s, 1H), 8.56 - 8.52 (m, 1H), 8.29 (d, *J* = 5.8 Hz, 1H), 7.84 - 7.80 (m, 1H), 7.76 - 7.74 (m, 1H), 7.54 (d, *J* = 8.3 Hz, 1H), 7.33 (dd, *J* = 8.3, 1.4 Hz, 1H), 6.69 (d, *J* = 5.8 Hz, 1H), 6.48 (dd, *J* = 2.6, 1.6 Hz, 1H), 6.27 (s, 2H), 5.55 (s, 2H), 3.76 - 3.70 (m, 4H), 3.72 - 3.65 (m, 2H), 3.66 (s, 2H), 3.65 - 3.59 (m, 2H), 2.50 (s, 4H), 1.01 - 0.94 (m, 2H), 0.91 - 0.85 (m, 2H), -0.02 (s, 9H), -0.12 (s, 9H). ¹³C NMR (126 MHz, CDCl₃) δ 159.45, 156.01, 147.12, 143.05, 142.40, 134.28, 132.84, 131.67, 128.80, 125.20, 124.97, 121.25, 119.71, 110.72, 108.15, 105.21, 81.36, 74.00, 67.19, 67.09, 66.08, 63.79, 53.69, 17.92, 17.83, -1.30, -1.37. LCMS (Finnigan, 10 → 90%): tr = 8.03 min, m/z: 703.2.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-(thiophen-3-yl)pyrimidin-4-amine (72)

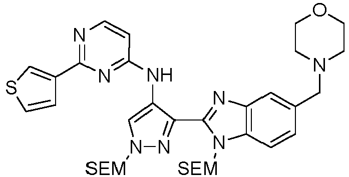

A microwave vial was charged with **65** (75.0 mg, 112 µmol), thiophene-3-boronic acid pinacol ester (27.5 mg, 134 µmol), K₂CO₃ (61.8 mg, 447 µmol), dioxane (0.8 mL) and H₂O (0.2 mL). N₂ was bubbled through the mixture for 1 min after which Pd(dppf)Cl₂·DCM (6.5 mg, 8.0 µmol) was added. N₂ was bubbled through the mixture for 30 sec after which the vial was sealed and the mixture was stirred at 100°C for 6 h. The mixture was diluted in 1:1:1 EtOAc/H₂O/brine (9 mL) and filtered over Celite. The mixture was poured into H₂O (10 mL) and EtOAc (10 mL) and the layers were separated. The organic layer was washed with brine (10 mL), dried over Na₂SO₄, filtered and concentrated. The crude was purified by automated column chromatography (0 - 10% MeOH/DCM) and used as such in subsequent reaction (yield: 43.2 mg). LCMS m/z: 719.3.

### N-(3-(5-(Morpholinomethyl)-1-((2-(trimethylsilyl)ethoxy)methyl)-1H-benzo[d]imidazol-2-yl)-1-((2-(trimethylsilyl)ethoxy)methyl)-1H-pyrazol-4-yl)-2-(thiophen-2-yl)pyrimidin-4-amine (73)

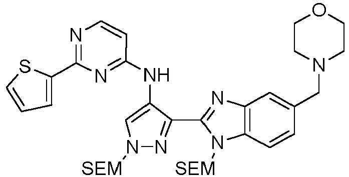

A microwave vial was charged with **65** (75.0 mg, 112 µmol), thiophene-2-boronic acid pinacol ester (27.5 mg, 134 µmol), K₂CO₃ (61.8 mg, 447 µmol), dioxane (0.8 mL) and H₂O (0.2 mL). N₂ was bubbled through the mixture for 1 min after which Pd(dppf)Cl₂·DCM (6.5 mg, 8.0 µmol) was added. N₂ was bubbled through the mixture for 30 sec after which the vial was sealed and the mixture was stirred at 100°C for 6 h. The mixture was poured into H₂O (10 mL) and the product extracted with DCM (10 mL). The organic layer was concentrated as such and purified by automated column chromatography (0 - 10% MeOH/DCM) and used as such in subsequent reaction (yield: 59.6 mg). LCMS (Finnigan, 10 → 90%): tr = 7.31 min, m/z: 719.3.

### 2-(4-Nitro-1H-pyrazol-3-yl)-1H-benzo[d]imidazole (74)

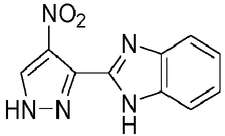

Benzene-1,2-diamine (9.39 g, 87.0 mmol), 4-nitro-1H-pyrazole-3-carboxylic acid (13.6 g, 87.0 mmol), EDC·HCl (16.7 g, 87.0 mmol) and HOBt (11.7 g, 87.0 mmol) were mixed in DMF (140 mL) and stirred for 18 h. The mixture was concentrated at 60°C after which AcOH (110 mL) was added and the reaction was stirred at 118°C for 75 min. Sat. NaHCO₃ (aq.) (440 mL) was added carefully while the mixture was stirred vigorously. The mixture was stirred for 1 h, filtered and the solids were washed with H₂O (2x50 mL). The solids were collected and dried by coevaporation with MeOH to afford the product (14.0 g, 60.9 mmol, 70%). ¹H NMR (400 MHz, DMSO) δ 14.55 (br s, 1H), 13.01 (br s, 1H), 8.84 (br s, 1H), 7.74 - 7.67 (m, 2H), 7.33 - 7.24 (m, 2H). ¹³C NMR (101 MHz, DMSO) δ 158.83, 141.58, 135.26, 134.13, 132.96, 122.89, 115.69 (br). LCMS (Finnigan, 0 → 90%): tr = 4.51 min, m/z: 230.1.

### 2-(4-Nitro-1-((2-(trimethylsilyl)ethoxy)methyl)-1H-pyrazol-3-yl)-1-((2-(trimethylsilyl)ethoxy)methyl)-1H-benzo[d]imidazole (75)

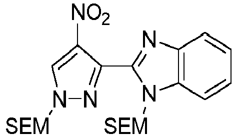

**74** (10.0 g, 43.6 mmol) was suspended in DCM (220 mL) and cooled down to 0°C. DIPEA (22.8 mL, 131 mmol) was added after which SEM-Cl (17 mL, 96 mmol) was added dropwise. The mixture was stirred at 0°C for 15 min and then allowed to warm to RT and stirred for 1.5 h. The mixture was poured into 0.05 M NaHCO₃ (aq.) (300 mL) and the organic layer was separated. The water layer was extracted with DCM (150 mL) after which the combined organic layers were washed with brine (300 mL). The brine layer was extracted with DCM (100 mL) after which the organic layers were combined, dried over Na₂SO₄, filtered and concentrated. The crude was purified by silica gel chromatography (twice, 0-3% MeOH/DCM) to afford both regioisomers in pure form (total yield: 17.2 g, 35.2 mmol, 81%). NMR data (sorted on descending isomer lipophilicity): Regioisomer 1 - yield (8.45 g, 17.3 mmol, 40%). ¹H NMR (400 MHz, CDCl₃) δ 8.30 (s, 1H), 7.86 (dd, *J* = 7.3, 1.0 Hz, 1H), 7.65 - 7.62 (m, 1H), 7.44 (td, *J* = 7.7, 1.3 Hz, 1H), 7.39 (td, *J* = 7.7, 1.3 Hz, 1H), 5.75 - 5.40 (br m, 2H), 5.39 - 5.29 (br m, 2H), 3.70 - 3.54 (br m, 2H), 3.44 - 3.36 (m, 2H), 0.90 - 0.70 (m, 4H), -0.05 (s, 9H), -0.08 (s, 9H). ¹³C NMR (101 MHz, CDCl₃) δ 143.11, 139.14, 136.31, 136.11, 135.19, 131.04, 124.92, 123.60, 120.90, 111.13, 79.52, 74.38, 68.15, 67.00, 17.86, 17.76, -1.40, -1.47. LCMS (Finnigan, 10 →- 90%): tr = 10.86 min, m/z: 490.0. Regioisomer 2 - yield (8.78 g, 17.9 mmol, 41%). ¹H NMR (400 MHz, CDCl₃) δ 8.49 (s, 1H), 7.85 (dd, *J* = 7.1, 1.4 Hz, 1H), 7.60 (dd, *J* = *7.0,* 1.3 Hz, 1H), 7.41 - 7.32 (m, 2H), 5.53 (s, 2H), 5.52 (s, 2H), 3.72 - 3.65 (m, 2H), 3.39 - 3.32 (m, 2H), 1.00 - 0.94 (m, 2H), 0.79 - 0.73 (m, 2H), 0.00 (s, 9H), - 0.13 (s, 9H). ¹³C NMR (101 MHz, CDCl₃) δ 143.03, 142.98, 137.81, 135.71, 135.04, 129.99, 124.23, 123.20, 120.73, 110.85, 82.21, 73.84, 68.55, 66.43, 17.95, 17.68, -1.32, -1.45. LCMS (Finnigan, 10 →- 90%): tr = 9.48 min, m/z: 490.0.

### 1-((2-(Trimethylsilyl)ethoxy)methyl)-3-(1-((2-(trimethylsilyl)ethoxy)methyl)-1H-benzo[d]imidazol-2-yl)-1H-pyrazol-4-amine (76)

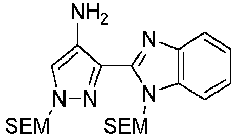

**75** (8.78 g, 17.9 mmol) was dissolved in degassed MeOH (220 mL). 10% Pd/C (900 mg) was added and the atmosphere was exchanged for H₂. The reaction was vigorously stirred for 5.5 h while bubbling H₂ through the mixture. The atmosphere was exchanged for N₂, the mixture was filtered over Celite and concentrated to afford the product (7.87 g, 17.1 mmol, 95%). ¹H NMR (400 MHz, CDCl₃) δ 7.78 - 7.72 (m, 1H), 7.60 - 7.54 (m, 1H), 7.32 - 7.26 (m, 2H), 7.21 (s, 1H), 6.23 (s, 2H), 5.36 (s, 2H), 4.75 (s, 2H), 3.64 - 3.55 (m, 4H), 0.96 - 0.91 (m, 2H), 0.91 - 0.85 (m, 2H), -0.01 (s, 9H), -0.10 (s, 9H). ¹³C NMR (101 MHz, CDCl₃) δ 147.51, 143.12, 135.09, 133.80, 131.81, 123.01, 122.63, 119.29, 115.95, 110.77, 81.07, 73.88, 66.78, 65.80, 17.92, 17.85, -1.29, -1.36. LCMS (Finnigan, 10 → 90%): tr = 8.61 min, m/z: 460.1.

### 2-Chloro-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (77)

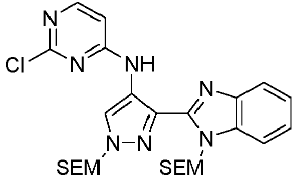

**76** (6.00 g, 13.1 mmol) was dissolved in EtOH (14 mL). DIPEA (6.8 mL, 39 mmol) and 2,4-dichloropyrimidine (2.33 g, 15.7 mmol) were added after which the mixture was stirred at 40°C for 2 days. 2,4-dichloropyrimidine (388 mg, 2.61 mmol) was added and the mixture was stirred for another day at 40°C. The mixture was poured into H₂O (200 mL) and the product extracted with DCM (2x200 mL). The combined organic layers were concentrated as such and purified by silica gel column chromatography (10 - 20% EtOAc/pentane) to afford the product (5.53 g, 9.66 mmol, 74%). ¹H NMR (500 MHz, CDCl₃) δ 11.03 (s, 1H), 8.57 (s, 1H), 8.11 (d, *J* = 5.8 Hz, 1H), 7.79 - 7.74 (m, 1H), 7.62 - 7.58 (m, 1H), 7.36 - 7.31 (m, 2H), 6.67 (d, *J* = 5.8 Hz, 1H), 6.26 (s, 2H), 5.50 (s, 2H), 3.69 - 3.65 (m, 2H), 3.65 - 3.61 (m, 2H), 1.00 - 0.95 (m, 2H), 0.91 - 0.86 (m, 2H), 0.01 (s, 9H), -0.11 (s, 9H). ¹³C NMR (126 MHz, CDCl₃) δ 160.90, 159.46, 156.28, 146.67, 142.15, 134.88, 131.60, 124.41, 123.72, 123.20, 121.14, 119.15, 111.06, 106.26, 81.26, 73.89, 67.15, 66.09, 17.90, 17.80, -1.29, -1.37. LCMS (Finnigan, 50 → 90%): tr = 11.35 min, m/z: 572.1.

### 2-Phenyl-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (86)

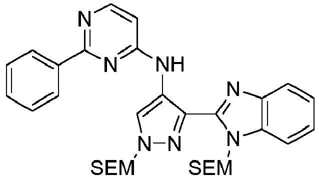

The title compound was synthesized according to general procedure F using phenylboronic acid. The crude was purified by silica gel column chromatography (40 - 50% Et₂O/pentane) to afford the product (57 mg, 93 µmol, 18%). ¹H NMR (500 MHz, CDCl₃) δ 10.77 (s, 1H), 8.77 (s, 1H), 8.44 - 8.40 (m, 3H), 7.85 - 7.81 (m, 1H), 7.63 - 7.59 (m, 1H), 7.56 - 7.49 (m, 3H), 7.38 - 7.34 (m, 2H), 6.72 (d, *J* = 5.8 Hz, 1H), 6.29 (s, 2H), 5.54 (s, 2H), 3.74 - 3.69 (m, 2H), 3.68 - 3.63 (m, 2H), 1.04 - 0.98 (m, 2H), 0.96 - 0.87 (m, 2H), 0.01 (s, 9H), -0.08 (s, 9H). ¹³C NMR (126 MHz, CDCl₃) δ 164.66, 158.69, 155.08, 146.90, 142.35, 142.23, 142.20, 138.68, 134.95, 131.53, 131.44, 130.37, 128.53, 128.18, 125.43, 123.57, 123.09, 120.36, 119.22, 111.00, 105.98, 81.37, 73.90, 67.12, 66.03, 17.91, 17.84, -1.29, -1.37. LCMS (Finnigan, 70 →- 90%): tr = 3.55 min, m/z: 614.4.

### 2-(o-Tolyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (87)

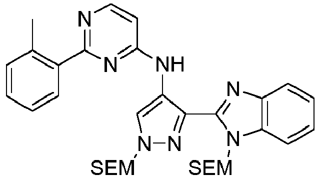

The title compound was synthesized from **77** (200 mg, 349 µmol) and *o-*tolylboronic acid (71.3 mg, 524 µmol) according to general procedure G (reaction time 1.5 h). The crude was purified by automated column chromatography (40 - 60% EtOAc/pentane) to afford the product (177 mg, 281 µmol, 80%). ¹H NMR (400 MHz, CDCl₃) δ 8.92 (br s, 1H), 8.39 (d, *J* = 5.9 Hz, 1H), 8.14 (br s, 1H), 7.80 (d, *J* = 7.3 Hz, 1H), 7.75 - 7.68 (m, 1H), 7.42 - 7.36 (m, 1H), 7.34 - 7.25 (m, 5H), 6.61 (d, *J* = 5.9 Hz, 1H), 5.55 (s, 2H), 5.38 (s, 2H), 3.56 - 3.45 (m, 4H), 2.56 (s, 3H), 0.93 - 0.87 (m, 2H), 0.80 - 0.73 (m, 2H), - 0.07 (s, 9H), -0.09 (s, 9H). ¹³C NMR (101 MHz, CDCl₃) δ 167.43, 160.34, 155.89, 142.74, 142.35, 138.70, 136.94, 134.78, 134.67, 131.09, 130.20, 128.99, 125.68, 124.60, 124.32, 123.34, 119.60, 110.81, 78.71, 73.72, 67.17, 66.96, 21.22, 17.77, -1.53 (not all quaternary carbons were observed, neither was one -CH of the pyrimidine). LCMS (Finnigan, 50 →- 90%): tᵣ = 5.18 min, m/z: 628.3.

### 2-(2-Chlorophenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (88)

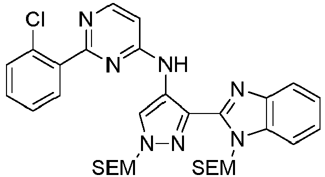

The title compound was synthesized according to general procedure F using 2-chlorophenylboronic acid. The crude was purified by silica gel column chromatography (40 - 50% Et₂O/pentane) to afford the product (268 mg, 413 µmol, 79%). ¹H NMR (300 MHz, CDCl₃) δ 10.82 (s, 1H), 8.80 (s, 1H), 8.45 (d, *J* = 5.9 Hz, 1H), 7.87 - 7.78 (m, 2H), 7.66 - 7.58 (m, 1H), 7.57 - 7.50 (m, 1H), 7.44 - 7.31 (m, 4H), 6.79 (d, *J* = 5.9 Hz, 1H), 6.30 (s, 2H), 5.47 (s, 2H), 3.69 - 3.59 (m, 4H), 0.99 - 0.85 (m, 4H), -0.02 (s, 9H), -0.10 (s, 9H). ¹³C NMR (75 MHz, CDCl₃) δ 165.14, 158.24, 155.06, 147.01, 142.41, 138.80, 135.00, 132.49, 131.86, 131.61, 130.62, 130.02, 126.80, 125.12, 123.58, 123.11, 121.31, 119.23, 111.06, 106.06, 81.27, 73.96, 67.11, 66.04, 17.93, 17.86, -1.33, -1.36. LCMS (Finnigan, 70 →- 90%): tr = 3.72 min, m/z: 648.4.

### 2-(2-Methoxyphenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (89)

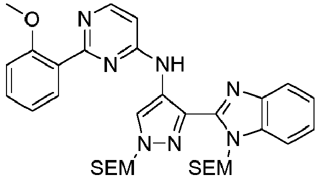

The title compound was synthesized according to general procedure F using 2-methoxyphenylboronic acid. The crude was purified by silica gel column chromatography (70 - 100% EtOAc/pentane) to afford the product (300 mg, 466 µmol, 89%). ¹H NMR (500 MHz, CDCl₃) δ 8.53 (br s, 1H), 8.39 (s, 1H), 8.35 (d, *J* = 5.9 Hz, 1H), 7.75 - 7.69 (m, 2H), 7.42 - 7.35 (m, 2H), 7.31 - 7.25 (m, 2H), 7.04 - 6.97 (m, 2H), 6.54 (d, *J* = 5.9 Hz, 1H), 5.51 (s, 2H), 5.37 (s, 2H), 3.88 (s, 3H), 3.52 - 3.43 (m, 4H), 0.86 (t, *J* = 8.2 Hz, 2H), 0.75 - 0.70 (m, 2H), -0.12 (s, 9H), -0.13 (s, 9H). ¹³C NMR (126 MHz, CDCl₃) δ 165.31, 157.69, 155.72, 142.88, 142.37, 134.80, 134.50, 131.70, 130.78, 128.44, 124.34, 123.42, 120.48, 119.83, 111.74, 110.80, 78.74, 73.76, 67.23, 66.99, 55.85, 17.85, 17.81, -1.51, -1.52 (not all quaternary carbons were observed, neither was one -CH of the pyrimidine). LCMS (Finnigan, 50 → 90%): tᵣ = 5.24 min, m/z: 644.3.

### 2-(m-Tolyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (90)

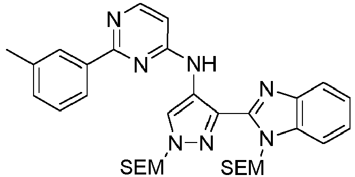

The title compound was synthesized according to general procedure F using m-tolylboronic acid. The crude was purified by silica gel chromatography (20 - 40% Et₂O/pentane) to afford the product (286 mg, 456 µmol, 87%). ¹H NMR (300 MHz, CDCl₃) δ 10.74 (s, 1H), 8.79 (s, 1H), 8.42 (d, *J* = 5.8 Hz, 1H), 8.27 (s, 1H), 8.23 (d, *J* = 7.8 Hz, 1H), 7.87 - 7.78 (m, 1H), 7.66 - 7.57 (m, 1H), 7.44 (t, *J* = 7.6 Hz, 1H), 7.39 - 7.30 (m, 3H), 6.70 (d, *J* = 5.8 Hz, 1H), 6.29 (s, 2H), 5.52 (s, 2H), 3.76 - 3.63 (m, 4H), 2.50 (s, 3H), 1.07 - 0.97 (m, 2H), 0.97 - 0.85 (m, 2H), 0.02 (s, 9H), -0.08 (s, 9H). ¹³C NMR (75 MHz, CDCl₃) δ 164.79, 158.64, 155.16, 146.92, 142.37, 138.70, 137.97, 134.95, 131.53, 131.09, 128.91, 128.41, 125.46, 125.28, 123.52, 123.04, 120.41, 119.21, 110.97, 105.85, 81.37, 73.90, 67.09, 65.74, 21.66, 17.92, 17.84, -1.30, -1.37. LCMS (Finnigan, 70 → 90%): tr = 4.15 min, m/z: 628.4.

### 2-(3-Chlorophenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (91)

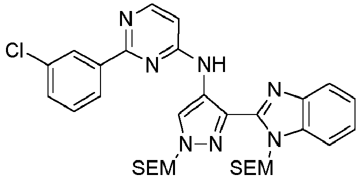

The title compound was synthesized according to general procedure F using 3-chlorophenylboronic acid. The crude was purified by silica gel column chromatography (45% EtOAc/pentane) and used as such in subsequent reaction (yield: 258 mg). LCMS (Finnigan, 70 → 90%): tᵣ = 2.86 min, m/z: 648.3.

### 2-(3-Methoxyphenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (92)

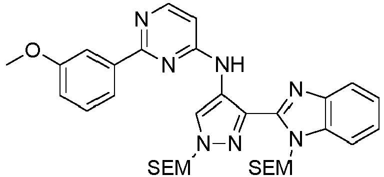

The title compound was synthesized from 77 (200 mg, 349 µmol) and 3-methoxyphenylboronic acid (80.0 mg, 524 µmol) according to general procedure G (reaction time 1.5 h). The crude was purified by automated column chromatography (40 - 60% EtOAc/pentane) to afford the product (184 mg, 286 µmol, 82%).¹H NMR (400 MHz, CDCl₃) δ 9.32 (br s, 1H), 8.39 (d, *J* = 5.8 Hz, 1H), 8.27 (br s, 1H), 8.08 - 8.01 (m, 2H), 7.69 - 7.63 (m, 1H), 7.37 (t, *J* = 7.9 Hz, 1H), 7.35 - 7.29 (m, 1H), 7.28 - 7.19 (m, 2H), 7.03 (dd, *J* = 8.1, 2.6 Hz, 1H), 6.66 (d, *J* = 5.8 Hz, 1H), 5.54 (s, 2H), 5.38 (s, 2H), 3.88 (s, 3H), 3.55 - 3.49 (m, 3H), 3.47 - 3.40 (m, 2H), 0.91 - 0.82 (m, 2H), 0.79 - 0.70 (m, 2H), -0.08 (s, 8H), -0.11 (s, 9H). ¹³C NMR (101 MHz, CDCl₃) δ 164.18, 160.51, 159.74, 156.07, 142.55, 142.25, 139.46, 134.58, 134.44, 129.33, 124.61, 124.28, 123.28, 120.65, 119.26, 117.04, 112.55, 110.89, 78.61, 73.64, 66.99, 66.91, 55.25, 17.70, -1.57 (not all quaternary carbons were observed, neither was one -CH of the pyrimidine). LCMS (Finnigan, 50 → 90%): tᵣ = 5.38 min, m/z: 644.3.

### 2-(3-Isopropylphenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (93)

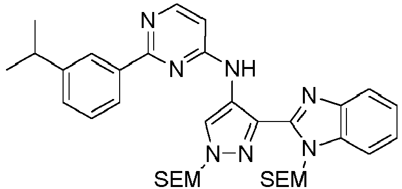

The title compound was synthesized from **77** (170 mg, 297 µmol), 3-isopropylphenylboronic acid (68.0 mg, 445 µmol), K₂CO₃ (165 mg, 1.19 mmol) and Pd(dppf)Cl₂·DCM (17 mg, 21 µmol) according to general procedure F (reaction time: 2 h). The crude was purified by silica gel column chromatography (40 - 60% EtOAc/pentane) to afford the product (89.6 mg, 137 µmol, 46%). ¹H NMR (400 MHz, CDCl₃) δ 10.76 (s, 1H), 8.84 (s, 1H), 8.44 (d, *J* = 5.8 Hz, 1H), 8.33 (t, *J* = 1.6 Hz, 1H), 8.23 (dt, *J* = 7.7, 1.4 Hz, 1H), 7.85 - 7.79 (m, 1H), 7.65 - 7.59 (m, 1H), 7.46 (t, *J* = 7.6 Hz, 1H), 7.40 - 7.33 (m, 3H), 6.75 (d, *J* = 5.9 Hz, 1H), 6.32 (s, 2H), 5.54 (s, 2H), 3.72 - 3.61 (m, 4H), 3.06 (hept, *J* = 6.8 Hz, 1H), 1.37 (d, *J* = 6.9 Hz, 6H), 1.02 - 0.93 (m, 2H), 0.93 - 0.86 (m, 2H), -0.01 (s, 9H), -0.11 (s, 9H). ¹³C NMR (101 MHz, CDCl₃) δ 164.98, 158.76, 155.15, 149.15, 147.03, 142.46, 138.67, 135.07, 131.71, 128.93, 128.65, 126.30, 125.85, 125.56, 123.69, 123.21, 120.65, 119.32, 111.12, 105.95, 81.50, 74.04, 67.17, 66.14, 34.37, 24.25, 18.00, 17.91, -1.24, -1.31. LCMS (Finnigan, 70 →- 90%): tr = 5.70 min, m/z: 656.5.

### 2-(p-Tolyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (95)

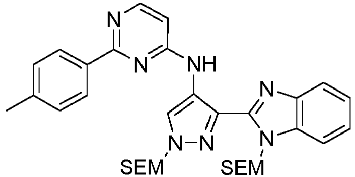

The title compound was synthesized according to general procedure F using p-tolylboronic acid. The crude was purified by silica gel column chromatography (20 - 80% Et₂O/pentane) to afford the product (175 mg, 279 µmol, 53%). ¹H NMR (300 MHz, CDCl₃) δ 10.72 (s, 1H), 8.78 (s, 1H), 8.41 (d, *J* = 5.8 Hz, 1H), 8.35 - 8.29 (m, 2H), 7.86 - 7.79 (m, 1H), 7.66 - 7.58 (m, 1H), 7.41 - 7.30 (m, 4H), 6.69 (d, *J* = 5.8 Hz, 1H), 6.29 (s, 2H), 5.54 (s, 2H), 3.76 - 3.62 (m, 4H), 2.46 (s, 3H), 1.06 - 0.97 (m, 2H), 0.95 - 0.88 (m, 2H), 0.02 (s, 9H), -0.08 (s, 9H). ¹³C NMR (75 MHz, CDCl₃) δ 164.78, 158.69, 155.21, 146.96, 142.41, 140.46, 136.10, 134.99, 131.54, 129.28, 128.14, 125.54, 123.55, 123.08, 120.37, 119.24, 111.01, 105.71, 81.38, 73.94, 67.12, 66.03, 21.55, 17.94, 17.89, -1.27, -1.35. LCMS (Finnigan, 70 →- 90%): tr = 3.97 min, m/z: 628.4.

### 2-(4-Chlorophenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (96)

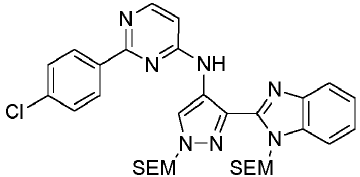

The title compound was synthesized according to general procedure F using 4-chlorophenylboronic acid. The crude was purified by silica gel column chromatography (25 - 35% EtOAc/pentane) and used as such in subsequent reaction (yield: 254 mg). LCMS (Finnigan, 70 → 90%): tᵣ = 2.23 min, m/z: 648.3.

### 2-(4-Methoxyphenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (97)

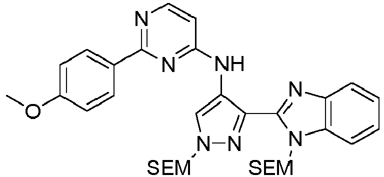

The title compound was synthesized according to general procedure F using 4-methoxyphenylboronic acid. The crude was purified by silica gel column chromatography (20% EtOAc/pentane) to afford the product (258 mg, 401 µmol, 77%). ¹H NMR (400 MHz, CDCl₃) δ 10.70 (s, 1H), 8.76 (s, 1H), 8.41 - 8.35 (m, 3H), 7.85 - 7.79 (m, 1H), 7.64 - 7.59 (m, 1H), 7.39 - 7.32 (m, 2H), 7.06 - 7.02 (m, 2H), 6.68 (d, *J* = 5.8 Hz, 1H), 6.30 (s, 2H), 5.56 (s, 2H), 3.90 (s, 3H), 3.73 - 3.68 (m, 2H), 3.68 - 3.62 (m, 2H), 1.04 - 0.97 (m, 2H), 0.94 - 0.87 (m, 2H), 0.01 (s, 9H), -0.10 (s, 9H). ¹³C NMR (101 MHz, CDCl₃) δ 164.47, 161.62, 158.71, 155.21, 146.99, 142.43, 135.02, 131.57, 131.42, 129.75, 125.62, 123.61, 123.13, 120.30, 119.28, 113.89, 111.05, 105.40, 81.44, 73.98, 67.17, 66.08, 55.46, 17.97, 17.91, -1.24, -1.33. LCMS (Finnigan, 70 →- 90%): tr = 3.43 min, m/z: 644.4.

### 2-(4-Fluorophenyl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (98)

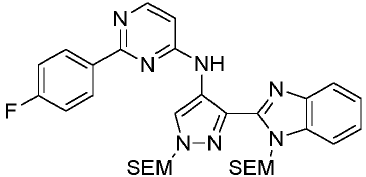

The title compound was synthesized according to general procedure F using 4-fluorophenylboronic acid. The crude was purified by silica gel column chromatography (35% EtOAc/pentane) and used as such in subsequent reaction (yield: 229 mg).

### 2-(Naphthalen-1-yl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (99)

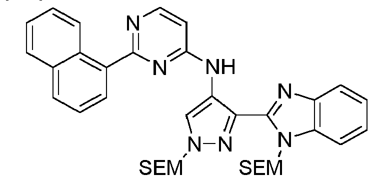

The title compound was synthesized from **77** (125 mg, 218 µmol) and naphthalen-1-boronic acid (43.2 mg, 251 µmol) according to general procedure G (reaction time 2 h). The crude was purified by automated column chromatography (40 - 60% EtOAc/pentane) to afford the product (134 mg, 201 µmol, 92%). ¹H NMR (400 MHz, CDCl₃) δ 8.89 (br s, 1H), 8.73 (d, *J* = 8.1 Hz, 1H), 8.45 (d, *J* = 5.8 Hz, 1H), 8.23 (br s, 1H), 8.04 (d, *J* = 7.1 Hz, 1H), 7.95 (d, *J* = 8.2 Hz, 1H), 7.93 - 7.89 (m, 1H), 7.78 - 7.72 (m, 1H), 7.59 - 7.55 (m, 1H), 7.54 - 7.50 (m, 2H), 7.43 - 7.38 (m, 1H), 7.33 - 7.28 (m, 2H), 6.66 (d, *J* = 5.9 Hz, 1H), 5.57 (s, 2H), 5.39 (s, 2H), 3.56 - 3.46 (m, 4H), 0.92 - 0.86 (m, 2H), 0.80 - 0.74 (m, 2H), -0.07 (s, 9H), -0.14 (s, 9H). ¹³C NMR (101 MHz, CDCl₃) δ 166.86, 160.45, 156.03, 142.81, 142.35, 136.53, 134.79, 134.53, 134.02, 131.04, 130.02, 128.70, 128.30, 126.49, 126.21, 125.72, 125.10, 124.74, 124.33, 123.37, 119.76, 110.72, 78.76, 73.69, 67.27, 67.00, 17.78, 17.76, -1.53, -1.59 (not all quaternary carbons were observed, neither was one -CH of the pyrimidine). LCMS (Finnigan, 50 → 90%): tᵣ = 5.46 min, m/z: 664.3.

### 2-(1H-indol-7-yl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (100)

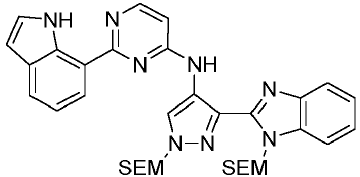

The title compound was synthesized from **77** (125 mg, 218 µmol) and indole-7-boronic acid pinacol ester (61.1 mg, 251 µmol) according to general procedure G (reaction time 16 h). The crude was purified by automated column chromatography (30 - 50% EtOAc/pentane) to afford the product (111 mg, 169 µmol, 78%). ¹H NMR (400 MHz, CDCl₃) δ 11.22 (s, 1H), 9.44 (br s, 1H), 8.46 (d, *J* = 5.9 Hz, 1H), 8.44 (dd, *J* = 7.7, 1.0 Hz, 1H), 8.14 (br s, 1H), 7.80 (d, *J* = 7.7 Hz, 1H), 7.66 - 7.60 (m, 1H), 7.33 - 7.28 (m, 1H), 7.26 - 7.17 (m, 3H), 7.09 (br s, 1H), 6.65 (d, *J* = 5.9 Hz, 1H), 6.56 (t, *J* = 2.7 Hz, 1H), 5.57 (s, 2H), 5.36 (s, 2H), 3.56 - 3.47 (m, 2H), 3.40 (dd, *J* = 8.8, 7.4 Hz, 2H), 0.84 - 0.78 (m, 2H), 0.76 - 0.70 (m, 2H), -0.10 (s, 9H), -0.15 (s, 9H). ¹³C NMR (101 MHz, CDCl₃) δ 165.23, 160.43, 156.00, 142.46, 142.23, 135.32, 134.77, 134.42, 129.17, 124.49, 124.46, 123.90, 123.43, 122.55, 120.19, 119.35, 119.32, 111.00, 102.01, 78.81, 73.76, 67.07, 67.04, 17.76, 17.72, -1.53, -1.56 (not all quaternary carbons were observed). LCMS (Finnigan, 10 → 90%): tᵣ = 9.13 min, m/z: 653.3.

### 2-(1H-Indazol-4-yl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (101)

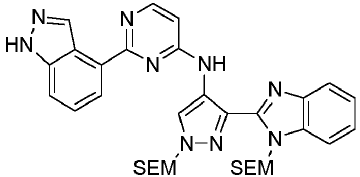

The title compound was synthesized from **77** (130 mg, 227 µmol) and indazole-4-boronic acid hydrochloride (63.1 mg, 318 µmol) according to general procedure G (reaction time 16 h). The crude was purified by automated column chromatography (40 - 100% EtOAc/pentane) to afford the product (101 mg, 155 µmol, 68%). ¹H NMR (500 MHz, CDCl₃) δ 9.00 (s, 1H), 8.89 (br s, 1H), 8.44 (d, *J* = 5.9 Hz, 1H), 8.29 - 8.12 (m, 2H), 7.76 - 7.72 (m, 1H), 7.54 (d, *J* = 8.3 Hz, 1H), 7.44 - 7.39 (m, 2H), 7.29 - 7.23 (m, 2H), 6.60 (d, *J* = 5.9 Hz, 1H), 5.57 (s, 2H), 5.42 (s, 2H), 3.60 - 3.54 (m, 2H), 3.51 - 3.44 (m, 2H), 0.88 - 0.82 (m, 2H), 0.80 - 0.75 (m, 2H), -0.10 (s, 9H), -0.17 (s, 9H) (the -NH of the benzimidazole was not observed). ¹³C NMR (126 MHz, CDCl₃) δ 164.66, 160.96 (br), 156.32 (br), 142.80, 142.27, 141.00, 136.36, 135.37, 134.84, 131.55, 126.37, 124.87, 124.43, 123.52, 122.09, 121.61, 119.88, 112.37, 110.81, 103.04 (br), 78.96, 73.83, 67.29, 67.17, 17.84, -1.48, -1.54. LCMS (Finnigan, 50 →- 90%): tr = 4.10 min, m/z: 654.3.

### 2-(Naphthalen-2-yl)-N-(1-((2-(trimethylsilyl)ethoxy)methyl)-3-(1-((2-(trimethylsilyl)ethoxy)methyl)-1H-benzo[d]imidazol-2-yl)-1H-pyrazol-4-yl)pyrimidin-4-amine (102)

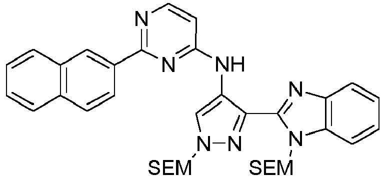

The title compound was synthesized from **77** (200 mg, 349 µmol), 2-naphthylboronic acid (90.1 mg, 524 µmol), K₂CO₃ (193 mg, 1.40 mmol) and Pd(dppf)Cl₂·DCM (20 mg, 24 µmol) according to general procedure F (reaction time: 2 h). The crude was purified by silica gel column chromatography (40 - 60% EtOAc/pentane) to afford the product (100 mg, 150 µmol, 43%). ¹H NMR (500 MHz, CDCl₃) δ 10.81 (s, 1H), 8.96 (s, 1H), 8.89 (s, 1H), 8.51 (dd, *J* = 8.6, 1.5 Hz, 1H), 8.48 (d, *J* = 5.8 Hz, 1H), 8.03 - 8.00 (m, 1H), 7.99 (d, *J* = 8.6 Hz, 1H), 7.93 - 7.88 (m, 1H), 7.87 - 7.80 (m, 1H), 7.65 - 7.60 (m, 1H), 7.57 - 7.51 (m, 2H), 7.39 - 7.34 (m, 2H), 6.78 (d, *J* = 5.8 Hz, 1H), 6.32 (s, 2H), 5.59 (s, 2H), 3.76 - 3.70 (m, 2H), 3.68 - 3.62 (m, 2H), 1.04 - 0.97 (m, 2H), 0.94 - 0.87 (m, 2H), -0.01 (s, 9H), -0.10 (s, 9H). ¹³C NMR (126 MHz, CDCl₃) δ 164.76, 158.89, 155.20, 147.02, 142.48, 136.03, 135.09, 134.70, 133.43, 131.76, 129.30, 128.56, 128.24, 127.84, 127.12, 126.36, 125.55, 125.33, 123.70, 123.22, 120.61, 119.33, 111.12, 106.07, 81.57, 77.41, 77.16, 76.91, 74.06, 67.28, 66.16, 18.02, 17.98, -1.23, -1.30. LCMS (Finnigan, 70 →- 90%): tr = 5.14 min, m/z: 664.4.

### Ethyl 4-nitro-1H-pyrazole-3-carboxylate (104)

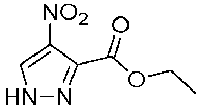

4-Nitro-1H-pyrazole-3-carboxylic acid (10.0 g, 63.7 mmol) was suspended in dry EtOH (160 mL) and cooled down to 0°C. SOCl₂ (6.0 mL, 83 mmol) was added dropwise, after which the mixture was allowed to warm to RT and stirred for 16 h. The mixture was concentrated and coevaporated with toluene (2x20 mL). The resulting solids were suspended in pentane and filtered to afford the product (11.3 g, 63.7 mmol, 96%). ¹H NMR (500 MHz, DMSO) δ 14.42 (br s, 1H), 8.95 (br s, 1H), 4.35 (q, *J* = 7.0 Hz, 2H), 1.29 (t, *J* = 7.1 Hz, 3H). ¹³C NMR (126 MHz, DMSO) δ 160.81, 138.53, 133.14, 130.81, 61.83, 13.83.

### Ethyl 4-nitro-1-((2-(trimethylsilyl)ethoxy)methyl)-1H-pyrazole-3-carboxylate (105) + regioisomer

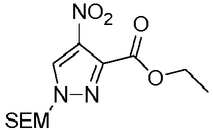

**104** (10.0 g, 54.0 mmol) was suspended in DCM (270 mL). DIPEA (19.8 mL, 113 mmol) was added after which the mixture was cooled down to 0°C. SEM-Cl (10.5 mL, 59.4 mmol) was added dropwise after which the mixture was stirred at 0°C for 10 min. The reaction was allowed to warm to RT and continued to stir for 3.5 h. The mixture was concentrated, coevaporated with toluene (2x20 mL), dissolved in DCM (200 mL) and poured into H₂O (250 mL). The organic layer was separated and the water layer extracted with DCM (200 mL). The combined organic layers were washed with brine (300 mL) and the layers were separated. The brine layer was extracted with DCM (100 mL) after which the combined organic layers were dried over Na₂SO₄, filtered and concentrated. The crude was purified by silica gel column chromatography (20 - 50% Et₂O/pentane) to afford both regioisomers in pure form (total yield: 11.1 g, 35.4 mmol, 66%). NMR data (sorted on descending isomer lipophilicity): Regioisomer 1 - yield (1.19 g, 3.76 mmol, 7%). ¹H NMR (500 MHz, CDCl₃) δ 7.98 (s, 1H), 5.55 (s, 2H), 4.43 (q, *J* = 7.2 Hz, 2H), 3.53 - 3.48 (m, 2H), 1.35 (t, *J* = 7.1 Hz, 3H), 0.86 - 0.79 (m, 2H), -0.09 (s, 9H). ¹³C NMR (126 MHz, CDCl₃) δ 158.03, 135.05, 131.82, 80.70, 67.70, 63.37, 17.58, 13.71, -1.57. Regioisomer 2 - yield (9.96 g, 31.6 mmol, 59%). ¹H NMR (500 MHz, CDCl₃) δ 8.32 (s, 1H), 5.45 (s, 2H), 4.42 (q, *J* = 7.1 Hz, 2H), 3.65 - 3.57 (m, 2H), 1.36 (t, *J* = 7.1 Hz, 3H), 0.94 - 0.87 (m, 2H), -0.04 (s, 9H). ¹³C NMR (126 MHz, CDCl₃) δ 160.14, 138.98, 134.89, 129.80, 82.02, 68.33, 62.54, 17.80, 13.99, -1.46.

### Ethyl 4-amino-1-((2-(trimethylsilyl)ethoxy)methyl)-1H-pyrazole-3-carboxylate (106)

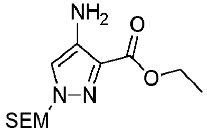

**105** (4.00 g, 12.7 mmol) was dissolved in degassed EtOH (140 mL). 10% Pd/C (400 mg) was added and the atmosphere was exchanged for H₂. The reaction was vigorously stirred for 5 h while bubbling H₂ through the mixture. The atmosphere was exchanged for N₂, the mixture was filtered over Celite and concentrated to afford the product (3.57 g, 12.5 mmol, 99%). ¹H NMR (500 MHz, CDCl₃) δ 7.09 (s, 1H), 5.30 (s, 2H), 4.35 (q, *J* = 7.1 Hz, 2H), 4.09 (br s, 2H), 3.50 - 3.44 (m, 2H), 1.34 (t, *J* = 7.1 Hz, 3H), 0.86 - 0.80 (m, 2H), -0.09 (s, 9H). ¹³C NMR (126 MHz, CDCl₃) δ 163.71, 135.54, 130.56, 115.82, 81.43, 66.89, 60.59, 17.79, 14.48, -1.46. LCMS (Finnigan, 10 →- 90%): tr = 5.88 min, m/z: 286.0.

### 4-((2-(4-Nitro-1-((2-(trimethylsilyl)ethoxy)methyl)-1H-pyrazol-3-yl)-1H-benzo[d]imidazol-5-yl)methyl)morpholine (114)

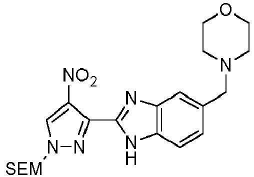

**105** (2.00 g, 6.34 mmol) was suspended in a mixture of MeOH and H₂O (1:1, 12 mL), LiOH·H₂O (1.06 g, 25.3 mmol) was added and the mixture was stirred vigorously for 1.5 h. The mixture was poured into a mixture of H₂O (60 mL) and DCM (75 mL). The pH of the water layer was adjusted by addition of 2 M HCl (aq.) to pH ~2. The organic layer was separated and the water layer extracted with DCM (75 mL). The combined organic layers were dried over Na₂SO₄, filtered and concentrated to yield the free acid intermediate. The intermediate was dissolved in DMF (10 mL), after which 4-(morpholinomethyl)benzene-1,2-diamine (1.45 g, 6.98 mmol), EDC·HCl (1.22 g, 6.34 mmol) and HOBt (857 mg, 6.34 mmol) were added and the mixture was stirred for 1 h. The mixture was concentrated after which AcOH (7 mL) was added and the mixture was stirred at 118°C for 30 min. The mixture was cooled down to RT, 10 M NaOH (aq.) was added until the pH was about 8. The mixture was poured into a mixture of H₂O (60 mL) and DCM (70 mL) after which the organic layer was separated. The water layer was extracted with DCM (70 mL) and the combined organic layers were dried over Na₂SO₄, filtered and concentrated. The crude was purified by automated column chromatography twice (0 - 10% MeOH/EtOAc) to afford the product (1.69 g, 3.69 mmol, 58%). ¹H NMR (400 MHz, CDCl₃) δ 11.42 (s, 1H), 8.51 (s, 1H), 7.85 - 7.66 (br m, 1H), 7.60 - 7.44 (br m, 1H), 7.35 - 7.22 (br m, 1H), 5.53 (s, 2H), 3.70 - 3.62 (m, 6H), 3.60 (s, 2H), 2.49 - 2.38 (m, 4H), 0.97 - 0.87 (m, 2H), -0.06 (s, 9H). ¹³C NMR (101 MHz, CDCl₃) δ 143.44 (br), 142.72 (br), 142.17, 136.67, 134.34 (br), 134.08, 133.41 (br), 132.80 (br), 132.56 (br), 131.56, 125.91 (br), 124.87 (br), 120.89 (br), 120.09 (br), 111.96 (br), 111.42 (br), 82.43, 68.65, 66.97, 63.60, 53.55, 17.98, -1.42. LCMS (Finnigan, 10 → 90%): tr = 5.57 min, m/z: 459.1.

### 3-(5-(Morpholinomethyl)-1H-benzo[d]imidazol-2-yl)-1-((2-(trimethylsilyl)ethoxy)methyl)-1H-pyrazol-4-amine (115)

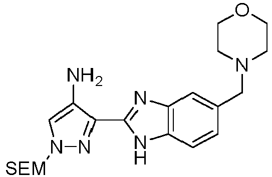

**114** (1.17 g, 2.56 mmol) was dissolved in degassed MeOH (30 mL). 10% Pd/C (120 mg) was added and the atmosphere was exchanged for H₂. The reaction was vigorously stirred for 30 min while bubbling H₂ through the mixture. The atmosphere was exchanged for N₂, the mixture was filtered over Celite and concentrated to afford the product (1.07 g, 2.49 mmol, 97%). ¹H NMR (400 MHz, CDCl₃) δ 7.55 (br s, 1H), 7.50 (br s, 1H), 7.21 (dd, *J* = 8.2, 1.6 Hz, 1H), 7.18 (s, 1H), 5.29 (s, 2H), 4.18 (s, 2H), 3.76 - 3.72 (m, 4H), 3.67 (s, 2H), 3.55 - 3.49 (m, 2H), 2.58 - 2.50 (m, 4H), 0.91 - 0.85 (m, 2H), -0.06 (s, 9H) (the -NH of the benzimidazole was not observed). ¹³C NMR (101 MHz, CDCl₃) δ 147.94, 131.91, 130.54 (br), 124.38 (br), 117.17, 80.90, 66.83, 66.56, 63.56, 53.29, 17.83, -1.35 (not all quaternary carbons were observed, neither were two -CH's of the benzimidazole). LCMS (Finnigan, 10 → 90%): tᵣ = 4.85 min, m/z: 429.2.

### 2-Chloro-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (116)

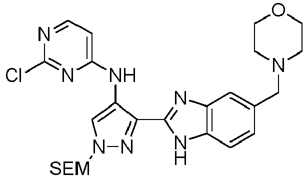

**115** (445 mg, 1.04 mmol) was dissolved in EtOH (1 mL). DIPEA (0.54 mL, 3.1 mmol) and 2,4-dichloropyrimidine (232 mg, 1.56 mmol) were added and the mixture was stirred for 4 days. The mixture was poured into H₂O (75 mL) and the product extracted with DCM (2x75 mL). The combined organic layers were dried over Na₂SO₄, filtered and concentrated. The crude was purified by automated column chromatography to afford the product (371 mg, 685 µmol, 66%). ¹H NMR (400 MHz, CDCl₃) δ 10.79 - 10.63 (2x s, 1H), 10.39 - 10.30 (2x s, 1H), 8.51 - 8.47 (2x s, 1H), 8.19 - 8.15 (2x d, *J* = 5.8 Hz, 1H), 7.74 - 7.67 (m, 1H), 7.44 - 7.36 (m, 1H), 7.30 - 7.22 (2x dd, *J* = 8.3, 1.3 Hz, 1H), 6.73 - 6.68 (2x d, *J* = 5.6 Hz, 1H), 5.44 (s, 2H), 3.76 - 3.70 (m, 4H), 3.66 - 3.57 (m, 4H), 2.56 - 2.45 (m, 4H), 0.97 - 0.90 (m, 2H), -0.02 - -0.04 (2x s, 9H). ¹³C NMR (101 MHz, CDCl₃) δ 160.90, 160.88, 159.55, 156.46, 147.11, 146.93, 143.26, 142.55, 133.59, 132.91, 132.23, 132.04, 131.66, 131.62, 125.28, 124.24, 123.00, 122.96, 121.91, 119.80, 118.77, 111.54, 110.74, 106.09, 81.09, 81.07, 67.11, 67.10, 67.07, 63.84, 63.77, 53.72, 53.68, 17.81, -1.33. LCMS (Fleet, 10 →- 90%): tr = 5.56 min, m/z: 541.2.

### 2-(3-Chlorophenyl)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (117)

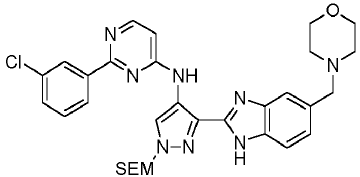

The title compound was synthesized from **116** (125 mg, 231 µmol) and 3-chlorophenylboronic acid (36.8 mg, 236 µmol) according to general procedure G (reaction time 16 h). The crude was purified by automated column chromatography (0 - 10% MeOH/EtOAc) to afford the product (87.3 mg, 141 µmol, 61%). ¹H NMR (500 MHz, CDCl₃) δ 10.96 - 10.79 (2x s, 1H), 10.14 - 10.06 (2x s, 1H), 8.58 (s, 1H), 8.41 - 8.37 (2x d, *J* = 5.9 Hz, 1H), 8.37 (s, 1H), 8.25 (d, *J* = 7.5 Hz, 1H), 7.76 - 7.71 (m, 1H), 7.45 - 7.42 (m, 1H), 7.40 (t, *J =* 7.7 Hz, 1H), 7.37 - 7.31 (m, 1H), 7.27 - 7.23 (m, 1H), 6.70 (d, *J* = 5.8 Hz, 1H), 5.43 (s, 2H), 3.79 - 3.70 (m, 4H), 3.68 - 3.60 (m, 4H), 2.57 - 2.48 (m, 4H), 0.98 - 0.93 (m, 2H), -0.05 (s, 9H). ¹³C NMR (126 MHz, CDCl₃) δ 163.40, 158.67, 155.27, 147.26, 147.10, 143.42, 142.74, 140.50, 134.53, 133.18, 133.00, 132.17, 131.89, 131.64, 130.26, 129.77, 128.39, 126.21, 125.18, 124.20, 123.80, 121.18, 119.93, 118.79, 111.56, 110.64, 106.21, 81.12, 67.16, 67.00, 63.77, 63.73, 53.64, 17.81, -1.35. LCMS (Fleet, 10 →- 90%): tr = 5.44 min, m/z: 617.1.

### N-(3-(5-(Morpholinomethyl)-1H-benzo[d]imidazol-2-yl)-1-((2-(trimethylsilyl)ethoxy)methyl)-1H-pyrazol-4-yl)-2-(3-((trimethylsilyl)ethynyl)phenyl)pyrimidin-4-amine (118)

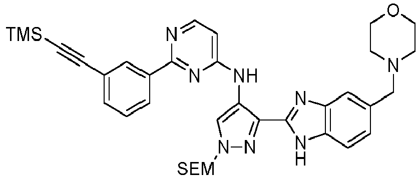

A microwave vial was charged with **116** (125 mg, 231 µmol), K₂CO₃ (128 mg, 924 µmol), 3-[(trimethylsilyl)ethynyl]phenylboronic acid pinacol ester (74.2 mg, 247 µmol) and 4:1 dioxane/H₂O (1.2 mL). N₂ was bubbled through the mixture for 1 min after which Pd(dppf)Cl₂·DCM (13.2 mg, 16.2 µmol) was added. N₂ was bubbled through the mixture for 30 sec after which the vial was sealed. The mixture was heated to 90°C and stirred for 16 h. Then, extra K₂CO₃ (64.0 mg, 462 µmol), 3-[(trimethylsilyl)ethynyl]phenylboronic acid pinacol ester (37.1 mg, 124 µmol) and Pd(dppf)Cl₂·DCM (6.7 mg, 8.0 µmol) were added, N₂ was bubbled through the mixture for 30 sec after which the vial was sealed and stirred at 90°C for another 8 h. The mixture was poured into H₂O (20 mL). The product was extracted with DCM (2x20 mL) and the combined organic layers were concentrated as such. The crude was purified by automated column chromatography (0 - 10% MeOH/EtOAc) to afford the product (44 mg, 65 µmol, 28%). ¹H NMR (400 MHz, CDCl₃) δ 10.87 - 10.70 (2x s, 1H), 10.13 - 10.06 (2x s, 1H), 8.65 (s, 1H), 8.52 (s, 1H), 8.44 - 8.39 (2x d, *J* = 5.5 Hz, 1H), 8.33 (d, *J =* 7.6 Hz, 1H), 7.77 - 7.71 (m, 1H), 7.58 (dt, *J* = 7.7, 1.4 Hz, 1H), 7.43 (t, *J* = 7.8 Hz, 1H), 7.41 - 7.35 (m, 1H), 7.26 (dd, *J* = 8.3, 1.3 Hz, 1H), 6.73 (d, *J* = 5.8 Hz, 1H), 5.48 - 5.45 (2x s, 2H), 3.78 - 3.70 (m, 4H), 3.68 - 3.59 (m, 4H), 2.57 - 2.47 (m, 4H), 0.97 - 0.91 (m, 2H), 0.28 (s, 9H), -0.06 (s, 9H). ¹³C NMR (101 MHz, CDCl₃) δ 163.88, 158.72, 155.34, 147.28, 147.13, 143.42, 142.75, 138.69, 133.61, 133.20, 133.01, 132.25, 132.16, 132.00, 131.90, 131.66, 128.52, 128.16, 125.24, 124.86, 124.25, 123.87, 123.48, 121.37, 119.96, 118.82, 111.64, 110.71, 106.12, 105.13, 94.37, 81.07, 67.05, 63.82, 63.78, 53.67, 53.64, 17.82, 0.12, -1.34. LCMS (Fleet, 10 → 90%): tr = 6.60 min, m/z: 679.2.

### N-(3-(5-(Morpholinomethyl)-1H-benzo[d]imidazol-2-yl)-1-((2-(trimethylsilyl)ethoxy)methyl)-1H-pyrazol-4-yl)-2-(3-(trifluoromethyl)phenyl)pyrimidin-4-amine (119)

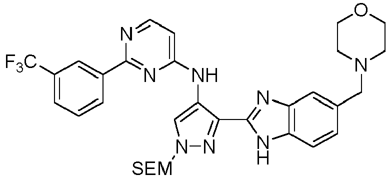

The title compound was synthesized from **116** (125 mg, 231 µmol) and (3-(trifluoromethyl)phenyl)boronic acid (46.9 mg, 247 µmol) according to general procedure G (reaction time 16 h). The crude was purified by automated column chromatography (0 - 10% MeOH/EtOAc) to afford the product (112 mg, 173 µmol, 75%). ¹H NMR (400 MHz, CDCl₃) δ 11.12 - 10.91 (2x s, 1H), 10.16 (s, 1H), 8.67 (s, 1H), 8.58 (s, 1H), 8.55 (d, *J* = 7.8 Hz, 1H), 8.41 (d, *J* = 5.8 Hz, 1H), 7.74 (br s, 1H), 7.72 (d, *J* = 7.7 Hz, 1H), 7.58 (t, *J* = 7.8 Hz, 1H), 7.41 - 7.31 (m, 1H), 7.25 (dd, *J* = 8.1, 1.8 Hz, 1H), 6.72 (d, *J* = 5.8 Hz, 1H), 5.41 (s, 2H), 3.79 - 3.71 (m, 4H), 3.69 - 3.58 (m, 4H), 2.60 - 2.49 (m, 4H), 0.97 - 0.90 (m, 2H), - 0.06 (s, 9H). ¹³C NMR (101 MHz, CDCl₃) δ 163.16, 158.64, 155.29, 147.17 (br), 147.08 (br), 143.35 (br), 142.71 (br), 139.34, 133.06 (br), 132.89 (br), 132.26 (br), 131.72, 131.57 (br), 131.27, 130.95, 130.63, 129.08, 126.85 (br), 126.81 (br), 125.70, 125.27 (br), 125.08 (br), 125.04 (br), 125.00 (br), 124.97 (br), 124.28 (br), 123.71, 122.99, 121.31, 120.00 (br), 118.77 (br), 111.75 (br), 110.74 (br), 106.39, 81.08, 67.13, 66.90, 63.67, 53.53, 17.74, -1.44. LCMS (Fleet, 10 → 90%): tr = 5.85 min, m/z: 651.1.

### 3-(4-((3-(5-(Morpholinomethyl)-1H-benzo[d]imidazol-2-yl)-1-((2-(trimethylsilyl)ethoxy)methyl)-1H-pyrazol-4-yl)amino)pyrimidin-2-yl)benzonitrile (120)

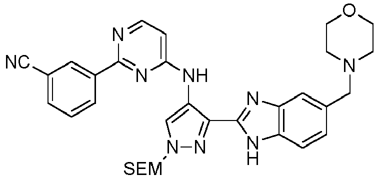

A microwave vial was charged with **116** (125 mg, 231 µmol), K₂CO₃ (128 mg, 924 µmol), (3-cyanophenyl)boronic acid (37.3 mg, 254 µmol) and 4:1 dioxane/H₂O (1.2 mL). N₂ was bubbled through the mixture for 1 min after which Pd(dppf)Cl₂·DCM (13.2 mg, 16.2 µmol) was added. N₂ was bubbled through the mixture for 30 sec after which the vial was sealed. The mixture was heated to 90°C and stirred for 5 h. Then, extra K₂CO₃ (60.0 mg, 434 µmol), (3-cyanophenyl)boronic acid (15.0 mg, 102 µmol) and Pd(dppf)Cl₂·DCM (6.0 mg, 7.3 µmol) were added, N₂ was bubbled through the mixture for 30 sec after which the vial was sealed and stirred at 90°C for another 4.5 h. The mixture was poured into H₂O (20 mL) and brine (1 mL). The product was extracted with 5% MeOH/DCM (20 mL) and DCM (3x20 mL) and the combined organic layers were concentrated as such. The crude was purified by automated column chromatography (0 - 15% MeOH/EtOAc) to afford the product (101 mg, 167 µmol, 72%). ¹H NMR (400 MHz, CDCl₃) δ 10.80 - 10.63 (2x s, 1H), 10.18 - 10.11 (2x s, 1H), 8.66 - 8.62 (2x t, *J* = 1.7 Hz, 1H), 8.59 - 8.54 (2x dt, *J* = 8.1, 1.5 Hz, 1H), 8.51 - 8.48 (2x s, 1H), 8.41 - 8.36 (2x d, *J* = 5.8 Hz, 1H), 7.75 - 7.68 (m, 2H), 7.55 (t, *J* = 7.8 Hz, 1H), 7.35 - 7.30 (m, 1H), 7.27 - 7.23 (m, 1H), 6.73 (d, *J* = 5.8 Hz, 1H), 5.47 - 5.42 (2x s, 2H), 3.79 - 3.70 (m, 4H), 3.67 - 3.59 (m, 4H), 2.58 - 2.46 (m, 4H), 0.94 (t, *J* = 8.2 Hz, 2H), -0.06 (s, 9H). ¹³C NMR (101 MHz, CDCl₃) δ 162.50, 162.47, 158.68, 155.25, 147.18, 147.00, 143.34, 142.62, 139.76, 133.36, 132.86, 132.10, 132.01, 131.90, 131.59, 131.57, 129.30, 125.17, 124.18, 123.67, 123.61, 120.80, 119.84, 118.84, 118.75, 112.63, 111.42, 110.56, 106.60, 81.17, 67.14, 67.05, 67.00, 63.79, 63.74, 53.68, 53.64, 17.76, -1.38. LCMS (Fleet, 10 →- 90%): tᵣ = 5.24 min, m/z: 608.3.

### 2-(3-Chloro-2-fluorophenyl)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (121)

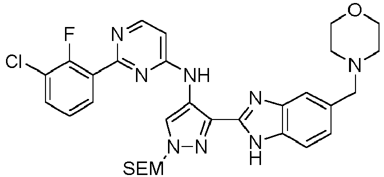

The title compound was synthesized from **116** (115 mg, 213 µmol) and (3-chloro-2-fluorophenyl)boronic acid (37.8 mg, 217 µmol) according to general procedure G (reaction time 16 h). The crude was purified by automated column chromatography (0 - 8% MeOH/EtOAc) to afford the product (104 mg, 164 µmol, 77%). ¹H NMR (400 MHz, CDCl₃) δ 10.90 - 10.75 (m, 1H), 10.16 - 10.08 (2x s, 1H), 8.72 - 8.69 (2x s, 1H), 8.46 - 8.41 (2x d, *J* = 5.9 Hz, 1H), 8.05 - 7.99 (m, 1H), 7.77 - 7.70 (m, 1H), 7.52 - 7.46 (m, 1H), 7.33 - 7.28 (m, 1H), 7.27 - 7.22 (m, 1H), 7.20 - 7.14 (m, 1H), 6.76 (dd, *J* = 5.9, 2.5 Hz, 1H), 5.44 - 5.41 (2x s, 2H), 3.77 - 3.70 (m, 4H), 3.67 - 3.58 (m, 4H), 2.56 - 2.45 (m, 4H), 0.98 - 0.91 (m, 2H), -0.06 - -0.07 (2x s, 9H). ¹³C NMR (101 MHz, CDCl₃) δ 162.09, 162.06, 162.04, 162.02, 158.35, 157.95, 155.40, 155.21, 155.18, 147.36, 147.20, 143.40, 142.70, 133.32, 132.95, 132.09, 132.05, 131.84, 131.70, 131.66, 130.29, 130.28, 128.75, 128.66, 125.10, 124.42, 124.38, 124.12, 123.57, 123.52, 122.49, 122.31, 122.01, 121.96, 119.82, 118.75, 111.46, 110.61, 106.12, 81.05, 67.09, 67.04, 63.85, 63.79, 53.70, 53.67, 17.74, -1.40. LCMS (Fleet, 10 →- 90%): tr = 5.37 min, m/z: 635.3.

### 2-(2-Fluoro-3-((trimethylsilyl)ethynyl)phenyl)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (122)

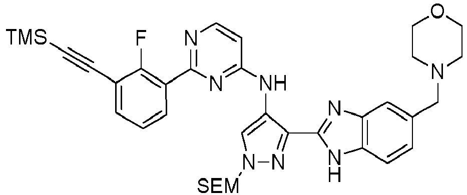

The title compound was synthesized from **121** (42 mg, 66 µmol) according to general procedure H. The crude was purified by automated column chromatography (0 - 8% MeOH/EtOAc) to afford the product (37.4 mg, 53.7 µmol, 81%). ¹H NMR (500 MHz, CDCl₃) δ 10.90 - 10.76 (2x s, 1H), 10.14 - 10.05 (2x s, 1H), 8.75 (s, 1H), 8.47 - 8.43 (2x d, *J* = 6.3 Hz, 1H), 8.12 - 8.06 (2x d, *J* = 7.3 Hz, 1H), 7.78 - 7.70 (m, 1H), 7.55 (ddd, *J* = 7.8, 6.3, 1.6 Hz, 1H), 7.36 - 7.29 (m, 1H), 7.27 - 7.23 (m, 1H), 7.18 (t, *J* = 7.7 Hz, 1H), 6.75 (d, *J =* 5.9 Hz, 1H), 5.43 (s, 2H), 3.78 - 3.71 (m, 4H), 3.69 - 3.57 (m, 4H), 2.61 - 2.45 (m, 4H), 0.96 - 0.87 (m, 2H), 0.29 (s, 9H), -0.08 (s, 9H). ¹³C NMR (126 MHz, CDCl₃) δ 162.61, 162.31, 162.28, 160.54, 158.35, 155.21, 147.42, 147.27, 143.43, 142.78, 135.09, 132.98, 132.16, 131.70, 127.36, 127.29, 125.13, 124.17, 123.78, 123.74, 123.59, 122.20, 119.92, 118.78, 113.31, 113.17, 111.58, 110.67, 105.95, 100.41, 100.38, 98.16, 80.95, 66.96, 66.92, 63.73, 53.60, 17.70, 0.00, -1.38. LCMS (Fleet, 10 →- 90%): tr = 6.51 min, m/z: 697.3.

### 2-(5-Chloro-2-fluorophenyl)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (123)

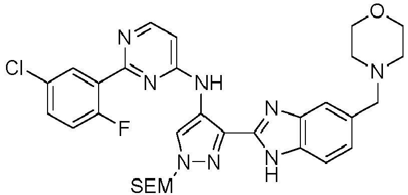

The title compound was synthesized from 116 (100 mg, 185 µmol) and (5-chloro-2-fluorophenyl)boronic acid (33.2 mg, 190 µmol) according to general procedure G (reaction time 16 h). The crude was purified by automated column chromatography (0 - 6% MeOH/EtOAc) to afford the product (65.5 mg, 103 µmol, 56%). ¹H NMR (400 MHz, CDCl₃) δ 10.76 - 10.61 (2x s, 1H), 10.17 - 10.08 (2x s, 1H), 8.71 - 8.68 (2x s, 1H), 8.45 - 8.41 (2x d, *J* = 6.2 Hz, 1H), 8.19 - 8.15 (2x dd, *J* = 6.7, 2.6 Hz, 1H), 7.76 - 7.71 (m, 1H), 7.40 - 7.35 (2x ddd, *J* = 8.6, 3.9, 2.7 Hz, 1H), 7.35 - 7.29 (m, 1H), 7.28 - 7.22 (2x dd, *J* = 8.1, 1.6 Hz, 1H), 7.16 - 7.10 (2x dd, *J* = 10.1, 8.8 Hz, 1H), 6.76 - 6.74 (2x d, *J* = 5.9 Hz, 1H), 5.45 - 5.42 (2x s, 2H), 3.79 - 3.69 (m, 4H), 3.67 - 3.57 (m, 4H), 2.57 - 2.45 (m, 4H), 0.97 - 0.90 (m, 2H), -0.05 - -0.07 (2x s, 9H). ¹³C NMR (101 MHz, CDCl₃) δ 161.64, 161.61, 161.59, 161.56, 161.04, 158.52, 158.37, 155.20, 155.16, 147.36, 147.19, 143.42, 142.72, 133.39, 132.92, 132.10, 132.06, 131.70, 131.68, 131.63, 131.60, 131.20, 131.11, 129.37, 129.33, 128.42, 128.32, 125.12, 124.15, 123.64, 123.59, 121.89, 121.84, 121.82, 119.85, 118.79, 118.47, 118.22, 111.42, 110.59, 106.18, 106.15, 81.11, 67.10, 67.08, 67.06, 63.86, 63.79, 53.72, 53.69, 17.79, -1.38. LCMS (Fleet, 10 →- 90%): tr = 5.41 min, m/z: 635.2.

### 2-(2-Chlorophenoxy)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (124)

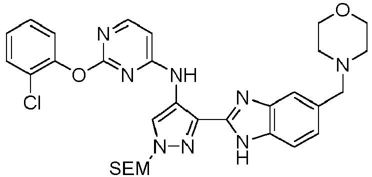

The title compound was synthesized according to general procedure I using 2-chlorophenol to afford the product (95.5 mg, 151 µmol, 91%). ¹H NMR (500 MHz, CDCl₃) δ 11.37 - 11.31 (2x s, 1H), 10.26 - 10.20 (2x s, *J* = 9.8 Hz, 1H), 8.33 - 8.30 (2x d, *J* = 5.8 Hz, 1H), 7.73 - 7.67 (m, 1H), 7.54 (dd, *J* = 8.0, 1.6 Hz, 1H), 7.47 - 7.33 (m, 3H), 7.30 - 7.24 (m, 2H), 7.24 - 7.19 (m, 1H), 6.58 - 6.52 (2x d, *J* = 5.4 Hz, 1H), 5.11 (s, 2H), 3.74 - 3.64 (m, 4H), 3.63 - 3.55 (2x s, 2H), 3.44 (t, *J* = 8.1 Hz, 2H), 2.56 - 2.38 (m, 4H), 0.92 - 0.83 (m, 2H), -0.03 - - 0.05 (2x s, 9H). ¹³C NMR (126 MHz, CDCl₃) δ 164.93, 160.03, 157.30, 157.28, 149.75, 147.21, 147.08, 143.26, 142.58, 133.11, 133.04, 132.25, 131.78, 131.70, 131.63, 130.54, 128.34, 128.01, 126.36, 125.00, 124.80, 124.01, 123.09, 123.04, 121.57, 119.69, 118.60, 111.83, 110.82, 102.52, 80.72, 66.99, 66.94, 66.71, 66.69, 63.79, 63.73, 53.59, 53.55, 17.77, -1.34, -1.37. LCMS (Fleet, 10 →- 90%): tr = 5.78 min, m/z: 633.2.

### 2-(3-Chlorophenoxy)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (125)

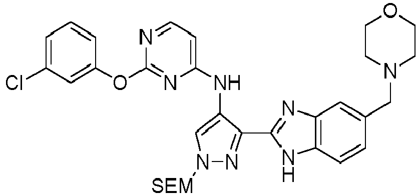

The title compound was synthesized according to general procedure I using 3-chlorophenol to afford the product (85.8 mg, 135 µmol, 81%). ¹H NMR (500 MHz, CDCl₃) δ 11.21 - 11.10 (m, 1H), 10.28 - 10.19 (m, 1H), 8.30 - 8.24 (2x d, *J* = 5.8 Hz, 1H), 7.73 - 7.68 (m, 1H), 7.48 - 7.44 (2x s, 1H), 7.43 - 7.35 (m, 2H), 7.35 - 7.33 (2x t, *J* = 2.3 Hz, 1H), 7.31 (dd, *J* = 8.0, 2.0 Hz, 1H), 7.26 - 7.22 (2x dd, *J* = 8.3, 1.5 Hz, 1H), 7.20 - 7.16 (2x dt, *J* = 8.1, 2.5 Hz, 1H), 6.58 - 6.52 (2x d, *J* = 5.8 Hz, 1H), 5.20 (s, 2H), 3.76 - 3.68 (m, 4H), 3.65 - 3.57 (2x s, 2H), 3.49 - 3.43 (m, 2H), 2.56 - 2.43 (m, 4H), 0.91 - 0.85 (m, 2H), -0.04 - -0.05 (2x s, *J* = 2.8 Hz, 9H). ¹³C NMR (126 MHz, CDCl₃) δ 165.46, 159.98, 159.88, 157.24, 157.21, 154.09, 147.19, 147.05, 146.92, 143.27, 142.60, 134.65, 133.19, 133.06, 132.92, 132.19, 132.04, 131.87, 131.66, 130.47, 125.49, 125.08, 124.07, 123.51, 123.00, 122.96, 122.88, 122.83, 121.76, 121.27, 119.78, 118.66, 111.68, 111.62, 110.72, 110.67, 102.63, 102.55, 80.86, 67.01, 66.97, 66.81, 66.79, 63.81, 63.75, 53.62, 17.75, -1.32. LCMS (Fleet, 10 → 90%): tr = 5.76 min, m/z: 633.2.

### 2-(4-Chlorophenoxy)-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (126)

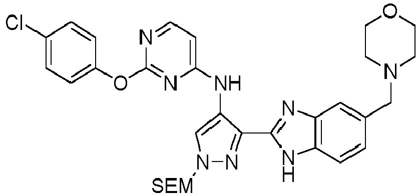

The title compound was synthesized according to general procedure I using 4-chlorophenol to afford the product (95.5 mg, 151 µmol, 91%). ¹H NMR (500 MHz, CDCl₃) δ 11.62 - 11.53 (2x s, 1H), 10.21 - 10.12 (2x s, 1H), 8.18 - 8.14 (2x d, *J* = 5.9 Hz, 1H), 7.70 - 7.64 (m, 1H), 7.44 - 7.37 (m, 3H), 7.31 (s, 1H), 7.24 - 7.16 (m, 3H), 6.57 - 6.53 (2x d, *J* = 5.8 Hz, 1H), 5.15 (s, 2H), 3.72 - 3.65 (m, 4H), 3.62 - 3.56 (2x s, 2H), 3.50 - 3.43 (m, 2H), 2.51 - 2.43 (m, 4H), 0.91 - 0.85 (m, 2H), -0.05 (s, 9H). ¹³C NMR (126 MHz, CDCl₃) δ 165.46, 159.92, 159.82, 156.84, 151.96, 146.97, 146.85, 146.74, 143.07, 142.46, 133.20, 133.07, 132.67, 132.40, 132.26, 131.84, 131.38, 130.43, 129.64, 125.14, 124.39, 124.15, 124.13, 122.74, 122.70, 122.61, 122.58, 121.93, 119.67, 118.45, 111.88, 110.88, 102.45, 102.40, 80.72, 66.81, 66.76, 66.72, 63.71, 53.48, 53.44, 17.70, -1.43, -1.49. LCMS (Fleet, 10 →- 90%): tr = 5.70 min, m/z: 633.2.

### 2-Chloro-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (127)

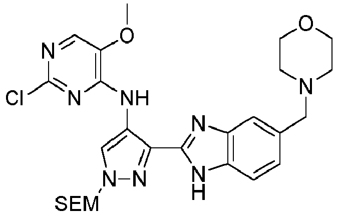

**115** (1.20 g, 2.80 mmol) was dissolved in EtOH (3 mL) after which DIPEA (1.5 mL 8.4 mmol) and 2,4-dichloro-5-methoxypyrimidine (752 mg, 4.20 mmol) were added. The mixture was heated to 40°C and stirred for 6 days. The mixture was poured into H₂O (75 mL) and the product extracted with DCM (2x50 mL). The combined organic layers were concentrated as such. The crude was purified by automated column chromatography (0 - 10% MeOH/EtOAc) to afford the product (1.12 g, 1.97 mmol, 70%). ¹H NMR (400 MHz, CDCl₃) δ 10.65 - 10.59 (2x s, 1H), 10.58 - 10.49 (2x s, 1H), 8.54 - 8.48 (2x s, 1H), 7.72 - 7.66 (m, 2H), 7.43 - 7.35 (m, 1H), 7.28 - 7.24 (m, 1H), 5.45 - 5.42 (2x s, 2H), 4.06 - 4.02 (2x s, 3H), 3.76 - 3.70 (m, 4H), 3.66 - 3.62 (2x s, 2H), 3.62 - 3.56 (m, 2H), 2.55 - 2.45 (m, 4H), 0.96 - 0.90 (m, 2H), -0.03 - -0.05 (2x s, 9H). ¹³C NMR (101 MHz, CDCl₃) δ 151.56, 151.54, 151.29, 147.07, 146.90, 143.48, 142.73, 140.15, 140.13, 133.70, 133.67, 133.52, 132.94, 132.09, 132.07, 132.06, 132.01, 125.29, 124.11, 122.83, 121.72, 121.67, 119.99, 118.90, 111.52, 110.65, 81.08, 81.05, 67.09, 63.92, 63.82, 56.71, 56.63, 53.74, 17.81, -1.34. LCMS (Fleet, 10 → 90%): tr = 6.13 min, m/z: 571.1.

### 2-(3-Chlorophenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (128)

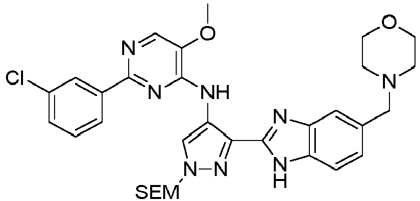

The title compound was synthesized from **127** (175 mg, 306 µmol) and (3-chlorophenyl)boronic acid (55.1 mg, 352 µmol) according to general procedure G (reaction time 16 h). The crude was purified by HPLC (Waters, 35 - 45% MeCN in 0.2% TFA (aq.)), the pH of the combined fractions was adjusted to pH ~8 using 10 M NaOH after which the mixture was concentrated. The product was dissolved in a mixture of H₂O (20 mL) and DCM (20 mL). The organic layer was separated and the water layer extracted with DCM (20 mL). The combined organic layers were dried over Na₂SO₄, filtered and concentrated to afford the product (103 mg, 159 µmol, 52%). ¹H NMR (400 MHz, CDCl₃) δ 10.89 - 10.81 (2x s, 1H), 10.47 - 10.41 (2x s, 1H), 8.61 - 8.58 (m, 1H), 8.32 - 8.29 (m, 1H), 8.20 - 8.15 (m, 1H), 7.93 - 7.88 (2x s, 1H), 7.74 - 7.69 (m, 1H), 7.41 - 7.36 (m, 2H), 7.32 - 7.27 (m, 1H), 7.27 - 7.22 (m, 1H), 5.39 (s, 2H), 4.06 - 4.03 (2x s, 3H), 3.78 - 3.70 (m, 4H), 3.68 - 3.58 (m, 4H), 2.51 (dt, *J* = 16.7, 4.7 Hz, 4H), 0.96 - 0.89 (m, 2H), -0.07 - -0.08 (2x s, 9H). ¹³C NMR (101 MHz, CDCl₃) δ 155.41, 155.39, 150.21, 150.19, 147.28, 147.12, 143.60, 142.85, 140.51, 140.04, 140.02, 134.39, 134.37, 133.21, 133.01, 132.69, 132.66, 132.16, 132.02, 131.98, 131.88, 129.69, 129.25, 127.85, 125.65, 125.15, 124.01, 123.57, 120.93, 120.87, 120.02, 118.87, 111.46, 110.52, 81.07, 67.07, 67.06, 67.03, 63.91, 63.82, 56.35, 56.26, 53.71, 17.76, -1.38. LCMS (Fleet, 10 → 90%): tr = 6.04 min, m/z: 647.3.

### 5-Methoxy-N-(3-(5-(morpholinomethyl)-1H-benzo[d]imidazol-2-yl)-1-((2-(trimethylsilyl)ethoxy)methyl)-1H-pyrazol-4-yl)-2-(3-((trimethylsilyl)ethynyl)phenyl)pyrimidin-4-amine (129)

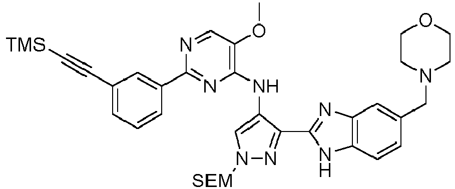

The title compound was synthesized from 128 (50 mg, 77 µmol) according to general procedure H. The crude was purified by automated column chromatography (0 - 10% MeOH/EtOAc) to afford the product (52.6 mg, 74.2 µmol, 96%). ¹H NMR (400 MHz, CDCl₃) δ 10.46 - 10.43 (2x s, 1H), 10.41 - 10.37 (2x s, 1H), 8.71 - 8.68 (2x s, 1H), 8.47 - 8.45 (2x t, *J* = 1.6 Hz, 1H), 8.29 - 8.25 (2x dt, *J* = 7.8, 1.3 Hz, 1H), 7.99 - 7.96 (2x s, 1H), 7.74 - 7.71 (m, 1H), 7.53 (dt, *J* = 7.5, 1.5 Hz, 1H), 7.41 (t, *J* = 8.1 Hz, 1H), 7.39 - 7.34 (m, 1H), 7.28 - 7.25 (m, 1H), 5.47 - 5.43 (2x s, 2H), 4.12 - 4.07 (2x s, 3H), 3.77 - 3.71 (m, 4H), 3.68 - 3.58 (m, 4H), 2.57 - 2.45 (m, 4H), 0.96 - 0.90 (m, 2H), 0.28 (s, 9H), -0.06 - -0.08 (2x s, 9H). ¹³C NMR (101 MHz, CDCl₃) δ 156.03, 150.36, 150.34, 147.31, 147.14, 143.67, 142.92, 140.04, 140.03, 138.72, 133.39, 132.94, 132.69, 132.08, 132.07, 132.01, 132.00, 131.48, 128.46, 127.69, 125.24, 124.09, 123.69, 123.35, 121.17, 121.13, 120.12, 119.00, 111.42, 110.54, 105.37, 94.12, 81.12, 67.12, 67.10, 67.04, 67.02, 63.96, 63.87, 56.46, 56.38, 53.76, 0.15, -1.33. LCMS (Fleet, 10 →- 90%): tᵣ = 7.08 min, m/z: 709.4.

### 5-Methoxy-N-(3-(5-(morpholinomethyl)-1H-benzo[d]imidazol-2-yl)-1-((2-(trimethylsilyl)ethoxy)methyl)-1H-pyrazol-4-yl)-2-(3-(trifluoromethyl) phenyl)pyrimidin-4-amine (130)

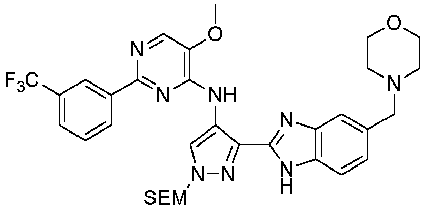

The title compound was synthesized from 127 (75.8 mg, 133 µmol) and (3-(trifluoromethyl)phenyl)boronic acid (29.0 mg, 153 µmol) according to general procedure G (reaction time 16 h). The crude was purified by automated column chromatography (0 - 10% MeOH/EtOAc) to afford the product (74.0 mg, 109 µmol, 82%). ¹H NMR (400 MHz, CDCl₃) δ 10.68 - 10.57 (2x s, 1H), 10.50 - 10.42 (2x s, 1H), 8.65 - 8.61 (m, 2H), 8.52 - 8.47 (m, 1H), 7.98 - 7.93 (2x s, 1H), 7.74 - 7.70 (m, 1H), 7.69 - 7.65 (m, 1H), 7.57 (t, *J* = 7.8 Hz, 1H), 7.37 - 7.30 (m, 1H), 7.28 - 7.23 (m, 1H), 5.40 - 5.37 (2x s, 2H), 4.09 - 4.05 (2x s, 3H), 3.78 - 3.70 (m, 4H), 3.68 - 3.58 (m, 4H), 2.58 - 2.46 (m, 4H), 0.95 - 0.90 (m, 2H), -0.06 - -0.08 (2x s, 9H). ¹³C NMR (101 MHz, CDCl₃) δ 155.28, 155.26, 150.30, 150.28, 147.28, 147.12, 143.64, 142.89, 140.21, 140.19, 139.42, 133.36, 132.98, 132.80, 132.77, 132.16, 132.13, 132.12, 131.99, 130.89, 130.69, 130.57, 128.99, 125.88, 125.85, 125.80, 125.22, 124.57, 124.54, 124.50, 124.46, 124.08, 123.53, 123.10, 121.06, 121.01, 120.08, 118.95, 111.44, 110.54, 81.15, 67.11, 67.08, 63.94, 63.85, 56.42, 56.33, 53.75, 17.75, - 1.43. LCMS (Fleet, 10 →- 90%): tr = 6.41 min, m/z: 681.3.

### 3-(5-Methoxy-4-((3-(5-(morpholinomethyl)-1H-benzo[d]imidazol-2-yl)-1-((2-(trimethylsilyl)ethoxy)methyl)-1H-pyrazol-4-yl)amino)pyrimidin-2-yl)benzonitrile (131)

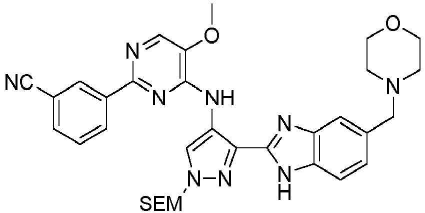

A microwave vial was charged with **127** (72,6 mg, 127 µmol), K₂CO₃ (70.3 mg, 508 µmol), (3-cyanophenyl)boronic acid (21.5 mg, 146 µmol) and 4:1 dioxane/H₂O (0.65 mL). N₂ was bubbled through the mixture for 1 min after which Pd(dppf)Cl₂·DCM (7.3 mg, 8.9 µmol) was added. N₂ was bubbled through the mixture for 30 sec after which the vial was sealed. The mixture was heated to 90°C and stirred for 16 h. Extra K₂CO₃ (70.3 mg, 508 µmol), (3-cyanophenyl)boronic acid (21.5 mg, 146 µmol), 4:1 dioxane/H₂O (0.2 mL) and Pd(dppf)Cl₂·DCM (7.3 mg, 8.9 µmol) were added and the mixture was continued to stir for another 16 h. The mixture was poured into H₂O (20 mL) and brine (2 mL). The product was extracted with DCM (2x20 mL) and the combined organic layers were concentrated as such. The crude was purified by automated column chromatography (0 - 10% MeOH/EtOAc) to afford the product (62.4 mg, 97.8 µmol, 77%). ¹H NMR (400 MHz, CDCl₃) δ 10.64 - 10.44 (m, 2H), 8.59 - 8.57 (2x t, *J* = 1.8 Hz, 1H), 8.55 - 8.53 (2x s, 1H), 8.51 - 8.47 (2x dt, *J* = 7.9, 1.4 Hz, 1H), 7.97 - 7.90 (2x s, 1H), 7.73 - 7.69 (m, 1H), 7.68 - 7.64 (2x dt, *J* = 7.7, 2.0 Hz, 1H), 7.53 (t, *J* = 7.8 Hz, 1H), 7.38 - 7.32 (m, 1H), 7.29 - 7.24 (m, 1H), 5.44 - 5.41 (2x s, *J* = 1.3 Hz, 2H), 4.11 - 4.07 (2x s, 3H), 3.77 - 3.70 (m, 4H), 3.68 - 3.58 (m, 4H), 2.57 - 2.46 (m, 4H), 0.96 - 0.90 (m, 2H), -0.06 - -0.08 (2x s, 9H). ¹³C NMR (101 MHz, CDCl₃) δ 154.55, 154.52, 150.33, 150.32, 147.18, 147.01, 143.57, 142.81, 140.33, 140.31, 139.81, 133.38, 132.93, 132.67, 132.65, 132.46, 132.08, 132.00, 131.96, 131.60, 131.38, 129.24, 125.24, 124.10, 123.50, 120.67, 120.62, 120.04, 119.05, 118.91, 112.54, 111.43, 110.54, 81.17, 67.14, 67.13, 67.08, 67.05, 63.91, 63.82, 56.47, 56.38, 53.73, 17.78, -1.37. LCMS (Fleet, 10 →- 90%): tr = 5.80 min, m/z: 638.3.

### 2-(3-Chloro-2-fluorophenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (132)

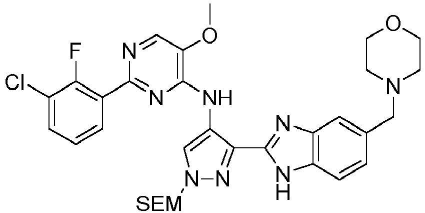

The title compound was synthesized from **127** (200 mg, 350 µmol) and (3-chloro-2-fluorophenyl)boronic acid (65.3 mg, 375 µmol) according to general procedure G (reaction time 64 h). The crude was purified by automated column chromatography (0 - 10% MeOH/EtOAc) to afford the product (160 mg, 241 µmol, 69%). ¹H NMR (400 MHz, CDCl₃) δ 10.71 - 10.58 (2x s, 1H), 10.53 - 10.45 (2x s, 1H), 8.81 - 8.75 (2x s, 1H), 8.07 - 8.04 (2x s, 1H), 8.01 (t, *J* = 7.8 Hz, 1H), 7.78 - 7.71 (m, 1H), 7.48 (t, *J* = 7.2 Hz, 1H), 7.39 - 7.31 (m, 1H), 7.31 - 7.24 (m, 1H), 7.18 (t, *J* = 7.9 Hz, 1H), 5.46 (s, 2H), 4.20 - 4.09 (2x s, 3H), 3.80 - 3.72 (m, 4H), 3.70 - 3.61 (m, 4H), 2.58 - 2.48 (m, 4H), 0.97 (t, *J* = 8.1 Hz, 2H), -0.03 (s, 9H). ¹³C NMR (101 MHz, CDCl₃) δ 157.74, 155.20, 154.02, 153.97, 150.06, 150.04, 147.35, 147.18, 143.64, 142.90, 139.82, 133.30, 132.99, 132.89, 132.87, 132.14, 132.09, 131.94, 131.10, 130.00, 128.74, 128.65, 125.13, 124.31, 124.27, 124.00, 123.42, 122.44, 122.25, 121.81, 121.76, 120.04, 118.93, 111.43, 110.55, 81.06, 67.11, 67.08, 63.94, 63.85, 56.42, 56.34, 53.74, 17.75, -1.40. LCMS (Finnigan, 10 →- 90%): tr = 6.71 min, m/z: 665.1.

### 2-(2-Fluoro-3-((trimethylsilyl)ethynyl)phenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (133)

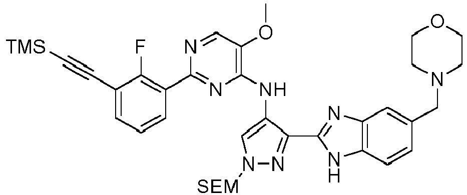

The title compound was synthesized from 132 (100 mg, 150 µmol) according to general procedure H. The crude was purified by automated column chromatography (0 - 10% MeOH/EtOAc) to afford the product (85.7 mg, 118 µmol, 78%). ¹H NMR (400 MHz, CDCl₃) δ 10.76 - 10.67 (2x s, 1H), 10.47 - 10.40 (2x s, *J* = 16.9 Hz, 1H), 8.80 - 8.77 (2x d, *J* = 1.9 Hz, 1H), 8.08 - 8.00 (m, 2H), 7.74 - 7.70 (m, 1H), 7.54 - 7.48 (m, 1H), 7.32 - 7.28 (m, 1H), 7.27 - 7.22 (m, 1H), 7.15 (td, *J* = 7.7, 1.4 Hz, 1H), 5.43 (s, 2H), 4.12 - 4.07 (2x s, 3H), 3.77 - 3.69 (m, 4H), 3.67 - 3.56 (m, 4H), 2.56 - 2.45 (m, 4H), 0.94 - 0.87 (m, 2H), 0.29 (s, 9H), -0.08 - -0.10 (2x s, 9H). ¹³C NMR (101 MHz, CDCl₃) δ 162.65, 160.07, 154.25, 154.21, 150.04, 150.02, 147.38, 147.21, 143.65, 142.91, 139.72, 139.70, 134.35, 133.20, 133.01, 132.91, 132.88, 132.18, 132.17, 132.13, 131.92, 131.89, 131.83, 127.33, 127.24, 125.11, 123.98, 123.67, 123.62, 123.44, 121.97, 121.92, 121.87, 120.03, 118.92, 113.21, 113.03, 111.44, 110.55, 100.15, 100.11, 98.40, 98.38, 80.97, 67.08, 67.04, 66.90, 66.88, 63.92, 63.82, 56.38, 56.30, 53.71, 17.70, 0.03, -1.39. LCMS (Finnigan, 10 →- 90%): tr = 7.71 min, m/z: 727.3.

### 2-(5-Chloro-2-fluorophenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (134)

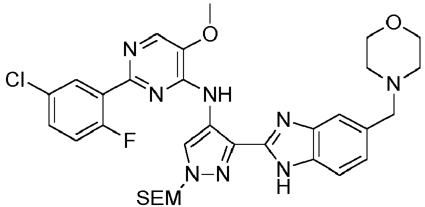

The title compound was synthesized from **127** (100 mg, 175 µmol) and (5-chloro-2-fluorophenyl)boronic acid (35.1 mg, 201 µmol) according to general procedure G (reaction time: 16 h). The crude was purified by automated column chromatography (0 - 10% MeOH/EtOAc) to afford the product (90.0 mg, 135 µmol, 77%). ¹H NMR (400 MHz, CDCl₃) δ 10.62 - 10.50 (2x s, 1H), 10.49 - 10.42 (2x s, 1H), 8.76 - 8.72 (2x d, *J* = 1.8 Hz, 1H), 8.15 - 8.11 (2x dd, *J* = 2.8, 1.9 Hz, 1H), 8.03 - 7.99 (2x s, 1H), 7.74 - 7.70 (m, 1H), 7.37 - 7.30 (m, 2H), 7.28 - 7.23 (m, 1H), 7.15 - 7.08 (2x dd, *J* = 10.3, 8.7 Hz, 1H), 5.45 - 5.42 (2x s, 2H), 4.13 - 4.09 (2x s, 3H), 3.77 - 3.70 (m, 4H), 3.66 - 3.58 (m, 4H), 2.56 - 2.45 (m, 4H), 0.96 - 0.89 (m, 2H), -0.07 (d, *J* = 1.3 Hz, 9H). ¹³C NMR (101 MHz, CDCl₃) δ 160.81, 158.29, 153.53, 153.48, 150.05, 150.03, 147.33, 147.16, 143.64, 142.89, 139.83, 133.34, 132.98, 132.82, 132.80, 132.12, 132.08, 132.03, 131.96, 131.38, 131.36, 130.39, 130.30, 129.23, 129.22, 129.20, 129.18, 128.40, 128.29, 125.16, 124.03, 123.47, 121.71, 121.66, 121.61, 120.06, 118.94, 118.40, 118.15, 111.42, 110.54, 81.10, 67.11, 67.08, 67.06, 63.95, 63.85, 56.42, 56.34, 53.74, 17.79, -1.38. LCMS (Fleet, 10 → 90%): tr = 6.01 min, m/z: 665.2.

### 2-(2-Fluoro-5-((trimethylsilyl)ethynyl)phenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (135)

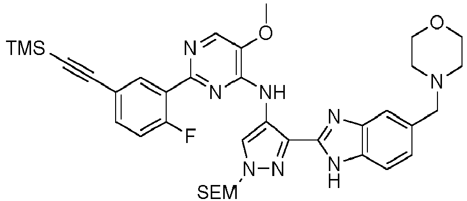

The title compound was synthesized from **134** (90.0 mg, 135 µmol) according to general procedure H. The crude was purified by automated column chromatography (0 - 10% MeOH/EtOAc) to afford the product (86.1 mg, 118 µmol, 88%). ¹H NMR (400 MHz, CDCl₃) δ 11.03 (br s, 1H), 10.59 - 10.47 (2x s, 1H), 8.79 - 8.76 (2x d, *J* = 1.9 Hz, 1H), 8.26 (dd, *J* = 7.6, 2.2 Hz, 1H), 8.05 - 8.01 (2x s, 1H), 7.73 - 7.69 (m, 1H), 7.48 (ddd, *J* = 8.5, 4.5, 2.3 Hz, 1H), 7.43 - 7.36 (m, 1H), 7.27 - 7.22 (m, 1H), 7.11 (dd, *J* = 11.1, 8.5 Hz, 1H), 5.42 (s, 2H), 4.13 - 4.06 (2x s, 3H), 3.77 - 3.71 (m, 4H), 3.71 - 3.64 (2x s, 2H), 3.62 - 3.56 (m, 2H), 2.61 - 2.49 (m, 4H), 0.96 - 0.88 (m, 2H), 0.25 (s, 9H), -0.08 (s, 9H). ¹³C NMR (101 MHz, CDCl₃) δ 162.08, 159.53, 153.88, 153.83, 150.01, 147.20, 147.04, 143.56, 142.94, 139.74, 135.63, 135.60, 134.12, 134.03, 133.19, 132.82, 132.46, 132.39, 132.03, 131.99, 130.94, 127.02, 126.92, 125.31, 124.19, 123.58, 121.99, 121.94, 120.27, 119.45, 119.41, 118.92, 117.21, 116.97, 111.93, 110.80, 104.05, 94.02, 80.88, 67.04, 66.77, 63.61, 56.40, 56.32, 53.43, 53.38, 17.76, 0.05, -1.41. LCMS (Fleet, 10 →- 90%): tr = 7.07 min, m/z: 727.3.

### Ethyl 4-((2-chloro-5-methoxypyrimidin-4-yl)amino)-1-((2-(trimethylsilyl)ethoxy)methyl)-1H-pyrazole-3-carboxylate (136)

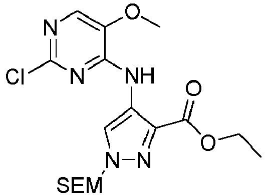

106 (1.54 g, 5.38 mmol) was dissolved in EtOH (5 mL) after which DIPEA (1.90 mL, 10.8 mmol) and 2,4-dichloro-5-methoxypyrimidine (1.45 g, 8.07 mmol) were added. The mixture heated to 40°C and stirred for 4 days and subsequently stirred at 50°C for 4 days. The mixture was poured into H₂O (75 mL) and the product extracted with DCM (2x75 mL). The organic layers were dried over Na₂SO₄, filtered and concentrated. The crude was purified by silica gel column chromatography (50% Et₂O/pentane) to afford the product (1.81 g, 4.22 mmol, 78%). ¹H NMR (400 MHz, CDCl₃) δ 9.21 (s, 1H), 8.52 (s, 1H), 7.67 (s, 1H), 5.48 (s, 2H), 4.45 (q, *J* = 7.1 Hz, 2H), 3.94 (s, 3H), 3.59 - 3.53 (m, 2H), 1.41 (t, *J =* 7.1 Hz, 3H), 0.92 - 0.86 (m, 2H), -0.06 (s, 9H). ¹³C NMR (101 MHz, CDCl₃) δ 163.90, 151.14, 151.02, 139.81, 134.01, 130.84, 125.79, 121.11, 81.72, 67.27, 61.49, 56.45, 17.78, 14.42, -1.41. LCMS (Fleet, 10 →- 90%): tr = 8.76 min, m/z: 428.3.

### Ethyl 4-((2-(3-chloro-2,6-difluorophenyl)-5-methoxypyrimidin-4-yl)amino)-1-((2-(trimethylsilyl)ethoxy)methyl)-1H-pyrazole-3-carboxylate (137)

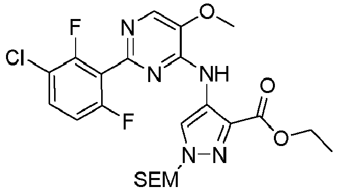

A microwave vial was charged with **136** (250 mg, 584 µmol), (3-chloro-2,6-difluorophenyl)boronic acid (169 mg, 876 µmol) and XPhos Pd G2 (92 mg, 117 µmol) after which the vial was purged with argon and subsequently sealed. Degassed THF (1 mL) and degassed 0.5 M K₃PO₄ (aq.) (2 mL) were added via syringe and the mixture was stirred for 1 h. The mixture was poured into H₂O (20 mL) and the product extracted with DCM (2x20 mL). The combined organic layers were concentrated as such. The crude was purified by automated column chromatography (50 - 80% Et₂O/pentane) to afford a mixture of starting material and product. The reaction was performed again using (3-chloro-2,6-difluorophenyl)boronic acid (113 mg, 584 µmol), XPhos Pd G2 (30 mg, 38 µmol), THF (0.5 mL) and 0.5 M K₃PO₄ (aq.) (1 mL). Performing a workup and purification as stated above, afforded a mixture of starting material and product which was subjected to another reaction using (3-chloro-2,6-difluorophenyl)boronic acid (169 mg, 876 µmol), XPhos Pd G2 (45 mg, 57 µmol) and THF (2 mL). The mixture was homogenized by stirring for 1 minute after which 1 M K₃PO₄ (aq.) (1 mL) was added and the mixture was stirred for 1 h. Performing a workup and purification as stated above, afforded a mixture of starting material and product (257 mg) which was used as such in subsequent reaction. LCMS (Fleet, 10 → 90%): tᵣ = 8.66 min, m/z: 540.3.

### 2-(3-Chloro-2,6-difluorophenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (138)

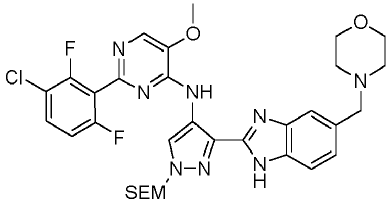

**137** (257 mg, crude) was suspended in MeOH:H₂O (1:1) (2 mL) after which LiOH·H₂O (232 mg, 5.53 mmol) was added. DCM (1 mL) was added to dissolve some insoluble parts of the mixture after which the reaction was heated to 65°C and stirred for 16 h. The mixture was poured into H₂O (30 mL) and DCM (30 mL), and the pH of the water layer was adjusted by addition of 2 M HCl (aq.) to pH ~ 2. The organic layer was separated and the water layer extracted with DCM (30 mL). The combined organic layers were dried over Na₂SO₄, filtered and concentrated. To the obtained intermediate was added 4-(morpholinomethyl)benzene-1,2-diamine (143 mg, 690 µmol), EDC·HCl (109 mg, 569 µmol), HOBt (77.3 mg, 572 µmol) and DMF (1 mL) after which the reaction was stirred for 16 h. The mixture was concentrated, AcOH (1.5 mL) was added and the reaction was stirred at 118°C for 1 h. The mixture was cooled down to RT and the pH was adjusted by addition of 10 M NaOH (aq.) (until 9 > pH > 7). The mixture was poured into H₂O (40 mL) and brine (1 mL) and the product extracted with DCM (2×40 mL). The combined organic layers were concentrated as such. The crude was purified by HPLC (Waters, 39 - 42% MeCN in 0.2% TFA (aq.)), the pH of the combined fractions was adjusted to pH ~8 using 10 M NaOH after which the mixture was concentrated. The product was dissolved in a mixture of H₂O (20 mL) and DCM (15 mL). The organic layer was separated and the water layer extracted with DCM (15 mL). The combined organic layers were dried over Na₂SO₄, filtered and concentrated to afford the product (32.5 mg, 49.8 µmol, 9% over 2 steps). ¹H NMR (400 MHz, CDCl₃) δ 10.54 - 10.47 (2x s, 1H), 10.32 - 10.21 (2x s, 1H), 8.56 - 8.53 (2x s, 1H), 8.09 - 8.06 (2x s, 1H), 7.75 - 7.72 (m, 1H), 7.45 - 7.35 (m, 2H), 7.29 - 7.25 (m, 1H), 7.01 - 6.94 (m, 1H), 5.44 - 5.41 (2x s, 2H), 4.18 - 4.13 (2x s, 3H), 3.77 - 3.70 (m, 4H), 3.67 - 3.62 (2x s, 2H), 3.61 - 3.55 (m, 2H), 2.56 - 2.45 (m, 4H), 0.94 - 0.88 (m, 2H), -0.06 - -0.07 (2x s, 9H). ¹³C NMR (101 MHz, CDCl₃) δ 160.65, 160.60, 158.15, 158.10, 157.71, 157.65, 155.19, 155.13, 150.37, 150.35, 149.94, 147.24, 147.07, 143.66, 142.91, 140.14, 133.51, 132.91, 132.84, 132.81, 132.11, 132.06, 132.01, 130.29, 130.20, 125.24, 124.10, 123.44, 121.56, 121.53, 120.12, 119.52, 119.34, 119.15, 119.05, 117.35, 117.17, 112.65, 112.61, 112.41, 112.37, 111.38, 110.55, 81.11, 81.10, 67.15, 67.13, 67.05, 67.03, 63.96, 63.87, 56.51, 56.44, 53.78, 17.81, -1.39. LCMS (Fleet, 10 → 90%): tr = 6.10 min, m/z: 683.3.

### 2-(2,6-Difluoro-3-((trimethylsilyl)ethynyl)phenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (139)

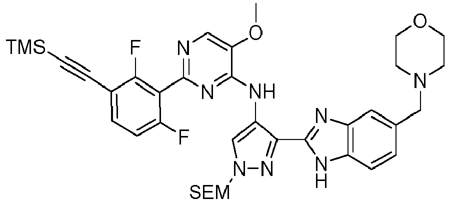

The title compound was synthesized from 138 (22 mg, 32 µmol) according to general procedure H. The crude was purified by automated column chromatography (0 - 10% MeOH/EtOAc) to afford the product (24 mg, 32 µmol, quant.). ¹H NMR (400 MHz, CDCl₃) δ 10.52 - 10.43 (2x s, 1H), 10.25 - 10.15 (2x s, 1H), 8.56 - 8.53 (2x s, 1H), 8.08 - 8.05 (2x s, *J* = 4.9 Hz, 1H), 7.76 - 7.71 (m, 1H), 7.51 - 7.45 (m, 1H), 7.43 - 7.36 (m, 1H), 7.30 - 7.25 (m, 1H), 6.99 - 6.91 (2× d, *J* = 8.8 Hz, 1H), 5.45 - 5.42 (2× s, 2H), 4.18 - 4.13 (2x s, 3H), 3.76 - 3.70 (m, 4H), 3.67 - 3.62 (2x s, 2H), 3.61 - 3.55 (m, 2H), 2.54 - 2.46 (m, 4H), 0.94 - 0.87 (m, 2H), 0.25 (s, 9H), -0.06 - -0.07 (2x s, 9H). ¹³C NMR (101 MHz, CDCl₃) δ 162.89, 162.82, 162.01, 161.95, 160.34, 160.27, 159.48, 159.42, 150.36, 150.33, 150.16, 150.15, 147.27, 147.09, 143.67, 142.92, 140.06, 133.85, 133.74, 133.53, 132.91, 132.87, 132.85, 132.13, 132.06, 132.04, 132.00, 125.24, 124.11, 123.48, 121.65, 121.63, 120.13, 119.06, 118.55, 118.37, 118.20, 112.07, 112.03, 111.84, 111.81, 111.38, 110.56, 108.91, 108.87, 108.74, 108.70, 100.09, 100.05, 97.26, 97.25, 81.09, 81.07, 67.17, 67.15, 67.01, 66.99, 63.98, 63.89, 56.50, 56.43, 53.79, 17.80, -0.02, -1.35. LCMS (Fleet, 10 →- 90%): tᵣ = 7.11 min, m/z: 745.3.

### 2-(4-Fluorophenyl)-5-methoxy-N-(3-(5-(morpholinomethyl)-1H-benzo[d]imidazol-2-yl)-1-((2-(trimethylsilyl)ethoxy)methyl)-1H-pyrazol-4-yl)pyrimidin-4-amine (140)

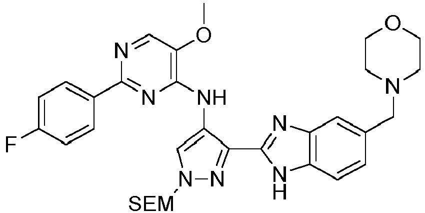

The title compound was synthesized from **127** (75.0 mg, 131 µmol) and (4-fluorophenyl)boronic acid (21.1 mg, 151µmol) according to general procedure G (reaction time 16 h). The crude was purified by automated column chromatography (0 - 10% MeOH/EtOAc) to afford the product (69.2 mg, 110 µmol, 84%). ¹H NMR (400 MHz, CDCl₃) δ 10.82 - 10.72 (2x s, 1H), 10.49 - 10.42 (2× s, *J* = 16.3 Hz, 1H), 8.61 - 8.59 (2x s, 1H), 8.32 - 8.27 (m, 2H), 7.94 (d, *J* = 8.1 Hz, 1H), 7.74 - 7.70 (m, 1H), 7.35 - 7.30 (m, 1H), 7.28 - 7.23 (m, 1H), 7.17 - 7.10 (m, 2H), 5.43 - 5.39 (2x s, 2H), 4.07 - 4.04 (2x s, 3H), 3.77 - 3.70 (m, 4H), 3.67 - 3.57 (m, 4H), 2.57 - 2.45 (m, 4H), 0.96 - 0.89 (m, 2H), - 0.06 - -0.07 (2x s, 9H). ¹³C NMR (101 MHz, CDCl₃) δ 165.07, 162.60, 156.04, 150.33, 150.31, 147.30, 147.14, 143.63, 142.88, 139.76, 139.74, 134.92, 134.89, 133.27, 133.02, 132.86, 132.16, 131.93, 131.88, 129.57, 129.48, 125.16, 124.03, 123.80, 120.67, 120.62, 120.05, 118.92, 115.41, 115.19, 111.44, 110.53, 81.14, 67.08, 67.06, 67.05, 63.83, 56.38, 56.31, 53.72, 17.82, - 1.37. LCMS (Fleet, 10 →- 90%): tr = 5.50 min, m/z: 631.3.

### (3,4-Dinitrophenyl)(morpholino)methanone (141)

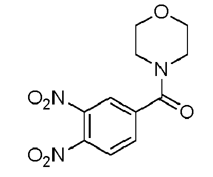

3,4-Dinitrobenzoic acid (15.0 g, 70.7 mmol) was dissolved in dry THF (150 mL) after which dry DMF (0.15 mL) and SOCl₂ (7.2 mL, 99 mmol) were added. The mixture was heated to 70°C and stirred for 2.5 h after which the mixture was cooled down to 0°C. Et₃N (14.9 mL, 107 mmol) was added dropwise over 15 min and subsequently morpholine (10.7 mL, 124 mmol) was added dropwise over 10 min. The mixture was allowed to warm up to RT and stirred overnight. H₂O (375 mL) was added and stirring was continued vigorously for 1 h after which the mixture was cooled down to 0°C and filtered. The solids were washed with ice cold H₂O (100 mL), collected and dried by coevaporation with MeOH several times to afford the product (18.4 g, 65.4 mmol, 93%). ¹H NMR (400 MHz, DMSO) δ 8.31 (d, *J* = 1.7 Hz, 1H), 8.30 (d, *J* = 8.2 Hz, 1H), 8.01 (dd, *J* = 8.3, 1.7 Hz, 1H), 3.71 - 3.60 (m, 4H), 3.59 - 3.49 (m, 2H), 3.37 - 3.27 (m, 2H). ¹³C NMR (101 MHz, DMSO) δ 165.09, 142.04, 141.35, 132.85, 126.22, 124.30, 65.91, 65.75, 47.41, 42.10.

### 4-(3,4-Dinitrobenzyl)morpholine (142)

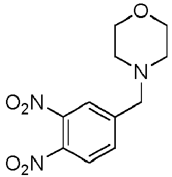

NaBH₄ (5.25 g, 139 mmol) was suspended in dry THF (193 mL) and cooled down to 0°C after which boron trifluoride etherate (17.1 mL, 139 mmol) was added. Subsequently, solid **141** (18.4 g, 65.4 mmol) was added after which the mixture was allowed to warm to RT and stirred for 3.5 h. The mixture was cooled down to 0°C and MeOH (160 mL) was added dropwise over 20 min (H₂ evolution). The resulting suspension was allowed to warm to RT, further heated to 70°C and stirred for 75 min (H₂ evolution). The mixture was concentrated, redissolved in EtOAc (200 mL) and poured into half sat. NaHCO₃ (200 mL). The organic layer was isolated and the water layer extracted with EtOAc (200 mL). The combined organic layers were washed with H₂O (200 mL), the organic layer was separated and the water layer extracted with EtOAc (100 mL). The combined organic layers were washed with brine (300 mL), dried over Na₂SO₄, filtered and concentrated. The obtained powder was grounded, suspended in MeOH (55 mL) and warmed up until fully dissolved. The solution was slowly cooled down to RT, further cooled on ice and kept on ice for 25 min. The mixture was filtered and the solids were washed with ice cold MeOH (40 mL) to afford the product (13.5 g, 50.4 mmol, 77%). ¹H NMR (400 MHz, CDCl₃) δ 7.87 (d, *J* = 1.6 Hz, 1H), 7.84 (d, *J* = 8.3 Hz, 1H), 7.71 (dd, *J* = 8.3, 1.6 Hz, 1H), 3.66 - 3.61 (m, 4H), 3.59 (s, 2H), 2.45 - 2.39 (m, 4H). ¹³C NMR (101 MHz, CDCl₃) δ 146.54, 143.01, 141.09, 132.98, 125.06, 124.66, 66.65, 61.35, 53.36. LCMS (Finnigan, 10 → 90%): tr = 4.03 min, m/z: 268.1.

### 4-(Morpholinomethyl)benzene-1,2-diamine (143)

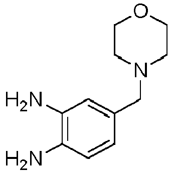

**142** (2.50 g, 9.35 mmol) was suspended in absolute EtOH (75 mL) and this mixture was subsequently degassed by bubbling N₂ through the mixture while sonicating for 20 min. 10% Pd/C (250 mg) was added and the atmosphere was exchanged for H₂. The reaction was vigorously stirred for 2 h while bubbling H₂ through the mixture. The atmosphere was exchanged for N₂, the mixture was filtered over Celite and subsequently concentrated to afford the product (1.58 g, 9.35 mmol, 81%). ¹H NMR (400 MHz, MeOD) δ 6.63 (d, *J* = 1.8 Hz, 1H), 6.60 (d, *J* = 7.8 Hz, 1H), 6.51 (dd, *J* = 7.9, 1.8 Hz, 1H), 3.63 - 3.58 (m, 4H), 3.27 (s, 2H), 2.41 - 2.31 (m, 4H). ¹³C NMR (101 MHz, MeOD) δ 135.81, 135.32, 128.48, 122.11, 119.00, 117.20, 67.51, 64.25, 54.39. LCMS (Fleet, 0 →- 50%): tᵣ = 0.86 min, m/z: 208.1.

### Biological Assays

### 1. Measurement of hBUB1 kinase activity assay

This protocol describes how the Kinase Activity Assay was performed to determine pIC₅₀ values of compounds of general formula (I) against hBUB1.

### Biochemical fluorescence polarization assay

Assays were performed in 384-well plates (Greiner, black, flat bottom, 781076) by sequential addition (final concentrations are indicated) of inhibitor (5 µL, 0.3 nM - 1 µM or 3 nM - 10 µM), BUB1/BUB3 (5 µL, 3.26 nM, Carna Biosciences (05-187), lot: 15CBS-0644 D), ATP (5 µL, 15 µM) and BUB1/BUB3 substrate (5 µL, 75 nM, Carna Biosciences (05-187MSSU)), all as 4x working solutions. The final concentration of DMSO was 1%. Assay reactions were stopped by addition of IMAP progressive binding reagent (20 µL, 1200x diluted, Molecular Devices (R8155), lot: 3117896). Each assay included the following controls: (i) a background control (treated with vehicle instead of inhibitor and BUB1/BUB3 substrate), (ii) MIN controls (treated with 5 µM BAY1816032 (MedChem Express) as inhibitor, used as 0% BUB1 activity) and (iii) MAX controls (treated with vehicle instead of inhibitor, used as 100% BUB1 activity). All inhibitors were tested in two separate assays and all inhibitor concentrations were tested in duplicate per assay (N=2, n=2).

For each assay, assay buffer (AB) was freshly prepared and consisted of 20 mM HEPES (prepared by diluting 1 M HEPES, pH 7.2), 5 mM MgCl₂, 0.01% Tween-20 and 1 mM DTT. Stocks of inhibitors (in DMSO) were diluted in AB to obtain 4x working solutions (4% DMSO) and 5 µL was added to the assay plate. BUB1/BUB3 (3.26 µM (486 µg/mL) in storage buffer) was diluted in AB to obtain 13.0 nM of which 5 µL was added to all wells of the assay plate. The assay plate was centrifuged (1 min, 200 g) and incubated at RT for 30 min. ATP (4 mM in MilliQ) was diluted in AB to obtain 60 µM of which 5 µL was added to each well. BUB1/BUB3 substrate (1 mM) was diluted in 20 mM HEPES (prepared by diluting 1 M HEPES (pH 7.2) in MilliQ) to obtain 80 µM (this solution was freshly prepared every assay) and further diluted in AB to obtain 300 nM after which 5 µL was added to each well of the assay plate except for background control wells. The assay plate was centrifuged (1 min, 200 g) and incubated at RT in the dark for 180 min. IMAP progressive binding buffer A (5x) and IMAP progressive binding buffer B (5x) were mixed in a ratio to obtain 30% buffer A and 70% buffer B, which was subsequently diluted 5x in MilliQ. IMAP progressive binding reagent was diluted 600x in aforementioned mixture of buffer A and B (to obtain a 2x working solution) of which 20 µL was added to each well of the assay plate. The assay plate was centrifuged (1 min, 200 g) and incubated at RT in the dark for 90 min. Fluorescence polarization was measured on a CLARIOstar plate reader using the following settings: (i) optic settings → excitation = F: 482-16, dichroic = F: LP 504, emission = F: 530-40, (ii) optic = top optic, (iii) speed/precision = maximum precision, (iv) focus adjustment was performed for every assay and (v) gain adjustment was done by setting the target mP value to 35 mP for one of the MIN control wells. Data was normalized between MIN and MAX controls and data was plotted using GraphPad Prism 8.0 using "Nonlinear regression (curve fit)" and "log(inhibitor) vs. normalized response - Variable slope" to determine pIC₅₀ values.

**Table 2:** shows activity data in the biochemical assay of hBUB1 kinase. Inhibition is indicated as pIC₅₀ ("-" = not measured). Compounds having an activity designated as "A" provided an pIC₅₀ ≥ 7.0 (IC₅₀ ≤100 nM); compounds having an activity designated as "B" provided an 6.3 ≤ pIC₅₀ < 7.0 (100 nM<IC₅₀ ≤ 500 nM); compounds having an activity designated as "C" provided an 6.0 ≤ pIC₅₀ < 6.3 (500 nM < IC₅₀ ≤ 1 µM); compounds having an activity designated as "D" provided an 6.0 < pIC₅₀ ≤ 5.0 (1 µM < IC₅₀ ≤ 10 µM); and compounds having an activity designated as "E" provided an pIC₅₀ < 5.0 (IC₅₀ >10 µM).

**Table 2.**

| **Example** | **hBUB1 pIC₅₀** |
|---|---|
| **15** | B |
| **16** | B |
| **17** | B |
| **18** | B |
| **19** | C |
| **20** | C |
| 21 | C |
| **22** | C |
| **23** | D |
| **24** | D |
| **25** | C |
| **26** | D |
| **27** | B |
| **28** | B |
| **29** | C |
| **30** | D |
| **32** | D |
| **33** | D |
| **34** | D |
| **36** | D |
| **37** | E |
| **38** | D |
| **39** | D |
| **41** | A |
| **42** | A |
| **43** | C |
| **44** | C |
| **45** | A |
| **46** | A |
| **47** | A |
| 48 | C |
| **49** | C |
| **50** | C |
| **51** | A |
| **52** | A |
| **53** | B |
| **54** | A |
| **55** | A |
| **56** | A |
| **57** | A |
| **58** | A |
| **59** | A |
| **60** | A |
| **61** | B |
| **62** | D |
| **63** | C |
| **64** | D |

### 2. Measurement of cellular proliferation

This protocol describes how the Cellular Proliferation Assay was performed to determine pIC₅₀ values of compounds of general formula (I) against U2OS cells.

### Cell culture

U2OS (human osteosarcoma) cells were purchased at ATCC and were tested on regular basis for mycoplasma contamination. Cultures were discarded after 2-3 months of use. Cells were cultured at 37°C under 7% CO₂ in DMEM (Sigma Aldrich, D6546) supplemented with GlutaMAX (2 mM, ThermoFisher), 10% (v/v) heat-inactivated newborn calf serum (Seradigm), penicillin and streptomycin (200 µg/mL each, Duchefa) (complete medium). Medium was refreshed every 2-3 days and cells were passaged by trypsinization twice a week at 80-90% confluence. Cell viability was assessed by Trypan Blue exclusion and cell quantification using a TC20^{™} Automated Cell Counter (Bio-Rad).

### SRB proliferation assay

Assays were performed in 96-well plates (Greiner, Cellstar, 655180) by seeding (day 0), treatment (day 1) and subsequent incubation for 72 h. Cells were fixed, stained and staining was subsequently dissolved after which absorbance was measured. Each assay included the following controls: (i) a background control (to which no cells were added), (ii) t₀ controls (separate assay plate in which cells were fixed on day 1, used as 0% proliferation), (iii) nontreated controls (present in each assay plate, treated with vehicle and used as 100% proliferation). Cells were treated with different concentrations of indicated inhibitor. All inhibitors were tested in two separate assays and all inhibitor concentrations were tested in triplicate per assay (N=2, n=3).

For each assay, U2OS cells from 10 cm dishes were seeded into 96-well plates (3,000 cells/well) and subsequently incubated overnight to allow for cell adherence. Stocks of inhibitor and vehicle (all in DMSO) were diluted in complete medium to obtain 2x working solutions (1% DMSO). Cell medium was replaced by fresh complete medium (50 µL), treatment was started by addition of the 2x working solutions (50 µL) and plates were incubated at 37°C for 72 h. Cell medium was replaced by fresh serum free medium (100 µL) and cells were fixed by addition of 30 µL 50% (w/v) aq. trichloroacetic acid after which the plates were incubated at 4°C for 60 min. Wells were emptied by shaking the plates upside down after which wells were washed three times with demineralized water and air-dried overnight. To each well, 60 µL of SRB solution (0.4% (w/v) in 1% aq. acetic acid) was added and plates were incubated for 30 min. The excess SRB was removed and the wells were washed three times with 1% aq. acetic acid and air-dried overnight. Bound SRB was redissolved by addition of 150 µL 10 mM TRIS (free-base) and absorbance was measured at 540 nm on a CLARIOstar plate reader. Data was normalized between t₀ and nontreated controls and plotted using GraphPad Prism 8.0 using "Nonlinear regression (curve fit)" and "log(inhibitor) vs. normalized response - Variable slope" to determine pIC₅₀ values.

**Table** 3 shows activity data in the U2OS cell proliferation assays. Inhibition is indicated as pIC₅₀ ("-" = not measured). Compounds having an activity designated as "A" provided an pIC₅₀ ≥ 6.0 (IC₅₀ ≤1 µM); compounds having an activity designated as "B" provided an 5.3 ≤ pIC₅₀ < 6.0 (1 µM<IC₅₀ ≤ 5 µM); compounds having an activity designated as "C" provided an 5.0 ≤ pIC₅₀ < 5.3 (5 µM < IC₅₀ ≤ 10 µM); compounds having an activity designated as "D" provided an 5.0 < pIC₅₀ ≤ 4.7 (10 µM < IC₅₀ ≤ 20 M); and compounds having an activity designated as "E" provided an pIC₅₀ < 4.7 (IC₅₀ >20 µM).

**Table 3.**

| **Example** | **U2OS / [nM]** |
|---|---|
| **15** | |
| **16** | B |
| **17** | C |
| **18** | B |
| **19** | - |
| **20** | - |
| **21** | - |
| **22** | - |
| **23** | - |
| **24** | - |
| **25** | - |
| **26** | - |
| **27** | - |
| **28** | - |
| **29** | - |
| **30** | - |
| **31** | - |
| **32** | - |
| **33** | - |
| **34** | - |
| **35** | - |
| **36** | - |
| **37** | - |
| **38** | - |
| **39** | - |
| **40** | - |
| **41** | B |
| **42** | B |
| **43** | B |
| **44** | B |
| **45** | B |
| **46** | A |
| **47** | B |
| **48** | - |
| **49** | - |
| **50** | - |
| **51** | A |
| **52** | A |
| **53** | A |
| **54** | D |
| **55** | A |
| **56** | A |
| **57** | A |
| **58** | A |
| **59** | A |
| **60** | A |
| **61** | A |
| **62** | - |
| **63** | - |
| **64** | - |
| **65** | - |

## Claims

1. A compound of the general formula (I) wherein 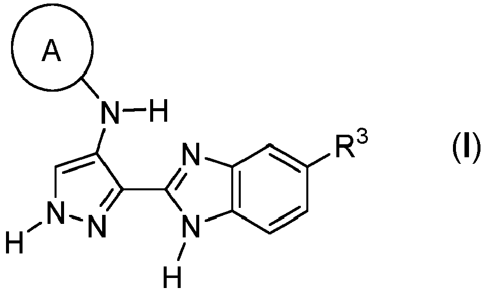
A represents 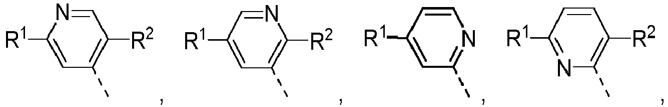 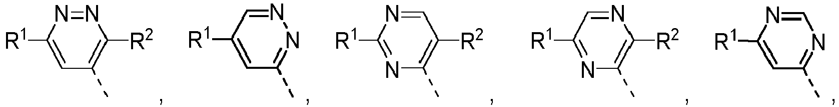 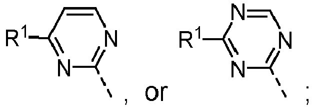
**R¹** represents -**R⁴** or -O-**L¹**-**R⁴**;
**L¹** represents a covalent bond, C₁-C₃ alkylene which is optionally substituted with one or more of -F, -Cl, -Br, -CN, C₁-C₃ alkyl, -CHF₂, -CF₃, and -cyclo-C₃H₅;
**R²** represents -H, -**R⁵**, or -O-**R⁵**;
**R³** represents -H, -**R⁶**, -(CH₂)ₘ-**R⁶**, or -O-(CH₂)ₘ-**R⁶**;
m represents an integer selected from 1, 2, and 3;
**R⁴** represents -C=C-L²-**R***, C₆-C₁₀ aryl, or C₁-C₁₀ heteroaryl, wherein C₆-C₁₀ aryl and C₁-C₁₀ heteroaryl are optionally substituted with one or more of **R^{N1}**, **Z¹**, **Z²**, **Z³ Z⁴** and **Z⁵**;
**L²** represents a covalent bond, -CH₂-, -CF₂-, or -CH₂CH₂-;
**R*** represents -H, C₁-C₆ alkyl, C₃-C₆ cycloalkyl, C₆-C₁₀ aryl, or C₁-C₁₀ heteroaryl, wherein C₃-C₆ cycloalkyl, C₆-C₁₀ aryl, and C₁-C₁₀ heteroaryl are optionally substituted with one or more of R^{N1}, Z¹, Z², Z³, Z⁴ and Z⁵;
**R⁵** represents -H, -CH₃, -CD₃, -C₂H₅, -C₃H₇, -CH(CH₃)₂, -CHF₂, -CF₃, -CN, -CH₂CN, -C₄H₉, -cyclo-C₃H₅, or -cyclo-C₄H₇;
**R⁶** represents -OH, -OCH₃, -OC₂H₅, -N(CH₃)₂, -N(C₂H₅)₂, -C₂H₄OH, -OC₂H₄OH, -NR⁷R⁸, -CH₂NR⁷R⁸, -CH₂CH₂NR⁷R⁸, -CH₂CH₂CH₂NR⁷R⁸, -NHCOR⁷, -NHSO₂R⁷, -OCH₂NR⁷R⁸, -OCH₂CH₂NR⁷R⁸, -OCH₂CH₂CH₂NR⁷R⁸, -NR⁹CH₂NR⁷R⁸, -NR⁹CH₂CH₂NR⁷R⁸, -NR⁹CH₂CH₂CH₂NR⁷R⁸, -CONR⁷R⁸, -CONHCH₂NR⁷R⁸, -CONHCH₂CH₂NR⁷R⁸, -CONHCH₂CH₂CH₂NR⁷R⁸, -(OC₂H₄)ₙOH, -(OC₂H₄)ₙOCH₃, -CN, 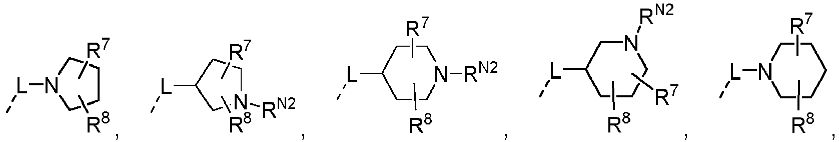 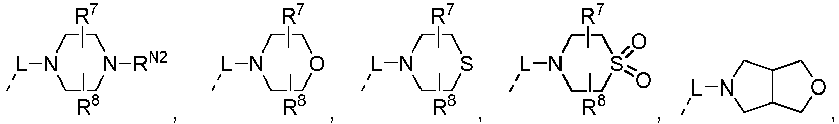 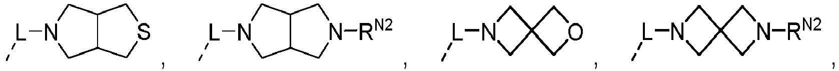 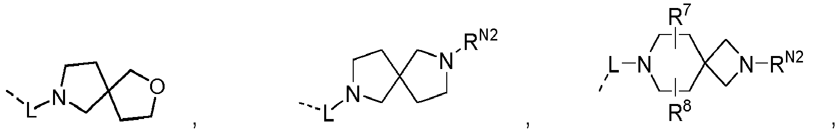 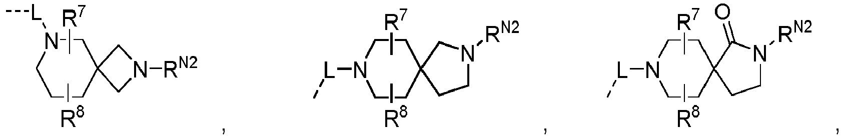 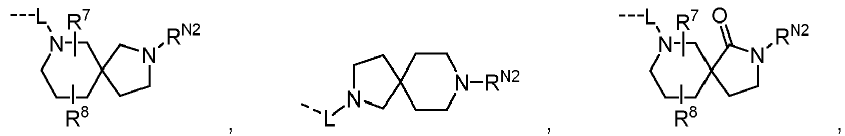 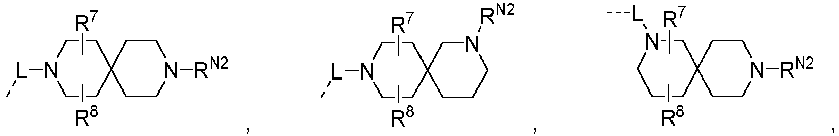 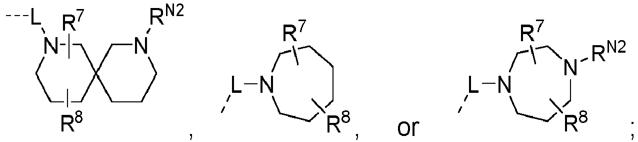
**n** represents an integer selected from 1, 2, and 3;
L represents a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OC-O-, -O-CO-, -SO₂-, -CH₂O-CO-, -CH₂-CO-O-, -CH₂CH₂O-CO-, or -CH₂CH₂-CO-O-;
**R⁷** and **R⁸** represent independently of each other -H, -F, -Br, -Cl, -CH₃, -C₂H₅, -C₃H₇, -CH(CH₃)₂, -CH₂F, -CHF₂, -CF₃, -CH₂CN, -CH₂Cl, -CH₂Br, -C(CH₃)₂-CN, -CH₂-C(CH₃)₂-CN, -C₄H₉, -CH₂-CH(CH₃)₂, -CH(CH₃)-C₂H₅, -C(CH₃)₃, -OCH₃, -OC₂H₅, -OC₃H₇, -OCH(CH₃)₂, -OCH₂F, -OCHF₂, -OCF₃, cyclo-C₃H₅, cyclo-C₄H₇, cyclo-C₅H₉, cyclo-C₆H₁₁, -O-cyclo-C₃H₅ -O-cyclo-C₄H₇, -O-cyclo-C₅H₉, -O-cyclo-C₆H₁₁, -CH=CH₂, -CH₂-CH=CH₂, -C(CH₃)=CH₂, -CH=CH-CH₃, -C(CH₃)=CH-CH₃, -CH=C(CH₃)₂, -C(CH₃)=C(CH₃)₂, -C₂H₄-CH=CH₂, -CH₂-CH=CH-CH₃, -CH₂-C(CH₃)=CH₂, -CH(CH₃)-CH=CH₂, -C(CH₃)=CH-CH₃, -CH₂-CH=C(CH₃)₂, -C=CH, -CH₂-C≡CH, -C₂H₄-C≡CH, -CH₂-C≡C-CH₃, -C≡C-CH₃, -C≡C-C₂H₅, -CH₂-OCF₃, -C₂H₄-OCF₃, -C₃H₆-OCF₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OH, -C₂H₄-OH, -C₃H₆-OH, -CH₂-SCH₃, -C₂H₄-SCH₃, -C₃H₆-SCH₃, -CH₂-SC₂H₅, -C₂H₄-SC₂H₅, -C₃H₆-SC₂H₅, -CH₂-SH, -C₂H₄-SH, or -C₃H₆-SH;
or **R⁷** and **R⁸** form together -CH₂-, -CH₂CH₂-, or -CH₂CH₂CH₂-;
**R⁹** and **R^{N1}** represent independently of each other -H, -CH₃, or -C₂H₅;
**R^{N2}** represents -H, -CH₃, -C₂H₅, -C₃H₇, -CH(CH₃)₂, -CF₃, 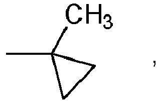 -cyclo-C₃H₅, -cyclo-C₄H₇, -cyclo-C₅H₉, -cyclo-C₆H₁₁, -cyclo-C₇H₁₃, -CH₂-cyclo-C₃H₅, -C₄H₉, -CH₂-CH(CH₃)₂, -CH(CH₃)-C₂H₅, -C(CH₃)₃, -C₅H₁₁, -CH(CH₃)-C₃H₇, -Ph, -CH₂-Ph, -CH₂-CH₂-Ph, -CH=CH₂, -CH₂-CH=CH₂, -C(CH₃)=CH₂, -CH=CH-CH₃;
**Z¹** - **Z⁵** represent independently of each other 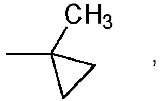 -cyclo-C₃H₅, -cyclo-C₄H₇, -cycl0-C₅H₉, -cyclo-C₆H₁₁, -cyclo-C₇H₁₃, -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-N H-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂-CH₂F, -CH₂-CHF₂, -CH₂-CF₃, cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C=CH, -CH=CH-CH=C(CH₃)₂, -CH₂-CH(CH₃)-CH₂-C=CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C=CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈≡C=CH, -C₂H₄-C=C≡C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C=C-C=CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, or -C≡C-C≡C-C₂H₅;
or an enantiomer, a diastereomer, a tautomer, a mixture of enantiomers, a mixture of diastereomers, a hydrate, a solvate, or a racemate of the above mentioned compounds or a pharmaceutically acceptable salt thereof.

2. The compound according to claim 1, wherein
**R⁴** represents -C=CH, -C≡C-CH₃, 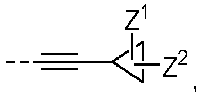 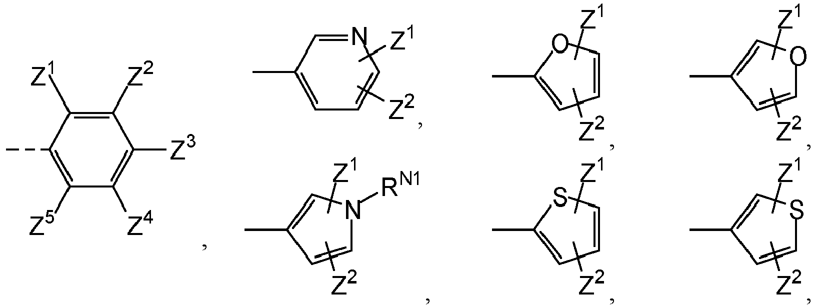 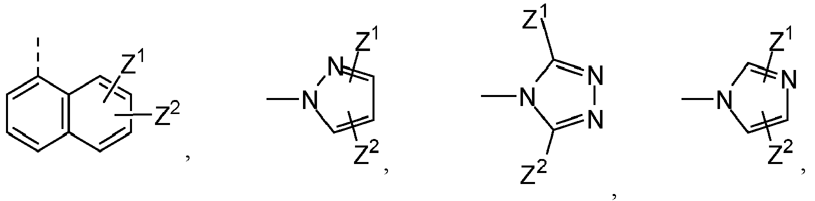 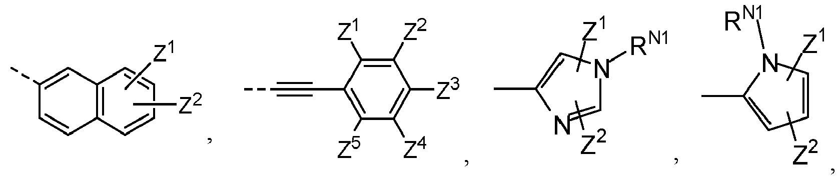 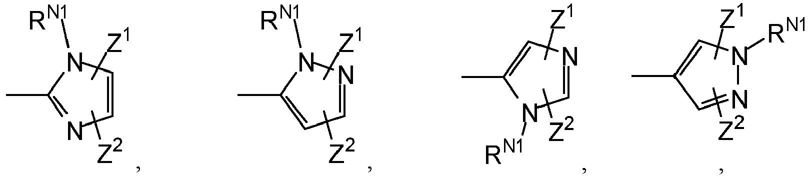 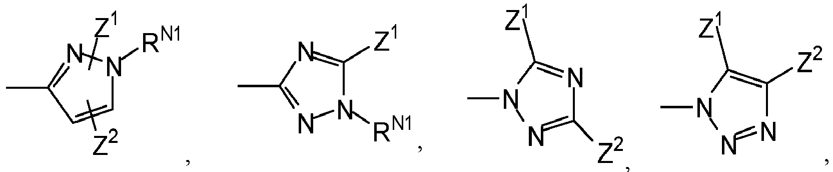 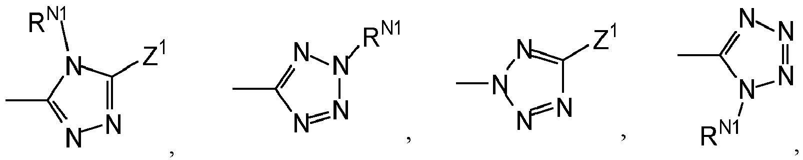 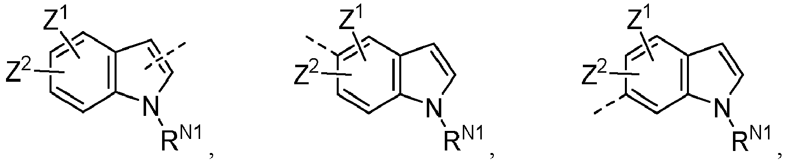 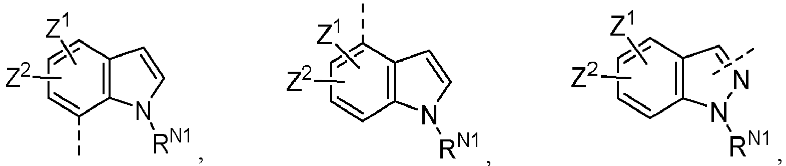 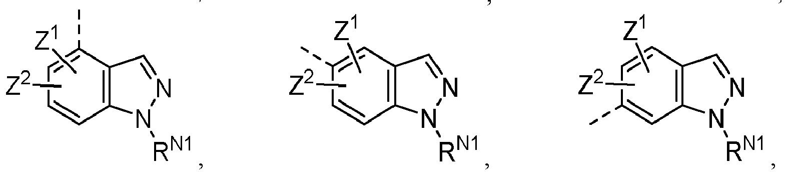 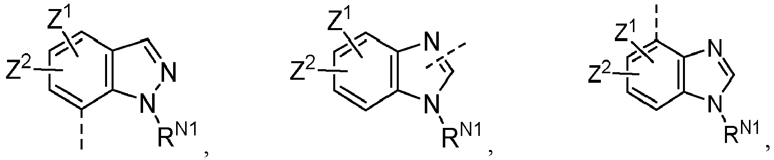 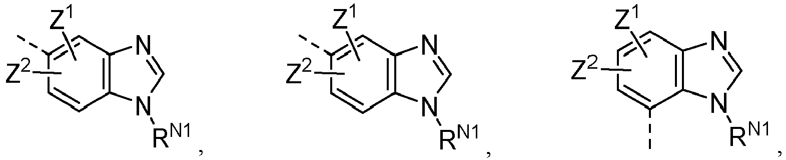
and Z¹, Z², Z³, Z⁴, Z⁵, and R^{N1} have the meanings as defined in claim 1.

3. The compound according to claim 1 or 2, wherein
R¹ represents -R⁴, -O-R⁴, -O-CH₂-R⁴ -O-C₂H₄-R⁴, or -O-C₃H₆-R⁴;
R⁴ represents -C=CH, -C≡C-CH₃, 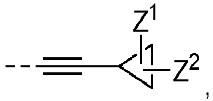 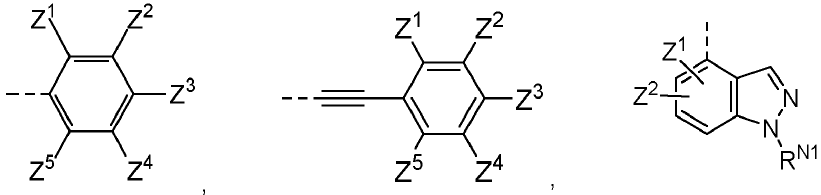 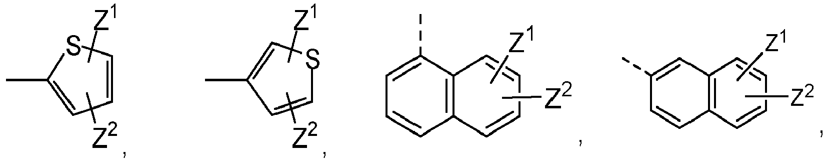 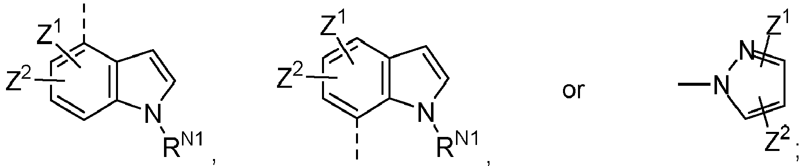
and Z¹, Z², Z³, Z⁴, Z⁵, and R^{N1} have the meanings as defined in claim 1.

4. The compound according to any one of the claims 1 to 3, wherein
R⁶ represents -OH, -OCH₃, -OC₂H₅, -N(CH₃)₂, -N(C₂H₅)₂, -NR⁷R⁸, -CH₂NR⁷R⁸, -NR⁹CH₂CH₂NR⁷R⁸, -(OC₂H₄)ₙOH, -(OC₂H₄)ₙOCH₃, 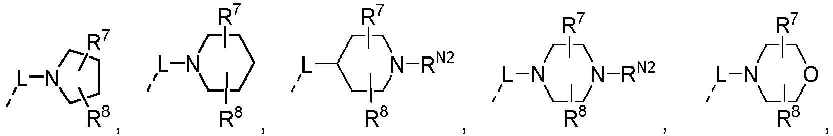 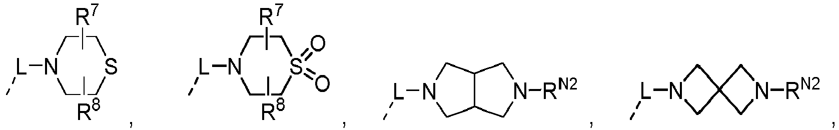 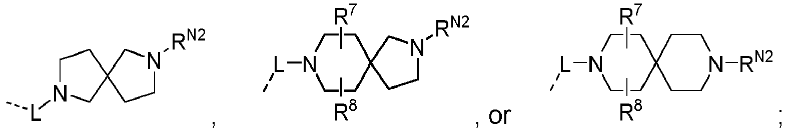
L represents a bond, -CH₂-, -CH₂CH₂-, -CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -SO₂-, -CH₂OCO-, or -CH₂CH₂OCO-; and
n, R⁷, R⁸, R⁹ and R^{N2} have the same meanings as defined in claim 1.

5. The compound according to any one of claims 1 to 4, wherein
**R³** represents -H, **-R⁶, -(CH₂)ₘ-R⁶,** or **-O-(CH₂)ₘ-R⁶;**
**R⁶** represents -OH, -OCH₃, -OC₂H₅, -NCH₃)₂, -N(C₂H₅)₂, 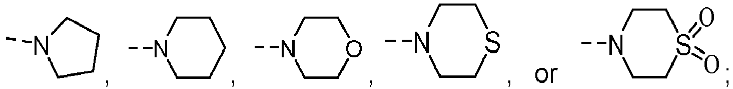 and
**m** represents the integer 1, 2 or 3.

6. The compound according to any one of the claims 1 to 5, having any one of the formulae **(I-1)** to **(I-8):** 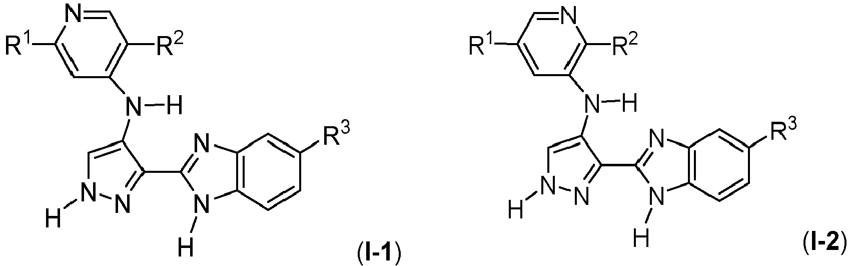 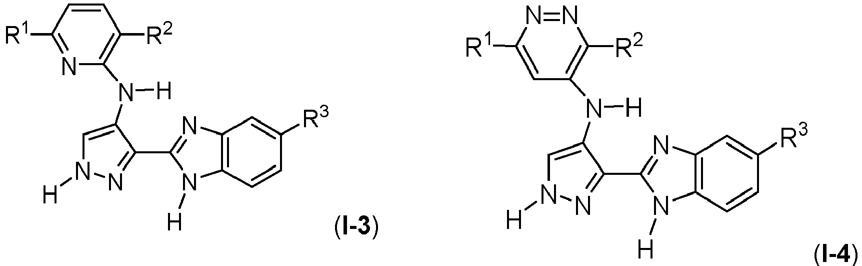 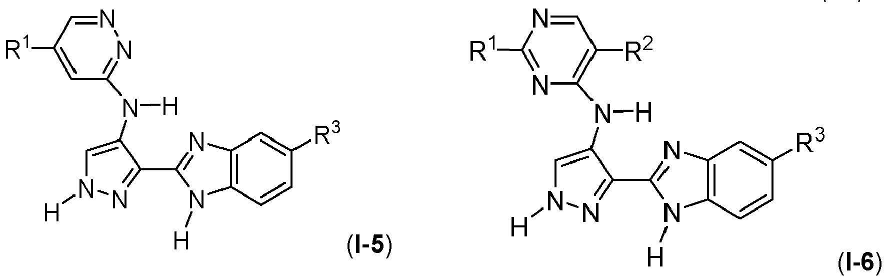 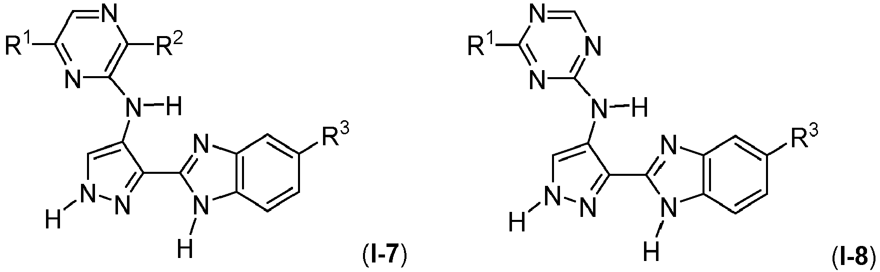 and **R¹, R²,** and **R³** have the meanings as defined in any one of claims 1 to 5.

7. The compound according to any one of the claims 1 to 6, wherein
**R¹** represents **-R⁴,** -O-**R⁴,** -O-CH₂-**R⁴** or -O-C₂H₄-**R⁴**;
**R⁴** represents -C≡CH, -C≡C-CH₃, -C≡C-cyclo-C₃H₅, -C=C-Ph, 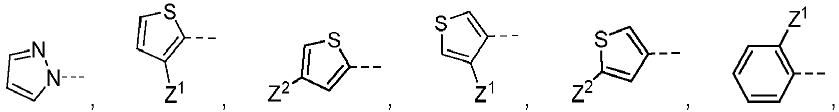 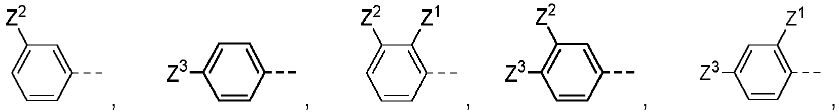 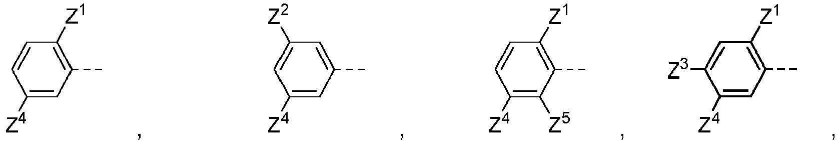 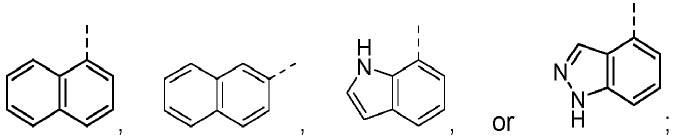 and
**Z¹, Z², Z³, Z⁴,** and **Z⁵** represent independently of each other -H, -F, -Cl, -CN, -CH₃, -CH(CH₃)₂, -cyclo-C₃H₅, -CF₃, -OCH₃, -OCF₃, -OPh, -SO₂NHCH₃, or -C=CH.

8. The compound according to any one of the claims 1 to 7, wherein
**R¹** represents -C≡CH, -C≡C-CH₃, -C≡C-cyclo-C₃H₅, -C=C-Ph, 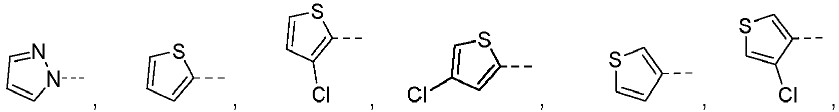 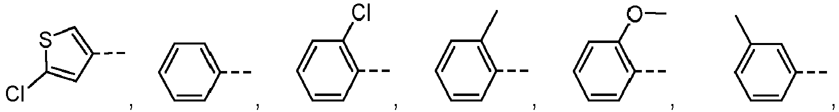 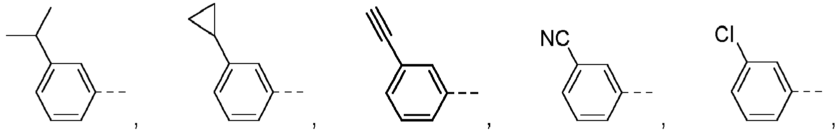 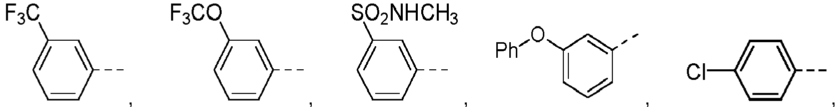 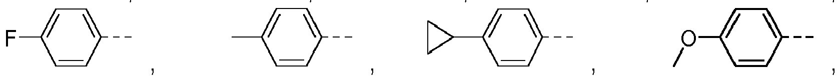 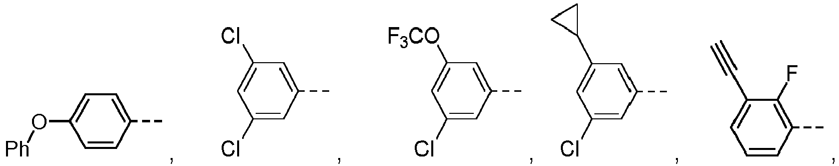 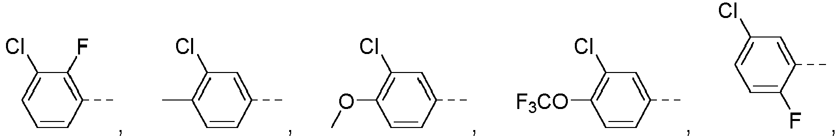 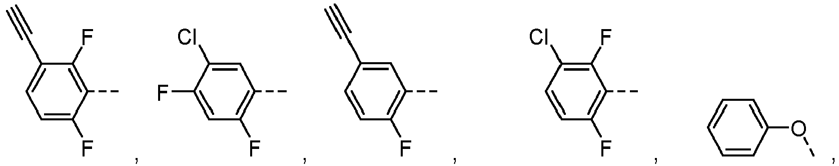 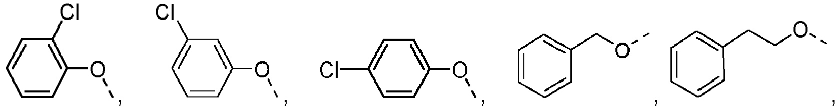 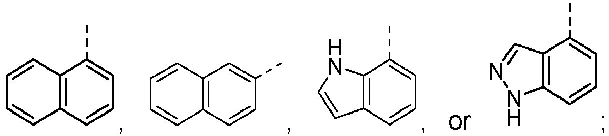
**R²** represents -H, -CH₃, -OCH₃, or -OCF₃;
**R³** represents -H, -CH₂-OH, -CH₂CH₂-OH, -CH₂-OCH₃, -CH₂CH₂-OCH₃, -CH₂N(CH₃)₂, -CH₂CH₂N(CH₃)₂, -CH₂N(C₂H₅)₂, -CH₂CH₂N(C₂H₅)₂, -OCH₂CH₂-OH, -OCH₂CH₂-OCH₃, -OCH₂CH₂N(CH₃)₂, -OCH₂CH₂N(C₂H₅)₂, 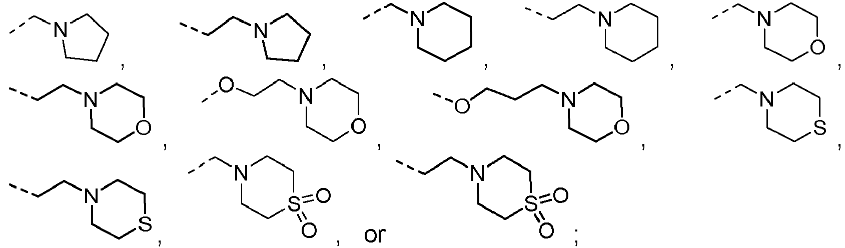 preferably **R³** represents -H, -CH₂-OH, -CH₂CH₂-OCH₃, -CH₂N(CH₃)₂, -OCH₂CH₂-OCH₃,

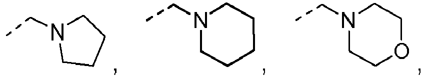

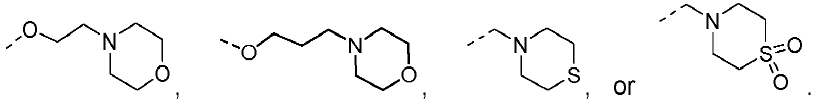

9. The compound according to any one of the claims 1 to 8, having any one of the formulae **(II-1)** to **(II-6)** or of the formulae **(III-1)** to **(III-10):** 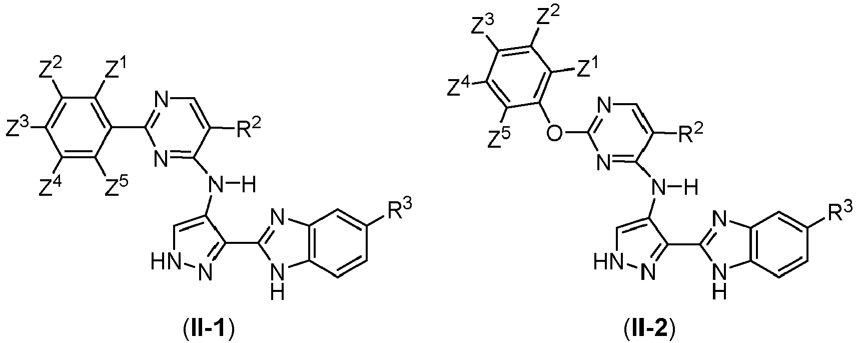 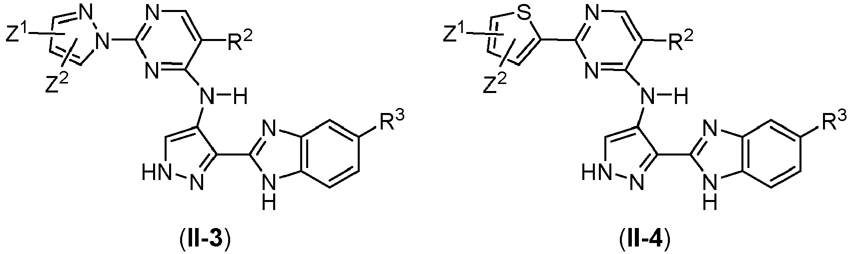 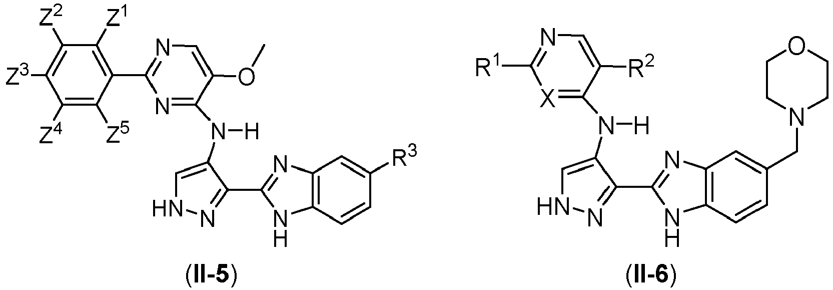 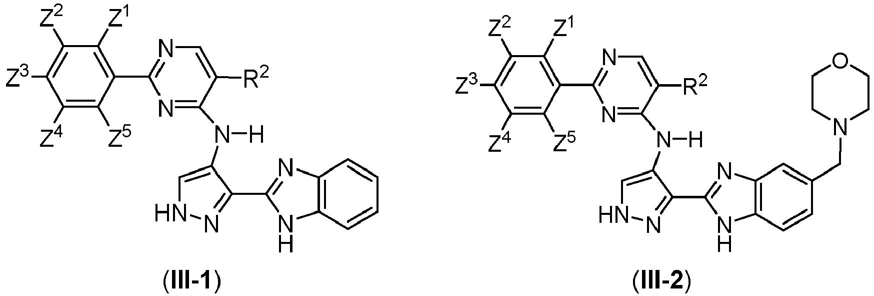 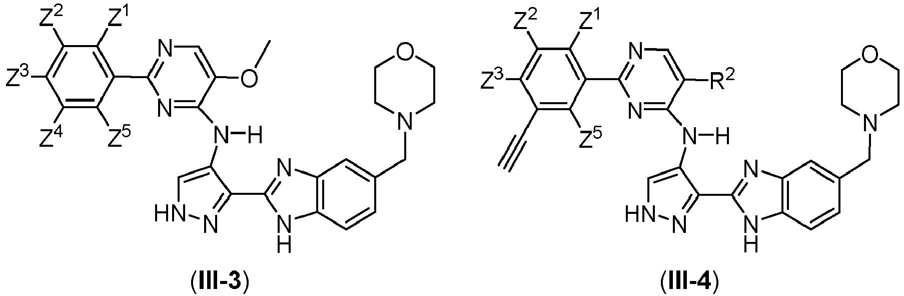 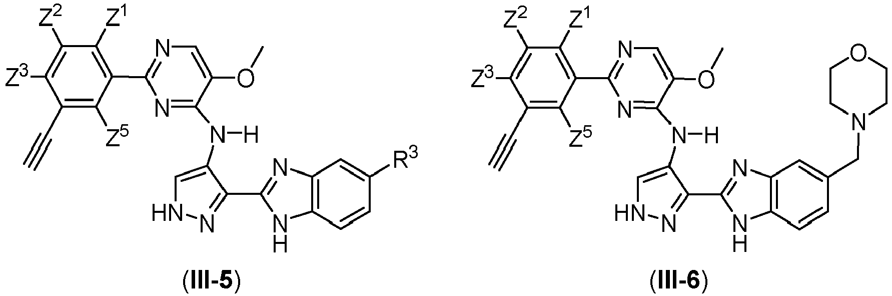 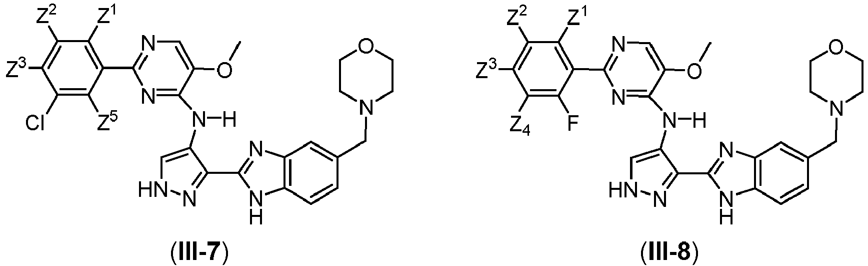 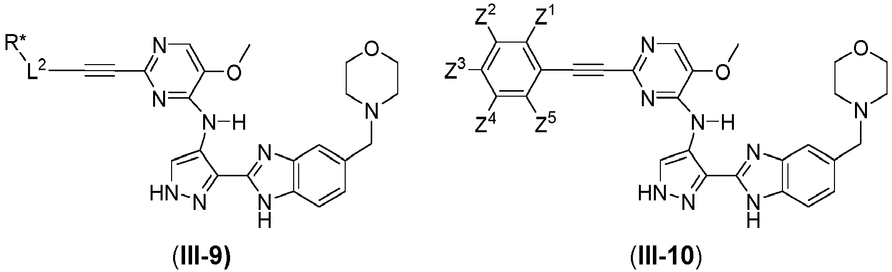
and X represents N or CH; and
**L², R¹, R², R³, R*, Z¹, Z², Z³, Z⁴**, and **Z⁵** have the meanings as defined in any one of the claims 1 to 8.

10. The compound according to claim 1 selected from the group consisting of:
| **Compound** | **Nomenclature** |
|---|---|
| **15** | 2-ethynyl-N-(3-(5-(morpholinomethyl)-1H-benzo[d]imidazol-2-yl)-1H-pyrazol-4-yl)pyrimidin-4-amine |
| **16** | N-(3-(5-(morpholinomethyl)-1H-benzo[d]imidazol-2-yl)-1H-pyrazol-4-yl)-2-phenylpyrimidin-4-amine |
| **17** | N-(3-(5-(morpholinomethyl)-1H-benzo[d]imidazol-2-yl)-1H-pyrazol-4-yl)-2-phenoxypyrimidin-4-amine |
| **18** | 2-(benzyloxy)-N-(3-(5-(morpholinomethyl)-1H-benzo[d]imidazol-2-yl)-1H-pyrazol-4-yl)pyrimidin-4-amine |
| **19** | N-(3-(5-(morpholinomethyl)-1H-benzo[d]imidazol-2-yl)-1H-pyrazol-4-yl)-2-phenethoxypyrimidin-4-amine |
| **20** | N-(3-(5-(morpholinomethyl)-1H-benzo[d]imidazol-2-yl)-1H-pyrazol-4-yl)-2-(1H-pyrazol-1-yl)pyrimidin-4-amine |
| **21** | N-(3-(5-(morpholinomethyl)-1H-benzo[d]imidazol-2-yl)-1H-pyrazol-4-yl)-2-(thiophen-3-yl)pyrimidin-4-amine hydrochloride |
| **22** | N-(3-(5-(morpholinomethyl)-1H-benzo[d]imidazol-2-yl)-1H-pyrazol-4-yl)-2-(thiophen-2-yl)pyrimidin-4-amine hydrochloride |
| **23** | N-(3-(1H-benzo[d]imidazol-2-yl)-1H-pyrazol-4-yl)-2-phenylpyrimidin-4-amine |
| **24** | N-(3-(1H-benzo[d]imidazol-2-yl)-1H-pyrazol-4-yl)-2-(o-tolyl)pyrimidin-4-amine |
| **25** | N-(3-(1H-benzo[d]imidazol-2-yl)-1H-pyrazol-4-yl)-2-(2-chlorophenyl)pyrimidin-4-amine |
| **26** | N-(3-(1H-benzo[d]imidazol-2-yl)-1H-pyrazol-4-yl)-2-(2-methoxyphenyl)pyrimidin-4-amine |
| **27** | N-(3-(1H-benzo[d]imidazol-2-yl)-1H-pyrazol-4-yl)-2-(m-tolyl)pyrimidin-4-amine |
| **28** | N-(3-(1H-benzo[d]imidazol-2-yl)-1H-pyrazol-4-yl)-2-(3-chlorophenyl)pyrimidin-4-amine |
| **29** | N-(3-(1H-benzo[d]imidazol-2-yl)-1H-pyrazol-4-yl)-2-(3-methoxyphenyl)pyrimidin-4-amine |
| **30** | N-(3-(1H-benzo[d]imidazol-2-yl)-1H-pyrazol-4-yl)-2-(3-isopropylphenyl)pyrimidin-4-amine |
| **32** | N-(3-(1H-benzo[d]imidazol-2-yl)-1H-pyrazol-4-yl)-2-(p-tolyl)pyrimidin-4-amine |
| **33** | N-(3-(1H-benzo[d]imidazol-2-yl)-1H-pyrazol-4-yl)-2-(4-chlorophenyl)pyrimidin-4-amine |
| **34** | N-(3-(1H-benzo[d]imidazol-2-yl)-1H-pyrazol-4-yl)-2-(4-methoxyphenyl)pyrimidin-4-amine |
| **36** | N-(3-(1H-benzo[d]imidazol-2-yl)-1H-pyrazol-4-yl)-2-(naphthalen-1-yl)pyrimidin-4-amine |
| **37** | N-(3-(1H-benzo[d]imidazol-2-yl)-1H-pyrazol-4-yl)-2-(1H-indol-7-yl)pyrimidin-4-amine |
| **38** | N-(3-(1H-benzo[d]imidazol-2-yl)-1H-pyrazol-4-yl)-2-(1H-indazol-4-yl)pyrimidin-4-amine |
| **39** | N-(3-(1H-benzo[d]imidazol-2-yl)-1H-pyrazol-4-yl)-2-(naphthalen-2-yl)pyrimidin-4-amine |
| **41** | 2-(3-chlorophenyl)-N-(3-(5-(morpholinomethyl)-1H-benzo[d]imidazol-2-yl)-1H-pyrazol-4-yl)pyrimidin-4-amine |
| **42** | 2-(3-ethynylphenyl)-N-(3-(5-(morpholinomethyl)-1H- |
| | benzo[d]imidazol-2-yl)-1H-pyrazol-4-yl)pyrimidin-4-amine |
| **43** | N-(3-(5-(morpholinomethyl)-1H-benzo[d]imidazol-2-yl)-1H-pyrazol-4-yl)-2-(3-(trifluoromethyl)phenyl)pyrimidin-4-amine |
| **44** | 3-(4-((3-(5-(morpholinomethyl)-1H-benzo[d]im idazol-2-yl)-1H-pyrazol-4-yl)amino)pyrimidin-2-yl)benzonitrile |
| **45** | 2-(3-chloro-2-fluorophenyl)-N-(3-(5-(morpholinomethyl)-1H-benzo[d]imidazol-2-yl)-1H-pyrazol-4-yl)pyrimidin-4-amine |
| **46** | 2-(3-ethynyl-2-fluorophenyl)-N-(3-(5-(morpholinomethyl)-1H-benzo[d]imidazol-2-yl)-1H-pyrazol-4-yl)pyrimidin-4-amine |
| **47** | 2-(5-chloro-2-fluorophenyl)-N-(3-(5-(morpholinomethyl)-1H-benzo[d]imidazol-2-yl)-1H-pyrazol-4-yl)pyrimidin-4-amine |
| **48** | 2-(2-chlorophenoxy)-N-(3-(5-(morpholinomethyl)-1H-benzo[d]imidazol-2-yl)-1H-pyrazol-4-yl)pyrimidin-4-amine |
| **49** | 2-(3-chlorophenoxy)-N-(3-(5-(morpholinomethyl)-1H-benzo[d]imidazol-2-yl)-1H-pyrazol-4-yl)pyrimidin-4-amine |
| **50** | 2-(4-chlorophenoxy)-N-(3-(5-(morpholinomethyl)-1H-benzo[d]imidazol-2-yl)-1H-pyrazol-4-yl)pyrimidin-4-amine |
| **51** | 2-(3-chlorophenyl)-5-methoxy-N-(3-(5-(morpholinomethyl)-1H-benzo[d]imidazol-2-yl)-1H-pyrazol-4-yl)pyrimidin-4-amine |
| **52** | 2-(3-ethynylphenyl)-5-methoxy-N-(3-(5-(morpholinomethyl)-1H-benzo[d]imidazol-2-yl)-1H-pyrazol-4-yl)pyrimidin-4-amine |
| **53** | 5-methoxy-N-(3-(5-(morpholinomethyl)-1H-benzo[d]imidazol-2-yl)-1H-pyrazol-4-yl)-2-(3-(trifluoromethyl)phenyl)pyrimidin-4-amine |
| **54** | 3-(5-methoxy-4-((3-(5-(morpholinomethyl)-1H-benzo[d]imidazol-2-yl)-1H-pyrazol-4-yl)amino)pyrimidin-2-yl)benzonitrile |
| **55** | 2-(3-chloro-2-fluorophenyl)-5-methoxy-N-(3-(5-(morpholinomethyl)-1H-benzo[d]imidazol-2-yl)-1H-pyrazol-4-yl)pyrimidin-4-amine |
| **56** | 2-(3-ethynyl-2-fluorophenyl)-5-methoxy-N-(3-(5-(morpholinomethyl)-1H-benzo[d]imidazol-2-yl)-1H-pyrazol-4-yl)pyrimidin-4-amine |
| **57** | 2-(5-chloro-2-fluorophenyl)-5-methoxy-N-(3-(5-(morpholinomethyl)-1H-benzo[d]imidazol-2-yl)-1H-pyrazol-4-yl)pyrimidin-4-amine |
| **58** | 2-(5-ethynyl-2-fluorophenyl)-5-methoxy-N-(3-(5-(morpholinomethyl)-1H-benzo[d]imidazol-2-yl)-1H-pyrazol-4-yl)pyrimidin-4-amine |
| **59** | 2-(3-chloro-2,6-difluorophenyl)-5-methoxy-N-(3-(5-(morpholinomethyl)-1H-benzo[d]imidazol-2-yl)-1H-pyrazol-4-yl)pyrimidin-4-amine |
| **60** | 2-(3-ethynyl-2,6-difluorophenyl)-5-methoxy-N-(3-(5-(morpholinomethyl)-1H-benzo[d]imidazol-2-yl)-1H-pyrazol-4-yl)pyrimidin-4-amine |
| **61** | 2-(4-fluorophenyl)-5-methoxy-N-(3-(5-(morpholinomethyl)-1H-benzo[d]imidazol-2-yl)-1H-pyrazol-4-yl)pyrimidin-4-amine |
| **144** | 5-methoxy-N-(3-(5-(morpholinomethyl)-1H-benzo[d]imidazol-2-yl)-1H-pyrazol-4-yl)-2-(3-phenoxyphenyl)pyrimidin-4-amine |
| **145** | 2-(3-chloro-5-cyclopropylphenyl)-5-methoxy-N-(3-(5-(morpholinomethyl)-1H-benzo[d]imidazol-2-yl)-1H-pyrazol-4-yl)pyrimidin-4-amine |
| **146** | 2-(3,5-dichlorophenyl)-5-methoxy-N-(3-(5-(morpholinomethyl)-1H-benzo[d]imidazol-2-yl)-1H-pyrazol-4-yl)pyrimidin-4-amine |
| **147** | 2-(3-chloro-4-methylphenyl)-5-methoxy-N-(3-(5-(morpholinomethyl)-1H-benzo[d]imidazol-2-yl)-1H-pyrazol-4-yl)pyrimidin-4-amine |
| **148** | 2-(3-chloro-4-methoxyphenyl)-5-methoxy-N-(3-(5-(morpholinomethyl)-1H-benzo[d]imidazol-2-yl)-1H-pyrazol-4-yl)pyrimidin-4-amine |
| **149** | 2-(3-chloro-4-(trifluoromethoxy)phenyl)-5-methoxy-N-(3-(5-(morpholinomethyl)-1H-benzo[d]imidazol-2-yl)-1H-pyrazol-4-yl)pyrimidin-4-amine |
| **150** | 2-(3-cyclopropylphenyl)-5-methoxy-N-(3-(5-(morpholinomethyl)-1H-benzo[d]imidazol-2-yl)-1H-pyrazol-4-yl)pyrimidin-4-amine |
| **151** | 2-(4-cyclopropylphenyl)-5-methoxy-N-(3-(5-(morpholinomethyl)-1H-benzo[d]imidazol-2-yl)-1H-pyrazol-4-yl)pyrimidin-4-amine |
| **152** | 5-methoxy-N-(3-(5-(morpholinomethyl)-1H-benzo[d]imidazol-2-yl)-1H-pyrazol-4-yl)-2-(3-(trifluoromethoxy)phenyl)pyrimidin-4-amine |
| **153** | 3-(5-methoxy-4-((3-(5-(morpholinomethyl)-1H-benzo[d]imidazol-2-yl)-1H-pyrazol-4-yl)amino)pyrimidin-2-yl)-N-methylbenzenesulfonamide |
| **154** | 5-methoxy-N-(3-(5-(morpholinomethyl)-1H-benzo[d]imidazol-2-yl)-1H-pyrazol-4-yl)-2-(4-phenoxyphenyl)pyrimidin-4-amine |
| **155** | 2-ethynyl-5-methoxy-N-(3-(5-(morpholinomethyl)-1H-benzo[d]imidazol-2-yl)-1H-pyrazol-4-yl)pyrimidin-4-amine |
| **156** | 2-(cyclopropylethynyl)-5-methoxy-N-(3-(5-(morpholinomethyl)-1H-benzo[d]imidazol-2-yl)-1H-pyrazol-4-yl)pyrimidin-4-amine |
| **157** | 5-methoxy-N-(3-(5-(morpholinomethyl)-1H-benzo[d]imidazol-2-yl)-1H-pyrazol-4-yl)-2-(phenylethynyl)pyrimidin-4-amine |
| **158** | 2-(3-chlorothiophen-2-yl)-5-methoxy-N-(3-(5-(morpholinomethyl)-1H-benzo[d]imidazol-2-yl)-1H-pyrazol-4-yl)pyrimidin-4-amine |
| **159** | 2-(4-chlorothiophen-2-yl)-5-methoxy-N-(3-(5-(morpholinomethyl)-1H-benzo[d]imidazol-2-yl)-1H-pyrazol-4-yl)pyrimidin-4-amine |
| **160** | 2-(4-chlorothiophen-3-yl)-5-methoxy-N-(3-(5-(morpholinomethyl)-1H-benzo[d]imidazol-2-yl)-1H-pyrazol-4-yl)pyrimidin-4-amine |
| **161** | 2-(5-chlorothiophen-3-yl)-5-methoxy-N-(3-(5-(morpholinomethyl)-1H-benzo[d]imidazol-2-yl)-1H-pyrazol-4-yl)pyrimidin-4-amine |
| **162** | 2-(5-chloro-2-fluorophenyl)-N-(3-(5-(morpholinomethyl)-1H-benzo[d]imidazol-2-yl)-1H-pyrazol-4-yl)-5-(trifluoromethyl)pyrimidin-4-amine |
| **163** | (2-(4-((2-(5-chloro-2-fluorophenyl)-5-methoxypyrimidin-4-yl)amino)-1H-pyrazol-3-yl)-1H-benzo[d]imidazol-5-yl)methanol |
| **164** | 2-(5-chloro-2-fluorophenyl)-N-(3-(5-((dimethylamino)methyl)-1H-benzo[d]imidazol-2-yl)-1H-pyrazol-4-yl)-5-methoxypyrimidin-4-amine |
| **165** | 2-(5-chloro-2-fluorophenyl)-5-methoxy-N-(3-(5-(pyrrolidin-1 -ylmethyl)-1H-benzo[d]imidazol-2-yl)-1H-pyrazol-4-yl)pyrimidin-4-amine |
| **166** | 2-(5-chloro-2-fluorophenyl)-5-methoxy-N-(3-(5-(thiomorpholinomethyl)-1H-benzo[d]imidazol-2-yl)-1H-pyrazol-4-yl)pyrimidin-4-amine |
| **167** | 4-((2-(4-((2-(5-chloro-2-fluorophenyl)-5-methoxypyrimidin-4-yl)amino)-1H-pyrazol-3-yl)-1H-benzo[d]imidazol-5-yl)methyl)thiomorpholine 1,1-dioxide |
| **168** | 2-(5-chloro-2-fluorophenyl)-5-methoxy-N-(3-(5-morpholino-1H-benzo[d]imidazol-2-yl)-1H-pyrazol-4-yl)pyrimidin-4-amine |
| **169** | 2-(5-chloro-2-fluorophenyl)-5-methoxy-N-(3-(5-(2-methoxyethoxy)-1H-benzo[d]imidazol-2-yl)-1H-pyrazol-4-yl)pyrimidin-4-amine |
| **170** | 2-(5-chloro-2-fluorophenyl)-5-methoxy-N-(3-(5-(2-morpholinoethoxy)-1H-benzo[d]imidazol-2-yl)-1H-pyrazol-4-yl)pyrimidin-4-amine |
| **171** | 2-(5-chloro-2-fluorophenyl)-5-methoxy-N-(3-(5-(3-morpholinopropoxy)-1H-benzo[d]imidazol-2-yl)-1H-pyrazol-4-yl)pyrimidin-4-amine |
| **172** | 2-(5-bromo-2-fluorophenyl)-5-methoxy-N-(3-(5-(morpholinomethyl)-1H-benzo[d]imidazol-2-yl)-1H-pyrazol-4-yl)pyrimidin-4-amine |
| **173** | 2-(2-fluoro-5-iodophenyl)-5-methoxy-N-(3-(5-(morpholinomethyl)-1H-benzo[d]imidazol-2-yl)-1H-pyrazol-4-yl)pyrimidin-4-amine |
| **174** | 4-chloro-2-(5-methoxy-4-((3-(5-(morpholinomethyl)-1H-benzo[d]imidazol-2-yl)-1H-pyrazol-4-yl)amino)pyrimidin-2-yl)benzonitrile |
| **175** | 2-(2,5-dichlorophenyl)-5-methoxy-N-(3-(5-(morpholinomethyl)-1H-benzo[d]imidazol-2-yl)-1H-pyrazol-4-yl)pyrimidin-4-amine |
| **176** | 2-(5-bromo-2-chlorophenyl)-5-methoxy-N-(3-(5-(morpholinomethyl)-1H-benzo[d]imidazol-2-yl)-1H-pyrazol-4-yl)pyrimidin-4-amine |
| **177** | 2-(2-fluoro-5-methylphenyl)-5-methoxy-N-(3-(5-(morpholinomethyl)-1H-benzo[d]imidazol-2-yl)-1H-pyrazol-4-yl)pyrimidin-4-amine |
| **178** | 2-(5-chloro-2,4-difluorophenyl)-5-methoxy-N-(3-(5-(morpholinomethyl)-1H-benzo[d]imidazol-2-yl)-1H-pyrazol-4-yl)pyrimidin-4-amine |
| **179** | 2-(5-chloro-2-fluorophenyl)-5-methoxy-N-(3-(5-(morpholinomethyl)-1H-benzo[d]imidazol-2-yl)-1H-pyrazol-4-yl)pyridin-4-amine |
or a tautomer, or a pharmaceutically acceptable salt thereof.

11. A pharmaceutical composition comprising at least one compound according to anyone of the claims 1 to 10 as an active ingredient, together with at least one pharmaceutically acceptable carrier, excipient and/or diluent.

12. A compound according to anyone of the claims 1 to 10 for use as a medicament.

13. A compound according to any one of claims 1 to 10 for use, or a pharmaceutical composition according to claim 11 for use in treatment of a cancer or a carcinoma associated with and/or caused by budding uninhibited by benzimidazole 1 kinase.

14. The compound for use or the pharmaceutical composition for use according to claim 13, wherein the cancer or the carcinoma associated with and/or caused by budding uninhibited by benzimidazole 1 kinase is selected from breast cancer, esophageal cancer, glioblastoma, hepatocellular carcinoma, gastric carcinomas, ovarian cancer, pancreatic cancer, prostate cancer, osteosarcoma, bladder cancer, cervical cancer, colon cancer, colorectal cancer, liver cancer, melanoma, non-small cell lung cancer (NSCLC).

15. A method for producing the compound of the formula **(Ia)** comprising the steps:
**Step 1:** providing a compound **1*** 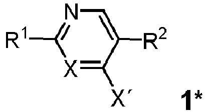 wherein **X'** is Br, Cl, or I;
**Step 2:** performing a coupling reaction of compound **1*** with a compound **2*** 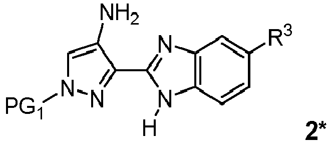 to obtain a compound **3*** 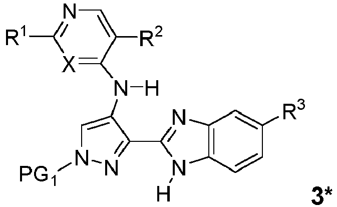
**Step 3:** removing a protecting group PG₁ from the compound **3*** to obtain the compound of the formula **(Ia)** 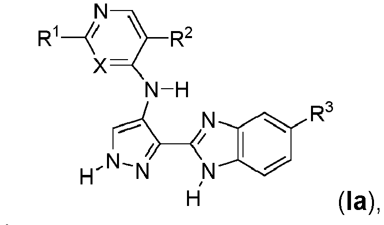 wherein **R¹, R², R³** and **X** have the same meanings as defined in the formula **(I)** and
**PG₁** is an amine protecting group;
or
a method for producing the compound of the formula **(Ia)** comprising the steps:
**Step 1A:** providing a compound **1b*** 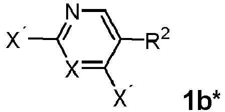 wherein **X'** is Br, Cl, or I;
**Step 2A:** performing a coupling reaction of compound **1b*** with a compound **2a*** 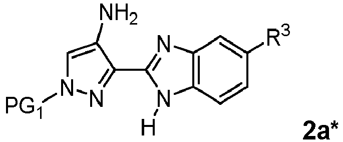 to obtain a compound **3a*** 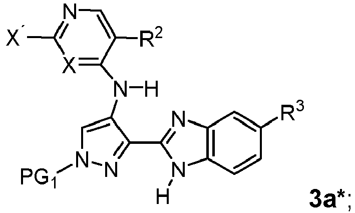
**Step 2B:** performing a coupling reaction of the compound **3a*** with a boronic acid compound **R¹**-B(O**R'**)(O**R**^) **4*** to obtain the compound **3*;** 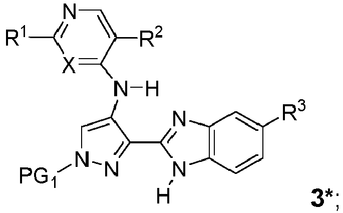
**Step 3:** removing a protecting group PG₁ from the compound **3*** to obtain the compound of the formula **(Ia)** 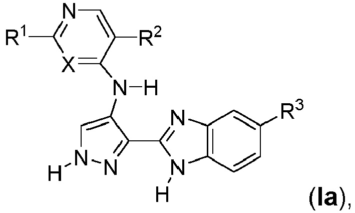 wherein
**R¹, R², R³**and **X** have the same meanings as defined in the formula **(I);**
**PG₁** is an amine protecting group; and
**R'** and **R"** represent independently of each other H or an alkyl chain with 1-10 carbon atoms or a cycloalkyl chain with 3 to 12 carbon atoms or both residues **R'** and **R"** form together a residue derived from pinacol;
or
a method for producing the compound of the formula (Ia) comprising:
**Step 1A:** providing the compound **1b*** 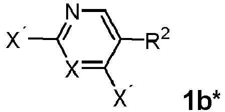 wherein **X'** is Br, Cl, or I;
**Step 2A:** performing a coupling reaction of compound **1b*** with a compound **2b*** 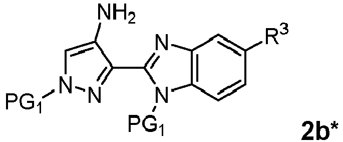 to obtain a compound **3b*** 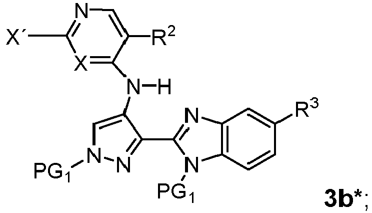
**Step 2B:** performing a coupling reaction of the compound **3b*** with a boronic acid compound **R¹**-B(O**R'**)(O**R**^) **4*** to obtain a compound **5b*;** 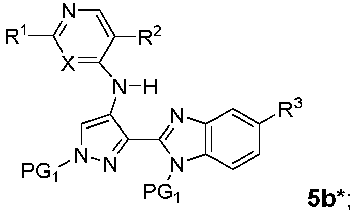
**Step 3B:** removing two protecting groups PG₁ from the compound **5b*** to obtain the compound of the formula **(Ia)** 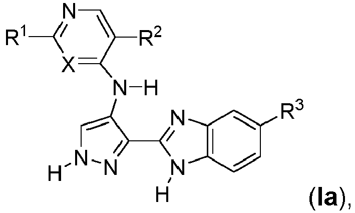 wherein
**R¹, R², R³** and **X** have the same meanings as defined in the formula **(I);**
**PG₁** is an amine protecting group; and
**R'** and **R"** represent independently of each other H or an alkyl chain with 1-10 carbon atoms or a cycloalkyl chain with 3 to 12 carbon atoms or both residues **R'** and **R"** form together a residue derived from pinacol;
or
a method for producing a compound of the formula **(Ia)** comprising:
**Step 1C:** providing a compound **6*** 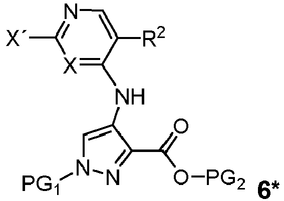 wherein **X'** is Br, Cl, or I;
**Step 2C:**
i) performing a coupling reaction of compound **6*** with a compound **R¹**-B(O**R'**)(O**R**^) **4*** to obtain a compound **7a*** 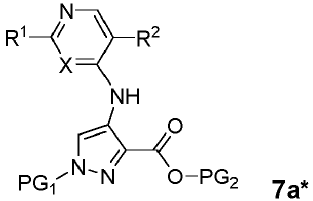
ii) removing a protecting group **PG₂** to obtain a compound **7b*** 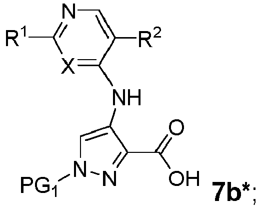
**Step 2D:** performing a condensation reaction of compound **7b*** with a compound **8*** 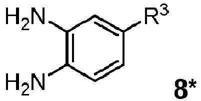 to obtain the compound **3*** 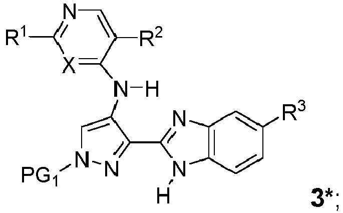
**Step 3:** removing a protecting group PG₁ from the compound **3*** to obtain the compound of the formula **(Ia)** 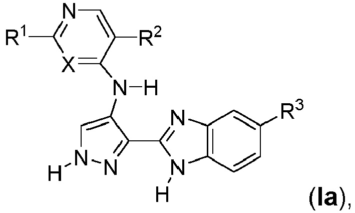 wherein
**R¹, R², R³** and **X** have the same meanings as defined in the formula **(I);**
**PG₁** is an amine protecting group;
**PG₂** is a carboxyl protecting group; and
**R'** and **R"** represent independently of each other H or an alkyl chain with 1-10 carbon atoms or a cycloalkyl chain with 3 to 12 carbon atoms or both residues **R'** and **R"** form together a residue derived from pinacol.
